(19) Europäisches Patentamt
European Patent Office
Office européen des brevets

(11) EP 4 129 987 A1

(12) EUROPEAN PATENT APPLICATION
published in accordance with Art. 153(4) EPC

(43) Date of publication:
**08.02.2023 Bulletin 2023/06**

(21) Application number: **21781797.2**

(22) Date of filing: **02.04.2021**

(51) International Patent Classification (IPC):
***C07D 241/18*** (1974.07) ***C07D 498/10*** (1974.07)
***C07D 471/10*** (1974.07) ***A61K 31/495*** (1974.07)
***A61P 35/00*** (2000.01)

(52) Cooperative Patent Classification (CPC):
**A61K 31/495; A61P 35/00; C07D 241/18; C07D 471/10; C07D 498/10**

(86) International application number:
**PCT/CN2021/085155**

(87) International publication number:
**WO 2021/197452 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2020 CN 202010261479**

(71) Applicants:
- **Shanghai Hansoh Biomedical Co., Ltd.**
**Shanghai 201203 (CN)**
- **Jiangsu Hansoh Pharmaceutical Group Co., Ltd.**
**Lianyungang, Jiangsu 222047 (CN)**

(72) Inventors:
- **ZHAN, Xiaolan**
**Shanghai 201203 (CN)**
- **GUO, Linsong**
**Shanghai 201203 (CN)**

(74) Representative: **Cabinet Beau de Loménie**
**158, rue de l'Université**
**75340 Paris Cedex 07 (FR)**

# (54) CRYSTAL FORM OF FREE ALKALI OF NITROGEN-CONTAINING AROMATIC DERIVATIVES

(57) The present invention relates to a crystal form of the free alkali of nitrogen-containing aromatic derivatives. In particular, the present invention relates to a crystal form of the compound represented by the general formula (I), its preparation method and a pharmaceutical composition containing a therapeutically effective amount of the crystal form, and the use thereof as a protein tyrosine phosphatase-2C (SHP2) inhibitor in the treatment of diseases or conditions such as leukemia, neuroblastoma, melanoma, breast cancer, lung cancer and colorectal cancer.

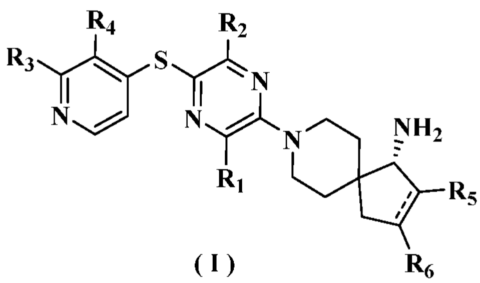

(I)

EP 4 129 987 A1

## Description

### FIELD OF THE INVENTION

The present invention belongs to the field of biomedicine, and in particular relates to a crystal form of free base of nitrogen-containing heteroaromatic derivative, a method for preparing the same, and a use thereof.

### BACKGROUND OF THE INVENTION

Src homology-2 domain-containing phosphatase 2 (SHP-2), also known as tyrosine-protein phosphatase non-receptor type 11 (PTPN11), is encoded by PTPN11 gene, and belongs to the protein tyrosine phosphatase (PTP) family. As a downstream signal molecule of cytokines, growth factors and other extracellular stimulating factors, SHP-2 is widely expressed in various tissues and cells of the body, participates in cell signal transduction, and regulates cell growth, differentiation, migration, metabolism, gene transcription, immune response and the like.

SHP-2 has three main structural parts: SH-2 domain (N-SH2 and C-SH2), PTP active domain, and C-terminal (having a tyrosine phosphorylation site). The SH2 domain is highly conserved, which is a phosphotyrosine binding site and mediates the binding of the PTP domain to its ligand.

SHP-2 has two main states *in vivo*: inactivated state and activated state. In the inactivated state, N-SH2 in SHP-2 binds to the PTP domain, because the PTP domain is occupied, SHP-2 is inactivated. When N-SH2 specifically binds to the phosphorylated tyrosine residue ligand, the PTP domain is re-exposed and SHP-2 resumes its activity. Latest studies show that SHP-2 can also form dimers *in vivo,* which can also lead to SHP-2 inactivation.

SHP-2 mainly functions by regulating signal pathways such as ERK/MAPK, JAK-STAT, PI3K/AKT, Hippo, Wnt/β-catenin, so as to maintain biological development and homeostasis. Specific studies show that SHP-2 participates in the activation of the ERK/MAPK pathway by directly binding to receptor tyrosine kinase (RTK) or scaffold. In addition, activated SHP-2 can also recruit GRB2/SOS, and indirectly promote the activation of the RAS signaling pathway. Moreover, SHP-2 is also involved in signal transduction that inhibits immune response. For example, SHP-2 and SHP-1 can bind to and activate immunosuppressive receptors (such as PD-1), and block T cell activation. As an important cell signaling factor, SHP-2 mutations are closely related to many diseases. Studies show that SHP-2 mutations are found in neuroblastoma, acute myeloid leukemia (AML, 4%), breast cancer, non-small cell lung cancer (NSCLC, 10%), lung adenocarcinoma (30%), esophageal cancer, head and neck tumor, melanoma and gastric cancer.

The mutation sites of SHP-2 mostly occur in N-SH2 and PTP active regions. The mutations reduce the mutual inhibition of N-CH2/PTP domains, and lead to highly active SHP-2, for example, Cys459Ser mutant, E76K mutant and the like will affect the activity of SHP-2. Studies show that highly active SHP-2 is closely related to inflammation, liver cirrhosis, the toxin CagA secreted by *Helicobacter pylori* and the like. Highly active SHP-2 can lead to tumor regeneration and development, and is equivalent to a proto-oncogene. With the deepening of the understanding of SHP-2, SHP-2 has been used as a tumor treatment target for drug development.

At present, several SHP-2 allosteric inhibitors have entered the clinical research phase. For example, TNO-155 developed by Novartis, RMC-4630 developed by Revolution Medicine, and JAB-3068 developed by Beijing Jacobio have all entered the phase I clinical research phase. However, there is no SHP-2 inhibitor on the market for the treatment of Noonan syndrome, leopard syndrome, leukemia, neuroblastoma, melanoma, breast cancer, esophageal cancer, head and neck tumor, lung cancer and colon cancer. Therefore, there is an urgent need to develop a class of SHP-2 inhibitor drugs with good pharmaceutical properties.

PCT patent application (application number: PCT/CN2019/110314) discloses the structure of a series of nitrogen-containing heteroaromatic derivative inhibitor. In the subsequent research and development, in order to obtain a product that can be readily processed, filtered and dried and to achieve features such as convenient storage, long-term stability and high bioavailability, the present invention has conducted a comprehensive study on the free base of the above substances, and is dedicated to obtaining the most suitable crystal forms.

### SUMMARY OF THE INVENTION

All contents involved in the patent application PCT/CN2019/110314 are incorporated into the present invention by way of reference.

The objective of the present invention is to provide a crystal form of a compound of formula (I), the structure of which is shown in formula (I):

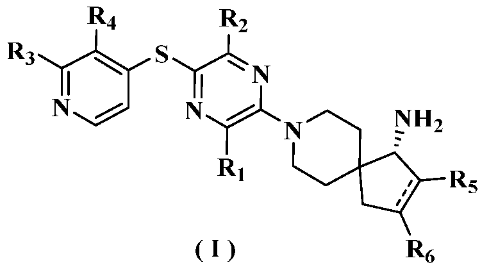

(I)

wherein:

$R_1$ is selected from the group consisting of hydrogen, halogen, $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-12}$ aryl and 5 to 12 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-12}$ aryl and 5 to 12 membered heteroaryl are each optionally further substituted by one or more substituent(s) selected from the group consisting of deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl;

$R_2$ is selected from the group consisting of hydrogen, amino and $C_{1-6}$ alkyl;

$R_3$ is selected from the group consisting of hydrogen, halogen, amino, cyano, $C_{1-6}$ alkyl, $C_{1-6}$ alkoxy, $C_{1-6}$ hydroxyalkyl, $C_{3-8}$ cycloalkyl and 3 to 12 membered heterocyclyl, wherein the $C_{1-6}$ alkyl, $C_{1-6}$ alkoxy, $C_{1-6}$ hydroxyalkyl, $C_{3-8}$ cycloalkyl and 3 to 12 membered heterocyclyl are each optionally further substituted by one or more substituent(s) selected from the group consisting of deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl;

$R_4$ is selected from the group consisting of hydrogen, deuterium, halogen, amino, nitro, hydroxy, cyano, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{3-8}$ cycloalkyl, 3 to 12 membered heterocyclyl, $C_{6-12}$ aryl and 5 to 12 membered heteroaryl;

$R_5$ and $R_6$ are each independently selected from the group consisting of hydrogen, deuterium, halogen, amino, nitro, hydroxy, cyano, $C_{1-6}$ alkyl, $C_{1-6}$ deuterated alkyl, $C_{1-6}$ haloalkyl, $C_{1-6}$ alkoxy, $C_{1-6}$ haloalkoxy, $C_{1-6}$ hydroxyalkyl, $C_{2-6}$ alkenyl, $C_{3-8}$ cycloalkyl, 3 to 12 membered heterocyclyl, $C_{6-12}$ aryl and 5 to 12 membered heteroaryl, wherein the $C_{1-6}$ alkyl, $C_{1-6}$ deuterated alkyl, $C_{1-6}$ haloalkyl, $C_{1-6}$ alkoxy, $C_{1-6}$ haloalkoxy, $C_{1-6}$ hydroxyalkyl, $C_{2-6}$ alkenyl, $C_{3-8}$ cycloalkyl, 3 to 12 membered heterocyclyl, $C_{6-12}$ aryl and 5 to 12 membered heteroaryl are each optionally further substituted by one or more substituent(s) selected from the group consisting of deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl;

or, $R_5$ and $R_6$ are bonded to form a $C_{3-8}$ cycloalkyl, 3 to 12 membered heterocyclyl, $C_{6-12}$ aryl or 5 to 12 membered heteroaryl, wherein the $C_{3-8}$ cycloalkyl, 3 to 12 membered heterocyclyl, $C_{6-12}$ aryl and 5 to 12 membered heteroaryl are each optionally further substituted by one or more substituent(s) selected from the group consisting of hydrogen, deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl.

In a further preferred embodiment of the present invention, $R_1$ is selected from the group consisting of hydrogen, halogen, $C_{1-6}$ alkyl, $C_{1-6}$ hydroxyalkyl, $C_{3-8}$ cycloalkyl and 3 to 12 membered heterocyclyl, wherein the $C_{1-6}$ alkyl, $C_{1-6}$ hydroxyalkyl, $C_{3-8}$ cycloalkyl and 3 to 12 membered heterocyclyl are each optionally further substituted by one or more substituent(s) selected from the group consisting of deuterium, halogen, hydroxy, oxo, $C_{1-6}$ alkyl and $C_{3-8}$ cycloalkyl; more preferably selected from the group consisting of hydrogen, halogen, $C_{1-3}$ alkyl, $C_{1-3}$ hydroxyalkyl, $C_{3-6}$ cycloalkyl and 3 to 6 membered heterocyclyl, wherein the $C_{1-3}$ alkyl, $C_{1-3}$ hydroxyalkyl, $C_{3-6}$ cycloalkyl and 3 to 6 membered heterocyclyl are each optionally further substituted by one or more substituent(s) selected from the group consisting of deuterium, halogen, hydroxy, oxo, $C_{1-3}$ alkyl and $C_{3-6}$ cycloalkyl; and further preferably selected from the group consisting of hydrogen, fluorine, bromine, methyl, hydroxyethyl,

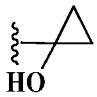, 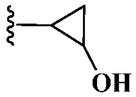, 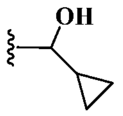, 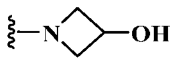, 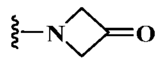

and

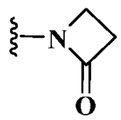.

In a further preferred embodiment of the present invention, $R_2$ is selected from the group consisting of hydrogen, amino and $C_{1-6}$ alkyl; preferably selected from the group consisting of hydrogen, amino and $C_{1-3}$ alkyl; more preferably selected from the group consisting of hydrogen, amino, methyl, ethyl and propyl; and further preferably selected from the group consisting of hydrogen, amino and methyl.

In a further preferred embodiment of the present invention, $R_3$ is selected from the group consisting of hydrogen, halogen, amino, $C_{1-6}$ alkyl, $C_{3-8}$ cycloalkyl and 3 to 12 membered heterocyclyl, wherein the $C_{1-6}$ alkyl, $C_{3-8}$ cycloalkyl and 3 to 12 membered heterocyclyl are each optionally further substituted by one or more substituent(s) selected from the group consisting of deuterium, halogen and oxo; preferably selected from the group consisting of hydrogen, halogen, amino, $C_{1-3}$ alkyl, $C_{3-6}$ cycloalkyl and 3 to 6 membered heterocyclyl, wherein the $C_{1-3}$ alkyl, $C_{3-6}$ cycloalkyl and 3 to 6 membered heterocyclyl are each optionally further substituted by one or more substituent(s) selected from the group consisting of deuterium, halogen and oxo; and further preferably selected from the group consisting of amino, chlorine, cyclopropyl, azacyclobutyl, tetrahydropyrrolyl, morpholinyl,

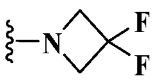, 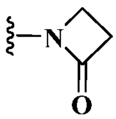 and 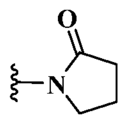.

In a further preferred embodiment of the present invention, $R_4$ is selected from the group consisting of hydrogen, halogen, $C_{1-6}$ alkyl and $C_{3-8}$ cycloalkyl; preferably selected from the group consisting of hydrogen, halogen, $C_{1-3}$ alkyl and $C_{3-6}$ cycloalkyl; and further preferably selected from the group consisting of hydrogen, fluorine, chlorine, methyl and cyclopropyl.

In a further preferred embodiment of the present invention, $R_5$ and $R_6$ are each independently selected from the group consisting of hydrogen, $C_{1-6}$ alkyl, $C_{1-6}$ haloalkyl, $C_{2-6}$ alkenyl and $C_{3-8}$ cycloalkyl, wherein the $C_{1-6}$ alkyl, $C_{1-6}$ haloalkyl, $C_{2-6}$ alkenyl and $C_{3-8}$ cycloalkyl are each optionally further substituted by one or more substituent(s) selected from the group consisting of deuterium, halogen and $C_{1-6}$ alkyl; preferably selected from the group consisting of hydrogen, $C_{1-3}$ alkyl, $C_{2-4}$ alkenyl and $C_{3-6}$ cycloalkyl, wherein the $C_{1-3}$ alkyl, $C_{2-4}$ alkenyl and $C_{3-6}$ cycloalkyl are each optionally further substituted by one or more substituent(s) selected from the group consisting of deuterium, halogen and $C_{1-3}$ alkyl; and further preferably selected from the group consisting of hydrogen, methyl, ethyl, isopropyl, vinyl, cyclopropyl,

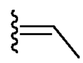

and

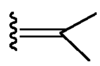;

or, $R_5$ and $R_6$ are bonded to form a $C_{3-8}$ cycloalkyl, $C_{6-12}$ aryl or 5 to 12 membered heteroaryl, wherein the $C_{3-8}$ cycloalkyl, $C_{6-12}$ aryl and 5 to 12 membered heteroaryl are each optionally further substituted by one or more substituent(s) selected from the group consisting of deuterium, halogen and $C_{1-6}$ alkyl; preferably form a $C_{3-6}$ cycloalkyl, phenyl or 5 to 6 membered heteroaryl, wherein the $C_{3-6}$ cycloalkyl, phenyl and 5 to 6 membered heteroaryl are each optionally further substituted by one or more substituent(s) selected from the group consisting of deuterium, halogen and $C_{1-3}$ alkyl; and further preferably form

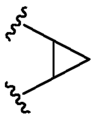 , 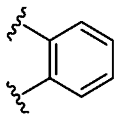 , 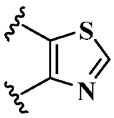 , 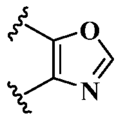 , 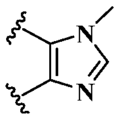 ,

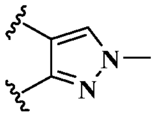 or 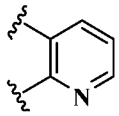 .

In a further preferred embodiment of the present invention, the structure of the compound is as shown in formula (II):

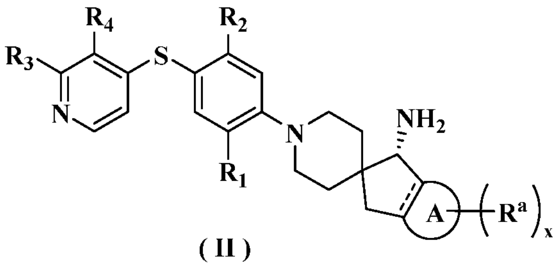

( II )

wherein:

ring A is selected from the group consisting of $C_{3-8}$ cycloalkyl, 3 to 12 membered heterocyclyl, $C_{6-10}$ aryl and 5 to 12 membered heteroaryl;
$R^a$ is selected from the group consisting of hydrogen, deuterium, halogen, amino, nitro, hydroxy, cyano, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl; and
x is 0, 1, 2 or 3.

In a further preferred embodiment of the present invention, ring A is phenyl.

The present invention also provides a method for preparing a crystal form of the compound of formula (I), specifically comprising the following steps of:

1) weighing an appropriate amount of free base, and suspending it in a poor solvent to obtain a suspension; the suspension density is preferably 50 to 200 mg/mL;
2) shaking the suspension obtained in step 1) at 0 to 40°C for 1 to 10 days;
3) rapidly centrifuging the suspension obtained in step 2), removing the supernatant, and drying the remaining solid in a vacuum drying oven at 40°C to constant weight to obtain the target product;

wherein:
the poor solvent in step 1) is selected from the group consisting of acetone, ethyl acetate, acetonitrile, ethanol, 88% acetone, tetrahydrofuran, dichloromethane, 1,4-dioxane, benzene, toluene, isopropanol, *n*-butanol, isobutanol, N,N-dimethylformamide, N,N-dimethylacetamide, n-propanol, *tert*-butanol, 2-butanone and 3-pentanone; and preferably selected from the group consisting of 3-pentanone, acetonitrile, dichloromethane and 1,4-dioxane.

The present invention also provides a method for preparing a crystal form of the compound of formula (I), specifically comprising the following steps of:

1) weighing an appropriate amount of free base, and dissolving it in a good solvent;
2) adding an anti-solvent to the solution obtained in step 1) at 0 to 25°C, and stirring the solution until a solid is precipitated;
3) rapidly centrifuging the suspension obtained in step 2), removing the supernatant, and drying the remaining solid in a vacuum drying oven at 40°C to constant weight to obtain the target product;

wherein:

the good solvent in step 1) is selected from the group consisting of methanol, acetone, ethyl acetate, acetonitrile, ethanol, 88% acetone, tetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone and 3-pentanone; and preferably selected from the group consisting of N,N-dimethylformamide and acetonitrile.

The anti-solvent in step 2) is selected from the group consisting of heptane, water, methyl *tert*-butyl ether, toluene and isopropyl ether; and preferably water.

In a further preferred embodiment of the present invention, the crystal form is crystal form A of the compound (S)-1'-(6-amino-5-((2-amino-3-chloropyridin-4-yl)thio) -3-fluoropyrazin-2-yl)-1,3-dihydrospiro[indene-2,4'-piperidin]-1-amine, the X-ray powder diffraction pattern thereof has diffraction peaks at 2θ of 9.0°, 15.2°, 20.2° and 23.0°; further has diffraction peaks at 2θ of .7°, 12.3°, 15.4°, 19.8°, 23.5° and 27.1°; and more further has diffraction peaks at 2θ of 4.5°, 13.7°, 14.6°, 16.8°, 18.1°, 21.5°, 27.7° and 28.2°.

Using Cu-Kα radiation, the characteristic X-ray diffraction peaks represented by 2θ angle and interplanar spacing d value are shown in Table 1.

Table 1

| No. | XRPD diffraction data of crystal form A | | | | | |
|---|---|---|---|---|---|---|
| | 2θ (±0.2°) | d value | Peak height | Proportion (1%) | Area | Proportion (1%) |
| 1 | 4.46 | 19.7967 | 336 | 59.9 | 2777 | 29.9 |
| 2 | 8.95 | 9.8727 | 553 | 98.6 | 6611 | 71.3 |
| 3 | 9.677 | 9.1323 | 292 | 52 | 2672 | 28.8 |
| 4 | 12.348 | 7.1619 | 257 | 45.8 | 3318 | 35.8 |
| 5 | 13.431 | 6.587 | 90 | 16 | 2814 | 30.3 |
| 6 | 13.732 | 6.4435 | 131 | 23.4 | 2807 | 30.3 |
| 7 | 14.58 | 6.0703 | 129 | 23 | 2026 | 21.8 |
| 8 | 15.213 | 5.8193 | 561 | 100 | 9274 | 100 |
| 9 | 15.435 | 5.7362 | 364 | 64.9 | 4743 | 51.1 |
| 10 | 16.75 | 5.2884 | 194 | 34.6 | 3530 | 38.1 |
| 11 | 17.072 | 5.1895 | 81 | 14.4 | 1497 | 16.1 |
| 12 | 18.129 | 4.8892 | 90 | 16 | 1303 | 14.1 |
| 13 | 19.838 | 4.4718 | 234 | 41.7 | 5413 | 58.4 |
| 14 | 20.199 | 4.3926 | 337 | 60.1 | 5137 | 55.4 |
| 15 | 21.454 | 4.1383 | 106 | 18.9 | 1754 | 18.9 |
| 16 | 22.974 | 3.868 | 537 | 95.7 | 8148 | 87.9 |
| 17 | 23.463 | 3.7884 | 335 | 59.7 | 6702 | 72.3 |
| 18 | 27.05 | 3.2936 | 183 | 32.6 | 2983 | 32.2 |
| 19 | 27.741 | 3.2132 | 175 | 31.2 | 3116 | 33.6 |
| 20 | 28.169 | 3.1653 | 93 | 16.6 | 1534 | 16.5 |

The crystal form of the present invention is crystal form A of the compound (S)-1'-(6-amino-5-((2-amino-3-chloropyridin-4-yl)thio)-3-fluoropyrazin-2-yl)-1,3-dihydrospiro[indene-2,4'-piperidin]-1-amine, the DSC spectrum thereof is substantially as shown in Figure 1; and the TGA spectrum thereof is substantially as shown in Figure 2.

In a further preferred embodiment of the present invention, the crystal form is crystal form B of the compound (S)-1'-(6-amino-5-((2-amino-3-chloropyridin-4-yl)thio) -3-fluoropyrazin-2-yl)-1,3-dihydrospiro[indene-2,4'-piperidin]-1-amine, the X-ray powder diffraction pattern thereof has diffraction peaks at 2θ of 7.9°, 14.6° and 16.2°; also has diffraction peaks at 2θ of 18.1°, 19.2°, 20.2°, 24.7° and 26.8°; further has diffraction peaks at 2θ of 14.9°, 27.2°, 28.1° and 30.5°; more further has diffraction peaks at 2θ of 13.9°, 15.6°, 16.9° and 19.8°; and most preferably, the XRPD pattern thereof

is substantially as shown in Figure 3.

Using Cu-Kα radiation, the characteristic X-ray diffraction peaks represented by 2θ angle and interplanar spacing d value are shown in Table 2.

Table 2

| No. | XRPD diffraction data of crystal form B | | | | | |
|---|---|---|---|---|---|---|
| | 2θ (±0.2°) | d value | Peak height | Proportion (1%) | Area | Proportion (1%) |
| 1 | 6.31 | 13.9956 | 80 | 8 | 396 | 2.5 |
| 2 | 7.853 | 11.2482 | 690 | 68.7 | 5922 | 37.1 |
| 3 | 9.923 | 8.9068 | 61 | 6.1 | 834 | 5.2 |
| 4 | 13.911 | 6.3607 | 221 | 22 | 1254 | 7.9 |
| 5 | 14.564 | 6.0771 | 733 | 72.9 | 9648 | 60.5 |
| 6 | 14.925 | 5.9309 | 547 | 54.4 | 6387 | 40 |
| 7 | 15.637 | 5.6625 | 105 | 10.4 | 1085 | 6.8 |
| 8 | 16.166 | 5.4781 | 1005 | 100 | 13053 | 81.8 |
| 9 | 16.92 | 5.2359 | 139 | 13.8 | 1536 | 9.6 |
| 10 | 18.11 | 4.8942 | 948 | 94.3 | 14765 | 92.6 |
| 11 | 19.183 | 4.6229 | 707 | 70.3 | 6787 | 42.5 |
| 12 | 19.831 | 4.4733 | 317 | 31.5 | 5379 | 33.7 |
| 13 | 20.219 | 4.3883 | 838 | 83.4 | 14241 | 89.3 |
| 14 | 21.899 | 4.0553 | 126 | 12.5 | 1340 | 8.4 |
| 15 | 23.135 | 3.8413 | 126 | 12.5 | 697 | 4.4 |
| 16 | 24.679 | 3.6044 | 447 | 44.5 | 7296 | 45.7 |
| 17 | 26.828 | 3.3204 | 820 | 81.6 | 15952 | 100 |
| 18 | 27.211 | 3.2745 | 232 | 23.1 | 8341 | 52.3 |
| 19 | 27.843 | 3.2016 | 219 | 21.8 | 6157 | 38.6 |
| 20 | 28.123 | 3.1703 | 265 | 26.4 | 6160 | 38.6 |
| 21 | 29.136 | 3.0624 | 131 | 13 | 1588 | 10 |
| 22 | 30.459 | 2.9323 | 227 | 22.6 | 4827 | 30.3 |
| 23 | 31.428 | 2.8441 | 131 | 13 | 2164 | 13.6 |
| 24 | 31.833 | 2.8088 | 80 | 8 | 470 | 2.9 |
| 25 | 32.483 | 2.7541 | 67 | 6.7 | 851 | 5.3 |
| 26 | 33.25 | 2.6923 | 78 | 7.8 | 709 | 4.4 |
| 27 | 34.473 | 2.5996 | 55 | 5.5 | 1185 | 7.4 |
| 28 | 34.915 | 2.5676 | 63 | 6.3 | 2273 | 14.2 |
| 29 | 38.185 | 2.3549 | 55 | 5.5 | 3052 | 19.1 |
| 30 | 38.787 | 2.3197 | 69 | 6.9 | 1824 | 11.4 |

The crystal form of the present invention is crystal form B of the compound (S)-1'-(6-amino-5-((2-amino-3-chloropyridin-4-yl)thio)-3-fluoropyrazin-2-yl)-1,3-dihydrospiro[indene-2,4'-piperidin]-1-amine, the DSC spectrum thereof is substantially as shown in Figure 4; and the TGA spectrum thereof is substantially as shown in Figure 5.

In a further preferred embodiment of the present invention, the crystal form is crystal form C of the compound (S)-1'-(6-amino-5-((2-amino-3-chloropyridin-4-yl)thio) -3-fluoropyrazin-2-yl)-1,3-dihydrospiro[indene-2,4'-piperidin]-1-amine, the X-ray powder diffraction pattern thereof has diffraction peaks at 2θ of 9.4°, 14.5° and 20.3°; also has diffraction peaks at 2θ of 22.2°, 22.7°, 22.9° and 26.2°; further has diffraction peaks at 2θ of 8.2°, 14.9°, 23.8°, 27.5°, 28.1°, 29.3°, 30.2° and 31.7°; and more further has diffraction peaks at 2θ of 12.0°, 16.4°, 25.5°, 28.6°, 34.8° and 35.4°.

Using Cu-Kα radiation, the characteristic X-ray diffraction peaks represented by 2θ angle and interplanar spacing d value are shown in Table 3.

Table 3

| No. | XRPD diffraction data of crystal form C | | | | | |
|---|---|---|---|---|---|---|
| | 2θ (±0.2°) | d value | Peak height | Proportion (1%) | Area | Proportion (1%) |
| 1 | 8.155 | 10.8327 | 137 | 20.9 | 1410 | 12.1 |
| 2 | 9.412 | 9.3886 | 231 | 35.3 | 2719 | 23.2 |
| 3 | 12.023 | 7.3549 | 108 | 16.5 | 578 | 4.9 |
| 4 | 14.522 | 6.0944 | 654 | 100 | 9549 | 81.6 |
| 5 | 14.926 | 5.9303 | 380 | 58.1 | 6492 | 55.5 |
| 6 | 16.351 | 5.4166 | 181 | 27.7 | 1568 | 13.4 |
| 7 | 19.076 | 4.6485 | 87 | 13.3 | 2344 | 20 |
| 8 | 19.63 | 4.5187 | 77 | 11.8 | 1134 | 9.7 |
| 9 | 20.32 | 4.3666 | 604 | 92.4 | 6581 | 56.2 |
| 10 | 22.206 | 4 | 414 | 63.3 | 5017 | 42.9 |
| 11 | 22.652 | 3.9222 | 384 | 58.7 | 11700 | 100 |
| 12 | 22.933 | 3.8748 | 465 | 71.1 | 10729 | 91.7 |
| 13 | 23.849 | 3.7279 | 277 | 42.4 | 3805 | 32.5 |
| 14 | 25.493 | 3.4912 | 116 | 17.7 | 2306 | 19.7 |
| 15 | 26.198 | 3.3987 | 367 | 56.1 | 6160 | 52.6 |
| 16 | 27.517 | 3.2388 | 258 | 39.4 | 3216 | 27.5 |
| 17 | 28.081 | 3.175 | 266 | 40.7 | 3946 | 33.7 |
| 18 | 28.55 | 3.1239 | 143 | 21.9 | 2439 | 20.8 |
| 19 | 29.341 | 3.0415 | 149 | 22.8 | 1702 | 14.5 |
| 20 | 30.153 | 2.9614 | 207 | 31.7 | 3160 | 27 |
| 21 | 31.673 | 2.8226 | 178 | 27.2 | 3637 | 31.1 |
| 22 | 33.013 | 2.7111 | 61 | 9.3 | 1617 | 13.8 |
| 23 | 34.816 | 2.5747 | 119 | 18.2 | 1530 | 13.1 |
| 24 | 35.384 | 2.5346 | 112 | 17.1 | 2291 | 19.6 |

The crystal form of the present invention is crystal form C of the compound (S)-1'-(6-amino-5-((2-amino-3-chloropyridin-4-yl)thio)-3-fluoropyrazin-2-yl)-1,3-dihydrospiro[indene-2,4'-piperidin]-1-amine, the DSC spectrum thereof is substantially as shown in Figure 6; and the TGA spectrum thereof is substantially as shown in Figure 7.

In a further preferred embodiment of the present invention, the crystal form is crystal form D of the compound (S)-1'-(6-amino-5-((2-amino-3-chloropyridin-4-yl)thio) -3-fluoropyrazin-2-yl)-1,3-dihydrospiro[indene-2,4'-piperidin]-1-amine, the X-ray powder diffraction pattern thereof has diffraction peaks at 2θ of 5.1°, 13.4° and 18.3°; also has diffraction peaks at 2θ of 18.0°, 19.9°, 20.7° and 22.6°; further has diffraction peaks at 2θ of 13.7°, 16.8°, 17.7°, 19.0°, 22.3° and 27.5°; and more further has diffraction peaks at 2θ of 16.1°, 22.9°, 24.9°, 25.5° and 28.9°.

Using Cu-Kα radiation, the characteristic X-ray diffraction peaks represented by 2θ angle and interplanar spacing d value are shown in Table 4.

Table 4

| No. | XRPD diffraction data of crystal form D | | | | | |
|---|---|---|---|---|---|---|
| | 2θ (±0.2°) | d value | Peak height | Proportion (1%) | Area | Proportion (1%) |
| 1 | 5.077 | 17.3927 | 634 | 100 | 4794 | 30.6 |
| 2 | 13.369 | 6.6176 | 377 | 59.5 | 4314 | 27.5 |
| 3 | 13.652 | 6.481 | 250 | 39.4 | 2141 | 13.7 |
| 4 | 16.137 | 5.4881 | 107 | 16.9 | 914 | 5.8 |
| 5 | 16.756 | 5.2866 | 300 | 47.3 | 2486 | 15.9 |
| 6 | 17.728 | 4.999 | 206 | 32.5 | 5075 | 32.4 |
| 7 | 17.928 | 4.9437 | 437 | 68.9 | 8117 | 51.8 |
| 8 | 18.251 | 4.8567 | 608 | 95.9 | 8751 | 55.8 |
| 9 | 19.025 | 4.6608 | 214 | 33.8 | 1681 | 10.7 |
| 10 | 19.855 | 4.468 | 346 | 54.6 | 4664 | 29.8 |
| 11 | 20.704 | 4.2866 | 434 | 68.5 | 5726 | 36.5 |
| 12 | 22.285 | 3.9859 | 372 | 58.7 | 12254 | 78.2 |
| 13 | 22.609 | 3.9295 | 467 | 73.7 | 15673 | 100 |
| 14 | 22.893 | 3.8815 | 196 | 30.9 | 3486 | 22.2 |
| 15 | 24.92 | 3.57 | 111 | 17.5 | 2065 | 13.2 |
| 16 | 25.468 | 3.4946 | 128 | 20.2 | 2543 | 16.2 |
| 17 | 27.517 | 3.2388 | 199 | 31.4 | 4405 | 28.1 |
| 18 | 28.486 | 3.1308 | 74 | 11.7 | 1554 | 9.9 |
| 19 | 28.898 | 3.0871 | 170 | 26.8 | 3959 | 25.3 |
| 20 | 32.646 | 2.7407 | 77 | 12.1 | 684 | 4.4 |
| 21 | 33.941 | 2.6391 | 74 | 11.7 | 1278 | 8.2 |

The crystal form of the present invention is crystal form D of the compound (S)-1'-(6-amino-5-((2-amino-3-chloropyridin-4-yl)thio)-3-fluoropyrazin-2-yl)-1,3-dihydrospiro[indene-2,4'-piperidin]-1-amine the DSC spectrum thereof is substantially as shown in Figure 8; and the TGA spectrum thereof is substantially as shown in Figure 9.

In a further preferred embodiment of the present invention, the crystal form is crystal form E of the compound (S)-1'-(6-amino-5-((2-amino-3-chloropyridin-4-yl)thio) -3-fluoropyrazin-2-yl)-1,3-dihydrospiro[indene-2,4'-piperidin]-1-amine, the X-ray powder diffraction pattern thereof has diffraction peaks at 2θ of 8.4°, 15.3° and 18.3°; also has diffraction peaks at 2θ of 20.7°, 22.5°, 26.7° and 27.6°; further has diffraction peaks at 2θ of 13.5°, 14.6°, 15.8°, 16.0°, 16.9°, 19.2°, 20.4°, 24.0°, 24.4°, 25.5°, 28.2°, 28.9°, 31.8° and 32.2°; and more further has diffraction peaks at 2θ of 5.2°, 9.5°, 10.4°, 17.2°, 17.7°, 19.5°, 20.0°, 26.3°, 28.7°, 30.5°, 31.0° and 34.1°.

Using Cu-Kα radiation, the characteristic X-ray diffraction peaks represented by 2θ angle and interplanar spacing d value are shown in Table 5.

Table 5

| No. | XRPD diffraction data of crystal form E | | | | | |
|---|---|---|---|---|---|---|
| | 2θ (±0.2°) | d value | Peak height | Proportion (1%) | Area | Proportion (1%) |
| 1 | 5.213 | 16.9394 | 142 | 14.1 | 1046 | 7.9 |
| 2 | 8.399 | 10.5189 | 557 | 55.4 | 6484 | 48.7 |
| 3 | 9.536 | 9.2669 | 131 | 13 | 1025 | 7.7 |
| 4 | 10.431 | 8.4736 | 119 | 11.8 | 1062 | 8 |

(continued)

| No. | XRPD diffraction data of crystal form E | | | | | |
|---|---|---|---|---|---|---|
| | 2θ (±0.2°) | d value | Peak height | Proportion (1%) | Area | Proportion (1%) |
| 5 | 13.525 | 6.5415 | 203 | 20.2 | 1975 | 14.8 |
| 6 | 14.586 | 6.0678 | 280 | 27.9 | 2862 | 21.5 |
| 7 | 15.333 | 5.7739 | 1005 | 100 | 8117 | 60.9 |
| 8 | 15.756 | 5.6198 | 369 | 36.7 | 5128 | 38.5 |
| 9 | 16.022 | 5.5271 | 309 | 30.7 | 4761 | 35.7 |
| 10 | 16.873 | 5.2504 | 284 | 28.3 | 2124 | 15.9 |
| 11 | 17.158 | 5.1637 | 138 | 13.7 | 1260 | 9.5 |
| 12 | 17.724 | 5.0002 | 180 | 17.9 | 2794 | 21 |
| 13 | 18.331 | 4.8358 | 959 | 95.4 | 11686 | 87.7 |
| 14 | 19.183 | 4.623 | 393 | 39.1 | 3758 | 28.2 |
| 15 | 19.527 | 4.5423 | 139 | 13.8 | 1435 | 10.8 |
| 16 | 20.055 | 4.4239 | 250 | 24.9 | 3421 | 25.7 |
| 17 | 20.463 | 4.3364 | 316 | 31.4 | 8881 | 66.7 |
| 18 | 20.745 | 4.2781 | 755 | 75.1 | 8937 | 67.1 |
| 19 | 22.528 | 3.9435 | 461 | 45.9 | 4722 | 35.4 |
| 20 | 23.927 | 3.716 | 304 | 30.2 | 6493 | 48.7 |
| 21 | 24.438 | 3.6394 | 328 | 32.6 | 3799 | 28.5 |
| 22 | 25.471 | 3.4942 | 227 | 22.6 | 2255 | 16.9 |
| 23 | 26.28 | 3.3883 | 113 | 11.2 | 589 | 4.4 |
| 24 | 26.667 | 3.3401 | 472 | 47 | 4830 | 36.3 |
| 25 | 27.558 | 3.234 | 624 | 62.1 | 13324 | 100 |
| 26 | 28.206 | 3.1612 | 187 | 18.6 | 1824 | 13.7 |
| 27 | 28.671 | 3.111 | 154 | 15.3 | 1821 | 13.7 |
| 28 | 28.939 | 3.0828 | 289 | 28.8 | 3092 | 23.2 |
| 29 | 30.498 | 2.9287 | 175 | 17.4 | 1729 | 13 |
| 30 | 31.023 | 2.8803 | 157 | 15.6 | 1960 | 14.7 |
| 31 | 31.756 | 2.8154 | 168 | 16.7 | 5043 | 37.8 |
| 32 | 32.221 | 2.7759 | 228 | 22.7 | 3442 | 25.8 |
| 33 | 34.124 | 2.6253 | 151 | 15 | 1585 | 11.9 |
| 34 | 36.496 | 2.4599 | 77 | 7.7 | 1877 | 14.1 |
| 35 | 38.162 | 2.3563 | 71 | 7.1 | 1082 | 8.1 |

The crystal form of the present invention is crystal form E of the compound (S)-1'-(6-amino-5-((2-amino-3-chloropyridin-4-yl)thio)-3-fluoropyrazin-2-yl)-1,3-dihydrospiro[indene-2,4'-piperidin]-1-amine, the DSC spectrum thereof is substantially as shown in Figure 10; and the TGA spectrum thereof is substantially as shown in Figure 11.

The objective of the present invention is also to provide a crystal form of the compound (S)-8-(6-amino-5-((2-amino-3-chloropyridin-4-yl)thio)pyrazin-2-yl)-2-cyclopropyl-8-azaspiro[4.5]dec-2-en-1-amine.

In a further preferred embodiment of the present invention, crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine is provided.

The X-ray powder diffraction pattern of crystal form A of the compound (S)-8-(6-amino-5-((2-amino-3-chloropyrdin-4-yl)thio)pyrazin-2-yl)-2-cyclopropyl-8-az aspiro[4.5]dec-2-en-1-amine has a diffraction peak at 2θ of 20.0±0.2°; or has a diffraction peak at 19.4±0.2°; or has a diffraction peak at 17.1±0.2°; or has a diffraction peak at 22.6±0.2°; or has a diffraction peak at 10.7±0.2°; or has a diffraction peak at 25.6±0.2°; or has a diffraction peak at 12.7±0.2°; or has a diffraction peak at 24.5±0.2°; or has a diffraction peak at 19.1±0.2°; or has a diffraction peak at 18.4±0.2°; preferably comprises any 2 to 5, or 3 to 5, or 3 to 6, or 3 to 8, or 5 to 8, or 6 to 8 of the above diffraction peaks; and more preferably comprises any 6, 7, or 8 of the above diffraction peaks.

The X-ray powder diffraction pattern of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine at least comprises one or more diffraction peaks at 2θ of 20.0±0.2°, 19.4±0.2° and 17.1±0.2°, preferably comprises 2 of the above diffraction peaks, and more preferably comprises 3 of the above diffraction peaks; optionally, can further comprises at least one diffraction peak at 2θ of 22.6±0.2°, 10.7±0.2°, 25.6±0.2°, 12.7±0.2° and 24.5±0.2, and preferably comprises 2, 3, 4, or 5 of the above diffraction peaks; for example, the X-ray powder diffraction pattern of crystal form A has diffraction peaks at 2θ of the following positions:

20.0±0.2° and 19.4±0.2°;
or, 19.4±0.2° and 17.1±0.2°;
or, 20.0±0.2° and 17.1±0.2°;
or, 20.0±0.2° and 22.6±0.2°;
or, 22.6±0.2° and 10.7±0.2°;
or, 20.0±0.2° and 10.7±0.2°;
or, 10.7±0.2° and 25.6±0.2°;
or, 10.7±0.2° and 12.7±0.2°;
or, 20.0±0.2°, 19.4±0.2° and 17.1±0.2°;
or, 20.0±0.2°, 19.4±0.2° and 22.6±0.2°;
or, 20.0±0.2°, 19.4±0.2° and 10.7±0.2°;
or, 20.0±0.2°, 19.4±0.2° and 25.6±0.2°;
or, 19.4±0.2°, 17.1±0.2° and 25.6±0.2°;
or, 19.4±0.2°, 17.1±0.2° and 22.6±0.2°;
or, 19.4±0.2°, 17.1±0.2° and 10.7±0.2°;
or, 17.1±0.2°, 22.6±0.2° and 10.7±0.2°;
or, 20.0±0.2°, 17.1±0.2°, 10.7±0.2° and 22.6±0.2°;
or, 20.0±0.2°, 17.1±0.2°, 10.7±0.2° and 25.6±0.2°;
or, 20.0±0.2°, 17.1±0.2°, 10.7±0.2° and 12.7±0.2°;
or, 20.0±0.2°, 19.4±0.2°, 10.7±0.2° and 24.5±0.2°;
or, 19.4±0.2°, 17.1±0.2°, 22.6±0.2° and 12.7±0.2°;
or, 19.4±0.2°, 17.1±0.2°, 22.6±0.2° and 25.6±0.2°;
or, 17.1±0.2°, 22.6±0.2°, 10.7±0.2° and 25.6±0.2°;
or, 17.1±0.2°, 22.6±0.2°, 10.7±0.2° and 24.5±0.2°;
or, 20.0±0.2°, 19.4±0.2°, 17.1±0.2°, 22.6±0.2° and 10.7±0.2°;
or, 20.0±0.2°, 19.4±0.2°, 17.1±0.2°, 22.6±0.2° and 25.6±0.2°;
or, 20.0±0.2°, 19.4±0.2°, 17.1±0.2°, 10.7±0.2° and 25.6±0.2°;
or, 20.0±0.2°, 19.4±0.2°, 22.6±0.2°, 10.7±0.2° and 12.7±0.2°;
or, 19.4±0.2°, 17.1±0.2°, 22.6±0.2°, 10.7±0.2° and 12.7±0.2°;
or, 19.4±0.2°, 22.6±0.2°, 10.7±0.2°, 25.6±0.2° and 12.7±0.2°;
or, 19.4±0.2°, 22.6±0.2°, 10.7±0.2°, 25.6±0.2° and 24.5±0.2°;
or, 19.4±0.2°, 22.6±0.2°, 10.7±0.2°, 25.6±0.2° and 24.5±0.2°;
or, 17.1±0.2°, 22.6±0.2°, 10.7±0.2°, 25.6±0.2° and 24.5±0.2°;
or, 20.0±0.2°, 19.4±0.2°, 17.1±0.2°, 22.6±0.2°, 10.7±0.2°, 25.6±0.2°, 12.7±0.2° and 24.5±0.2°;
or, 19.4±0.2°, 17.1±0.2°, 22.6±0.2°, 10.7±0.2°, 25.6±0.2°, 12.7±0.2° and 24.5±0.2°;
or, 20.0±0.2°, 17.1±0.2°, 22.6±0.2°, 10.7±0.2°, 25.6±0.2°, 12.7±0.2° and 24.5±0.2°;
or, 20.0±0.2°, 19.4±0.2°, 22.6±0.2°, 10.7±0.2°, 25.6±0.2°, 12.7±0.2° and 24.5±0.2°;
or, 20.0±0.2°, 19.4±0.2°, 17.1±0.2°, 10.7±0.2°, 25.6±0.2°, 12.7±0.2° and 24.5±0.2°;
or, 20.0±0.2°, 19.4±0.2°, 17.1±0.2°, 22.6±0.2°, 25.6±0.2°, 12.7±0.2° and 24.5±0.2°;
or, 20.0±0.2°, 19.4±0.2°, 17.1±0.2°, 22.6±0.2°, 10.7±0.2°, 12.7±0.2° and 24.5±0.2°;
or, 20.0±0.2°, 19.4±0.2°, 17.1±0.2°, 22.6±0.2°, 10.7±0.2°, 25.6±0.2° and 24.5±0.2°;
or, 20.0±0.2°, 19.4±0.2°, 17.1±0.2°, 22.6±0.2°, 10.7±0.2°, 25.6±0.2° and 12.7±0.2°;
or, 17.1±0.2°, 22.6±0.2°, 10.7±0.2°, 25.6±0.2°, 12.7±0.2° and 24.5±0.2°;

or, 20.0±0.2°, 22.6±0.2°, 10.7±0.2°, 25.6±0.2°, 12.7±0.2° and 24.5±0.2°;
or, 20.0±0.2°, 19.4±0.2°, 10.7±0.2°, 25.6±0.2°, 12.7±0.2° and 24.5±0.2°;
or, 20.0±0.2°, 19.4±0.2°, 17.1±0.2°, 25.6±0.2°, 12.7±0.2° and 24.5±0.2°;
or, 20.0±0.2°, 19.4±0.2°, 17.1±0.2°, 22.6±0.2°, 12.7±0.2° and 24.5±0.2°;
or, 20.0±0.2°, 19.4±0.2°, 17.1±0.2°, 22.6±0.2°, 10.7±0.2° and 24.5±0.2°;
or, 20.0±0.2°, 19.4±0.2°, 17.1±0.2°, 22.6±0.2°, 10.7±0.2° and 25.6±0.2°;
or, 19.4±0.2°, 22.6±0.2°, 10.7±0.2°, 25.6±0.2°, 12.7±0.2° and 24.5±0.2°;
or, 19.4±0.2°, 17.1±0.2°, 10.7±0.2°, 25.6±0.2°, 12.7±0.2° and 24.5±0.2°;
or, 19.4±0.2°, 17.1±0.2°, 22.6±0.2°, 25.6±0.2°, 12.7±0.2° and 24.5±0.2°;
or, 19.4±0.2°, 17.1±0.2°, 22.6±0.2°, 10.7±0.2°, 12.7±0.2° and 24.5±0.2°;
or, 19.4±0.2°, 17.1±0.2°, 22.6±0.2°, 10.7±0.2°, 25.6±0.2° and 24.5±0.2°;
or, 19.4±0.2°, 17.1±0.2°, 22.6±0.2°, 10.7±0.2°, 25.6±0.2° and 12.7±0.2°;
or, 20.0±0.2°, 17.1±0.2°, 10.7±0.2°, 25.6±0.2°, 12.7±0.2° and 24.5±0.2°;
or, 20.0±0.2°, 17.1±0.2°, 22.6±0.2°, 25.6±0.2°, 12.7±0.2° and 24.5±0.2°;
or, 20.0±0.2°, 17.1±0.2°, 22.6±0.2°, 10.7±0.2°, 12.7±0.2° and 24.5±0.2°;
or, 20.0±0.2°, 17.1±0.2°, 22.6±0.2°, 10.7±0.2°, 25.6±0.2° and 12.7±0.2°;
or, 20.0±0.2°, 19.4±0.2°, 22.6±0.2°, 25.6±0.2°, 12.7±0.2° and 24.5±0.2°;
or, 20.0±0.2°, 19.4±0.2°, 22.6±0.2°, 10.7±0.2°, 25.6±0.2° and 24.5±0.2°;
or, 20.0±0.2°, 19.4±0.2°, 22.6±0.2°, 10.7±0.2°, 25.6±0.2° and 12.7±0.2°;
or, 20.0±0.2°, 19.4±0.2°, 17.1±0.2°, 10.7±0.2°, 25.6±0.2° and 24.5±0.2°;
or, 20.0±0.2°, 19.4±0.2°, 17.1±0.2°, 10.7±0.2°, 25.6±0.2° and 12.7±0.2°;
or, 20.0±0.2°, 19.4±0.2°, 17.1±0.2°, 22.6±0.2°, 25.6±0.2° and 24.5±0.2°;
or, 20.0±0.2°, 19.4±0.2°, 17.1±0.2°, 22.6±0.2°, 25.6±0.2° and 12.7±0.2°;
or, 22.6±0.2°, 10.7±0.2°, 25.6±0.2°, 12.7±0.2° and 24.5±0.2°;
or, 17.1±0.2°, 10.7±0.2°, 25.6±0.2°, 12.7±0.2° and 24.5±0.2°;
or, 17.1±0.2°, 22.6±0.2°, 25.6±0.2°, 12.7±0.2° and 24.5±0.2°;
or, 17.1±0.2°, 22.6±0.2°, 10.7±0.2°, 12.7±0.2° and 24.5±0.2°;
or, 17.1±0.2°, 22.6±0.2°, 10.7±0.2°, 25.6±0.2° and 12.7±0.2°;
or, 19.4±0.2°, 22.6±0.2°, 25.6±0.2°, 12.7±0.2° and 24.5±0.2°;
or, 19.4±0.2°, 22.6±0.2°, 10.7±0.2°, 12.7±0.2° and 24.5±0.2°;
or, 19.4±0.2°, 22.6±0.2°, 10.7±0.2°, 25.6±0.2° and 24.5±0.2°;
or, 19.4±0.2°, 22.6±0.2°, 10.7±0.2°, 25.6±0.2° and 12.7±0.2°;
or, 20.0±0.2°, 10.7±0.2°, 25.6±0.2°, 12.7±0.2° and 24.5±0.2°;
or, 20.0±0.2°, 19.4±0.2°, 25.6±0.2°, 12.7±0.2° and 24.5±0.2°;
or, 20.0±0.2°, 19.4±0.2°, 17.1±0.2°, 12.7±0.2° and 24.5±0.2°;
or, 20.0±0.2°, 19.4±0.2°, 17.1±0.2°, 22.6±0.2° and 24.5±0.2°;
or, 20.0±0.2°, 19.4±0.2°, 17.1±0.2°, 22.6±0.2° and 10.7±0.2°.

The X-ray powder diffraction pattern of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine optionally also comprises one or more diffraction peaks at 2θ of 19.1±0.2°, 18.4±0.2°, 6.2±0.2°, 31.5±0.2°, 29.2±0.2°, 20.6±0.2° and 21.4±0.2°; preferably at least comprises any 2 to 3, or 4 to 5, or 6 to 7 of the above diffraction peaks; and further preferably comprises any 2, 3, 4, 5, 6, or 7 of the above diffraction peaks; for example, the X-ray powder diffraction pattern of crystal form A has diffraction peaks at 2θ of the following positions:

20.0±0.2°, 19.4±0.2°, 17.1±0.2°, 22.6±0.2°, 25.6±0.2°, 24.5±0.2°, 18.4±0.2° and 19.1±0.2°;
or, 20.0±0.2°, 12.7±0.2°, 17.1±0.2°, 22.6±0.2°, 25.6±0.2°, 24.5±0.2°, 18.4±0.2° and 19.1±0.2°;
or, 20.0±0.2°, 19.4±0.2°, 22.6±0.2°, 25.6±0.2°, 12.7±0.2°, 24.5±0.2°, 18.4±0.2° and 19.1±0.2°;
or, 20.0±0.2°, 19.4±0.2°, 10.7±0.2°, 25.6±0.2°, 12.7±0.2°, 24.5±0.2°, 18.4±0.2° and 19.1±0.2°;
or, 20.0±0.2°, 19.4±0.2°, 17.1±0.2°, 22.6±0.2°, 10.7±0.2°, 25.6±0.2°, 12.7±0.2°, 24.5±0.2° and 19.1±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 19.1±0.2° and 18.4±0.2°;
or, 19.4±0.2°, 17.1±0.2°, 22.6±0.2°, 10.7±0.2°, 25.6±0.2°, 12.7±0.2°, 24.5±0.2° and 19.1±0.2°;
or, 19.4±0.2°, 17.1±0.2°, 22.6±0.2°, 10.7±0.2°, 25.6±0.2°, 12.7±0.2°, 24.5±0.2°, 19.1±0.2° and 18.4±0.2°;
or, 20.0±0.2°, 17.1±0.2°, 22.6±0.2°, 10.7±0.2°, 25.6±0.2°, 12.7±0.2°, 24.5±0.2° and 19.1±0.2°;
or, 20.0±0.2°, 17.1±0.2°, 22.6±0.2°, 10.7±0.2°, 25.6±0.2°, 12.7±0.2°, 24.5±0.2°, 19.1±0.2° and 18.4±0.2°;
or, 20.0±0.2°, 22.6±0.2°, 10.7±0.2°, 25.6±0.2°, 12.7±0.2°, 24.5±0.2° and 19.1±0.2°;
or, 20.0±0.2°, 22.6±0.2°, 10.7±0.2°, 25.6±0.2°, 12.7±0.2°, 24.5±0.2°, 19.1±0.2° and 6.2±0.2°;

or, 20.0±0.2°, 19.4±0.2°, 10.7±0.2°, 25.6±0.2°, 12.7±0.2°, 24.5±0.2° and 19.1±0.2°;
or, 20.0±0.2°, 19.4±0.2°, 10.7±0.2°, 25.6±0.2°, 12.7±0.2°, 24.5±0.2°, 19.1±0.2° and 29.2±0.2°;
or, 20.0±0.2°, 19.4±0.2°, 17.1±0.2°, 22.6±0.2°, 24.5±0.2° and 19.1±0.2°;
or, 20.0±0.2°, 19.4±0.2°, 17.1±0.2°, 22.6±0.2°, 24.5±0.2°, 19.1±0.2° and 18.4±0.2°;
or, 20.0±0.2°, 19.4±0.2°, 17.1±0.2°, 22.6±0.2°, 10.7±0.2° and 19.1±0.2°;
or, 20.0±0.2°, 19.4±0.2°, 17.1±0.2°, 22.6±0.2°, 10.7±0.2°, 19.1±0.2° and 18.4±0.2°.

The X-ray powder diffraction pattern of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine comprises one or more diffraction peaks at 2θ of 20.0±0.2°, 19.4±0.2°, 17.1±0.2°, 22.6±0.2°, 10.7±0.2°, 25.6±0.2°, 12.7±0.2°, 24.5±0.2, 19.1±0.2°, 18.4±0.2°, 6.2±0.2°, 31.5±0.2°, 29.2±0.2°, 20.6±0.2° and 21.4±0.2°; and preferably comprises any 4, 5, 6, 8, or 10 of the above diffraction peaks; for example, the X-ray powder diffraction pattern of crystal form A has diffraction peaks at 2θ of the following positions:

20.0±0.2°, 19.4±0.2°, 17.1±0.2° and 22.6±0.2°;
or, 20.0±0.2°, 19.4±0.2°, 17.1±0.2° and 10.7±0.2°;
or, 20.0±0.2°, 19.4±0.2°, 17.1±0.2° and 25.6±0.2°;
or, 20.0±0.2°, 19.4±0.2°, 17.1±0.2° and 12.7±0.2°;
or, 20.0±0.2°, 19.4±0.2°, 17.1±0.2° and 24.5±0.2°;
or, 20.0±0.2°, 19.4±0.2°, 17.1±0.2° and 19.1±0.2°;
or, 20.0±0.2°, 19.4±0.2°, 17.1±0.2° and 18.4±0.2°;
or, 20.0±0.2°, 19.4±0.2°, 17.1±0.2° and 6.2±0.2°;
or, 20.0±0.2°, 19.4±0.2°, 17.1±0.2° and 31.5±0.2°;
or, 19.4±0.2°, 17.1±0.2°, 22.6±0.2° and 10.7±0.2°;
or, 19.4±0.2°, 17.1±0.2°, 22.6±0.2° and 25.6±0.2°;
or, 19.4±0.2°, 17.1±0.2°, 22.6±0.2° and 12.7±0.2°;
or, 19.4±0.2°, 17.1±0.2°, 22.6±0.2° and 24.5±0.2°;
or, 19.4±0.2°, 17.1±0.2°, 22.6±0.2° and 19.1±0.2°;
or, 19.4±0.2°, 17.1±0.2°, 22.6±0.2° and 18.4±0.2°;
or, 19.4±0.2°, 17.1±0.2°, 22.6±0.2° and 6.2±0.2°;
or, 19.4±0.2°, 17.1±0.2°, 22.6±0.2° and 31.5±0.2°;
or, 17.1±0.2°, 22.6±0.2°, 10.7±0.2° and 25.6±0.2°;
or, 17.1±0.2°, 22.6±0.2°, 10.7±0.2° and 12.7±0.2°;
or, 17.1±0.2°, 22.6±0.2°, 10.7±0.2° and 24.5±0.2°;
or, 17.1±0.2°, 22.6±0.2°, 10.7±0.2° and 19.1±0.2°;
or, 17.1±0.2°, 22.6±0.2°, 10.7±0.2° and 18.4±0.2°;
or, 17.1±0.2°, 22.6±0.2°, 10.7±0.2° and 6.2±0.2°;
or, 17.1±0.2°, 22.6±0.2°, 10.7±0.2° and 31.5±0.2°;
or, 20.0±0.2°, 17.1±0.2°, 22.6±0.2° and 10.7±0.2°;
or, 20.0±0.2°, 17.1±0.2°, 22.6±0.2° and 25.6±0.2°;
or, 20.0±0.2°, 17.1±0.2°, 22.6±0.2° and 12.7±0.2°;
or, 20.0±0.2°, 17.1±0.2°, 22.6±0.2° and 24.5±0.2°;
or, 20.0±0.2°, 17.1±0.2°, 22.6±0.2° and 19.1±0.2°;
or, 20.0±0.2°, 17.1±0.2°, 22.6±0.2° and 18.4±0.2°;
or, 20.0±0.2°, 17.1±0.2°, 22.6±0.2° and 6.2±0.2°;
or, 20.0±0.2°, 17.1±0.2°, 22.6±0.2° and 31.5±0.2°;
or, 20.0±0.2°, 19.4±0.2°, 22.6±0.2° and 10.7±0.2°;
or, 20.0±0.2°, 19.4±0.2°, 22.6±0.2° and 25.6±0.2°;
or, 20.0±0.2°, 19.4±0.2°, 22.6±0.2° and 12.7±0.2°;
or, 20.0±0.2°, 19.4±0.2°, 22.6±0.2° and 24.5±0.2°;
or, 20.0±0.2°, 19.4±0.2°, 22.6±0.2° and 19.1±0.2°;
or, 20.0±0.2°, 19.4±0.2°, 22.6±0.2° and 18.4±0.2°;
or, 20.0±0.2°, 19.4±0.2°, 22.6±0.2° and 6.2±0.2°;
or, 20.0±0.2°, 19.4±0.2°, 22.6±0.2° and 31.5±0.2°;
or, 20.0±0.2°, 19.4±0.2°, 17.1±0.2°, 22.6±0.2°, 10.7±0.2° and 25.6±0.2°;
or, 20.0±0.2°, 19.4±0.2°, 17.1±0.2°, 22.6±0.2°, 10.7±0.2° and 12.7±0.2°;
or, 20.0±0.2°, 19.4±0.2°, 17.1±0.2°, 22.6±0.2°, 10.7±0.2° and 24.5±0.2°;
or, 20.0±0.2°, 19.4±0.2°, 17.1±0.2°, 22.6±0.2°, 25.6±0.2° and 12.7±0.2°;

or, 20.0±0.2°, 19.4±0.2°, 17.1±0.2°, 22.6±0.2°, 25.6±0.2° and 24.5±0.2°;
or, 20.0±0.2°, 19.4±0.2°, 17.1±0.2°, 22.6±0.2°, 12.7±0.2° and 24.5±0.2°;
or, 20.0±0.2°, 19.4±0.2°, 17.1±0.2°, 10.7±0.2°, 25.6±0.2° and 12.7±0.2°;
or, 20.0±0.2°, 19.4±0.2°, 17.1±0.2°, 10.7±0.2°, 12.7±0.2° and 24.5±0.2°;
or, 20.0±0.2°, 19.4±0.2°, 17.1±0.2°, 10.7±0.2°, 25.6±0.2° and 24.5±0.2°;
or, 20.0±0.2°, 19.4±0.2°, 22.6±0.2°, 10.7±0.2°, 25.6±0.2° and 12.7±0.2°;
or, 20.0±0.2°, 19.4±0.2°, 22.6±0.2°, 10.7±0.2°, 12.7±0.2° and 24.5±0.2°;
or, 20.0±0.2°, 19.4±0.2°, 22.6±0.2°, 25.6±0.2°, 12.7±0.2° and 24.5±0.2°;
or, 20.0±0.2°, 19.4±0.2°, 22.6±0.2°, 10.7±0.2°, 25.6±0.2° and 24.5±0.2°;
or, 19.4±0.2°, 17.1±0.2°, 22.6±0.2°, 10.7±0.2°, 25.6±0.2° and 12.7±0.2°;
or, 19.4±0.2°, 17.1±0.2°, 22.6±0.2°, 10.7±0.2°, 12.7±0.2° and 24.5±0.2°;
or, 19.4±0.2°, 17.1±0.2°, 22.6±0.2°, 25.6±0.2°, 12.7±0.2° and 24.5±0.2°;
or, 19.4±0.2°, 17.1±0.2°, 22.6±0.2°, 10.7±0.2°, 25.6±0.2° and 24.5±0.2°;
or, 20.0±0.2°, 17.1±0.2°, 22.6±0.2°, 10.7±0.2°, 25.6±0.2° and 12.7±0.2°;
or, 20.0±0.2°, 17.1±0.2°, 22.6±0.2°, 10.7±0.2°, 12.7±0.2° and 24.5±0.2°;
or, 20.0±0.2°, 17.1±0.2°, 22.6±0.2°, 25.6±0.2°, 12.7±0.2° and 24.5±0.2°;
or, 20.0±0.2°, 17.1±0.2°, 22.6±0.2°, 10.7±0.2°, 25.6±0.2° and 24.5±0.2°;
or, 20.0±0.2°, 19.4±0.2°, 17.1±0.2°, 22.6±0.2°, 10.7±0.2°, 25.6±0.2°, 12.7±0.2° and 19.1±0.2°;
or, 20.0±0.2°, 19.4±0.2°, 17.1±0.2°, 22.6±0.2°, 10.7±0.2°, 25.6±0.2°, 12.7±0.2° and 18.4±0.2°;
or, 20.0±0.2°, 19.4±0.2°, 17.1±0.2°, 22.6±0.2°, 10.7±0.2°, 25.6±0.2°, 12.7±0.2° and 6.2±0.2°;
or, 20.0±0.2°, 19.4±0.2°, 17.1±0.2°, 22.6±0.2°, 10.7±0.2°, 25.6±0.2°, 12.7±0.2° and 31.5±0.2°;
or, 20.0±0.2°, 19.4±0.2°, 17.1±0.2°, 22.6±0.2°, 10.7±0.2°, 25.6±0.2°, 24.5±0.2° and 19.1±0.2°;
or, 20.0±0.2°, 19.4±0.2°, 17.1±0.2°, 22.6±0.2°, 10.7±0.2°, 25.6±0.2°, 24.5±0.2° and 18.4±0.2°;
or, 20.0±0.2°, 19.4±0.2°, 17.1±0.2°, 22.6±0.2°, 10.7±0.2°, 25.6±0.2°, 19.1±0.2° and 18.4±0.2°;
or, 20.0±0.2°, 19.4±0.2°, 17.1±0.2°, 22.6±0.2°, 10.7±0.2°, 12.7±0.2°, 24.5±0.2° and 19.1±0.2°;
or, 20.0±0.2°, 19.4±0.2°, 17.1±0.2°, 22.6±0.2°, 10.7±0.2°, 12.7±0.2°, 24.5±0.2° and 18.4±0.2°;
or, 20.0±0.2°, 19.4±0.2°, 17.1±0.2°, 22.6±0.2°, 10.7±0.2°, 25.6±0.2°, 19.1±0.2° and 18.4±0.2°;
or, 20.0±0.2°, 19.4±0.2°, 17.1±0.2°, 22.6±0.2°, 25.6±0.2°, 12.7±0.2°, 24.5±0.2° and 19.1±0.2°;
or, 20.0±0.2°, 19.4±0.2°, 17.1±0.2°, 22.6±0.2°, 25.6±0.2°, 12.7±0.2°, 24.5±0.2° and 18.4±0.2°;
or, 20.0±0.2°, 19.4±0.2°, 17.1±0.2°, 22.6±0.2°, 12.7±0.2°, 24.5±0.2°, 19.1±0.2° and 18.4±0.2°;
or, 20.0±0.2°, 19.4±0.2°, 17.1±0.2°, 22.6±0.2°, 25.6±0.2°, 24.5±0.2°, 19.1±0.2° and 18.4±0.2°;
or, 19.4±0.2°, 17.1±0.2°, 22.6±0.2°, 10.7±0.2°, 25.6±0.2°, 12.7±0.2°, 24.5±0.2° and 19.1±0.2°;
or, 19.4±0.2°, 17.1±0.2°, 22.6±0.2°, 10.7±0.2°, 25.6±0.2°, 12.7±0.2°, 24.5±0.2° and 18.4±0.2°;
or, 19.4±0.2°, 22.6±0.2°, 10.7±0.2°, 25.6±0.2°, 12.7±0.2°, 24.5±0.2°, 19.1±0.2° and 18.4±0.2°;
or, 19.4±0.2°, 22.6±0.2°, 10.7±0.2°, 25.6±0.2°, 12.7±0.2°, 24.5±0.2°, 19.1±0.2° and 18.4±0.2°;
or, 19.4±0.2°, 22.6±0.2°, 10.7±0.2°, 25.6±0.2°, 12.7±0.2°, 24.5±0.2°, 19.1±0.2° and 18.4±0.2°;
or, 20.0±0.2°, 17.1±0.2°, 22.6±0.2°, 10.7±0.2°, 12.7±0.2°, 24.5±0.2°, 19.1±0.2° and 18.4±0.2°;
or, 17.1±0.2°, 22.6±0.2°, 10.7±0.2°, 25.6±0.2°, 12.7±0.2°, 24.5±0.2°, 19.1±0.2° and 18.4±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 19.1±0.2° and 18.4±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 18.4±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 18.4±0.2° and 31.5±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 6.2±0.2° and 31.5±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 18.4±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 19.1±0.2°, 18.4±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 19.1±0.2°, 18.4±0.2° and 31.5±0.2°;
or, 20.0±0.2°, 19.4±0.2°, 17.1±0.2°, 22.6±0.2°, 10.7±0.2°, 25.6±0.2°, 12.7±0.2°, 19.1±0.2°, 6.2±0.2° and 31.5±0.2°;
or, 20.0±0.2°, 19.4±0.2°, 17.1±0.2°, 22.6±0.2°, 10.7±0.2°, 25.6±0.2°, 24.5±0.2°, 19.1±0.2°, 18.4±0.2° and 6.2±0.2°;
or, 20.0±0.2°, 19.4±0.2°, 17.1±0.2°, 22.6±0.2°, 10.7±0.2°, 25.6±0.2°, 24.5±0.2°, 19.1±0.2°, 18.4±0.2° and 31.5±0.2°;

or, 20.0±0.2°, 19.4±0.2°, 17.1±0.2°, 22.6±0.2°, 10.7±0.2°, 25.6±0.2°, 19.1±0.2°, 18.4±0.2°, 6.2±0.2° and 31.5±0.2°;
or, 20.0±0.2°, 19.4±0.2°, 17.1±0.2°, 22.6±0.2°, 10.7±0.2°, 12.7±0.2°, 24.5±0.2°, 19.1±0.2°, 18.4±0.2° and 6.2±0.2°;
or, 20.0±0.2°, 19.4±0.2°, 17.1±0.2°, 22.6±0.2°, 10.7±0.2°, 12.7±0.2°, 24.5±0.2°, 19.1±0.2°, 6.2±0.2° and 31.5±0.2°;
or, 20.0±0.2°, 19.4±0.2°, 17.1±0.2°, 22.6±0.2°, 10.7±0.2°, 24.5±0.2°, 19.1±0.2°, 18.4±0.2°, 6.2±0.2° and 31.5±0.2°;
or, 19.4±0.2°, 17.1±0.2°, 22.6±0.2°, 10.7±0.2°, 25.6±0.2°, 12.7±0.2°, 24.5±0.2°, 19.1±0.2°, 18.4±0.2° and 6.2±0.2°;
or, 19.4±0.2°, 22.6±0.2°, 10.7±0.2°, 25.6±0.2°, 12.7±0.2°, 24.5±0.2°, 19.1±0.2°, 18.4±0.2°, 6.2±0.2° and 31.5±0.2°;
or, 19.4±0.2°, 17.1±0.2°, 10.7±0.2°, 25.6±0.2°, 12.7±0.2°, 24.5±0.2°, 19.1±0.2°, 18.4±0.2°, 6.2±0.2° and 31.5±0.2°;
or, 17.1±0.2°, 22.6±0.2°, 10.7±0.2°, 25.6±0.2°, 12.7±0.2°, 24.5±0.2°, 19.1±0.2°, 18.4±0.2°, 6.2±0.2° and 31.5±0.2°.

The X-ray powder diffraction pattern of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine comprises one or more diffraction peaks at 2θ of 20.0±0.2°, 19.4±0.2°, 17.1±0.2°, 22.6±0.2°, 10.7±0.2°, 25.6±0.2°, 12.7±0.2°, 24.5±0.2, 19.1±0.2°, 18.4±0.2°, 6.2±0.2°, 31.5±0.2°, 29.2±0.2°, 20.6±0.2°, 21.4±0.2°, 21.1±0.2°, 15.8±0.2°, 27.4±0.2°, 33.1±0.2° and 14.5±0.2°; preferably at least comprises any 2 to 3, or 4 to 5, or 7 to 8, or 10 to 12, or 15 to 18 of the above diffraction peaks; and further preferably comprises any 2, 3, 4, 5, 6, 8, 10, 12, 16, 18 of the above diffraction peaks; for example, the X-ray powder diffraction pattern of crystal form A has diffraction peaks at 2θ of the following positions:

20.0±0.2°, 19.4±0.2°, 17.1±0.2°, 21.1±0.2°, 15.8±0.2° and 22.6±0.2°;
or, 20.0±0.2°, 19.4±0.2°, 17.1±0.2°, 21.1±0.2°, 15.8±0.2° and 10.7±0.2°;
or, 20.0±0.2°, 19.4±0.2°, 17.1±0.2°, 21.1±0.2°, 15.8±0.2° and 25.6±0.2°;
or, 20.0±0.2°, 19.4±0.2°, 17.1±0.2°, 21.1±0.2°, 15.8±0.2° and 12.7±0.2°;
or, 20.0±0.2°, 19.4±0.2°, 17.1±0.2°, 21.1±0.2°, 15.8±0.2° and 24.5±0.2°;
or, 20.0±0.2°, 19.4±0.2°, 17.1±0.2°, 21.1±0.2°, 15.8±0.2° and 19.1±0.2°;
or, 20.0±0.2°, 19.4±0.2°, 17.1±0.2°, 21.1±0.2°, 15.8±0.2° and 18.4±0.2°;
or, 20.0±0.2°, 19.4±0.2°, 17.1±0.2°, 21.1±0.2°, 15.8±0.2° and 6.2±0.2°;
or, 20.0±0.2°, 19.4±0.2°, 17.1±0.2°, 21.1±0.2°, 15.8±0.2° and 31.5±0.2°;
or, 19.4±0.2°, 17.1±0.2°, 22.6±0.2°, 21.1±0.2°, 15.8±0.2° and 10.7±0.2°;
or, 19.4±0.2°, 17.1±0.2°, 22.6±0.2°, 21.1±0.2°, 15.8±0.2° and 25.6±0.2°;
or, 19.4±0.2°, 17.1±0.2°, 22.6±0.2°, 21.1±0.2°, 15.8±0.2° and 12.7±0.2°;
or, 19.4±0.2°, 17.1±0.2°, 22.6±0.2°, 21.1±0.2°, 15.8±0.2° and 24.5±0.2°;
or, 19.4±0.2°, 17.1±0.2°, 22.6±0.2°, 21.1±0.2°, 15.8±0.2° and 19.1±0.2°;
or, 19.4±0.2°, 17.1±0.2°, 22.6±0.2°, 21.1±0.2°, 15.8±0.2° and 18.4±0.2°;
or, 19.4±0.2°, 17.1±0.2°, 22.6±0.2°, 21.1±0.2°, 15.8±0.2° and 6.2±0.2°;
or, 19.4±0.2°, 17.1±0.2°, 22.6±0.2°, 21.1±0.2°, 15.8±0.2° and 31.5±0.2°;
or, 17.1±0.2°, 22.6±0.2°, 10.7±0.2°, 21.1±0.2°, 15.8±0.2° and 25.6±0.2°;
or, 17.1±0.2°, 22.6±0.2°, 10.7±0.2°, 21.1±0.2°, 15.8±0.2° and 12.7±0.2°;
or, 17.1±0.2°, 22.6±0.2°, 10.7±0.2°, 21.1±0.2°, 15.8±0.2° and 24.5±0.2°;
or, 17.1±0.2°, 22.6±0.2°, 10.7±0.2°, 21.1±0.2°, 15.8±0.2° and 19.1±0.2°;
or, 17.1±0.2°, 22.6±0.2°, 10.7±0.2°, 21.1±0.2°, 15.8±0.2° and 18.4±0.2°;
or, 17.1±0.2°, 22.6±0.2°, 10.7±0.2°, 21.1±0.2°, 15.8±0.2° and 6.2±0.2°;
or, 17.1±0.2°, 22.6±0.2°, 10.7±0.2°, 21.1±0.2°, 15.8±0.2° and 31.5±0.2°;
or, 20.0±0.2°, 17.1±0.2°, 22.6±0.2°, 21.1±0.2°, 15.8±0.2° and 10.7±0.2°;
or, 20.0±0.2°, 17.1±0.2°, 22.6±0.2°, 21.1±0.2°, 15.8±0.2° and 25.6±0.2°;
or, 20.0±0.2°, 17.1±0.2°, 22.6±0.2°, 21.1±0.2°, 15.8±0.2° and 12.7±0.2°;
or, 20.0±0.2°, 17.1±0.2°, 22.6±0.2°, 21.1±0.2°, 15.8±0.2° and 24.5±0.2°;
or, 20.0±0.2°, 17.1±0.2°, 22.6±0.2°, 21.1±0.2°, 15.8±0.2° and 19.1±0.2°;
or, 20.0±0.2°, 17.1±0.2°, 22.6±0.2°, 21.1±0.2°, 15.8±0.2° and 18.4±0.2°;
or, 20.0±0.2°, 17.1±0.2°, 22.6±0.2°, 21.1±0.2°, 15.8±0.2° and 6.2±0.2°;
or, 20.0±0.2°, 17.1±0.2°, 22.6±0.2°, 21.1±0.2°, 15.8±0.2° and 31.5±0.2°;
or, 20.0±0.2°, 19.4±0.2°, 22.6±0.2°, 21.1±0.2°, 15.8±0.2° and 10.7±0.2°;

or, 20.0±0.2°, 19.4±0.2°, 22.6±0.2°, 21.1±0.2°, 15.8±0.2° and 25.6±0.2°;
or, 20.0±0.2°, 19.4±0.2°, 22.6±0.2°, 21.1±0.2°, 15.8±0.2° and 12.7±0.2°;
or, 20.0±0.2°, 19.4±0.2°, 22.6±0.2°, 21.1±0.2°, 15.8±0.2° and 24.5±0.2°;
or, 20.0±0.2°, 19.4±0.2°, 22.6±0.2°, 21.1±0.2°, 15.8±0.2° and 19.1±0.2°;
or, 20.0±0.2°, 19.4±0.2°, 22.6±0.2°, 21.1±0.2°, 15.8±0.2° and 18.4±0.2°;
or, 20.0±0.2°, 19.4±0.2°, 22.6±0.2°, 21.1±0.2°, 15.8±0.2° and 6.2±0.2°;
or, 20.0±0.2°, 19.4±0.2°, 22.6±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 21.1±0.2°, 15.8±0.2° and 25.6±0.2°;
or, 20.0±0.2°, 19.4±0.2°, 17.1±0.2°, 22.6±0.2°, 10.7±0.2°, 21.1±0.2°, 15.8±0.2° and 12.7±0.2°;
or, 20.0±0.2°, 19.4±0.2°, 17.1±0.2°, 22.6±0.2°, 10.7±0.2°, 21.1±0.2°, 15.8±0.2° and 24.5±0.2°;
or, 20.0±0.2°, 19.4±0.2°, 17.1±0.2°, 22.6±0.2°, 25.6±0.2°, 21.1±0.2°, 15.8±0.2° and 12.7±0.2°;
or, 20.0±0.2°, 19.4±0.2°, 17.1±0.2°, 22.6±0.2°, 25.6±0.2°, 21.1±0.2°, 15.8±0.2° and 24.5±0.2°;
or, 20.0±0.2°, 19.4±0.2°, 17.1±0.2°, 22.6±0.2°, 12.7±0.2°, 21.1±0.2°, 15.8±0.2° and 24.5±0.2°;
or, 20.0±0.2°, 19.4±0.2°, 17.1±0.2°, 10.7±0.2°, 25.6±0.2°, 21.1±0.2°, 15.8±0.2° and 12.7±0.2°;
or, 20.0±0.2°, 19.4±0.2°, 17.1±0.2°, 10.7±0.2°, 12.7±0.2°, 21.1±0.2°, 15.8±0.2° and 24.5±0.2°;
or, 20.0±0.2°, 19.4±0.2°, 17.1±0.2°, 10.7±0.2°, 25.6±0.2°, 21.1±0.2°, 15.8±0.2° and 24.5±0.2°;
or, 20.0±0.2°, 19.4±0.2°, 22.6±0.2°, 10.7±0.2°, 25.6±0.2°, 21.1±0.2°, 15.8±0.2° and 12.7±0.2°;
or, 20.0±0.2°, 19.4±0.2°, 22.6±0.2°, 10.7±0.2°, 12.7±0.2°, 21.1±0.2°, 15.8±0.2° and 24.5±0.2°;
or, 20.0±0.2°, 19.4±0.2°, 22.6±0.2°, 25.6±0.2°, 12.7±0.2°, 21.1±0.2°, 15.8±0.2° and 24.5±0.2°;
or, 20.0±0.2°, 19.4±0.2°, 22.6±0.2°, 10.7±0.2°, 25.6±0.2°, 21.1±0.2°, 15.8±0.2° and 24.5±0.2°;
or, 19.4±0.2°, 17.1±0.2°, 22.6±0.2°, 10.7±0.2°, 25.6±0.2°, 21.1±0.2°, 15.8±0.2° and 12.7±0.2°;
or, 19.4±0.2°, 17.1±0.2°, 22.6±0.2°, 10.7±0.2°, 12.7±0.2°, 21.1±0.2°, 15.8±0.2° and 24.5±0.2°;
or, 19.4±0.2°, 17.1±0.2°, 22.6±0.2°, 25.6±0.2°, 12.7±0.2°, 21.1±0.2°, 15.8±0.2° and 24.5±0.2°;
or, 19.4±0.2°, 17.1±0.2°, 22.6±0.2°, 10.7±0.2°, 25.6±0.2°, 21.1±0.2°, 15.8±0.2° and 24.5±0.2°;
or, 20.0±0.2°, 17.1±0.2°, 22.6±0.2°, 10.7±0.2°, 25.6±0.2°, 21.1±0.2°, 15.8±0.2° and 12.7±0.2°;
or, 20.0±0.2°, 17.1±0.2°, 22.6±0.2°, 10.7±0.2°, 12.7±0.2°, 21.1±0.2°, 15.8±0.2° and 24.5±0.2°;
or, 20.0±0.2°, 17.1±0.2°, 22.6±0.2°, 25.6±0.2°, 12.7±0.2°, 21.1±0.2°, 15.8±0.2° and 24.5±0.2°;
or, 20.0±0.2°, 17.1±0.2°, 22.6±0.2°, 10.7±0.2°, 25.6±0.2°, 21.1±0.2°, 15.8±0.2° and 24.5±0.2°;
or, 20.0±0.2°, 19.4±0.2°, 17.1±0.2°, 22.6±0.2°, 10.7±0.2°, 25.6±0.2°, 12.7±0.2°, 21.1±0.2°, 15.8±0.2° and 19.1±0.2°;
or, 20.0±0.2°, 19.4±0.2°, 17.1±0.2°, 22.6±0.2°, 10.7±0.2°, 25.6±0.2°, 12.7±0.2°, 21.1±0.2°, 15.8±0.2° and 18.4±0.2°;
or, 20.0±0.2°, 19.4±0.2°, 17.1±0.2°, 22.6±0.2°, 10.7±0.2°, 25.6±0.2°, 12.7±0.2°, 21.1±0.2°, 15.8±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 25.6±0.2°, 24.5±0.2°, 21.1±0.2°, 15.8±0.2° and 19.1±0.2°;
or, 20.0±0.2°, 19.4±0.2°, 17.1±0.2°, 22.6±0.2°, 10.7±0.2°, 25.6±0.2°, 24.5±0.2°, 21.1±0.2°, 15.8±0.2° and 18.4±0.2°;
or, 20.0±0.2°, 19.4±0.2°, 17.1±0.2°, 22.6±0.2°, 10.7±0.2°, 25.6±0.2°, 19.1±0.2°, 21.1±0.2°, 15.8±0.2° and 18.4±0.2°;
or, 20.0±0.2°, 19.4±0.2°, 17.1±0.2°, 22.6±0.2°, 10.7±0.2°, 12.7±0.2°, 24.5±0.2°, 21.1±0.2°, 15.8±0.2° and 19.1±0.2°;
or, 20.0±0.2°, 19.4±0.2°, 17.1±0.2°, 22.6±0.2°, 10.7±0.2°, 12.7±0.2°, 24.5±0.2°, 21.1±0.2°, 15.8±0.2° and 18.4±0.2°;
or, 20.0±0.2°, 19.4±0.2°, 17.1±0.2°, 22.6±0.2°, 10.7±0.2°, 25.6±0.2°, 19.1±0.2°, 21.1±0.2°, 15.8±0.2° and 18.4±0.2°;
or, 20.0±0.2°, 19.4±0.2°, 17.1±0.2°, 22.6±0.2°, 25.6±0.2°, 12.7±0.2°, 24.5±0.2°, 21.1±0.2°, 15.8±0.2° and 19.1±0.2°;
or, 20.0±0.2°, 19.4±0.2°, 17.1±0.2°, 22.6±0.2°, 25.6±0.2°, 12.7±0.2°, 24.5±0.2°, 21.1±0.2°, 15.8±0.2° and 18.4±0.2°;
or, 20.0±0.2°, 19.4±0.2°, 17.1±0.2°, 22.6±0.2°, 12.7±0.2°, 24.5±0.2°, 19.1±0.2°, 21.1±0.2°, 15.8±0.2° and 18.4±0.2°;
or, 20.0±0.2°, 19.4±0.2°, 17.1±0.2°, 22.6±0.2°, 25.6±0.2°, 24.5±0.2°, 19.1±0.2°, 21.1±0.2°, 15.8±0.2° and 18.4±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 19.1±0.2°, 21.1±0.2°, 15.8±0.2° and 18.4±0.2°;

or, 20.0±0.2°, 19.4±0.2°, 17.1±0.2°, 22.6±0.2°, 10.7±0.2°, 25.6±0.2°, 12.7±0.2°, 24.5±0.2°, 18.4±0.2°, 21.1±0.2°, 15.8±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 18.4±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 6.2±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 25.6±0.2°, 12.7±0.2°, 24.5±0.2°, 18.4±0.2°, 21.1±0.2°, 15.8±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 19.1±0.2°, 18.4±0.2°, 21.1±0.2°, 15.8±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 19.1±0.2°, 18.4±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 25.6±0.2°, 12.7±0.2°, 19.1±0.2°, 6.2±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 25.6±0.2°, 24.5±0.2°, 19.1±0.2°, 18.4±0.2°, 21.1±0.2°, 15.8±0.2° and 6.2±0.2°;
or, 20.0±0.2°, 19.4±0.2°, 17.1±0.2°, 22.6±0.2°, 10.7±0.2°, 25.6±0.2°, 24.5±0.2°, 19.1±0.2°, 18.4±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 25.6±0.2°, 19.1±0.2°, 18.4±0.2°, 6.2±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 12.7±0.2°, 24.5±0.2°, 19.1±0.2°, 18.4±0.2°, 21.1±0.2°, 15.8±0.2° and 6.2±0.2°;
or, 20.0±0.2°, 19.4±0.2°, 17.1±0.2°, 22.6±0.2°, 10.7±0.2°, 12.7±0.2°, 24.5±0.2°, 19.1±0.2°, 6.2±0.2°, 21.1±0.2°, 15.8±0.2° and 31.5±0.2°;
or, 20.0±0.2°, 19.4±0.2°, 17.1±0.2°, 22.6±0.2°, 10.7±0.2°, 24.5±0.2°, 19.1±0.2°, 18.4±0.2°, 6.2±0.2°, 21.1±0.2°, 15.8±0.2° and 31.5±0.2°.

Most preferably, using Cu-Kα radiation, the characteristic X-ray diffraction peaks represented by 2θ angle and interplanar spacing d value are shown in Table 6.

Table 6

| No. | XRPD diffraction data of crystal form A | | | |
|---|---|---|---|---|
| | 2θ (±0.2°) | d value | Peak height | Proportion (1%) |
| 1 | 6.205 | 14.2327 | 335 | 15.2 |
| 2 | 10.747 | 8.2255 | 727 | 33 |
| 3 | 12.715 | 6.9565 | 630 | 28.6 |
| 4 | 14.545 | 6.0847 | 184 | 8.4 |
| 5 | 15.761 | 5.6181 | 218 | 9.9 |
| 6 | 17.116 | 5.1763 | 1073 | 48.7 |
| 7 | 18.374 | 4.8247 | 365 | 16.6 |
| 8 | 19.061 | 4.6521 | 424 | 19.3 |
| 9 | 19.428 | 4.5651 | 1271 | 57.7 |
| 10 | 19.976 | 4.4411 | 2202 | 100 |
| 11 | 20.606 | 4.3067 | 278 | 12.6 |
| 12 | 21.111 | 4.2048 | 229 | 10.4 |
| 13 | 21.44 | 4.141 | 264 | 12 |
| 14 | 22.551 | 3.9396 | 838 | 38.1 |
| 15 | 24.532 | 3.6257 | 470 | 21.3 |
| 16 | 25.612 | 3.4751 | 717 | 32.6 |

(continued)

| No. | XRPD diffraction data of crystal form A | | | |
|---|---|---|---|---|
| | 2θ (±0.2°) | d value | Peak height | Proportion (1%) |
| 17 | 27.379 | 3.2548 | 196 | 8.9 |
| 18 | 29.22 | 3.0537 | 318 | 14.4 |
| 19 | 31.514 | 2.8365 | 333 | 15.1 |
| 20 | 33.054 | 2.7078 | 191 | 8.7 |

The crystal form of the present invention is crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine, the X-ray powder diffraction pattern thereof is substantially as shown in Figure 12; the DSC spectrum thereof is as shown in Figure 13; and the TGA spectrum thereof is as shown in Figure 14.

In a preferred embodiment of the present invention, crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine is a solvent-containing crystal form or a solvent-free crystal form, wherein the solvent is one or more selected from the group consisting of water, methanol, acetone, ethyl acetate, acetonitrile, ethanol, 88% acetone, tetrahydrofuran, 2-methyl-tetrahydrofuran, dichloromethane, 1,4-dioxane, benzene, toluene, isopropanol, *n*-butanol, isobutanol, N,N-dimethylformamide, N,N-dimethylacetamide, N-methylpyrrolidone, dimethyl sulfoxide, *n*-propanol, *tert*-butanol, 2-butanone, 3-pentanone, *n*-heptane, heptane, ethyl formate, isopropyl acetate, cyclohexane, methyl *tert*-butyl ether and isopropyl ether.

In a preferred embodiment of the present invention, the number of solvent molecule contained in crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine is 0.2 to 3, preferably 0.2, 0.5, 1, 1.5, 2, 2.5 or 3, and more preferably 0.5, 1, 2 or 3.

In a preferred embodiment of the present invention, crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine is a solvent-free crystal form, and preferably an anhydrous crystal form.

In a preferred embodiment of the present invention, crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine is a hydrate crystal form, and the number of water molecule is 0.2 to 3, preferably 0.2, 0.5, 1, 1.5, 2, 2.5 or 3, and more preferably 0.5, 1, 2 or 3.

In a further preferred embodiment of the present invention, crystal form B of the compound (S)-8-(6-amino-5-((2-amino-3-chloropyridin-4-yl)thio)pyrazin-2-yl) -2-cyclopropyl-8-azaspiro[4.5]dec-2-en-1-amine is provided.

The X-ray powder diffraction pattern of crystal form B of the compound (S)-8-(6-amino-5-((2-amino-3-chloropyrdin-4-yl)thio)pyrazin-2-yl)-2-cyclopropyl-8-az aspiro[4.5]dec-2-en-1-amine has a diffraction peak at 2θ of 20.2±0.2°; or has a diffraction peak at 22.2±0.2°; or has a diffraction peak at 12.2±0.2°; or has a diffraction peak at 25.1±0.2°; or has a diffraction peak at 21.5±0.2°; or has a diffraction peak at 18.5±0.2°; or has a diffraction peak at 17.8±0.2°; or has a diffraction peak at 20.0±0.2°; or has a diffraction peak at 28.6±0.2°; or has a diffraction peak at 5.1±0.2°; preferably comprises any 2 to 5, or 3 to 5, or 3 to 6, or 3 to 8, or 5 to 8, or 6 to 8 of the above diffraction peaks; and more preferably comprises any 6, 7, or 8 of the above diffraction peaks.

The X-ray powder diffraction pattern of crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine at least comprises one or more diffraction peaks at 2θ of 20.2±0.2°, 22.2±0.2° and 12.2±0.2°, preferably comprises 2 of the above diffraction peaks, and more preferably comprises 3 of the above diffraction peaks; optionally, can further comprises at least one diffraction peak at 2θ of 25.1±0.2°, 21.5±0.2°, 18.5±0.2°, 17.8±0.2°, 20.0±0.2, and preferably comprises 2, 3, 4, or 5 of the above diffraction peaks; for example, the X-ray powder diffraction pattern of crystal form B has diffraction peaks at 2θ of the following positions:

20.2±0.2° and 22.2±0.2°;
or, 20.2±0.2° and 12.2±0.2°;
or, 22.2±0.2° and 12.2±0.2°;
or, 22.2±0.2° and 25.1±0.2°;
or, 20.2±0.2°, 22.2±0.2° and 25.1±0.2°;
or, 22.2±0.2°, 25.1±0.2° and 21.5±0.2°;
or, 20.2±0.2°, 22.2±0.2° and 21.5±0.2°;
or, 20.2±0.2°, 12.2±0.2° and 25.1±0.2°;
or, 20.2±0.2°, 22.2±0.2°, 12.2±0.2° and 25.1±0.2°;

or, 20.2±0.2°, 22.2±0.2°, 25.1±0.2° and 21.5±0.2°;
or, 20.2±0.2°, 12.2±0.2°, 25.1±0.2° and 21.5±0.2°;
or, 22.2±0.2°, 25.1±0.2°, 21.5±0.2° and 18.5±0.2°;
or, 20.2±0.2°, 22.2±0.2°, 12.2±0.2°, 25.1±0.2° and 21.5±0.2°;
or, 20.2±0.2°, 22.2±0.2°, 25.1±0.2°, 21.5±0.2° and 18.5±0.2°;
or, 22.2±0.2°, 12.2±0.2°, 25.1±0.2°, 21.5±0.2° and 18.5±0.2°;
or, 22.2±0.2°, 12.2±0.2°, 25.1±0.2°, 18.5±0.2° and 17.8±0.2°;
or, 20.2±0.2°, 22.2±0.2°, 12.2±0.2°, 25.1±0.2°, 21.5±0.2° and 18.5±0.2°;
or, 20.2±0.2°, 22.2±0.2°, 25.1±0.2°, 21.5±0.2°, 18.5±0.2° and 17.8±0.2°;
or, 20.2±0.2°, 12.2±0.2°, 25.1±0.2°, 21.5±0.2°, 18.5±0.2° and 17.8±0.2°;
or, 12.2±0.2°, 22.2±0.2°, 12.2±0.2°, 25.1±0.2°, 21.5±0.2° and 18.5±0.2°;
or, 20.2±0.2°, 22.2±0.2°, 12.2±0.2°, 25.1±0.2°, 21.5±0.2° and 17.8±0.2°;
or, 20.2±0.2°, 22.2±0.2°, 25.1±0.2°, 21.5±0.2°, 18.5±0.2° and 20.0±0.2°;
or, 20.2±0.2°, 12.2±0.2°, 25.1±0.2°, 21.5±0.2°, 18.5±0.2° and 20.0±0.2°;
or, 12.2±0.2°, 22.2±0.2°, 12.2±0.2°, 25.1±0.2°, 21.5±0.2° and 17.8±0.2°.

The X-ray powder diffraction pattern of crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine optionally also comprises one or more diffraction peaks at 2θ of 28.6±0.2°, 5.1±0.2°, 14.3±0.2°, 24.5±0.2°, 13.2±0.2°, 12.4±0.2° and 29.2±0.2°; preferably at least comprises any 2 to 3, or 4 to 5, or 6 to 7 of the above diffraction peaks; and further preferably comprises any 2, 3, 4, 5, 6, or 7 of the above diffraction peaks; for example, the X-ray powder diffraction pattern of crystal form B has diffraction peaks at 2θ of the following positions:

20.2±0.2°, 22.2±0.2°, 12.2±0.2°, 25.1±0.2°, 21.5±0.2°, 18.5±0.2°, 28.6±0.2° and 5.1±0.2°;
or, 20.2±0.2°, 22.2±0.2°, 25.1±0.2°, 21.5±0.2°, 18.5±0.2°, 17.8±0.2°, 28.6±0.2°, 5.1±0.2° and 14.3±0.2°;
or, 20.2±0.2°, 12.2±0.2°, 25.1±0.2°, 21.5±0.2°, 18.5±0.2°, 17.8±0.2°, 28.6±0.2°, 5.1±0.2°, 14.3±0.2° and 24.5±0.2°;
20.2±0.2°, 22.2±0.2°, 12.2±0.2°, 25.1±0.2°, 21.5±0.2°, 18.5±0.2°, 28.6±0.2° and 14.3±0.2°;
or, 20.2±0.2°, 22.2±0.2°, 25.1±0.2°, 21.5±0.2°, 18.5±0.2°, 17.8±0.2°, 28.6±0.2°, 5.1±0.2° and 24.5±0.2°;
or, 20.2±0.2°, 12.2±0.2°, 25.1±0.2°, 21.5±0.2°, 18.5±0.2°, 17.8±0.2°, 28.6±0.2°, 5.1±0.2°, 14.3±0.2° and 13.2±0.2°;
20.2±0.2°, 22.2±0.2°, 12.2±0.2°, 25.1±0.2°, 21.5±0.2°, 18.5±0.2°, 28.6±0.2° and 24.5±0.2°;
or, 20.2±0.2°, 22.2±0.2°, 25.1±0.2°, 21.5±0.2°, 18.5±0.2°, 17.8±0.2°, 28.6±0.2°, 5.1±0.2° and 13.2±0.2°;
or, 20.2±0.2°, 12.2±0.2°, 25.1±0.2°, 21.5±0.2°, 18.5±0.2°, 17.8±0.2°, 28.6±0.2°, 5.1±0.2°, 14.3±0.2° and 12.4±0.2°.

The X-ray powder diffraction pattern of crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine comprises one or more diffraction peaks at 2θ of 20.2±0.2°, 22.2±0.2°, 12.2±0.2°, 25.1±0.2°, 21.5±0.2°, 18.5±0.2°, 17.8±0.2°, 20.0±0.2, 28.6±0.2°, 5.1±0.2°, 14.3±0.2°, 24.5±0.2°, 13.2±0.2°, 12.4±0.2° and 29.2±0.2°; and preferably comprises any 4, 5, 6, 8, or 10 of the above diffraction peaks; for example, the X-ray powder diffraction pattern of crystal form B has diffraction peaks at 2θ of the following positions:

20.2±0.2°, 22.2±0.2°, 12.2±0.2° and 25.1±0.2°;
or, 20.2±0.2°, 22.2±0.2°, 12.2±0.2° and 21.5±0.2°;
or, 20.2±0.2°, 22.2±0.2°, 12.2±0.2° and 18.5±0.2°;
or, 20.2±0.2°, 22.2±0.2°, 12.2±0.2° and 17.8±0.2°;
or, 20.2±0.2°, 22.2±0.2°, 12.2±0.2° and 20.0±0.2°;
or, 20.2±0.2°, 22.2±0.2°, 12.2±0.2° and 22.2±0.2°;
or, 22.2±0.2°, 12.2±0.2°, 25.1±0.2° and 21.5±0.2°;
or, 22.2±0.2°, 12.2±0.2°, 21.5±0.2° and 18.5±0.2°;
or, 12.2±0.2°, 25.1±0.2°, 21.5±0.2° and 18.5±0.2°;
or, 12.2±0.2°, 25.1±0.2°, 18.5±0.2° and 28.6±0.2°;
or, 20.2±0.2°, 22.2±0.2°, 12.2±0.2°, 25.1±0.2°, 21.5±0.2° and 28.6±0.2°;
or, 20.2±0.2°, 22.2±0.2°, 12.2±0.2°, 21.5±0.2°, 18.5±0.2° and 28.6±0.2°;
or, 20.2±0.2°, 22.2±0.2°, 12.2±0.2°, 25.1±0.2°, 21.5±0.2° and 5.1±0.2°;
or, 20.2±0.2°, 22.2±0.2°, 12.2±0.2°, 21.5±0.2°, 18.5±0.2° and 5.1±0.2°;
or, 20.2±0.2°, 22.2±0.2°, 12.2±0.2°, 25.1±0.2°, 21.5±0.2° and 14.3±0.2°;

or, 20.2±0.2°, 22.2±0.2°, 12.2±0.2°, 21.5±0.2°, 18.5±0.2° and 14.3±0.2°;
or, 22.2±0.2°, 12.2±0.2°, 25.1±0.2°, 21.5±0.2°, 18.5±0.2° and 28.6±0.2°;
or, 22.2±0.2°, 12.2±0.2°, 25.1±0.2°, 21.5±0.2°, 28.6±0.2° and 14.3±0.2°;
or, 12.2±0.2°, 25.1±0.2°, 21.5±0.2°, 18.5±0.2°, 28.6±0.2° and 14.3±0.2°;
or, 12.2±0.2°, 25.1±0.2°, 17.8±0.2°, 20.0±0.2, 28.6±0.2° and 14.3±0.2°;
or, 20.2±0.2°, 22.2±0.2°, 12.2±0.2°, 25.1±0.2°, 21.5±0.2°, 18.5±0.2°, 28.6±0.2° and 14.3±0.2°;
or, 20.2±0.2°, 22.2±0.2°, 12.2±0.2°, 25.1±0.2°, 21.5±0.2°, 18.5±0.2°, 5.1±0.2° and 14.3±0.2°;
or, 20.2±0.2°, 22.2±0.2°, 12.2±0.2°, 25.1±0.2°, 21.5±0.2°, 18.5±0.2°, 28.6±0.2° and 24.5±0.2°;
or, 20.2±0.2°, 22.2±0.2°, 12.2±0.2°, 25.1±0.2°, 21.5±0.2°, 18.5±0.2°, 5.1±0.2° and 24.5±0.2°;
or, 20.2±0.2°, 22.2±0.2°, 12.2±0.2°, 25.1±0.2°, 21.5±0.2°, 18.5±0.2°, 28.6±0.2° and 13.2±0.2°;
or, 20.2±0.2°, 22.2±0.2°, 12.2±0.2°, 25.1±0.2°, 21.5±0.2°, 18.5±0.2°, 5.1±0.2° and 13.2±0.2°;
or, 22.2±0.2°, 12.2±0.2°, 25.1±0.2°, 21.5±0.2°, 18.5±0.2°, 17.8±0.2°, 20.0±0.2 and 28.6±0.2°;
or, 22.2±0.2°, 12.2±0.2°, 25.1±0.2°, 21.5±0.2°, 18.5±0.2°, 17.8±0.2°, 28.6±0.2° and 14.3±0.2°;
or, 12.2±0.2°, 25.1±0.2°, 21.5±0.2°, 18.5±0.2°, 17.8±0.2°, 20.0±0.2, 28.6±0.2° and 14.3±0.2°;
or, 12.2±0.2°, 25.1±0.2°, 21.5±0.2°, 18.5±0.2°, 17.8±0.2°, 20.0±0.2, 28.6±0.2° and 24.5±0.2°;
or, 20.2±0.2°, 22.2±0.2°, 12.2±0.2°, 25.1±0.2°, 21.5±0.2°, 18.5±0.2°, 17.8±0.2°, 20.0±0.2, 28.6±0.2° and 14.3±0.2°;
or, 20.2±0.2°, 22.2±0.2°, 12.2±0.2°, 25.1±0.2°, 21.5±0.2°, 18.5±0.2°, 17.8±0.2°, 20.0±0.2, 5.1±0.2° and 14.3±0.2°;
or, 20.2±0.2°, 22.2±0.2°, 12.2±0.2°, 25.1±0.2°, 21.5±0.2°, 18.5±0.2°, 17.8±0.2°, 20.0±0.2, 28.6±0.2° and 24.5±0.2°;
or, 20.2±0.2°, 22.2±0.2°, 12.2±0.2°, 25.1±0.2°, 21.5±0.2°, 18.5±0.2°, 17.8±0.2°, 20.0±0.2, 5.1±0.2° and 24.5±0.2°;
or, 20.2±0.2°, 22.2±0.2°, 12.2±0.2°, 25.1±0.2°, 21.5±0.2°, 18.5±0.2°, 17.8±0.2°, 20.0±0.2, 28.6±0.2° and 13.2±0.2°;
or, 20.2±0.2°, 22.2±0.2°, 12.2±0.2°, 25.1±0.2°, 21.5±0.2°, 18.5±0.2°, 17.8±0.2°, 20.0±0.2, 5.1±0.2° and 13.2±0.2°;
or, 22.2±0.2°, 12.2±0.2°, 25.1±0.2°, 21.5±0.2°, 18.5±0.2°, 17.8±0.2°, 20.0±0.2, 28.6±0.2°, 5.1±0.2° and 13.2±0.2°;
or, 22.2±0.2°, 12.2±0.2°, 25.1±0.2°, 21.5±0.2°, 18.5±0.2°, 17.8±0.2°, 20.0±0.2, 28.6±0.2°, 5.1±0.2° and 14.3±0.2°;
or, 12.2±0.2°, 25.1±0.2°, 21.5±0.2°, 18.5±0.2°, 17.8±0.2°, 20.0±0.2, 28.6±0.2°, 5.1±0.2°, 14.3±0.2° and 13.2±0.2°;
or, 12.2±0.2°, 25.1±0.2°, 21.5±0.2°, 18.5±0.2°, 17.8±0.2°, 20.0±0.2, 28.6±0.2°, 5.1±0.2°, 14.3±0.2° and 24.5±0.2°.

The X-ray powder diffraction pattern of crystal form B of the compound (S)-8-(6-amino-5-((2-amino-3-chloropyrdin-4-yl)thio)pyrazin-2-yl)-2-cyclopropyl-8-az aspiro[4.5]dec-2-en-1-amine comprises one or more diffraction peaks at 2θ of 20.2±0.2°, 22.2±0.2°, 12.2±0.2°, 25.1±0.2°, 21.5±0.2°, 18.5±0.2°, 17.8±0.2°, 20.0±0.2, 28.6±0.2°, 5.1±0.2°, 14.3±0.2°, 24.5±0.2°, 13.2±0.2°, 12.4±0.2°, 29.2±0.2°, 10.8±0.2°, 9.9±0.2°, 16.9±0.2°, 30.5±0.2° and 15.5±0.2°; preferably at least comprises any 2 to 3, or 4 to 5, or 7 to 8, or 10 to 12, or 15 to 18 of the above diffraction peaks; and further preferably comprises any 2, 3, 4, 5, 6, 8, 10, 12, 16, 18 of the above diffraction peaks; for example, the X-ray powder diffraction pattern of crystal form B has diffraction peaks at 2θ of the following positions:

20.2±0.2°, 22.2±0.2°, 12.2±0.2° and 25.1±0.2°;
or, 20.2±0.2°, 22.2±0.2°, 12.2±0.2°, 10.8±0.2°, 9.9±0.2° and 21.5±0.2°;
or, 20.2±0.2°, 22.2±0.2°, 12.2±0.2°, 10.8±0.2°, 9.9±0.2° and 18.5±0.2°;
or, 20.2±0.2°, 22.2±0.2°, 12.2±0.2°, 10.8±0.2°, 9.9±0.2° and 17.8±0.2°;
or, 20.2±0.2°, 22.2±0.2°, 12.2±0.2°, 10.8±0.2°, 9.9±0.2° and 20.0±0.2°;
or, 22.2±0.2°, 12.2±0.2°, 10.8±0.2°, 9.9±0.2°, 10.8±0.2° and 9.9±0.2°;
or, 22.2±0.2°, 12.2±0.2°, 10.8±0.2°, 10.8±0.2°, 9.9±0.2° and 22.2±0.2°;
or, 12.2±0.2°, 10.8±0.2°, 9.9±0.2°, 10.8±0.2°, 9.9±0.2° and 22.2±0.2°;
or, 12.2±0.2°, 25.1±0.2°, 21.5±0.2°, 10.8±0.2°, 9.9±0.2° and 28.6±0.2°;
or, 20.2±0.2°, 22.2±0.2°, 12.2±0.2°, 10.8±0.2°, 9.9±0.2°, 10.8±0.2°, 9.9±0.2° and 22.2±0.2°;
or, 20.2±0.2°, 22.2±0.2°, 12.2±0.2°, 25.1±0.2°, 21.5±0.2°, 10.8±0.2°, 9.9±0.2° and 28.6±0.2°;
or, 20.2±0.2°, 22.2±0.2°, 12.2±0.2°, 21.5±0.2°, 18.5±0.2°, 10.8±0.2°, 9.9±0.2° and 28.6±0.2°;
or, 20.2±0.2°, 22.2±0.2°, 12.2±0.2°, 25.1±0.2°, 21.5±0.2°, 10.8±0.2°, 9.9±0.2° and 5.1±0.2°;
or, 20.2±0.2°, 22.2±0.2°, 12.2±0.2°, 21.5±0.2°, 18.5±0.2°, 10.8±0.2°, 9.9±0.2° and 5.1±0.2°;

or, 20.2±0.2°, 22.2±0.2°, 12.2±0.2°, 25.1±0.2°, 21.5±0.2°, 10.8±0.2°, 9.9±0.2° and 14.3±0.2°;
or, 20.2±0.2°, 22.2±0.2°, 12.2±0.2°, 21.5±0.2°, 18.5±0.2°, 10.8±0.2°, 9.9±0.2° and 14.3±0.2°;
or, 22.2±0.2°, 12.2±0.2°, 25.1±0.2°, 21.5±0.2°, 18.5±0.2°, 28.6±0.2°, 10.8±0.2° and 9.9±0.2°;
or, 22.2±0.2°, 12.2±0.2°, 25.1±0.2°, 21.5±0.2°, 18.5±0.2°, 28.6±0.2°, 9.9±0.2° and 14.3±0.2°;
or, 12.2±0.2°, 25.1±0.2°, 21.5±0.2°, 18.5±0.2°, 28.6±0.2°, 10.8±0.2°, 9.9±0.2° and 14.3±0.2°;
or, 12.2±0.2°, 25.1±0.2°, 21.5±0.2°, 18.5±0.2°, 28.6±0.2°, 10.8±0.2°, 9.9±0.2° and 24.5±0.2°;
or, 20.2±0.2°, 22.2±0.2°, 12.2±0.2°, 25.1±0.2°, 21.5±0.2°, 18.5±0.2°, 28.6±0.2°, 10.8±0.2°, 9.9±0.2° and 14.3±0.2°;
or, 20.2±0.2°, 22.2±0.2°, 12.2±0.2°, 25.1±0.2°, 21.5±0.2°, 18.5±0.2°, 5.1±0.2°, 10.8±0.2°, 9.9±0.2° and 14.3±0.2°;
or, 20.2±0.2°, 22.2±0.2°, 12.2±0.2°, 25.1±0.2°, 21.5±0.2°, 18.5±0.2°, 28.6±0.2°, 10.8±0.2°, 9.9±0.2° and 24.5±0.2°;
or, 20.2±0.2°, 22.2±0.2°, 12.2±0.2°, 25.1±0.2°, 21.5±0.2°, 18.5±0.2°, 5.1±0.2°, 10.8±0.2°, 9.9±0.2° and 24.5±0.2°;
or, 20.2±0.2°, 22.2±0.2°, 12.2±0.2°, 25.1±0.2°, 21.5±0.2°, 18.5±0.2°, 28.6±0.2°, 10.8±0.2°, 9.9±0.2° and 13.2±0.2°;
or, 20.2±0.2°, 22.2±0.2°, 12.2±0.2°, 25.1±0.2°, 21.5±0.2°, 18.5±0.2°, 5.1±0.2°, 10.8±0.2°, 9.9±0.2° and 13.2±0.2°;
or, 22.2±0.2°, 12.2±0.2°, 25.1±0.2°, 21.5±0.2°, 18.5±0.2°, 17.8±0.2°, 28.6±0.2°, 10.8±0.2°, 9.9±0.2° and 13.2±0.2°;
or, 22.2±0.2°, 12.2±0.2°, 25.1±0.2°, 21.5±0.2°, 18.5±0.2°, 17.8±0.2°, 20.0±0.2°, 10.8±0.2°, 9.9±0.2° and 13.2±0.2°;
or, 12.2±0.2°, 25.1±0.2°, 21.5±0.2°, 18.5±0.2°, 17.8±0.2°, 20.0±0.2°, 28.6±0.2°, 10.8±0.2°, 9.9±0.2° and 13.2±0.2°;
or, 12.2±0.2°, 25.1±0.2°, 21.5±0.2°, 18.5±0.2°, 17.8±0.2°, 20.0±0.2°, 28.6±0.2°, 5.1±0.2°, 9.9±0.2° and 13.2±0.2°.

Most preferably, using Cu-Kα radiation, the characteristic X-ray diffraction peaks represented by 2θ angle and interplanar spacing d value are shown in Table 7.

Table 7

| No. | XRPD diffraction data of crystal form B | | | |
|---|---|---|---|---|
| | 2θ (±0.2°) | d value | Peak height | Proportion (1%) |
| 1 | 5.095 | 17.3287 | 325 | 22 |
| 2 | 9.939 | 8.892 | 189 | 12.8 |
| 3 | 10.831 | 8.162 | 191 | 12.9 |
| 4 | 12.188 | 7.256 | 815 | 55.2 |
| 5 | 12.414 | 7.1243 | 231 | 15.7 |
| 6 | 13.162 | 6.7211 | 265 | 18 |
| 7 | 14.316 | 6.1817 | 306 | 20.7 |
| 8 | 15.493 | 5.7145 | 173 | 11.7 |
| 9 | 16.854 | 5.2563 | 179 | 12.1 |
| 10 | 17.788 | 4.9822 | 371 | 25.1 |
| 11 | 18.452 | 4.8045 | 440 | 29.8 |
| 12 | 19.976 | 4.4412 | 359 | 24.3 |
| 13 | 20.238 | 4.3842 | 1476 | 100 |
| 14 | 21.455 | 4.1382 | 502 | 34 |
| 15 | 22.185 | 4.0037 | 1166 | 79 |
| 16 | 24.496 | 3.631 | 295 | 20 |

(continued)

| No. | XRPD diffraction data of crystal form B | | | |
|---|---|---|---|---|
| | 2θ (±0.2°) | d value | Peak height | Proportion (1%) |
| 17 | 25.081 | 3.5476 | 634 | 43 |
| 18 | 28.551 | 3.1238 | 327 | 22.2 |
| 19 | 29.239 | 3.0518 | 216 | 14.6 |
| 20 | 30.478 | 2.9305 | 177 | 12 |

The crystal form of the present invention is crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine, the X-ray powder diffraction pattern thereof is substantially as shown in Figure 15; and the DSC spectrum thereof is as shown in Figure 16.

In a preferred embodiment of the present invention, crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine is a solvent-containing crystal form or a solvent-free crystal form, wherein the solvent is one or more selected from the group consisting of water, methanol, acetone, ethyl acetate, acetonitrile, ethanol, 88% acetone, tetrahydrofuran, 2-methyl-tetrahydrofuran, dichloromethane, 1,4-dioxane, benzene, toluene, isopropanol, *n*-butanol, isobutanol, N,N-dimethylformamide, N,N-dimethylacetamide, N-methylpyrrolidone, dimethyl sulfoxide, *n*-propanol, *tert*-butanol, 2-butanone, 3-pentanone, *n*-heptane, heptane, ethyl formate, isopropyl acetate, cyclohexane, methyl *tert*-butyl ether and isopropyl ether.

In a preferred embodiment of the present invention, the number of solvent molecule contained in crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine is 0.2 to 3, preferably 0.2, 0.5, 1, 1.5, 2, 2.5 or 3, and more preferably 0.5, 1, 2 or 3.

In a preferred embodiment of the present invention, crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine is a solvent-free crystal form, and preferably an anhydrous crystal form.

In a preferred embodiment of the present invention, crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine is a hydrate crystal form, and the number of water molecule is 0.2 to 3, preferably 0.2, 0.5, 1, 1.5, 2, 2.5 or 3, and more preferably 0.5, 1, 2 or 3.

In a further preferred embodiment of the present invention, crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine is provided.

The X-ray powder diffraction pattern of crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine has a diffraction peak at 2θ of 17.8±0.2°; or has a diffraction peak at 19.8±0.2°; or has a diffraction peak at 26.1±0.2°; or has a diffraction peak at 18.2±0.2°; or has a diffraction peak at 24.0±0.2°; or has a diffraction peak at 22.8±0.2°; or has a diffraction peak at 10.5±0.2°; or has a diffraction peak at 21.5±0.2°; or has a diffraction peak at 17.5±0.2°; or has a diffraction peak at 21.8±0.2°; preferably comprises any 2 to 5, or 3 to 5, or 3 to 6, or 3 to 8, or 5 to 8, or 6 to 8 of the above diffraction peaks; and more preferably comprises any 6, 7, or 8 of the above diffraction peaks.

The X-ray powder diffraction pattern of crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine at least comprises one or more diffraction peaks at 2θ of 17.8±0.2°, 19.8±0.2° and 26.1±0.2°, preferably comprises 2 of the above diffraction peaks, and more preferably comprises 3 of the above diffraction peaks; optionally, can further comprises at least one diffraction peak at 2θ of 18.2±0.2°, 24.0±0.2°, 22.8±0.2°, 10.5±0.2°, 21.5±0.2, and preferably comprises 2, 3, 4, or 5 of the above diffraction peaks; for example, the X-ray powder diffraction pattern of crystal form C has diffraction peaks at 2θ of the following positions:

17.8±0.2° and 19.8±0.2°;
or, 17.8±0.2° and 26.1±0.2°;
or, 19.8±0.2° and 26.1±0.2°;
or, 26.1±0.2° and 18.2±0.2°;
or, 17.8±0.2°, 19.8±0.2° and 26.1±0.2°;
or, 17.8±0.2°, 26.1±0.2° and 18.2±0.2°;
or, 19.8±0.2°, 26.1±0.2° and 18.2±0.2°;
or, 26.1±0.2°, 18.2±0.2° and 24.0±0.2°;
or, 17.8±0.2°, 19.8±0.2°, 26.1±0.2° and 18.2±0.2°;
or, 17.8±0.2°, 19.8±0.2°, 18.2±0.2° and 24.0±0.2°;

or, 19.8±0.2°, 26.1±0.2°, 18.2±0.2° and 24.0±0.2°;
or, 26.1±0.2°, 18.2±0.2°, 24.0±0.2° and 22.8±0.2°;
or, 17.8±0.2°, 19.8±0.2°, 26.1±0.2°, 18.2±0.2° and 24.0±0.2°;
or, 19.8±0.2°, 26.1±0.2°, 18.2±0.2°, 24.0±0.2° and 22.8±0.2°;
or, 17.8±0.2°, 19.8±0.2°, 18.2±0.2°, 24.0±0.2° and 10.5±0.2°;
or, 26.1±0.2°, 18.2±0.2°, 24.0±0.2°, 22.8±0.2° and 10.5±0.2°;
or, 17.8±0.2°, 19.8±0.2°, 26.1±0.2°, 18.2±0.2°, 24.0±0.2° and 22.8±0.2°;
or, 17.8±0.2°, 19.8±0.2°, 18.2±0.2°, 24.0±0.2°, 22.8±0.2° and 10.5±0.2°;
or, 17.8±0.2°, 26.1±0.2°, 18.2±0.2°, 24.0±0.2°, 22.8±0.2° and 10.5±0.2°;
or, 26.1±0.2°, 19.8±0.2°, 26.1±0.2°, 18.2±0.2°, 24.0±0.2° and 22.8±0.2°;
or, 17.8±0.2°, 19.8±0.2°, 26.1±0.2°, 18.2±0.2°, 24.0±0.2° and 10.5±0.2°;
or, 17.8±0.2°, 19.8±0.2°, 18.2±0.2°, 24.0±0.2°, 22.8±0.2° and 21.5±0.2°;
or, 17.8±0.2°, 26.1±0.2°, 18.2±0.2°, 24.0±0.2°, 22.8±0.2° and 21.5±0.2°;
or, 19.8±0.2°, 26.1±0.2°, 18.2±0.2°, 24.0±0.2°, 22.8±0.2° and 10.5±0.2°.

The X-ray powder diffraction pattern of crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine optionally also comprises one or more diffraction peaks at 2θ of 17.5±0.2°, 21.8±0.2°, 19.5±0.2°, 22.3±0.2°, 24.3±0.2°, 14.5±0.2° and 30.1±0.2°; preferably at least comprises any 2 to 3, or 4 to 5, or 6 to 7 of the above diffraction peaks; and further preferably comprises any 2, 3, 4, 5, 6, or 7 of the above diffraction peaks; for example, the X-ray powder diffraction pattern of crystal form C has diffraction peaks at 2θ of the following positions:

17.8±0.2°, 19.8±0.2°, 26.1±0.2°, 18.2±0.2°, 24.0±0.2°, 22.8±0.2°, 17.5±0.2° and 21.8±0.2°;
or, 19.8±0.2°, 18.2±0.2°, 24.0±0.2°, 22.8±0.2°, 10.5±0.2°, 17.5±0.2°, 21.8±0.2° and 19.5±0.2°;
or, 17.8±0.2°, 19.8±0.2°, 18.2±0.2°, 24.0±0.2°, 22.8±0.2°, 10.5±0.2°, 17.5±0.2°, 21.8±0.2° and 19.5±0.2°;
or, 19.8±0.2°, 26.1±0.2°, 18.2±0.2°, 24.0±0.2°, 22.8±0.2°, 10.5±0.2°, 17.5±0.2°, 21.8±0.2° and 22.3±0.2°;
or, 26.1±0.2°, 18.2±0.2°, 24.0±0.2°, 22.8±0.2°, 10.5±0.2°, 17.5±0.2°, 21.8±0.2°, 19.5±0.2° and 22.3±0.2°;
or, 17.8±0.2°, 26.1±0.2°, 18.2±0.2°, 24.0±0.2°, 22.8±0.2°, 10.5±0.2°, 17.5±0.2°, 21.8±0.2°, 19.5±0.2° and 22.3±0.2°;
or, 19.8±0.2°, 26.1±0.2°, 18.2±0.2°, 24.0±0.2°, 22.8±0.2°, 10.5±0.2°, 17.5±0.2°, 21.8±0.2°, 19.5±0.2° and 22.3±0.2°;
or, 17.8±0.2°, 19.8±0.2°, 26.1±0.2°, 18.2±0.2°, 24.0±0.2°, 22.8±0.2°, 17.5±0.2° and 19.5±0.2°;
or, 19.8±0.2°, 26.1±0.2°, 18.2±0.2°, 24.0±0.2°, 22.8±0.2°, 10.5±0.2°, 17.5±0.2° and 19.5±0.2°;
or, 17.8±0.2°, 19.8±0.2°, 18.2±0.2°, 24.0±0.2°, 22.8±0.2°, 10.5±0.2°, 17.5±0.2°, 21.8±0.2° and 22.3±0.2°;
or, 19.8±0.2°, 26.1±0.2°, 18.2±0.2°, 24.0±0.2°, 22.8±0.2°, 10.5±0.2°, 17.5±0.2°, 21.8±0.2° and 22.3±0.2°;
or, 17.8±0.2°, 26.1±0.2°, 18.2±0.2°, 24.0±0.2°, 22.8±0.2°, 10.5±0.2°, 17.5±0.2°, 21.8±0.2°, 19.5±0.2° and 24.3±0.2°;
or, 19.8±0.2°, 26.1±0.2°, 18.2±0.2°, 24.0±0.2°, 22.8±0.2°, 10.5±0.2°, 17.5±0.2°, 21.8±0.2°, 19.5±0.2° and 24.3±0.2°;
or, 17.8±0.2°, 19.8±0.2°, 26.1±0.2°, 18.2±0.2°, 24.0±0.2°, 22.8±0.2°, 17.5±0.2° and 22.3±0.2°;
or, 19.8±0.2°, 26.1±0.2°, 18.2±0.2°, 24.0±0.2°, 22.8±0.2°, 17.5±0.2°, 21.8±0.2°, and 22.3±0.2°;
or, 17.8±0.2°, 19.8±0.2°, 18.2±0.2°, 24.0±0.2°, 22.8±0.2°, 10.5±0.2°, 17.5±0.2°, 21.8±0.2° and 24.3±0.2°;
or, 19.8±0.2°, 26.1±0.2°, 18.2±0.2°, 24.0±0.2°, 22.8±0.2°, 10.5±0.2°, 17.5±0.2°, 21.8±0.2° and 24.3±0.2°;
or, 17.8±0.2°, 26.1±0.2°, 18.2±0.2°, 24.0±0.2°, 22.8±0.2°, 10.5±0.2°, 17.5±0.2°, 21.8±0.2°, 19.5±0.2° and 14.5±0.2°;
or, 19.8±0.2°, 26.1±0.2°, 18.2±0.2°, 24.0±0.2°, 22.8±0.2°, 10.5±0.2°, 17.5±0.2°, 21.8±0.2°, 19.5±0.2° and 14.5±0.2°.

The X-ray powder diffraction pattern of crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine comprises one or more diffraction peaks at 2θ of 17.8±0.2°, 19.8±0.2°, 26.1±0.2°, 18.2±0.2°, 24.0±0.2°, 22.8±0.2°, 10.5±0.2°, 21.5±0.2, 17.5±0.2°, 21.8±0.2°, 19.5±0.2°, 22.3±0.2°, 24.3±0.2°, 14.5±0.2° and 30.1±0.2°; and preferably comprises any 4, 5, 6, 8, or 10 of the above diffraction peaks; for example, the X-ray powder diffraction pattern of crystal form C has diffraction peaks at 2θ of the following positions:

17.8±0.2°, 19.8±0.2°, 26.1±0.2° and 18.2±0.2°;
or, 17.8±0.2°, 19.8±0.2°, 26.1±0.2° and 24.0±0.2°;
or, 17.8±0.2°, 19.8±0.2°, 26.1±0.2° and 22.8±0.2°;

or, 17.8±0.2°, 19.8±0.2°, 26.1±0.2° and 10.5±0.2°;
or, 17.8±0.2°, 19.8±0.2°, 26.1±0.2° and 21.5±0.2°;
or, 17.8±0.2°, 19.8±0.2°, 26.1±0.2° and 17.5±0.2°;
or, 17.8±0.2°, 19.8±0.2°, 26.1±0.2°, 18.2±0.2°, 24.0±0.2° and 17.5±0.2°;
or, 17.8±0.2°, 19.8±0.2°, 26.1±0.2°, 24.0±0.2°, 22.8±0.2° and 17.5±0.2°;
or, 17.8±0.2°, 19.8±0.2°, 26.1±0.2°, 18.2±0.2°, 24.0±0.2° and 21.8±0.2°;
or, 17.8±0.2°, 19.8±0.2°, 26.1±0.2°, 24.0±0.2°, 22.8±0.2° and 21.8±0.2°;
or, 17.8±0.2°, 19.8±0.2°, 26.1±0.2°, 18.2±0.2°, 24.0±0.2° and 19.5±0.2°;
or, 17.8±0.2°, 19.8±0.2°, 26.1±0.2°, 24.0±0.2°, 22.8±0.2° and 19.5±0.2°;
or, 17.8±0.2°, 19.8±0.2°, 26.1±0.2°, 18.2±0.2°, 24.0±0.2°, 22.8±0.2°, 17.5±0.2° and 19.5±0.2°;
or, 17.8±0.2°, 19.8±0.2°, 26.1±0.2°, 18.2±0.2°, 24.0±0.2°, 22.8±0.2°, 21.8±0.2° and 19.5±0.2°;
or, 17.8±0.2°, 19.8±0.2°, 26.1±0.2°, 18.2±0.2°, 24.0±0.2°, 22.8±0.2°, 17.5±0.2° and 22.3±0.2°;
or, 17.8±0.2°, 19.8±0.2°, 26.1±0.2°, 18.2±0.2°, 24.0±0.2°, 22.8±0.2°, 21.8±0.2° and 22.3±0.2°;
or, 17.8±0.2°, 19.8±0.2°, 26.1±0.2°, 18.2±0.2°, 24.0±0.2°, 22.8±0.2°, 17.5±0.2° and 24.3±0.2°;
or, 17.8±0.2°, 19.8±0.2°, 26.1±0.2°, 18.2±0.2°, 24.0±0.2°, 22.8±0.2°, 21.8±0.2° and 24.3±0.2°;
or, 17.8±0.2°, 19.8±0.2°, 26.1±0.2°, 18.2±0.2°, 24.0±0.2°, 22.8±0.2°, 10.5±0.2°, 21.5±0.2, 17.5±0.2° and 19.5±0.2°;
or, 17.8±0.2°, 19.8±0.2°, 26.1±0.2°, 18.2±0.2°, 24.0±0.2°, 22.8±0.2°, 10.5±0.2°, 21.5±0.2, 21.8±0.2° and 19.5±0.2°;
or, 17.8±0.2°, 19.8±0.2°, 26.1±0.2°, 18.2±0.2°, 24.0±0.2°, 22.8±0.2°, 10.5±0.2°, 21.5±0.2, 17.5±0.2° and 22.3±0.2°;
or, 17.8±0.2°, 19.8±0.2°, 26.1±0.2°, 18.2±0.2°, 24.0±0.2°, 22.8±0.2°, 10.5±0.2°, 21.5±0.2, 21.8±0.2° and 22.3±0.2°;
or, 17.8±0.2°, 19.8±0.2°, 26.1±0.2°, 18.2±0.2°, 24.0±0.2°, 22.8±0.2°, 10.5±0.2°, 21.5±0.2, 17.5±0.2° and 24.3±0.2°;
or, 17.8±0.2°, 19.8±0.2°, 26.1±0.2°, 18.2±0.2°, 24.0±0.2°, 22.8±0.2°, 10.5±0.2°, 21.5±0.2, 21.8±0.2° and 24.3±0.2°.

The X-ray powder diffraction pattern of crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine comprises one or more diffraction peaks at 2θ of 17.8±0.2°, 19.8±0.2°, 26.1±0.2°, 18.2±0.2°, 24.0±0.2°, 22.8±0.2°, 10.5±0.2°, 21.5±0.2, 17.5±0.2°, 21.8±0.2°, 19.5±0.2°, 22.3±0.2°, 24.3±0.2°, 14.5±0.2°, 30.1±0.2°, 23.4±0.2°, 12.0±0.2°, 24.8±0.2°, 13.2±0.2° and 12.5±0.2°; preferably at least comprises any 2 to 3, or 4 to 5, or 7 to 8, or 10 to 12, or 15 to 18 of the above diffraction peaks; and further preferably comprises any 2, 3, 4, 5, 6, 8, 10, 12, 16, 18 of the above diffraction peaks; for example, the X-ray powder diffraction pattern of crystal form C has diffraction peaks at 2θ of the following positions:

17.8±0.2°, 19.8±0.2°, 26.1±0.2°, 23.4±0.2°, 12.0±0.2° and 18.2±0.2°;
or, 17.8±0.2°, 19.8±0.2°, 26.1±0.2°, 23.4±0.2°, 12.0±0.2° and 24.0±0.2°;
or, 17.8±0.2°, 19.8±0.2°, 26.1±0.2°, 23.4±0.2°, 12.0±0.2° and 22.8±0.2°;
or, 17.8±0.2°, 19.8±0.2°, 26.1±0.2°, 23.4±0.2°, 12.0±0.2° and 10.5±0.2°;
or, 17.8±0.2°, 19.8±0.2°, 26.1±0.2°, 23.4±0.2°, 12.0±0.2° and 21.5±0.2°;
or, 17.8±0.2°, 19.8±0.2°, 26.1±0.2°, 23.4±0.2°, 12.0±0.2° and 17.5±0.2°;
or, 17.8±0.2°, 19.8±0.2°, 26.1±0.2°, 18.2±0.2°, 24.0±0.2°, 23.4±0.2°, 12.0±0.2° and 17.5±0.2°;
or, 17.8±0.2°, 19.8±0.2°, 26.1±0.2°, 24.0±0.2°, 22.8±0.2°, 23.4±0.2°, 12.0±0.2° and 17.5±0.2°;
or, 17.8±0.2°, 19.8±0.2°, 26.1±0.2°, 18.2±0.2°, 24.0±0.2°, 23.4±0.2°, 12.0±0.2° and 21.8±0.2°;
or, 17.8±0.2°, 19.8±0.2°, 26.1±0.2°, 24.0±0.2°, 22.8±0.2°, 23.4±0.2°, 12.0±0.2° and 21.8±0.2°;
or, 17.8±0.2°, 19.8±0.2°, 26.1±0.2°, 18.2±0.2°, 24.0±0.2°, 23.4±0.2°, 12.0±0.2° and 19.5±0.2°;
or, 17.8±0.2°, 19.8±0.2°, 26.1±0.2°, 24.0±0.2°, 22.8±0.2°, 23.4±0.2°, 12.0±0.2° and 19.5±0.2°;
or, 17.8±0.2°, 19.8±0.2°, 26.1±0.2°, 18.2±0.2°, 24.0±0.2°, 22.8±0.2°, 17.5±0.2°, 23.4±0.2°, 12.0±0.2° and 19.5±0.2°;
or, 17.8±0.2°, 19.8±0.2°, 26.1±0.2°, 18.2±0.2°, 24.0±0.2°, 22.8±0.2°, 21.8±0.2°, 23.4±0.2°, 12.0±0.2° and 19.5±0.2°;
or, 17.8±0.2°, 19.8±0.2°, 26.1±0.2°, 18.2±0.2°, 24.0±0.2°, 22.8±0.2°, 17.5±0.2°, 23.4±0.2°, 12.0±0.2° and 22.3±0.2°;
or, 17.8±0.2°, 19.8±0.2°, 26.1±0.2°, 18.2±0.2°, 24.0±0.2°, 22.8±0.2°, 21.8±0.2°, 23.4±0.2°, 12.0±0.2° and 22.3±0.2°;
or, 17.8±0.2°, 19.8±0.2°, 26.1±0.2°, 18.2±0.2°, 24.0±0.2°, 22.8±0.2°, 17.5±0.2°, 23.4±0.2°, 12.0±0.2° and 24.3±0.2°;

or, 17.8±0.2°, 19.8±0.2°, 26.1±0.2°, 18.2±0.2°, 24.0±0.2°, 22.8±0.2°, 21.8±0.2°, 23.4±0.2°, 12.0±0.2° and 24.3±0.2°.

Most preferably, using Cu-Kα radiation, the characteristic X-ray diffraction peaks represented by 2θ angle and interplanar spacing d value are shown in Table 8.

Table 8

| No. | XRPD diffraction data of crystal form C | | | |
|---|---|---|---|---|
| | 2θ (±0.2°) | d value | Peak height | Proportion (1%) |
| 1 | 10.544 | 8.3832 | 296 | 36.4 |
| 2 | 12.046 | 7.3412 | 138 | 17 |
| 3 | 12.476 | 7.0891 | 120 | 14.7 |
| 4 | 13.182 | 6.7106 | 129 | 15.8 |
| 5 | 14.461 | 6.1201 | 173 | 21.3 |
| 6 | 17.522 | 5.0572 | 242 | 29.7 |
| 7 | 17.807 | 4.9769 | 814 | 100 |
| 8 | 18.249 | 4.8573 | 541 | 66.5 |
| 9 | 19.451 | 4.5598 | 210 | 25.8 |
| 10 | 19.835 | 4.4725 | 607 | 74.6 |
| 11 | 21.473 | 4.1349 | 287 | 35.3 |
| 12 | 21.762 | 4.0806 | 233 | 28.6 |
| 13 | 22.306 | 3.9822 | 201 | 24.7 |
| 14 | 22.813 | 3.8949 | 341 | 41.9 |
| 15 | 23.4 | 3.7984 | 156 | 19.2 |
| 16 | 24.049 | 3.6974 | 371 | 45.6 |
| 17 | 24.332 | 3.655 | 198 | 24.3 |
| 18 | 24.798 | 3.5873 | 132 | 16.2 |
| 19 | 26.117 | 3.4091 | 567 | 69.7 |
| 20 | 30.11 | 2.9655 | 160 | 19.7 |

The crystal form of the present invention is crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine, the X-ray powder diffraction pattern thereof is substantially as shown in Figure 17; and the DSC spectrum thereof is as shown in Figure 18.

In a preferred embodiment of the present invention, crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine is a solvent-containing crystal form or a solvent-free crystal form, wherein the solvent is one or more selected from the group consisting of water, methanol, acetone, ethyl acetate, acetonitrile, ethanol, 88% acetone, tetrahydrofuran, 2-methyl-tetrahydrofuran, dichloromethane, 1,4-dioxane, benzene, toluene, isopropanol, *n*-butanol, isobutanol, N,N-dimethylformamide, N,N-dimethylacetamide, N-methylpyrrolidone, dimethyl sulfoxide, *n*-propanol, *tert*-butanol, 2-butanone, 3-pentanone, *n*-heptane, heptane, ethyl formate, isopropyl acetate, cyclohexane, methyl tert-butyl ether and isopropyl ether.

In a preferred embodiment of the present invention, the number of solvent molecule contained in crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine is 0.2 to 3, preferably 0.2, 0.5, 1, 1.5, 2, 2.5 or 3, and more preferably 0.5, 1, 2 or 3.

In a preferred embodiment of the present invention, crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine is a solvent-free crystal form, and preferably an anhydrous crystal form.

In a preferred embodiment of the present invention, crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine is a hydrate crystal form, and the number of water molecule is 0.2 to 3, preferably 0.2, 0.5, 1, 1.5, 2, 2.5 or 3, and more preferably 0.5, 1, 2 or 3.

In a further preferred embodiment of the present invention, in the X-ray powder diffraction patterns of the crystal forms, the 2θ error between the diffraction peak position having top-ten relative peak intensity and the reference diffraction peak position is ±0.2° to ±0.5°, preferably ±0.2° to ±0.3°, and most preferably ±0.2°.

In a further preferred embodiment of the present invention, in the X-ray powder diffraction patterns of crystal form A, crystal form B and crystal form C of the compound (S)-8-(6-amino-5-((2-amino-3-chloropyridin-4-yl)thio)pyrazin-2-yl)-2-cyclopropyl-8-azaspiro[4.5]dec-2-en-1-amine, the 2θ error between the diffraction peak having top-ten relative peak intensity and the reference diffraction peak position in Figure 1, Figure 4 and Figure 6 respectively is ±0.2° to ±0.5°, preferably ±0.2° to ±0.3°, and most preferably ±0.2°.

The present invention also provides a method for preparing the crystal form of the compound (S)-8-(6-amino-5-((2-amino-3-chloropyridin-4-yl)thio)pyrazin-2-yl)-2-cyclopropyl-8-azaspiro[4.5]dec-2-en-1-amine, specifically comprising the following steps of:

1) weighing an appropriate amount of free base of the compound (S)-8-(6-amino-5-((2-amino-3-chloropyridin-4-yl)thio)pyrazin-2-yl)-2-cyclopropyl-8-azaspiro[4.5]dec-2-en-1-amine, and suspending it in a poor solvent, the suspension density is preferably 50 to 200 mg/mL;
2) shaking the suspension obtained in step 1) at a certain temperature for a certain period of time, the temperature is preferably 0 to 60°C, and the time is preferably 1 to 10 days;
3) rapidly centrifuging the suspension obtained in step 2), removing the supernatant, and drying the remaining solid in a vacuum drying oven at 40°C to constant weight to obtain the target product;

wherein:

the poor solvent is one or more selected from the group consisting of acetone, ethyl acetate, isopropyl acetate, acetonitrile, ethanol, 88% acetone, tetrahydrofuran, 2-methyltetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone, 3-pentanone, methyl *tert*-butyl ether and water, and preferably one or more selected from the group consisting of acetone, ethyl acetate, isopropyl acetate, acetonitrile, ethanol, 88% acetone, tetrahydrofuran, 2-methyltetrahydrofuran, dichloromethane, 1,4-dioxane, toluene, isopropanol, 2-butanone, 3-pentanone, methyl tert-butyl ether and water;
or, specifically comprising the following steps of:

1) weighing an appropriate amount of free base of the compound (S)-8-(6-amino-5-((2-amino-3-chloropyridin-4-yl)thio)pyrazin-2-yl)-2-cyclopropyl-8-azaspiro[4.5]dec-2-en-1-amine, and dissolving itin a good solvent;
2) adding an anti-solvent to the solution obtained in step 1) at a certain temperature, and stirring the solution until a solid is precipitated, the temperature is preferably 0 to 25°C;
3) rapidly centrifuging the suspension obtained in step 2), removing the supernatant, and drying the remaining solid in a vacuum drying oven at 40°C to constant weight to obtain the target product;

wherein:

the good solvent is one or more selected from the group consisting of methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone, 3-pentanone and N-methylpyrrolidone, and preferably N-methylpyrrolidone;
the anti-solvent is selected from the group consisting of heptane, water, methyl *tert*-butyl ether, toluene and isopropyl ether;
or, specifically comprising the following steps of:

1) weighing an appropriate amount of free base of the compound (S)-8-(6-amino-5-((2-amino-3-chloropyridin-4-yl)thio)pyrazin-2-yl)-2-cyclopropyl-8-azaspiro[4.5]dec-2-en-1-amine, and dissolving it in a good solvent under heating;
2) rapidly placing the solution obtained in step 1) at a low temperature, and stirring it until a solid is precipitated, the temperature is preferably -10 to 5°C;
3) rapidly centrifuging the suspension obtained in step 2), removing the supernatant, and drying the remaining solid in a vacuum drying oven at 40°C to constant weight to obtain the target product;

wherein:

the good solvent is one or more selected from the group consisting of methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone, 3-pentanone and N-methylpyrrolidone, and preferably 2-methyltetrahydrofuran.

The objective of the present invention is also to provide a pharmaceutical composition comprising a therapeutically effective amount of the crystal form of the compound of formula (I) or the compound (S)-1'-(6-amino-5-((2-amino-3-chloropyridin-4-yl)thio) -3-fluoropyrazin-2-yl)-1,3-dihydrospiro[indene-2,4'-piperidin]-1-amine, and one or more pharmaceutically acceptable carrier(s), diluent(s) or excipient(s).
The objective of the present invention is also to provide a pharmaceutical composition comprising a therapeutically effective amount of the crystal form of the compound of formula (I) or the compound (S)-8-(6-amino-5-((2-amino-3-chloropyridin-4-yl)thio) pyrazin-2-yl)-2-cyclopropyl-8-azaspiro[4.5]dec-2-en-1-amine, and one or more pharmaceutically acceptable carrier(s), diluent(s) or excipient(s).
The objective of the present invention is also to provide a use of the crystal form of the compound of formula (I) or the compound (S)-1'-(6-amino-5-((2-amino-3-chloropyridin-4-yl)thio)-3-fluoropyrazin-2-yl)-1,3-dihydrospiro[indene-2,4'-piperidin]-1-amine or the pharmaceutical composition comprising the same in the preparation of a SHP-2 inhibitor medicament.
The objective of the present invention is also to provide a use of the crystal form of the compound of formula (I) or the compound (S)-8-(6-amino-5-((2-amino-3-chloropyridin-4-yl)thio)pyrazin-2-yl)-2-cyclopropyl-8-azaspiro[4.5]dec-2-en-1-amine or the pharmaceutical composition comprising the same in the preparation of a SHP-2 inhibitor medicament.
In the above use, the use is a use in the preparation of a medicament for treating a disease or condition such as Noonan syndrome, leopard skin syndrome, leukemia, neuroblastoma, melanoma, esophageal cancer, head and neck tumor, breast cancer, lung cancer and colon cancer; and preferably non-small cell lung cancer, esophageal cancer or head and neck tumor.

## DESCRIPTION OF THE DRAWINGS

Figure 1 is the DSC spectrum of crystal form A of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 2 is the TGA spectrum of crystal form A of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 3 is the XRPD pattern of crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 4 is the DSC spectrum of crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 5 is the TGA spectrum of crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 6 is the DSC spectrum of crystal form C of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 7 is the TGA spectrum of crystal form C of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 8 is the DSC spectrum of crystal form D of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 9 is the TGA spectrum of crystal form D of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 10 is the DSC spectrum of crystal form E of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 11 is the TGA spectrum of crystal form E of (S)-1'-(6-amino-5-((2-amino-3-chloropyridin-4-yl)thio)-3-fluoropyrazin-2-yl)-1,3-dihydrospiro[indene-2,4'-piperidin]-1-amine.
Figure 12 is the XRPD pattern of crystal form A of (S)-8-(6-amino-5-((2-amino-3-chloropyridin-4-yl)thio)pyrazin-2-yl)-2-cyclopropyl-8-azaspiro[4.5]dec-2-en-1-amine.
Figure 13 is the DSC spectrum of crystal form A of (S)-8-(6-amino-5-((2-amino-3-chloropyridin-4-yl)thio)pyrazin-2-yl)-2-cyclopropyl-8-azaspiro[4.5]dec-2-en-1-amine.

Figure 14 is the TGA spectrum of crystal form A of (S)-8-(6-amino-5-((2-amino-3-chloropyridin-4-yl)thio)pyrazin-2-yl)-2-cyclopropyl-8-azaspiro[4.5]dec-2-en-1-amine.

Figure 15 is the XRPD pattern of crystal form B of (S)-8-(6-amino-5-((2-amino-3-chloropyridin-4-yl)thio)pyrazin-2-yl)-2-cyclopropyl-8-azaspiro[4.5]dec-2-en-1-amine.

Figure 16 is the DSC spectrum of crystal form B of (S)-8-(6-amino-5-((2-amino-3-chloropyridin-4-yl)thio)pyrazin-2-yl)-2-cyclopropyl-8-azaspiro[4.5]dec-2-en-1-amine.

Figure 17 is the XRPD pattern of crystal form C of (S)-8-(6-amino-5-((2-amino-3-chloropyridin-4-yl)thio)pyrazin-2-yl)-2-cyclopropyl-8-azaspiro[4.5]dec-2-en-1-amine.

Figure 18 is the DSC spectrum of crystal form C of (S)-8-(6-amino-5-((2-amino-3-chloropyridin-4-yl)thio)pyrazin-2-yl)-2-cyclopropyl-8-azaspiro[4.5]dec-2-en-1-amine.

## DETAILED DESCRIPTION OF THE INVENTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl, hydroxy-substituted alkyl and cyano-substituted alkyl.

The term "cycloalkyl" refers to a saturated or partially unsaturated monocyclic or polycyclic hydrocarbon substituent group having 3 to 20 carbon atoms, preferably 3 to 8 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably cyclopropyl, cyclobutyl, cyclohexyl, cyclopentyl or cycloheptyl. The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The term "heterocyclyl" refers to a 3 to 20 membered saturated or partially unsaturated monocyclic or polycyclic hydrocarbon group, wherein one or more ring atoms are heteroatoms selected from the group consisting of nitrogen, oxygen and $S(O)_m$ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; and most preferably 3 to 8 ring atoms. Non-limiting examples of monocyclic heterocyclyl include oxetanyl, pyrrolidinyl, pyrrolidonyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, preferably oxetanyl, pyrrolidonyl, tetrahydrofuranyl, pyrazolidinyl, morpholinyl, piperazinyl and pyranyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring. The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring

in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of oxygen, sulfur and nitrogen. The heteroaryl is preferably a 5 to 10 membered heteroaryl, and more preferably a 5 or 6 membered heteroaryl, for example imidazolyl, furyl, thienyl, thiazolyl, pyrazolyl, oxazolyl, pyrrolyl, triazolyl, tetrazolyl, pyridyl, pyrimidinyl, thiadiazolyl, oxadiazolyl, pyrazinyl and the like, preferably oxazolyl, oxadiazolyl, tetrazolyl, triazolyl, thienyl, imidazolyl, pyridyl, pyrazolyl, pyrimidinyl and thiazolyl, and more preferably oxazolyl, oxadiazolyl, tetrazolyl, triazolyl, thienyl, pyridyl, thiazolyl and pyrimidinyl. The heteroaryl ring can be fused to the ring of aryl, heterocyclyl or cycloalkyl, wherein the ring bound to the parent structure is heteroaryl ring. The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. The alkoxy is preferably an alkoxy having 1 to 8 carbon atoms, more preferably an alkoxy having 1 to 6 carbon atoms, and most preferably an alkoxy having 1 to 3 carbon atoms. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Haloalkyl" refers to an alkyl group substituted by one or more halogen(s), wherein the alkyl is as defined above.

"Haloalkoxy" refers to an alkoxy group substituted by one or more halogen(s), wherein the alkoxy is as defined above.

"Hydroxyalkyl" refers to an alkyl group substituted by hydroxy(s), wherein the alkyl is as defined above.

"Alkenyl" refers to a chain alkenyl, also known as alkene group. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Hydroxy" refers to an -OH group.

"Halogen" refers to fluorine, chlorine, bromine or iodine.

"Amino" refers to a $-NH_2$ group.

"Cyano" refers to a -CN group.

"Nitro" refers to a $-NO_2$ group.

"THF" refers to tetrahydrofuran.

"EtOAc" refers to ethyl acetate.

"DMSO" refers to dimethyl sulfoxide.

"LDA" refers to lithium diisopropylamide.

"DMAP" refers to 4-dimethylaminopyridine.

"EtMgBr" refers to ethylmagnesium bromide.

"HOSu" refers to N-hydroxysuccinimide.

"EDCI" refers to 1-(3-dimethylaminopropyl)-3-ethylcarbodiimide hydrochloride.

"IPA" refers to isopropanol.

"MeOH" refers to methanol.

"EtOH" refers to ethanol.

"DMF" refers to N,N-dimethylformamide.

"DIPEA" refers to N,N-diisopropylethylamine.

"HEPES" refers to 4-hydroxyethylpiperazineethanesulfonic acid.

Different expressions such as "X is selected from the group consisting of A, B or C", "X is selected from the group consisting of A, B and C", "X is A, B or C", "X is A, B and C" and the like, express the same meaning, that is, X can be any one or more of A, B and C. "Optional" or "optionally" means that the event or circumstance described subsequently can, but need not, occur, and such a description includes the situation in which the event or circumstance does or does not occur.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

"Stereoisomerism" includes geometric isomerism (cis-trans isomerism), optical isomerism, and conformational isomerism.

The hydrogen atom of the present invention can be substituted by its isotope deuterium. Any of the hydrogen atoms in the compounds of the examples of the present invention can also be substituted by deuterium atom(s).

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

X-ray powder diffraction (XRPD) pattern refers to the experimentally observed diffraction pattern or the parameters derived from it, and the X-ray powder diffraction pattern is characterized by peak position (abscissa) and peak intensity (ordinate). Those skilled in the art will appreciate that the experimental error therein depends on the conditions of the instrument, the preparation of the sample, and the purity of the sample. In particular, it is well known to those skilled in the art that X-ray diffraction pattern generally varies with the conditions of the instrument. Those skilled in the art will appreciate that suitable error tolerances for XRPD may be: $2\theta\pm0.5°$, $2\theta\pm0.4°$, $2\theta\pm0.3°$, $2\theta\pm0.2°$. In particular, it is important to point out that the relative intensity in X-ray diffraction pattern may vary with experimental conditions, so the order of peak intensity cannot be used as the sole or decisive factor. In addition, due to the influence of experimental factors such as the height of the sample, the overall deviation of the peak angle will occur, and a certain deviation is usually allowed. Therefore, those skilled in the art can understand that any crystal form having the same or similar characteristic peaks as the pattern of the present invention falls within the scope of the present invention.

"TGA" refers to thermogravimetric analysis (TGA) test.

"DSC" refers to differential scanning calorimetry (DSC) test.

"HPLC" refers to high performance liquid chromatography (HPLC) test.

"PK" refers to pharmacokinetic (PK) test.

The present invention will be further described with reference to the following examples, but the examples should not be considered as limiting the scope of the present invention.

## I. Preparation of the Compounds

The structures of the compounds of the present invention were identified by nuclear magnetic resonance (NMR) and/or liquid chromatography-mass spectrometry (LC-MS). NMR shifts ($\delta$) are given in parts per million (ppm). NMR was determined by a Bruker AVANCE-400 machine. The solvents for determination were deuterated-dimethyl sulfoxide (DMSO-$d_6$), deuterated-methanol ($CD_3OD$) and deuterated-chloroform ($CDCl_3$), and the internal standard was tetramethylsilane (TMS).

Liquid chromatography-mass spectrometry (LC-MS) was determined on an Agilent 1200 Infinity Series mass spectrometer. High performance liquid chromatography (HPLC) was determined on an Agilent 1200DAD high pressure liquid chromatograph (Sunfire C18 150×4.6 mm chromatographic column), and a Waters 2695-2996 high pressure liquid chromatograph (Gimini C18 150×4.6 mm chromatographic column). Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm. Yantai Huanghai 200 to 300 mesh silica gel was generally used as a carrier for column chromatography.

The raw materials used in the examples of the present invention are known and commercially available, or can be synthesized by adopting or according to known methods in the art.

Unless otherwise stated, all reactions of the present invention were carried out under continuous magnetic stirring under a dry nitrogen or argon atmosphere, the solvent was dry, and the reaction temperature was in degrees celsius.

**Example 1**

Preparation of (S)-1'-(6-amino-5-((2-amino-3-cyclopropylpyridin-4-yl)thio)pyrazin-2-yl)-1,3-dihydro-spiro[indene-2,4'-piperidin]-1-amine

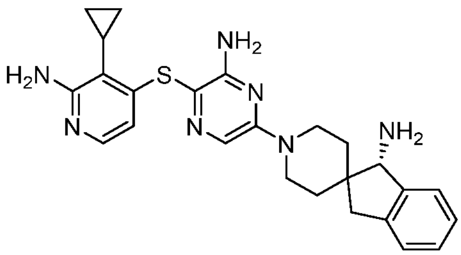

Step 1: Preparation of 2-ethylhexyl 3-((2-amino-3-chloropyridin-4-yl)thio)propanoate

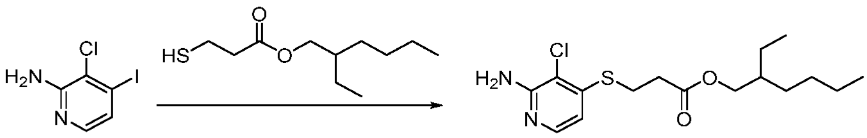

In a microwave reaction tube, 3-chloro-4-iodopyridin-2-amine (800 mg, 3.15 mmol) and 2-ethylhexyl 3-mercaptopropanoate (687 mg, 3.15 mmol) were dissolved in 1,4-dioxane (7 mL), followed by the addition of DIPEA (813 mg, 6.3 mmol), palladium acetate (35 mg, 0.16 mmol) and Xantphos (109 mg, 0.19 mmol). The reaction solution was bubbled with nitrogen for 3 minutes, heated under microwave to 100°C and reacted for 1 hour. The reaction solution was cooled, diluted with 20 mL of ethyl acetate, and filtered through diatomaceous earth to remove insoluble substance. The filtrate was concentrated to dryness by rotary evaporation, and the resulting residue was purified by column chromatography (15 to 25% ethyl acetate/petroleum ether) to obtain the product (1.03 g, yield: 95%) as a brown solid.

MS m/z (ESI): 345.1 $[M+H]^+$.

Step 2: Preparation of 2-ethylhexyl 3-((2-amino-3-cyclopropylpyridin-4-yl)thio)propanoate

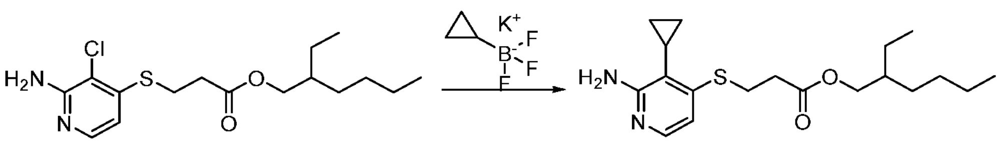

In a sealed tube, 2-ethylhexyl 3-((2-amino-3-chloropyridin-4-yl)thio)propanoate (500 mg, 1.45 mmol) was dissolved in a mixed solvent of toluene and water (toluene/water=10 mL/1 mL), followed by the addition of potassium cyclopropyl trifluoroborate (280 mg, 1.89 mmol), n-butylbis(1-adamantyl)phosphine (52 mg, 0.145 mmol), palladium acetate (16 mg, 0.073 mmol) and cesium carbonate (1.41 g, 4.35 mmol). The reaction solution was bubbled with nitrogen for 3 minutes, heated to 100°C and reacted for 5 hours. The reaction solution was cooled to room temperature, and then 20 mL of saturated $NH_4Cl$ solution was added. The reaction solution was extracted with ethyl acetate (20 mL×3). The ethyl acetate layer was washed with saturated sodium chloride solution, dried over anhydrous sodium sulfate, and purified by column chromatography (50% ethyl acetate/petroleum ether) to obtain the product (220 mg, yield: 43%) as a brown oil.

MS m/z (ESI): 350.1 $[M+H]^+$.

Step 3: Preparation of potassium 2-amino-3-cyclopropylpyridine-4-thiolate

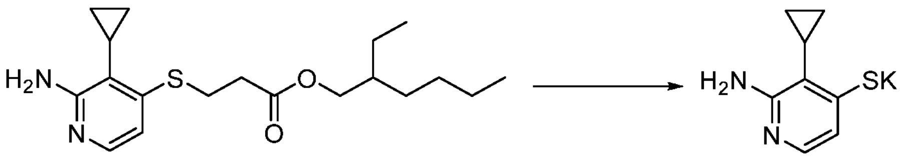

2-Ethylhexyl 3-((2-amino-3-cyclopropylpyridin-4-yl)thio)propanoate (220 mg, 0.63 mmol) was dissolved in 10 mL of ethanol. Potassium tert-butoxide (74 mg, 0.66 mmol) was added, and the reaction solution was stirred at room temperature for 1 hour. The reaction solution was concentrated to dryness, and the resulting residue was used directly in the next step.

MS m/z (ESI): 165.1 $[M-H]^-$.

Step 4: Preparation of 3-((2-amino-3-cyclopropylpyridin-4-yl)thio)-6-chloropyrazin-2-amine

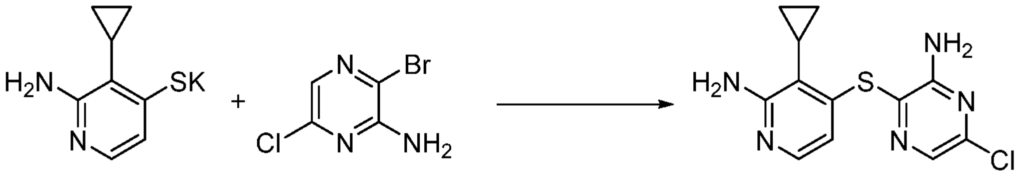

In a microwave reaction tube, potassium 2-amino-3-cyclopropylpyridine-4-thiolate (128 mg, 0.63 mmol) and 2-amino-3-bromo-6-chloropyrazine (149 mg, 0.72 mmol) were dissolved in 7 mL of 1,4-dioxane, followed by the addition of tris(dibenzylideneacetone)dipalladium (33 mg, 0.036 mmol), Xantphos (42 mg, 0.072 mmol) and DIPEA (279 mg, 2.16 mmol). The reaction solution was bubbled with nitrogen for 3 minutes, heated under microwave to 110°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (50 to 70% ethyl acetate/petroleum ether) to obtain the product (50 mg, yield: 24%) as a brown solid.

MS m/z (ESI): 294.1 $[M+H]^+$.

Step 5: Preparation of (R)-N-((S)-1'-(6-amino-5-((2-amino-3-cyclopropylpyridin-4-yl)thio)pyrazin-2-yl)-1,3-dihydrospiro[indene-2,4'-piperidin]-1-yl)-2-methylpropane-2-sulfinamide

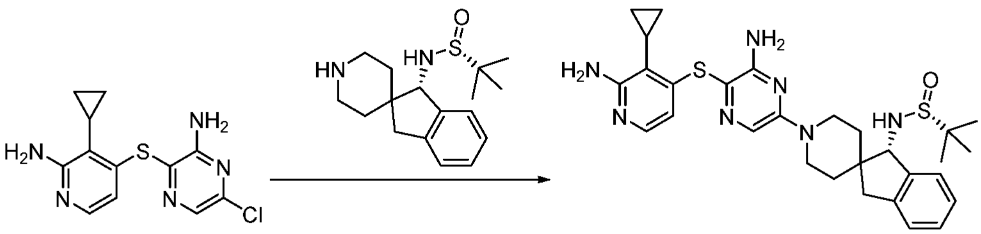

(S)-1-(((R)-tert-butylsulfinyl)amino)-1,3-dihydrospiro[indene-2,4'-piperidine]-1'-carboxylate (69 mg, 0.17 mmol) was dissolved in 3 mL of dichloromethane. 1 mL of trifluoroacetic acid was added, and the reaction solution was reacted at room temperature for 1 hour. The reaction solution was concentrated to dryness, and dissolved in 5 mL of DMF. Potassium carbonate (352 mg, 2.55 mmol) and 3-((2-amino-3-cyclopropylpyridin-4-yl)thio)-6-chloropyrazin-2-amine (50 mg, 0.17 mmol) were added, and the reaction solution was heated to 100°C under a nitrogen atmosphere and reacted for 12 hours. The reaction solution was cooled, and then 20 mL of water was added. The reaction solution was extracted with ethyl acetate (20 mL×3). The ethyl acetate layer was washed with saturated sodium chloride solution (20 mL×3),

dried over anhydrous sodium sulfate, and purified by column chromatography (75 to 80% ethyl acetate/petroleum ether) to obtain the product (20 mg, yield: 21%) as an oil.

MS m/z (ESI): 564.1 $[M+H]^+$.

Step 6: Preparation of (S)-1'-(6-amino-5-((2-amino-3-cyclopropylpyridin-4-yl)-thio)pyrazin-2-yl)-1,3-dihydrospiro[indene-2,4'-piperidin]-1-amine

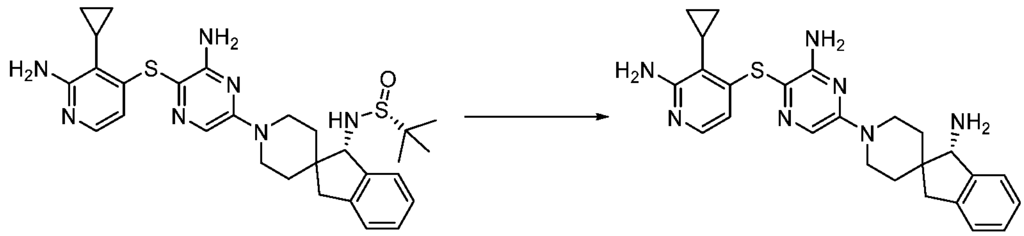

(R)-N-((S)-1'-(6-Amino-5-((2-amino-3-cyclopropylpyridin-4-yl)thio)pyrazin-2-yl)-1,3-dihydrospiro[indene-2,4'-piperidin]-1-yl)-2-methylpropane-2-sulfinamide (20 mg, 0.035 mmol) was dissolved in 5 mL of dichloromethane. 1 mL of 4M HCl in dioxane was added, and the reaction solution was reacted at room temperature for 1 hour. The reaction solution was concentrated to dryness and dissolved in 5 mL of methanol, and the pH was adjusted to alkaline with 7M $NH_3$ in methanol. The reaction solution was concentrated to dryness by rotary evaporation, and the resulting residue was purified by column chromatography (0 to 10% MeOH in DCM). The resulting crude product was purified by thin layer chromatography (dichloromethane:methanol=10:1) to obtain the product (9.0 mg, yield: 56%) as a brown solid.

$^1H$ NMR (400 MHz, Methanol-$d_4$) $\delta$ 7.90 (d, $J$ = 5.4 Hz, 1H), 7.61 (s, 1H), 7.40 - 7.33 (m, 1H), 7.27 - 7.16 (m, 3H), 6.57 (d, $J$ = 5.4 Hz, 1H), 4.28 (d, $J$ = 13.6 Hz, 2H), 3.96 (s, 1H), 3.30 - 3.19 (m, 2H), 3.15 (d, $J$ = 15.7 Hz, 1H), 2.81 (d, $J$ = 15.7 Hz, 1H), 1.90 - 1.78 (m, 2H), 1.82 - 1.70 (m, 1H), 1.58 (d, $J$ = 13.5 Hz, 1H), 1.43 (d, $J$ = 13.1 Hz, 1H), 1.26 - 1.15 (m, 2H), 0.94 - 0.85 (m, 2H).

MS m/z (ESI): 460.1 $[M+H]^+$.

**Example 2**

Preparation of

(S)-1'-(6-amino-5-((2-chloro-3-cyclopropylpyridin-4-yl)thio)pyrazin-2-yl)-1,3-dihydro-spiro[indene-2,4'-piperidin]-1-amine

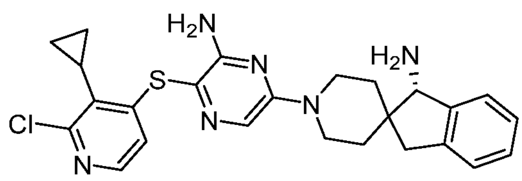

The compound of Example 2 was prepared by referring to the experimental scheme of Example 1.

$^1H$ NMR (400 MHz, Methanol-$d_4$) $\delta$ 7.88 (d, $J$ = 5.5 Hz, 1H), 7.60 (s, 1H), 7.38 - 7.30 (m, 1H), 7.25 - 7.15 (m, 3H), 6.54 (d, $J$ = 5.5 Hz, 1H), 4.26 (d, $J$ = 13.7 Hz, 2H), 3.95 (s, 1H), 3.28 - 3.17 (m, 2H), 3.13 (d, $J$ = 15.6 Hz, 1H), 2.79(d, $J$ = 15.6 Hz, 1H), 1.88 - 1.74 (m, 2H), 1.80 - 1.70 (m, 1H), 1.56 (d, $J$ = 13.7 Hz, 1H), 1.43 (d, $J$ = 13.1 Hz, 1H), 1.24 - 1.13 (m, 2H), 0.93 - 0.83 (m, 2H).

MS m/z (ESI): 479.1 $[M+H]^+$, 481.1 $[M+2+H]^+$.

**Example 3**

Preparation of (S)-1'-(6-amino-5-((3-chloro-2-cyclopropylpyridin-4-yl)thio)-pyrazin-2-yl)-1,3-dihydrospiro[indene-2,4'-piperidin]-1-amine

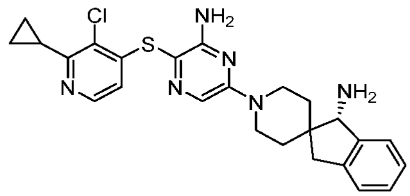

Step 1: Preparation of 2-ethylhexyl 3-((2,3-dichloropyridin-4-yl)thio)propanoate

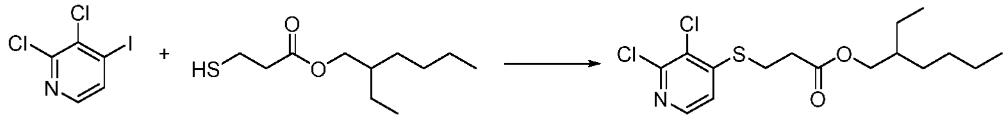

In a microwave reaction tube, 2,3-dichloro-4-iodopyridine (1.0 g, 3.65 mmol) and 2-ethylhexyl 3-mercaptopropanoate (0.88 g, 4.03 mmol) were dissolved in 1,4-dioxane (7 mL), followed by the addition of DIPEA (0.95 g, 7.34 mmol), palladium acetate (41 mg, 0.18 mmol) and Xantphos (127 mg, 0.22 mmol). The reaction solution was bubbled with nitrogen for 3 minutes, heated under microwave to 100°C and reacted for 1 hour. The reaction solution was cooled, diluted with 20 mL of ethyl acetate, and filtered through diatomaceous earth to remove insoluble substance. The filtrate was concentrated to dryness by rotary evaporation, and the resulting residue was purified by column chromatography (10 to 15% ethyl acetate/petroleum ether) to obtain a brown oil (1.29 g, yield: 97%).

MS m/z (ESI): 364.1 $[M+H]^+$.

Step 2: Preparation of 2-ethylhexyl 3-((3-chloro-2-cyclopropylpyridin-4-yl)thio)propanoate

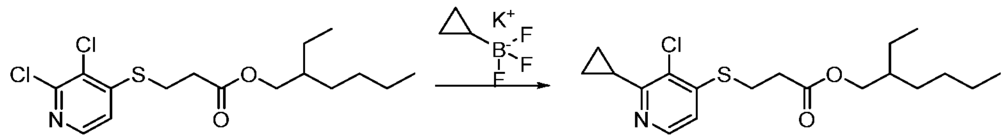

In a sealed tube, 2-ethylhexyl 3-((2,3-dichloropyridin-4-yl)thio)propanoate (1.29 g, 3.54 mmol) was dissolved in a mixed solvent of toluene and water (toluene/water=20 mL/2 mL), followed by the addition of potassium cyclopropyl trifluoroborate (624 mg, 4.25 mmol), *n*-butylbis(1-adamantyl)phosphine (95 mg, 0.267 mmol), palladium acetate (40 mg, 0.178 mmol) and cesium carbonate (3.43 g, 3.54 mmol). The reaction solution was bubbled with nitrogen for 3 minutes, heated to 100°C and reacted for 5 hours. The reaction solution was cooled to room temperature, and then 20 mL of saturated $NH_4Cl$ solution was added. The reaction solution was extracted with ethyl acetate (20 mL×3). The ethyl acetate layer was washed with saturated sodium chloride solution, dried over anhydrous sodium sulfate, and purified by column chromatography (5 to 8% ethyl acetate/petroleum ether) to obtain a yellow oil (600 mg, yield: 45%).

MS m/z (ESI): 370.1 $[M+H]^+$.

Step 3: Preparation of potassium 3-chloro-2-cyclopropylpyridine-4-thiolate

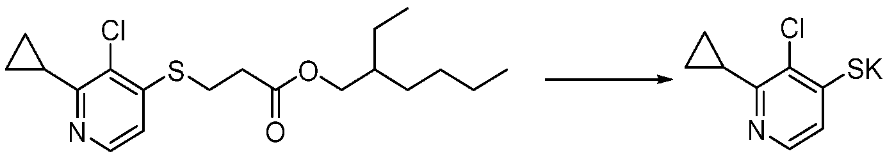

2-Ethylhexyl 3-((3-chloro-2-cyclopropylpyridin-4-yl)thio)propanoate (600 mg, 1.62 mmol) was dissolved in 15 mL of ethanol. Potassium *tert*-butoxide (190 mg, 1.70 mmol) was added, and the reaction solution was stirred at room temperature for 1 hour. The reaction solution was concentrated to dryness, and the resulting residue was used directly in the next step.

MS m/z (ESI): 186.1 $[M+H]^+$.

Step 4: Preparation of 6-chloro-3-((3-chloro-2-cyclopropylpyridin-4-yl)thio)-pyrazin-2-amine

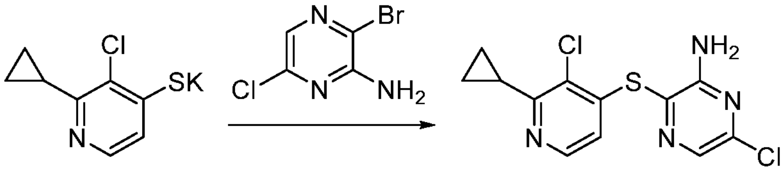

In a microwave reaction tube, potassium 3-chloro-2-cyclopropylpyridine-4-thiolate (120 mg, 0.54 mmol) and 2-amino-3-bromo-6-chloropyrazine (112 mg, 0.54 mmol) were dissolved in 5 mL of 1,4-dioxane, followed by the addition of tris(dibenzylideneacetone)dipalladium (25 mg, 0.027 mmol), Xantphos (31 mg, 0.054 mmol) and DIPEA (209 mg, 1.62 mmol). The reaction solution was bubbled with nitrogen for 3 minutes, heated under microwave to 110°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (10 to 20% ethyl acetate/petroleum ether) to obtain the product (135 mg, yield: 80%) as an off-white solid.

MS m/z (ESI): 313.1 $[M+H]^+$.

Step 5: Preparation of *tert*-butyl 1-oxo-1,3-dihydrospiro[indene-2,4'-piperidine]-1'-carboxylate

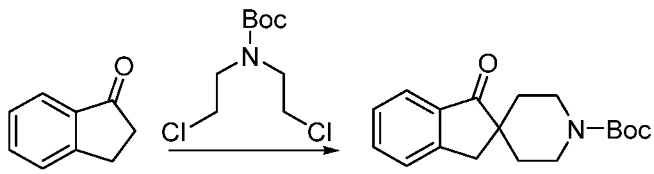

1-Indenone (5.14 g, 38.9 mmol) and tert-butyl N,N-bis(2-chloroethyl)carbamate (9.42 g, 38.9 mmol) were dissolved in 100 mL of DMF. 60% Sodium hydride (3.89 g, 97.3 mmol) was added in batches in an ice bath. The reaction solution was heated to 60°C in an oil bath under a nitrogen atmosphere and reacted overnight. The reaction solution was cooled, and then 250 mL of saturated sodium chloride solution was added. The reaction solution was extracted with ethyl acetate (150 mL×2). The ethyl acetate layer was washed with saturated sodium chloride solution (100 dried over anhydrous $MgSO_4$, and purified by column chromatography (10 to 20% ethyl acetate/petroleum ether) to obtain the crude product (2.80 g, yield: 23%) as a brown oil.

MS m/z (ESI): 202.1 $[M-Boc+H]^+$.

Step 6: Preparation of *tert*-butyl (R,E)-1-((*tert*-butylsulfinyl)imino)-1,3-dihydrospiro[indene-2,4'-piperidine]-1'-carboxylate

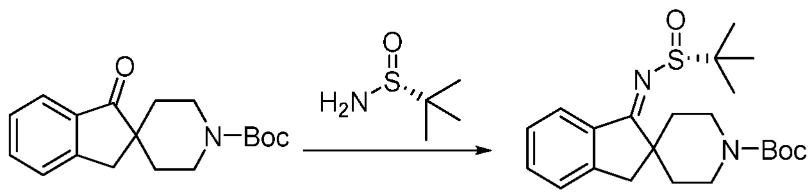

Tetraethyl titanate (40 mL) was heated to 90°C. *Tert*-butyl 1-oxo-1,3-dihydrospiro[indene-2,4'-piperidine]-1'-carboxylate (2.80 g, 9.27 mmol) and (R)-(+)-*tert*-butylsulfinamide (3.36 g, 27.8 mmol) were added, and the reaction solution was reacted for 24 hours at 90°C under a nitrogen atmosphere. After completion of the reaction, the reaction solution was poured into 400 mL of ethyl acetate. 400 mL of saturated sodium chloride solution was slowly added under stirring, and the reaction solution was stirred at room temperature for 20 minutes. The reaction solution was filtered through diatomaceous earth to remove the precipitated solid. After the resulting filtrate was separated into two layers, the ethyl acetate layer was dried over anhydrous magnesium sulfate, and purified by column chromatography (20 to 30% ethyl acetate/petroleum ether) to obtain the product (2.20 g, yield: 59%) as a brown oil.

MS m/z (ESI): 405.1 $[M+H]^+$.

Step 7: Preparation of *tert*-butyl (S)-1-(((R)-*tert*-butylsulfinyl)amino)-1,3-dihydrospiro[indene-2,4'-piperidine]-1'-carboxylate

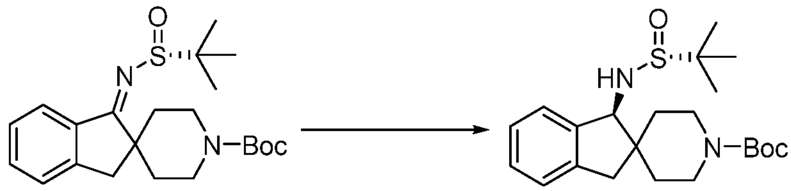

*Tert*-butyl (R,E)-1-((*tert*-butyl sulfinyl)imino)-1,3-dihydrospiro-[indene-2,4'-piperidine]-1'-carboxylate (2.20 g, 5.44 mmol) was dissolved in 30 mL of tetrahydrofuran. The solution was cooled to -78°C, and sodium borohydride (308 mg, 8.16 mmol) was added in batches at this temperature. The reaction solution was gradually warmed up to room temperature under a nitrogen atmosphere and stirred overnight. 200 mL of saturated sodium chloride solution was added, and the reaction solution was extracted with ethyl acetate (100 mL×2). The ethyl acetate layer was washed with saturated sodium chloride solution (100 mL), dried over anhydrous sodium sulfate, and purified by column chromatography (20 to 35% ethyl acetate/petroleum ether) to obtain a brown foamy solid (1.21 g, yield: 54%).

MS m/z (ESI): 407.1 $[M+H]^+$.

Step 8: Preparation of (R)-N-((S)-1'-(6-amino-5-((3-chloro-2-cyclopropylpyridin-4-yl)thio)-pyrazin-2-yl)-1,3-dihydrospiro[indene-2,4'-piperidin]-1-yl)-2-methylpropane-2-sulfinamide

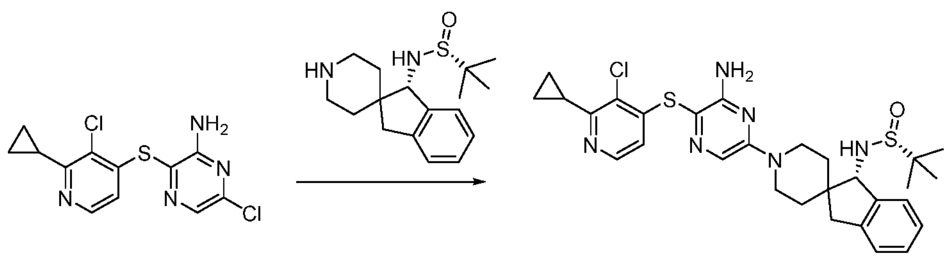

*Tert*-butyl (S)-1-(((R)-tert-butyl sulfinyl)amino)-1,3-dihydrospiro-[indene-2,4'-piperidine]-1'-carboxylate (100 mg, 0.25 mmol) was dissolved in 3 mL of dichloromethane. 1 mL of trifluoroacetic acid was added, and the reaction solution was reacted at room temperature for 1 hour. The reaction solution was concentrated to dryness, and dissolved

in 5 mL of DMF. Potassium carbonate (517 mg, 3.75 mmol) and 6-chloro-3-((3-chloro-2-cyclopropylpyridin-4-yl)thio)pyrazin-2-amine (78 mg, 0.25 mmol) were added, and the reaction solution was heated to 100°C under a nitrogen atmosphere and reacted for 12 hours. The reaction solution was cooled, and then 20 mL of water was added. The reaction solution was extracted with ethyl acetate (20 mL×3). The ethyl acetate layer was washed with saturated sodium chloride solution (20 dried over anhydrous sodium sulfate, and purified by column chromatography (50% ethyl acetate/petroleum ether) to obtain the product (26 mg, yield: 18%) as an oil.

MS m/z (ESI): 583.1 $[M+H]^+$.

Step 9: Preparation of (S)-1'-(6-amino-5-((3-chloro-2-cyclopropylpyridin-4-yl)thio)pyrazin-2-yl)-1,3-dihydros piro[indene-2,4'-piperidin]-1-amine

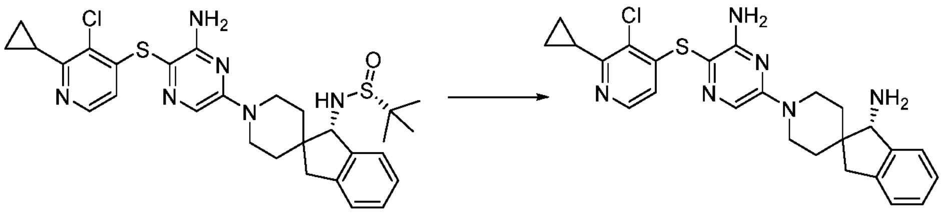

(R)-N-((S)-1'-(6-Amino-5-((3-chloro-2-cyclopropylpyridin-4-yl)thio)pyrazin-2-yl)-1,3-dihydrospiro[indene-2,4'-piperidin]-1-yl)-2-methylpropane-2-sulfinamide (26 mg, 0.044 mmol) was dissolved in 5 mL of dichloromethane. 1 mL of 4M HCl in dioxane was added, and the reaction solution was reacted at room temperature for 1 hour. The reaction solution was concentrated to dryness and dissolved in 5 mL of methanol, and the pH was adjusted to alkaline with 7M $NH_3$ in methanol. The reaction solution was concentrated to dryness by rotary evaporation, and the resulting residue was purified by column chromatography (5 to 8% MeOH in DCM) to obtain the product (12 mg, yield: 57%) as a light yellow solid.

$^1H$ NMR (400 MHz, Chloroform-d) δ 8.03 (d, *J* = 5.2 Hz, 1H), 7.67 (s, 1H), 7.44 - 7.37 (m, 1H), 7.24 (dd, *J* = 6.4, 3.9 Hz, 3H), 6.39 (d, *J* = 5.2 Hz, 1H), 4.85 (s, 2H), 4.21 - 4.13 (m, 2H), 4.07 (s, 1H), 3.23 - 3.21 (m, 2H), 3.13 (d, *J* = 15.8 Hz, 1H), 2.79 (d, *J* = 15.7 Hz, 1H), 2.57 - 2.48 (m, 1H), 1.88 - 1.73 (m, 2H), 1.63 - 1.56 (m, 1H), 1.50 - 1.42 (m, 1H), 1.09 - 1.00 (m, 4H).

MS m/z (ESI): 479.1 $[M+H]^+$.

## Example 4

Preparation of (2R)-1'-(6-amino-5-((2-amino-3-cyclopropylpyridin-4-yl)thio)pyrazin-2-yl)spiro-[bicyclo[3.1.0]hexane-3,4'-piperidin]-2-amine

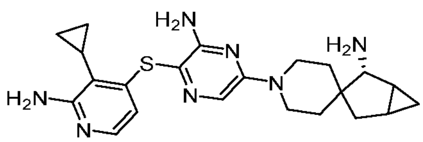

$^1H$ NMR (400 MHz, Methanol-$d_4$) δ 7.73 - 7.67 (m, 1H), 7.65 - 7.60 (m, 1H), 6.08 - 6.01 (m, 1H), 4.53 - 4.41 (m, 2H), 3.63 - 3.51 (m, 2H), 3.14 (p, *J* = 9.6 Hz, 1H), 2.41 - 2.33 (m, 1H), 1.56 - 1.40 (m, 3H), 1.44 - 1.35 (m, 1H), 1.39 - 1.19 (m, 4H), 0.89 - 0.81 (m, 2H), 0.76 - 0.60 (m, 2H), 0.44 - 0.28 (m, 2H).

MS m/z (ESI): 424.1 $[M+H]^+$.

The compound of Example 4 was prepared by referring to the experimental scheme of Example 1.

**Example 5**

Preparation of (S)-1'-(6-amino-5-((2-amino-3-chloropyridin-4-yl)thio)-3-bromopyrazin-2-yl)-1,3-dihydrospiro[indene-2,4'-piperidin]-1-amine

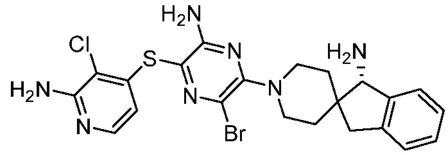

Step 1: Preparation of 2-ethylhexyl 3-((3-amino-5-chloropyrazin-2-yl)thio)propanoate

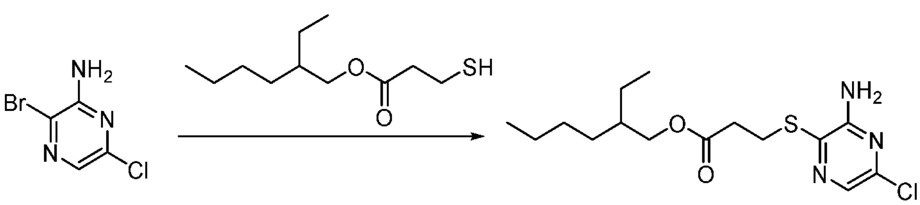

3-Bromo-6-chloropyrazin-2-amine (4 g, 20 mmol), 2-ethylhexyl 3-mercaptopropanoate (5.2 g, 24 mmol), tris(dibenzylideneacetone)dipalladium (916 mg, 1 mmol), 4,5-bis(diphenylphosphino)-9,9-dimethylxanthene (1.16 g, 2 mmol) and N,N-diisopropylethylamine (5.12 g, 40 mmol) were stirred in dioxane (35 mL) at 100°C for 18 hours. The reaction solution was filtered, and the filter cake was washed with ethyl acetate (30 mL) twice. The filtrate was concentrated, and purified by column chromatography [eluent: petroleum ether ~ petroleum ether/ethyl acetate (90:10)] to obtain 2-ethylhexyl 3-((3-amino-5-chloropyrazin-2-yl)thio)propanoate (5.5 g, yield: 82%) as a brown oil.

MS m/z (ESI): 146.1 $[M+H]^+$, 148.1 $[M+2+H]^+$.

Step 2: Preparation of 3-amino-5-chloropyrazine-2-thiol

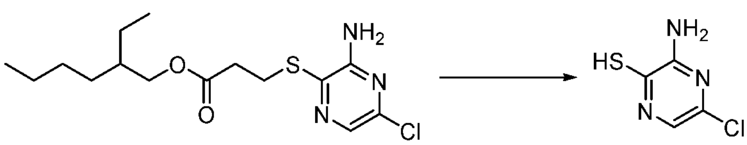

Potassium tert-butoxide (2.7 g, 23.9 mmol) was added to a solution of 2-ethylhexyl 3-((3-amino-5-chloropyrazin-2-yl)thio)propanoate (5.5 g, 15.9 mmol) in ethanol (100 mL). After completion of the addition, the reaction solution was stirred at room temperature for 3 hours. The reaction solution was concentrated by rotary evaporation to remove about 50 mL of ethanol. The remaining reaction solution was poured into aqueous ammonium chloride solution (100 mL), and extracted with ethyl acetate (100 mL) twice and dichloromethane (100 mL) twice. The organic phase was concentrated, and purified by column chromatography [eluent: dichloromethane ~ dichloromethane/methanol (95:5)] to obtain 3-amino-5-chloropyrazine-2-thiol (1.8 g, yield: 70%) as a dark green solid.

MS m/z (ESI): 162.0$[M+H]^+$, 164.0 $[M+2+H]^+$

Step 3: Preparation of 3-((2-amino-3-chloropyridin-4-yl)thio)-6-chloropyrazin-2-amine

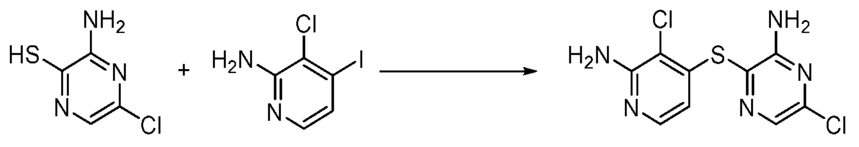

3-Amino-5-chloropyrazine-2-thiol (500 mg, 3.1 mmol), 3-chloro-4-iodopyridin-2-amine (789 mg, 3.1 mmol), tris(dibenzylideneacetone)dipalladium (142 mg, 0.16 mmol), 4,5-bis(diphenylphosphino)-9,9-dimethylxanthene (179 mg, 0.31 mmol) and N,N-diisopropylethylamine (1.2 g, 9.3 mmol) were stirred in dioxane (10 mL) at 130°C under microwave for 1 hour. The reaction solution was concentrated, and purified by column chromatography [eluent: dichloromethane ~ dichloromethane/methanol (99:1)] to obtain 1 g of a crude product. The crude product was pulped in ethanol (5 mL), and filtered to obtain 3-((2-amino-3-chloropyridin-4-yl)thio)-6-chloropyrazin-2-amine (580 mg, yield: 65%) as a grey solid.
MS m/z (ESI): 288.0 $[M+H]^+$, 290.0 $[M+2+H]^+$.

Step 4: Preparation of N-((S)-1,3-dihydrospiro[indene-2,4'-piperidin]-1-yl)-2-methylpropane-2-sulfinamide

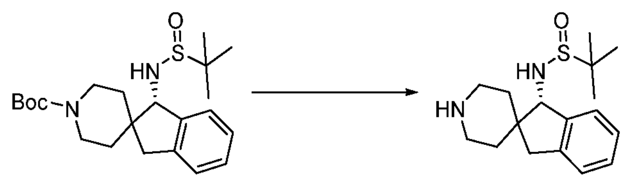

Trifluoroacetic acid (1 mL) was added to a solution of tert-butyl (1 S)-1-((*tert*-butylsulfinyl<sulfenyl>)amino)-1,3-dihydrospiro[indene-2,4'-piperidine]-1'-carboxylate (150 mg, 0.37 mmol) in dichloromethane (3 mL). After completion of the addition, the reaction solution was stirred at room temperature for 2 hours. The reaction solution was concentrated to obtain N-((S)-1,3-dihydrospiro-[indene-2,4'-piperidin]-1-yl)-2-methylpropane-2-sulfinamide hydrochloride (150 mg, yield: 100%) as a light yellow solid.
MS m/z (ESI): 307.2 $[M+H]^+$.

$$[\alpha]^{20}_{D}=1.773.$$

Step 5: Preparation of N-((S)-1'-(6-amino-5-((2-amino-3-chloropyridin-4-yl)thio)pyrazin-2-yl)-1,3-dihydrospiro[indene-2,4'-piperidin]-1-yl)-2-methylpropane-2-sulfinamide

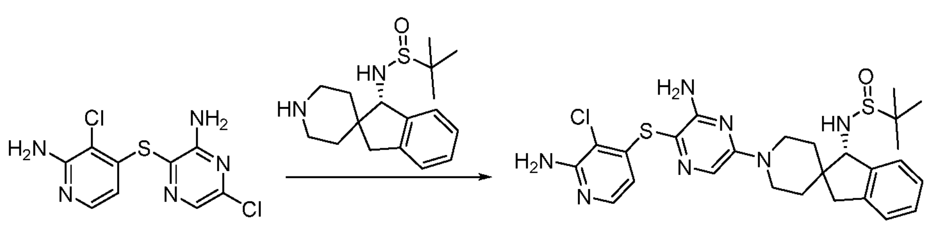

N-((S)-1,3-Dihydrospiro[indene-2,4'-piperidin]-1-yl)-2-methylpropane-2-sulfinamide hydrochloride (150 mg, 0.37 mmol), 3-((2-amino-3-chloropyridin-4-yl)thio)-6-chloropyrazin-2-amine (100 mg, 0.35 mmol) and potassium carbonate (335 mg, 2.43 mmol) were stirred in N,N-dimethylformamide (4 mL) at 100°C for 18 hours. The reaction solution was concentrated, and purified by column chromatography [eluent: dichloromethane ~ dichloromethane/methanol (97:3)] to obtain N-((S)-1'-(6-amino-5-((2-amino-3-chloropyridin-4-yl)thio)pyrazin-2-yl)-1,3-dihydro-spiro[indene-2,4'-piperidin]-1-yl)-2-methylpropane-2-sulfinamide (100 mg, yield: 52%) as a purple solid.
MS m/z (ESI): 558.1 $[M+H]^+$, 560.2 $[M+2+H]^+$.

Step 6: Preparation of (S)-1'-(6-amino-5-((2-amino-3-chloropyridin-4-yl)thio)-3-bromopyrazin-2-yl)-1,3-dihydrospiro[indene-2,4'-piperidin]-1-amine

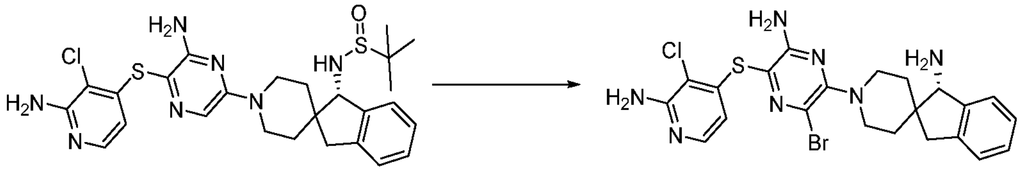

N-((S)-1'-(6-Amino-5-((2-amino-3-chloropyridin-4-yl)thio)pyrazin-2-yl)-1,3-dihydro-spiro[indene-2,4'-piperidin]-1-yl)-2-methylpropane-2-sulfinamide (100 mg, 0.18 mmol) and N-bromosuccinimide (64 mg, 0.36 mmol) were stirred in N,N-dimethylformamide (1 mL) at room temperature for 18 hours. The reaction solution was concentrated to obtain a crude product, which was purified by column chromatography [eluent: dichloromethane ~ dichloromethane/methanol (95:5)] to obtain (S)-1'-(6-amino-5-((2-amino-3-chloropyridin-4-yl)thio)-3-bromopyrazin-2-yl)-1,3-dihydrospiro[indene-2,4'-piperidin]-1-amine (80 mg, yield: 84%) as a khaki solid.

$^1$H NMR (400 MHz, DMSO-d6) $\delta$ 7.72-7.64 (m, 1H), 7.36 (d, *J*= 4 Hz, 1H), 7.27-7.16 (m, 3H), 6.58 (s, 2H), 6.33 (s, 2H), 5.80 (d, *J* = 4 Hz, 1H), 3.99 (s, 1H), 3.94-3.83 (m, 2H), 3.17 (d, *J* = 4 Hz, 1H), 3.14-2.98 (m, 3H), 2.77-2.64 (m, 1H), 1.97-1.74 (m, 2H), 1.55 (d, *J* = 12 Hz, 1H).

MS m/z (ESI): 532.0 $[M+H]^+$, 534.0 $[M+2+H]^+$.

**Example 6**

Preparation of (S)-1'-(6-amino-5-((2-amino-3-chloropyridin-4-yl)thio)-3-methylpyrazin-2-yl)-1,3-dihydrospiro[indene-2,4'-piperidin]-1-amine

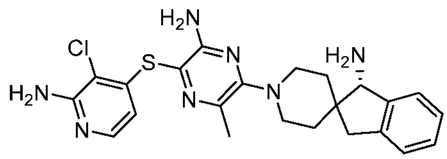

Step 1: Preparation of (S)-1'-(6-amino-5-((2-amino-3-chloropyridin-4-yl)thio)-3-methylpyrazin-2-yl)-1,3-dihydrospiro[indene-2,4'-piperidin]-1-amine

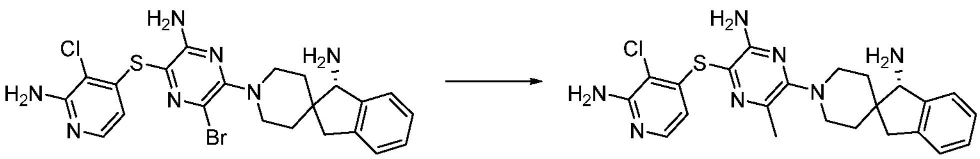

(S)-1'-(6-Amino-5-((2-amino-3-chloropyridin-4-yl)thio)-3-bromopyrazin-2-yl)-1,3-dihydrospiro[indene-2,4'-piperidin]-1-amine (30 mg, 0.056 mmol), trimethylboroxine (789 mg, 3.1 mmol), [1,1'-bis(diphenylphosphino)ferrocene]dichloropalladium (4 mg, 0.0056 mmol) and potassium carbonate (15 mg, 0.11 mmol) were stirred in N,N-dimethylformamide (10 mL) at 130°C under microwave for 1 hour. The reaction solution was concentrated and purified by high performance liquid chromatography to obtain (S)-1'-(6-amino-5-((2-amino-3-chloropyridin-4-yl)thio)-3-methylpyrazin-2-yl)-1,3-dihydrospiro[indene-2,4'-piperidin]-1-amine (0.7 mg, yield: 3%) as a grey solid.

$^1$H NMR (400 MHz, DMSO) $\delta$ 7.73-7.62 (m, 1H), 7.35 (d, *J*= 4 Hz, 1H), 7.28-7.16 (m, 3H), 6.57 (s, 2H), 6.32 (s, 2H), 5.78 (d, *J* = 4 Hz, 1H), 3.97 (s, 1H), 3.94-3.81 (m, 2H), 3.15 (d, *J* = 4 Hz, 1H), 3.12-2.95 (m, 3H), 2.75-2.64 (m, 1H), 2.41 (s, 3H), 1.99-1.75 (m, 2H), 1.58 (d, *J*= 12 Hz, 1H).

MS m/z (ESI): 468.1 $[M+H]^+$, 470.1 $[M+2+H]^+$.

**Example 7**

Preparation of (S)-1'-(5-((3-chloro-2-cyclopropylpyridin-4-yl)thio)pyrazin-2-yl)-1,3-dihydrospiro-[indene-2,4'-piperidin]-1-amine

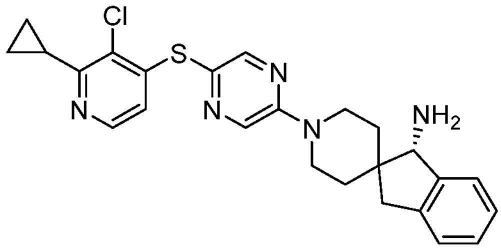

Step 1: Preparation of 2-chloro-5-((3-chloro-2-cyclopropylpyridin-4-yl)thio)pyrazine

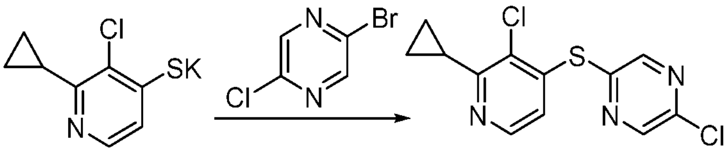

In a microwave reaction tube, potassium 3-chloro-2-cyclopropylpyridine-4-thiolate (120 mg, 0.54 mmol) and 2-bromo-5-chloropyrazine (104 mg, 0.54 mmol) were dissolved in 5 mL of 1,4-dioxane, followed by the addition of tris(dibenzylideneacetone)dipalladium (25 mg, 0.027 mmol), Xantphos (31 mg, 0.054 mmol) and DIPEA (209 mg, 1.62 mmol). The reaction solution was bubbled with nitrogen for 3 minutes, heated under microwave to 110°C and reacted for 1 hour. The reaction solution was cooled to room temperature, diluted with 20 mL of ethyl acetate, and filtered through diatomaceous earth. The filtrate was concentrated to dryness by rotary evaporation, and the resulting residue was purified by column chromatography (10 to 20% ethyl acetate/petroleum ether) to obtain a light yellow oil (120 mg, yield: 74%).
MS m/z (ESI): 298.0 $[M+H]^+$.

Step 2: Preparation of (R)-N-((S)-1'-(5-((3-chloro-2-cyclopropylpyridin-4-yl)thio)-pyrazin-2-yl)-1,3-dihydrospiro[indene-2,4'-piperidin]-1-yl)-2-methylpropane-2-sulfinamide

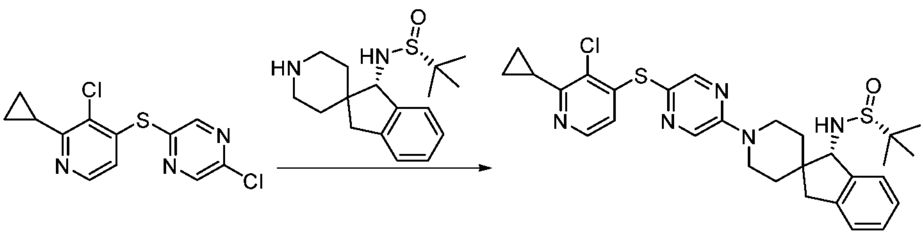

*Tert-butyl*

(S)-1-(((R)-*tert*-butylsulfinyl)amino)-1,3-dihydrospiro[indene-2,4'-piperidine]-1'-carboxylate (81 mg, 0.20 mmol) was dissolved in 3 mLof dichloromethane. 1 mLof trifluoroacetic acid was added, and the reaction solution was reacted at room temperature for 1 hour. The reaction solution was concentrated to dryness, and dissolved in 5 mL of DMF. Potassium carbonate (373 mg, 2.70 mmol) and 2-chloro-5-((3-chloro-2-cyclopropylpyridin-4-yl)thio)pyrazine (55 mg, 0.18 mmol) were added, and the reaction solution was heated to 100°C under a nitrogen atmosphere and reacted for 12 hours. The reaction solution was cooled, and then 20 mL of water was added. The reaction solution was extracted with ethyl acetate (20 mL×3). The ethyl acetate layer was washed with saturated sodium chloride solution (20 mL×3),

dried over anhydrous sodium sulfate, and purified by column chromatography (50% ethyl acetate/petroleum ether) to obtain the product (48 mg, yield: 47%) as an oil.

MS m/z (ESI): 568.1 $[M+H]^+$.

Step 3: Preparation of (S)-1'-(5-((3-chloro-2-cyclopropylpyridin-4-yl)thio)-pyrazin-2-yl)-1,3-dihydrospiro[indene-2,4'-piperidin]-1-amine

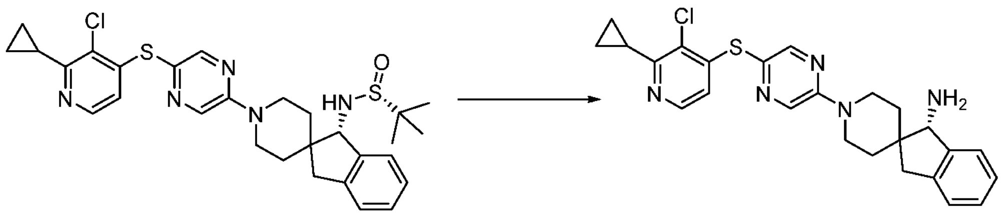

(R)-N-((S)-1'-(5-((3-Chloro-2-cyclopropylpyridin-4-yl)thio)pyrazin-2-yl)-1,3-dihydro-spiro[indene-2,4'-piperidin]-1-yl)-2-methylpropane-2-sulfinamide (48 mg, 0.085 mmol) was dissolved in 5 mL of dichloromethane. 1 mL of 4M HCl in dioxane was added, and the reaction solution was reacted at room temperature for 1 hour. The reaction solution was concentrated to dryness and dissolved in 5 mL of methanol, and the pH was adjusted to alkaline with 7M $NH_3$ in methanol. The reaction solution was concentrated to dryness by rotary evaporation, and the resulting residue was purified by column chromatography (0 to 10% MeOH in DCM) to obtain the product (23 mg, yield: 58%) as a light yellow solid.

$^1$H NMR (400 MHz, Methanol-$d_4$) δ 8.36 (s, 1H), 8.30 (s, 1H), 7.98 (d, *J*= 5.3 Hz, 1H), 7.42 (d, *J* = 6.8 Hz, 1H), 7.32 - 7.21 (m, 3H), 6.43 (d, *J* = 5.3 Hz, 1H), 4.44 - 4.29 (m, 2H), 4.14 (s, 1H), 3.41 - 3.30 (m, 2H), 3.19 (d, *J* = 15.9 Hz, 1H), 2.96 (d, *J* = 15.9 Hz, 1H), 2.60 - 2.49 (m, 1H), 1.90 - 1.76 (m, 2H), 1.67 - 1.54 (m, 2H), 1.09 - 0.99 (m, 4H).

MS m/z (ESI): 464.1 $[M+H]^+$.

**Example 8**

Preparation of (S)-1'-(6-amino-5-((3-chloro-2-cyclopropylpyridin-4-yl)thio)-pyrazin-2-yl)-4,6-dihydrospiro[cyclopenta[d]thiazole-5,4'-piperidin]-6-amine

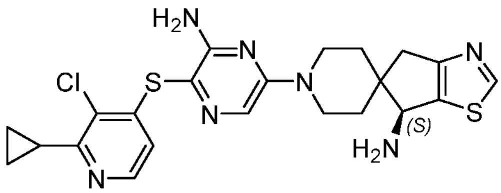

Step 1: Preparation of *tert*-butyl (5-bromo-1,3,4-thiadiazol-2-yl)carbamate

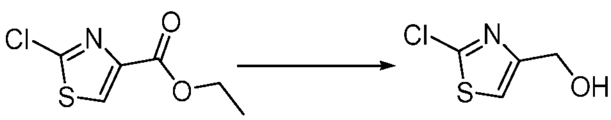

Ethyl 2-chlorothiazole-4-carboxylate (9.0 g, 46.97 mmol) was dissolved in 150 mL of methanol and stirred at room temperature. $NaBH_4$ (7.1 g, added in three batches) was added, and the reaction solution was stirred until the starting materials disappeared. The reaction solution was slowly added to brine under stirring to quench the reaction. The reaction solution was concentrated to remove the solvent, and extracted with ethyl acetate (200 mL) three times. The organic layers were combined, dried over anhydrous sodium sulfate, and concentrated to obtain a crude product, which was purified by column chromatography (ethyl acetate/petroleum ether=3:1) to obtain the target product (2-chlorothiazol-4-yl)methanol (4.2 g, yield: 60%) as a colorless oily liquid.

$^{1}$H NMR (400 MHz, $CDCl_3$) δ 7.11 (s, 1H), 4.71 (s, 2H), 2.22 (s, 1H).

MS m/z (ESI): 150.0 $[M+H]^+$, 152.0 $[M+2+H]^+$.

Step 2: Preparation of (2-chlorothiazol-4-yl)methyl methanesulfonate

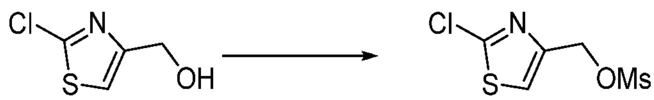

(2-Chlorothiazol-4-yl)methanol (2.6 g, 17.4 mmol) was dissolved in 40 mL of dichloromethane. Methanesulfonyl chloride (1.6 mL, 20.88 mmol, d=1.48 g/mL) was added dropwise under a nitrogen atmosphere in an ice water bath, and the reaction solution was stirred in the ice water bath for 30 minutes. After completion of the reaction, the reaction solution was added dropwise to saturated sodium chloride solution (100 mL), and extracted with dichloromethane (100 mL) three times. The organic layers were combined, dried over anhydrous sodium sulfate, and concentrated to obtain the target product (2-chlorothiazol-4-yl)methyl methanesulfonate (3.9 g, yield: 97%) as a white solid.

$^{1}$H NMR (400 MHz, $CDCl_3$) δ 7.35 (s, 1H), 5.25 (s, 2H), 3.07 (s, 3H).

Step 3: Preparation of 1-(tert-butyl) 4-ethyl 4-((2-chlorothiazol-4-yl)methyl)piperidine-1,4-dicarboxylate

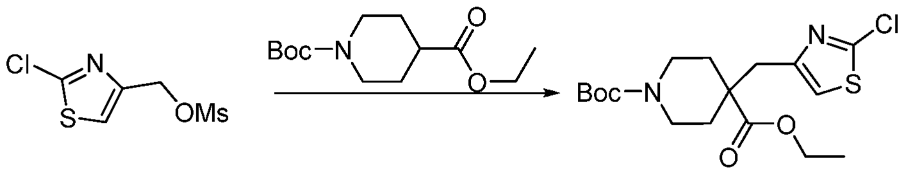

1-(*Tert*-butyl) 4-ethyl piperidine-1,4-dicarboxylate (4.8 g, 18.59 mmol) was dissolved in THF (40 mL). The solution was cooled to -60°C under a nitrogen atmosphere, and then lithium diisopropylamide (12.7 mL, 25.35 mmol) was added dropwise. After completion of the addition, the reaction solution was stirred at -60°C to -50°C for 30 minutes. A solution of (2-chlorothiazol-4-yl)methyl methanesulfonate in THF (15 mL) was added dropwise. After completion of the addition, the reaction solution was stirred at -60°C for 30 minutes, then slowly warmed up to room temperature and stirred for 2 hours. After completion of the reaction, the reaction solution was added dropwise to saturated brine (150 mL), and extracted with ethyl acetate (100 mL) three times. The organic layers were combined, dried over anhydrous sodium sulfate, and concentrated to obtain a crude product, which was purified by column chromatography (ethyl acetate/petroleum ether=3:1) to obtain the target product 1-(tert-butyl) 4-ethyl 4-((2-chlorothiazol-4-yl)methyl)piperidine-1,4-dicarboxylate (3.7 g, yield: 56%) as a yellow liquid.

$^{1}$H NMR (400 MHz, $CDCl_3$) δ 6.79 (s, 1H), 4.14 (dd, *J* = 7.1, 3.1 Hz, 2H), 3.91 - 3.85 (m, 2H), 2.92 (s, 2H), 2.86 (dd, *J* = 16.3, 13.6 Hz, 2H), 2.10 (d, *J* = 13.5 Hz, 2H), 1.53 (s, 2H), 1.44 (s, 9H), 1.25 - 1.22 (m, 3H).

MS m/z (ESI): 289 $[M-100+H]^+$.

Step 4: Preparation of *tert*-butyl 2-chloro-6-oxo-4,6-dihydrospiro[cyclopenta[d]thiazole-5,4'-piperidine]-1'-carboxylate

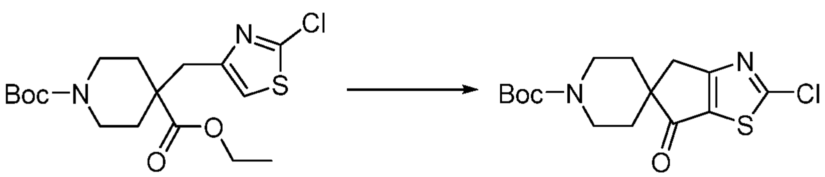

1- (*Tert*-butyl) 4-ethyl 4-((2-chlorothiazol-4-yl)methyl)piperidine-1,4-dicarboxylate (4.1 g, 10.6 mmol) was dissolved in THF (40 mL). The solution was cooled to -70°C under a nitrogen atmosphere, and then lithium diisopropylamide (13.4 mL, 26.5 mmol) was added dropwise. After completion of the addition, the reaction solution was stirred at -70°C for 30 minutes. After completion of the reaction, the reaction solution was added dropwise to saturated brine (150 mL), and extracted with ethyl acetate (100 mL) three times. The organic layers were combined, dried over anhydrous sodium sulfate, and concentrated to obtain a crude product, which was purified by column chromatography (ethyl acetate/petroleum ethet=3:1) to obtain the target product *tert*-butyl 2-chloro-6-oxo-4,6-dihydrospiro[cyclopenta[d]thiazole-5,4'-piperidine]-1'-carboxylate (1.2 g, yield: 33%) as a yellow solid.

$^1$H NMR (400 MHz, $CDCl_3$) δ 4.15 (s, 4H), 3.05 (s, 2H), 2.06 - 1.93 (m, 2H), 1.60 (d, *J* = 12.7 Hz, 2H), 1.48 (s, 9H).

MS m/z (ESI): 243 $[M-100+H]^+$.

Step 5: Preparation of *tert*-butyl (R,Z)-6-((*tert*-butylsulfinyl<sulfenyl>)imino)-2-chloro-4,6-dihydrospiro[cyclopenta[d]t hiazole-5,4'-piperidine]-1'-carboxylate

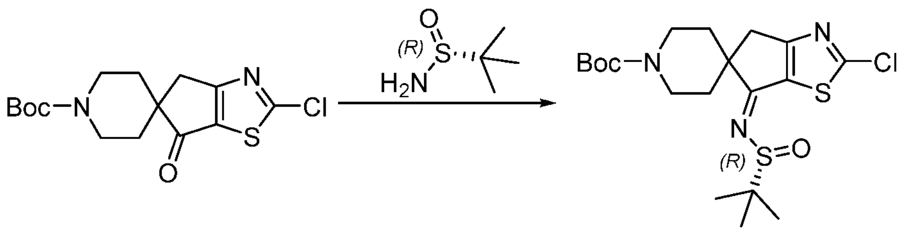

*Tert*-butyl 2-chloro-6-oxo-4,6-dihydrospiro[cyclopenta[d]thiazole-5,4'-piperidine]-1'-carboxylate (1.2 g, 3.5 mmol) was dissolved in THF (10 mL). $Ti(OEt)_4$ (10 mL) was added, and the reaction solution was heated to 95°C under a nitrogen atmosphere and stirred for 10 hours. The reaction solution was cooled to room temperature, and diluted with ethyl acetate (200 mL). Water (20 mL) was added under stirring, and the solution was stirred until cloudy. Anhydrous sodium sulfate was added thereto until the solid in the mixture was sandy. The mixture was filtered, and the filtrate was concentrated to obtain a crude product, which was purified by column chromatography (PE/EA=5:1) to obtain the target product *tert*-butyl (R,Z)-6-((*tert*-butylsulfinyl<sulfenyl>)imino)-2-chloro-4,6-dihydro-spiro[cyclopenta[d]thiazole-5,4'-piperidine]-1'-carboxylate (1.1 g, yield: 75%) as a yellow solid.

$^1$H NMR (400 MHz, $CDCl_3$) δ 4.13 (dd, *J* = 22.7, 15.6 Hz, 4H), 2.92 (d, *J* = 2.9 Hz, 2H), 2.05 (s, 2H), 1.57 (s, 2H), 1.48 (s, 9H), 1.29 - 1.22 (m, 9H).

MS m/z (ESI): 346 $[M-100+H]^+$.

Step 6: Preparation of *tert*-butyl (S)-6-(((R)-*tert*-butylsulfinyl<sulfenyl>)amino)-4,6-dihydrospiro[cyclopenta[d]thiazole-5,4'-piperidine]-1'-carboxylate and *tert-butyl* (S)-6-(((R)-*tert*-butylsulfinyl<sulfenyl>)amino)-2-chloro-4,6-dihydrospiro-[cyclopenta[d]thiazole-5,4'-piperidine]-1'-carboxylate

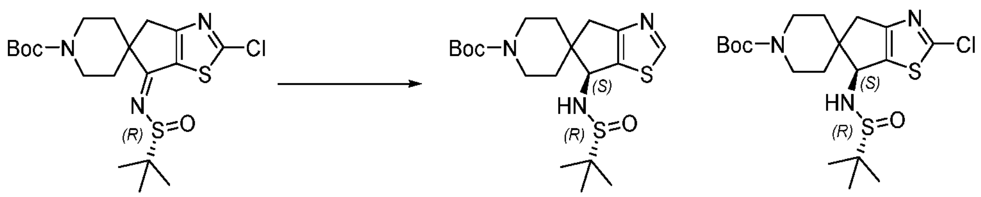

Tert-butyl (R,Z)-6-((*tert*-butylsulfinyl<sulfenyl>)imino)-2-chloro-4,6-dihydrospiro[cyclopenta[d]thiazole-5,4'-piperidine]-1'-carboxylate (600 mg, 1.35 mmol) was dissolved in anhydrous THF (10 mL) in a 50 mL three-neck flask. The solution was cooled to -70°C under a nitrogen atmosphere, and then $BH_3$/THF (4.0 mL, D=1 M) was added dropwise. After completion of the addition, the reaction solution was slowly warmed to room temperature and stirred for 10 hours. The reaction solution was added dropwise to water, and saturated sodium bicarbonate solution was added to adjust the pH to 9. The reaction solution was extracted with ethyl acetate (100 mL) three times. The organic layers were combined, dried over anhydrous sodium sulfate, and concentrated to obtain a crude product, which was purified by column chromatography (PE/EA=1:1) to obtain the target products *tert*-butyl (S)-6-(((R)-*tert*-butylsulfinyl<sulfenyl>)amino)-4,6-dihydrospiro[cyclopenta[d]thiazole-5,4'-piperidine]-1'-carboxylate (112 mg, yield: 20%) as a yellow liquid and *tert*-butyl (S)-6-(((R)-*tert*-butylsulfinyl<sulfenyl>)amino)-2-chloro-4,6-dihydrospiro-[cyclopenta[d]thiazole-5,4'-piperidine]-1'-carboxylate (56 mg, yield: 9%) as a yellow liquid.

MS m/z (ESI): 414.0 $[M+H]^+$;

MS m/z (ESI): 448.1 $[M+H]^+$, 450.1 $[M+H+2]^+$.

Step 7: Preparation of (R)-N-((S)-4,6-dihydrospiro[cyclopenta[d]thiazole-5,4'-piperidin]-6-yl)-2-methylpropane-2-sulfinamide

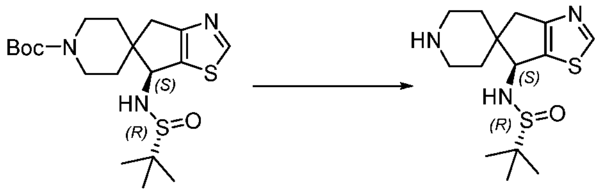

*Tert*-butyl (S)-6-(((R)-*tert*-butylsulfinyl<sulfenyl>)amino)-4,6-dihydro-spiro[cyclopenta[d]thiazole-5,4'-piperidine]-1'-carboxylate (112 mg, 0.27 mmol) was dissolved in anhydrous $CH_2Cl_2$(5 mL). Trifluoroacetic acid (1.0 mL) was added, and the reaction solution was stirred at room temperature for 2 hours. The reaction solution was concentrated to obtain the crude product (R)-N-((S)-4,6-dihydrospiro[cyclopenta[d]thiazole-5,4'-piperidin]-6-yl)-2-methylpropane-2-sulfinamide (67 mg, yield: 76%) as a yellow liquid.

MS m/z (ESI): 314.0 $[M+H]^+$.

Step 8: Preparation of (R)-N-((S)-1'-(6-amino-5-((3-chloro-2-cyclopropylpyridin-4-yl)thio)pyrazin-2-yl)-4,6-dihydrospiro-[cyclopenta[d]thiazole-5,4'-piperidin]-6-yl)-2-methylpropane-2-sulfinamide

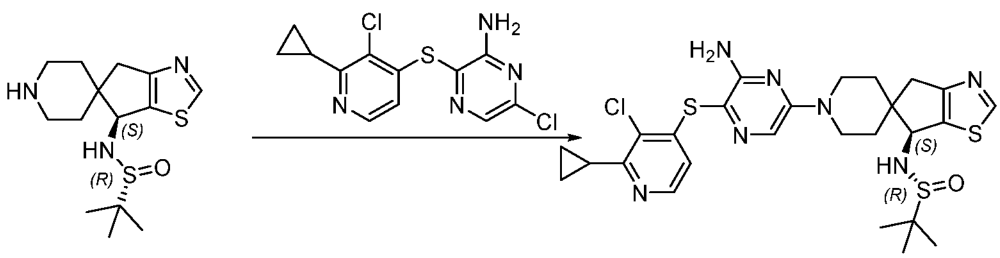

(R)-N-((S)-4,6-Dihydrospiro[cyclopenta[d]thiazole-5,4'-piperidin]-6-yl)-2-methylpropane-2-sulfinamide (67 mg, 0.21 mmol), 6-chloro-3-((3-chloro-2-cyclopropylpyridin-4-yl)thio)pyrazin-2-amine (78 mg, 0.25 mmol) and DIPEA (0.4 mL) were dissolved in DMF (2.0 mL). The reaction solution was heated to 110°C and stirred for 10 hours. The reaction solution was dissolved in ethyl acetate (100 mL), washed with water (50 mL) twice, and washed with saturated brine (50 mL) three times.

The organic layers were combined, dried over anhydrous sodium sulfate, and concentrated to obtain a crude product, which was purified by column chromatography (EtOAc) to obtain the target product (R)-N-((S)-1'-(6-amino-5-((3-chloro-2-cyclopropylpyridin-4-yl)thio)pyrazin-2-yl)-4,6-dihydrospiro[cyclopenta[d]thiazole-5,4'-piperidin]-6-yl)-2-methylpropane-2-sulfinamide (30 mg, yield: 19%) as a yellow liquid.

MS m/z (ESI): 590.0 $[M+H]^+$.

Step 9: Preparation of (S)-1'-(6-amino-5-((3-chloro-2-cyclopropylpyridin-4-yl)thio)pyrazin-2-yl)-4,6-dihydro spiro [cyclopenta[d] thiazole- 5,4' -piperidin] -6-amine

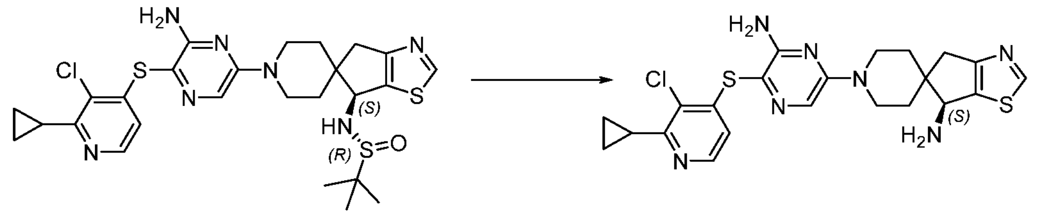

(R)-N-((S)-1'-(6-Amino-5-((3-chloro-2-cyclopropylpyridin-4-yl)thio)pyrazin-2-yl)-4,6-dihydrospiro[cyclopenta[d]thiazole-5,4'-piperidin]-6-yl)-2-methylpropane-2-sulfinamide (30 mg, 0.05 mmol) was dissolved in anhydrous methanol (3.0 mL). Hydrochloric acid/1,4-dioxane (1.0 mL) was added, and the reaction solution was stirred at room temperature for 0.5 hour. The reaction solution was concentrated to obtain a crude product, which was dissolved in water. Saturated sodium bicarbonate solution was added to adjust the pH to 10, and the reaction solution was extracted with dichloromethane (50 mL) twice. The organic layers were combined, dried over anhydrous sodium sulfate, and concentrated to obtain a crude product, which was purified by preparative HPLC to obtain the target product (S)-1'-(6-amino-5-((3-chloro-2-cyclopropylpyridin-4-yl)thio)pyrazin-2-yl)-4,6-dihydro-spiro[cyclopenta[d]thiazole-5,4'-piperidin]-6-amine (3.4 mg, yield: 49%) as a yellow solid.

$^1$H NMR (500 MHz, $CDCl_3$ ) δ 8.77 (s, 1H), 8.48 (s, 1H), 7.45 (d, *J*= 9.7 Hz, 2H), 4.45 (s, 1H), 3.38 (d, *J* = 61.4 Hz, 4H), 2.91 (s, 2H), 2.22 (s, 1H), 1.92 (s, 1H), 1.71 (s, 1H), 1.52 (s, 2H), 1.33 - 0.92 (m, 8H).

MS m/z (ESI): 486 $[M+H]^+$.

**Example 9**

Preparation of (S)-1'-(6-amino-5-((3-chloro-2-cyclopropylpyridin-4-yl)thio)pyrazin-2-yl)-2-chloro-4,6-dihydrospiro[cyclopenta[d]thiazole-5,4'-piperidin]-6-amine

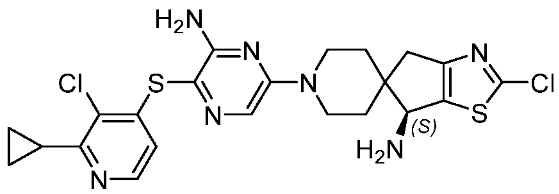

Step 1: Preparation of (R)-N-((S)-2-chloro-4,6-dihydrospiro[cyclopenta[d]thiazole-5,4'-piperidin]-6-yl)-2-methylpropane-2-sulfinamide

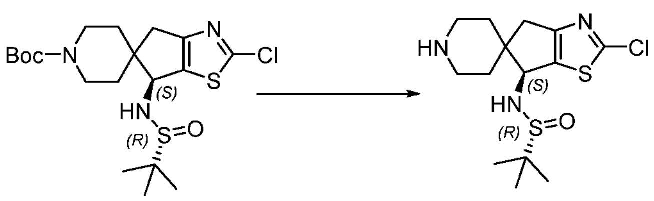

*Tert*-butyl (S)-6-(((R)-*tert*-butylsulfinyl<sulfenyl>)amino)-2-chloro-4,6-dihydrospiro[cyclopenta[d]thiazole-5,4'-piperidine]-1'-carboxylate (56 mg, 0.13 mmol) was dissolved in anhydrous $CH_2Cl_2$(5 mL). Trifluoroacetic acid (1.0 mL) was added, and the reaction solution was stirred at room temperature for 2 hours. The reaction solution was concentrated to obtain the crude product (R)-N-((S)-2-chloro-4,6-dihydrospiro[cyclopenta[d]thiazole-5,4'-piperidin]-6-yl)-2-methylpropane-2-sulfinamide (40 mg, yield: 100%) as a yellow liquid.

MS m/z (ESI): 348.0 $[M+H]^+$.

Step 2: Preparation of (R)-N-((S)-1'-(6-amino-5-((3-chloro-2-cyclopropylpyridin-4-yl)thio)-pyrazin-2-yl)-2-chloro-4, 6-dihydro spiro [cyclopenta [d] thiazole-5, 4' -piperidin] -6-yl)-2-methylprop ane-2-sulfinamide

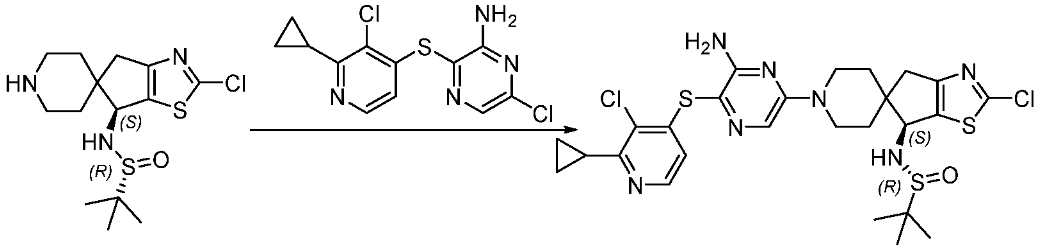

(R)-N-((S)-2-Chloro-4,6-dihydrospiro[cyclopenta[d]thiazole-5,4'-piperidin]-6-yl)-2-methylpropane-2-sulfinamide (40 mg, 0.1 mmol), 6-chloro-3-((3-chloro-2-cyclopropylpyridin-4-yl)thio)pyrazin-2-amine (15 mg, 0.05 mmol) and DIPEA (0.1 mL) were dissolved in DMF (0.8 mL). The reaction solution was heated to 110°C and stirred for 10 hours. The reaction solution was dissolved in ethyl acetate (100 mL), washed with water (50 mL) twice, and washed with saturated brine (50 mL) three times. The organic layers were combined, dried over anhydrous sodium sulfate, and concentrated to obtain a crude product, which was purified by column chromatography (EtOAc) to obtain the target product (R)-N-((S)-1'-(6-amino-5-((3-chloro-2-cyclopropylpyridin-4-yl)thio)pyrazin-2-yl)-4,6-dihydrospiro[cyclopenta[d]thiazole-5,4'-piperidin]-6-yl)-2-methylpropane-2-sulfinamide (8.2 mg, yield: 26%) as a yellow liquid.

MS m/z (ESI): 624.1 [M+H]$^+$.

Step 3: Preparation of (S)-1'-(6-amino-5-((3-chloro-2-cyclopropylpyridin-4-yl)thio)pyrazin-2-yl)-2-chloro-4,6-dihydrospiro[cyclopenta[d]thiazole-5,4'-piperidin]-6-amine

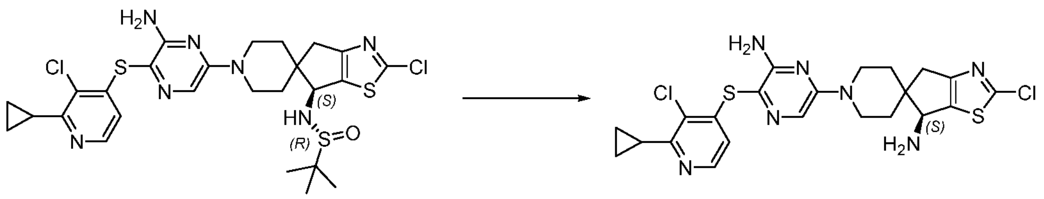

(R)-N-((S)-1'-(6-Amino-5-((3-chloro-2-cyclopropylpyridin-4-yl)thio)pyrazin-2-yl)-4,6-dihydrospiro[cyclopenta[d]thiazole-5,4'-piperidin]-6-yl)-2-methylpropane-2-sulfinamide (8.2 mg, 0.01 mmol) was dissolved in anhydrous methanol (2.0 mL). Hydrochloric acid/dioxane (0.5 mL) was added, and the reaction solution was stirred at room temperature for 0.5 hour. The reaction solution was concentrated to obtain a crude product, which was dissolved in water. Saturated sodium bicarbonate solution was added to adjust the pH to 10, and the reaction solution was extracted with dichloromethane (50 mL) twice. The organic layers were combined, dried over anhydrous sodium sulfate, and concentrated to obtain a crude product, which was purified by preparative HPLC to obtain the target product (S)-1'-(6-amino-5-((3-chloro-2-cyclopropylpyridin-4-yl)thio)pyrazin-2-yl)-2-chloro-4,6-dihydrospiro[cyclopenta[d]thiazole-5,4'-piperidin]-6-amine (1.5 mg, yield: 22%) as a yellow solid.

$^1$H NMR (500 MHz, $CDCl_3$ ) $\delta$ 8.48 (s, 1H), 7.45 (d, $J$ = 10.3 Hz, 2H), 4.71 (s, 1H), 3.41 (d, $J$ = 74.5 Hz, 4H), 2.92 (s, 2H), 2.16 (d, $J$ = 56.0 Hz, 2H), 1.74 (s, 1H), 1.52 (s, 2H), 1.26 (d, $J$ = 15.5 Hz, 4H), 1.03 (d, $J$ = 39.0 Hz, 4H).

MS m/z (ESI): 520.1 [M+H]$^+$.

**Example 10**

Preparation of (S)-1'-(6-amino-5-((2-(azetidin-1-yl)-3-chloropyridin-4-yl)thio)pyrazin-2-yl)-1,3-dihydrospiro[indene-2,4'-piperidin]-1-amine

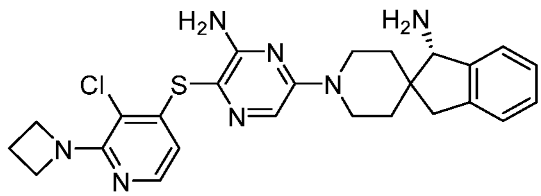

Step 1: Preparation of 2-ethylhexyl 3-((2,3-dichloropyridin-4-yl)thio)propanoate

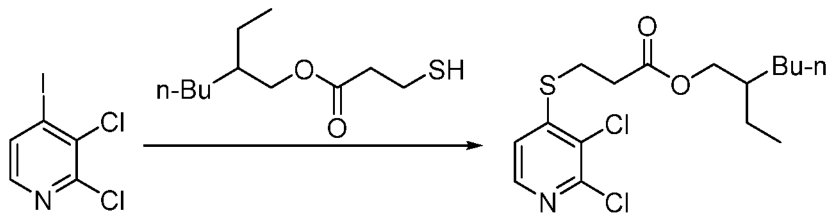

2,3-Dichloro-4-iodopyridine (2.0 g, 7.31 mmol), 2-ethylhexyl 3-mercaptopropanoate (1.8 g, 8.24 mmol), palladium acetate (82 mg, 0.37 mmol), Xantphos (254 mg, 0.44 mmol) and diisopropylethylamine (1.9 g, 14.70 mmol) were stirred in 15 mL of dioxane under a nitrogen atmosphere at 100°C for 5 hours. The reaction solution was cooled to room temperature, and then ethyl acetate was added. The reaction solution was filtered, and the filtrate was concentrated to dryness. The resulting residue was purified by column chromatography (petroleum ether/ethyl acetate=3:1) to obtain the target product 2-ethylhexyl 3-((2,3-dichloropyridin-4-yl)thio)propanoate (910 mg, yield: 34%).

MS m/z (ESI): 364.1 $[M+H]^+$

Step 2: Preparation of 2-ethylhexyl 3-((2-(azetidin-1-yl)-3-chloropyridin-4-yl)thio)propanoate

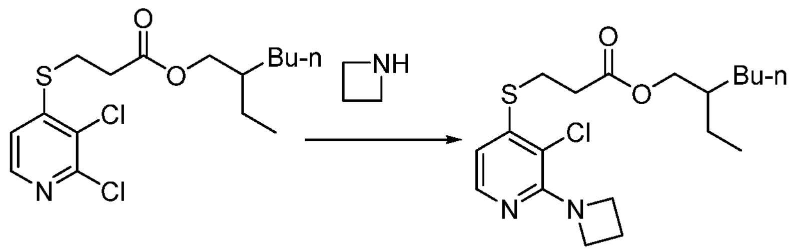

2-Ethylhexyl 3-((2,3-dichloropyridin-4-yl)thio)propanoate (200 mg, 0.55 mmol), azetidine (63 mg, 1.10 mmol) and 1,4-dioxane (5 mL) were stirred at 120°C under microwave for 3 hours. The reaction solution was concentrated to dryness, and then water was added. The solution was extracted with dichloromethane, and the organic phase was concentrated to dryness. The resulting residue was purified by column chromatography (petroleum ether/ethyl acetate=3:1) to obtain the product (130 mg, yield: 62%).

MS m/z (ESI): 385.1 $[M+H]^+$.

Step 3: Preparation of 2-(azetidin-1-yl)-3-chloropyridine-4-thiol

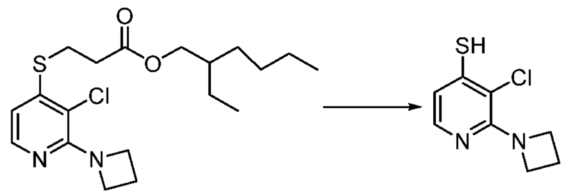

2-Ethylhexyl 3-((2-(azetidin-1-yl)-3-chloropyridin-4-yl)thio)propanoate (130 mg, 0.34 mmol) and potassium *tert*-butoxide (38 mg, 0.34 mmol) were stirred in anhydrous ethanol (5 mL) at room temperature for 1 hour. The reaction solution was concentrated to dryness, and the resulting residue was used directly in the next step.

MS m/z (ESI): 201.0 $[M+H]^+$.

Step 4: Preparation of 3-((2-(azetidin-1-yl)-3-chloropyridin-4-yl)thio)-6-chloropyrazin-2-amine

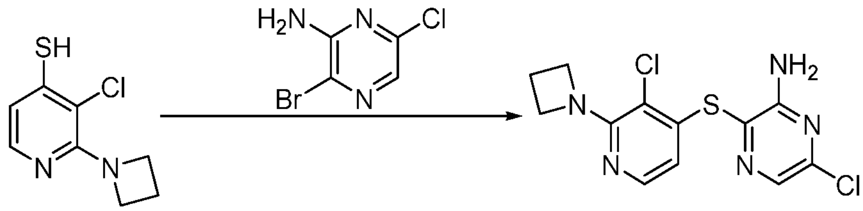

2-(Azetidin-1-yl)-3-chloropyridine-4-thiol (obtained in the previous step), 3-bromo-6-chloropyrazin-2-amine (84 mg, 0.40 mmol), $Pd_2(dba)_3$ (15 mg, 0.016 mmol), Xantphos (20 mg, 0.035 mmol) and diisopropylethylamine (87 mg, 0.67 mmol) were added to 5 mL of 1,4-dioxane, and the reaction solution was stirred under a nitrogen atmosphere at 100°C overnight. The reaction solution was cooled to room temperature and concentrated to dryness, and the resulting residue was purified by column chromatography (petroleum ether/ethyl acetate=1:1) to obtain a light yellow solid (71 mg, yield: 64%).

MS m/z (ESI): 328.0 $[M+H]^+$.

Step 5: Preparation of (R)-N-((S)-1,3-dihydrospiro[indene-2,4'-piperidin]-1-yl)-2-methylpropane-2-sulfinamide

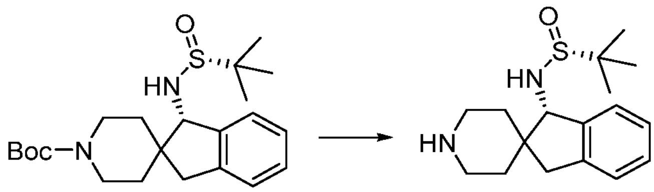

*Tert-butyl* (S)-1-(((R)-*tert*-butylsulfinyl<sulfenyl>)amino)-1,3-dihydrospiro-[indene-2,4'-piperidine]-1'-carboxylate (100 mg, 0.25 mmol) was dissolved in dichloromethane (1 mL). 1 mL of trifluoroacetic acid was added, and the reaction solution was stirred at room temperature for 1 hour. The reaction solution was concentrated to dryness to obtain an oil, which was used directly in the next step.

MS m/z (ESI): 307.1 $[M+H]^+$.

$$[\alpha]^{20}_{D}=1.773.$$

Step 6: Preparation of (R)-N-((S)-1'-(6-amino-5-((2-(azetidin-1-yl)-3-chloropyridin-4-yl)thio)pyrazin-2-yl)-1,3-dihydrospiro[indene-2,4'-piperidin]-1-yl)-2-methylpropane-2-sulfinamide

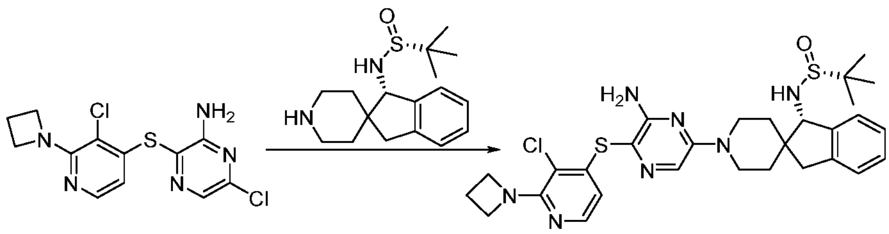

3-((2-(Azetidin-1-yl)-3-chloropyridin-4-yl)thio)-6-chloropyrazin-2-amine (71 mg, 0.22 mmol), (R)-N-((S)-1,3-dihydrospiro[indene-2,4'-piperidin]-1-yl)-2-methylpropane-2-sulfinamide (obtained in the previous step) and potassium carbonate (299 mg, 2.16 mmol) were stirred in DMF (5 mL) under a nitrogen atmosphere at 100°C under microwave for 2 hours. Water was added, and the reaction solution was extract with ethyl acetate. The organic phase was dried over anhydrous sodium sulfate, and concentrated to dryness to obtain an oil, which was used directly in the next step.

MS m/z (ESI): 598.2[M+H]$^+$.

Step 7: Preparation of (S)-1'-(6-amino-5-((2-(azetidin-1-yl)-3-chloropyridin-4-yl)thio)pyrazin-2-yl)-1,3-dihydrospiro[indene-2,4'-piperidin]-1-amine

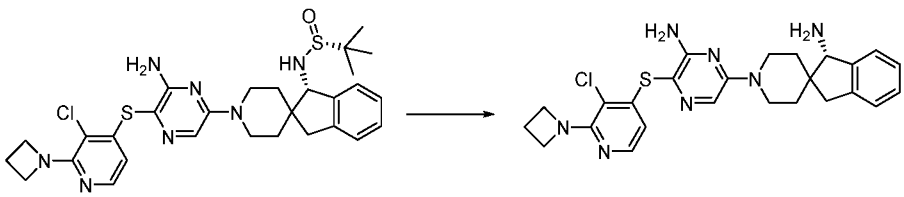

(R)-N-((S)-1'-(6-Amino-5-((2-(azetidin-1-yl)-3-chloropyridin-4-yl)thio)pyrazin-2-yl)-1,3-dihydrospiro[indene-2,4'-piperidin]-1-yl)-2-methylpropane-2-sulfinamide (obtained in the previous step) was dissolved in methanol (5 mL). 4M HCl/1,4-dioxane (5 mL) was added, and the reaction solution was stirred at room temperature for 1 hour. The reaction solution was concentrated to dryness, and the resulting residue was purified by column chromatography to obtain the target product (S)-1'-(6-amino-5-((2-(azetidin-1-yl)-3-chloropyridin-4-yl)thio)pyrazin-2-yl)-1,3-dihydrospiro[indene-2,4'-piperidin]-1-amine (8.7 mg, yield: 8%).

$^1$H NMR (400 MHz, Methanol-d4) $\delta$ 7.69 (d, *J* = 5.4 Hz, 1H), 7.58 (s, 1H), 7.42 - 7.32 (m, 1H), 7.28 - 7.14 (m, 3H), 5.97 (d, *J* = 5.5 Hz, 1H), 4.35 - 4.16 (m, 6H), 3.96 (s, 1H), 3.28 - 3.19 (m, 2H), 3.15 (d, *J* = 15.7 Hz, 1H), 2.81 (d, *J*= 15.7 Hz, 1H), 2.32 (q, *J* = 7.5 Hz, 2H), 1.80 (dtd, *J* = 32.3, 12.6, 4.3 Hz, 2H), 1.58 (d, *J*= 13.4 Hz, 1H), 1.42 (d, *J*= 13.4 Hz, 1H), 1.31 (d, *J* = 16.2 Hz, 1H).

MS m/z (ESI): 494.1 [M+H]$^+$.

**Example 11**

Preparation of (S)-1'-(6-amino-5-((3-chloro-2-morpholinopyridin-4-yl)thio)-pyrazin-2-yl)-1,3-dihydrospiro[indene-2,4'-piperidin]-1-amine

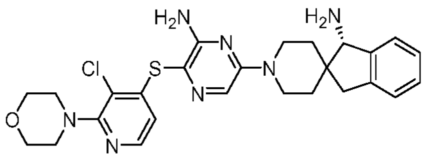

Step 1: Preparation of 2-ethylhexyl 3-((3-chloro-2-morpholinopyridin-4-yl)thio)propanoate

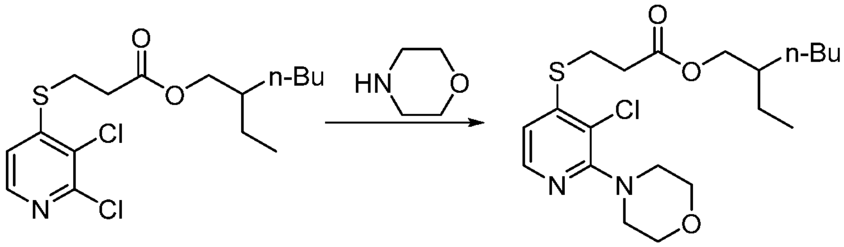

2-Ethylhexyl 3-((2,3-dichloropyridin-4-yl)thio)propanoate (200 mg, 0.55 mmol), morpholine (96 mg, 1.10 mmol) and 1,4-dioxane (5 mL) were stirred at 120°C under microwave for 8 hours. The reaction solution was concentrated to dryness, and then water was added. The solution was extracted with dichloromethane, and the organic phase was concentrated to dryness. The resulting residue was purified by column chromatography (petroleum ether/ethyl acetate=3:1) to obtain the product (152 mg, yield: 67%).

MS m/z (ESI): 415.2 $[M+H]^+$.

Step 2: Preparation of potassium 3-chloro-2-morpholinopyridine-4-thiolate

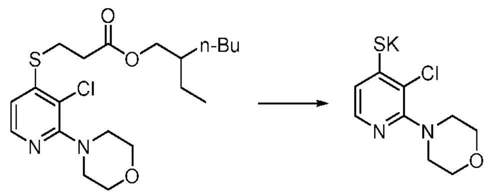

2-Ethylhexyl 3-((3-chloro-2-morpholinopyridin-4-yl)thio)propanoate (100 mg, 0.24 mmol) and potassium *tert*-butoxide (27 mg, 0.24 mmol) were stirred in anhydrous ethanol (5 mL) at room temperature for 1 hour. The reaction solution was concentrated to dryness to obtain the product, which was used directly in the next step.

MS m/z (ESI): 231.0 $[M+H]^+$.

Step 3: Preparation of 6-chloro-3-((3-chloro-2-morpholinopyridin-4-yl)thio)pyrazin-2-amine

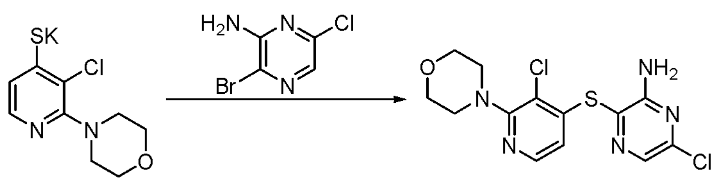

Potassium 3-chloro-2-morpholinopyridine-4-thiolate (obtained in the previous step), 3-bromo-6-chloropyrazin-2-amine (60 mg, 0.29 mmol), $Pd_2(dba)_3$ (11 mg, 0.012 mmol), Xantphos (14 mg, 0.024 mmol) and diisopropylethylamine (62 mg, 0.48 mmol) were stirred in 5 mL of dioxane under a nitrogen atmosphere at 100°C overnight. The reaction solution was cooled to room temperature and concentrated to dryness, and the resulting residue was purified by column chromatography (petroleum ether/ethyl acetate=1:1) to obtain a light yellow solid (60 mg, yield: 70%).

MS m/z (ESI): 358.0 $[M+H]^+$.

Step 4: Preparation of (R)-N-((S)-1'-(6-amino-5-((3-chloro-2-morpholinopyridin-4-yl)thio)pyrazin-2-yl)-1,3-dihydrospiro[indene-2,4'-piperidin]-1-yl)-2-methylpropane-2-sulfinamide

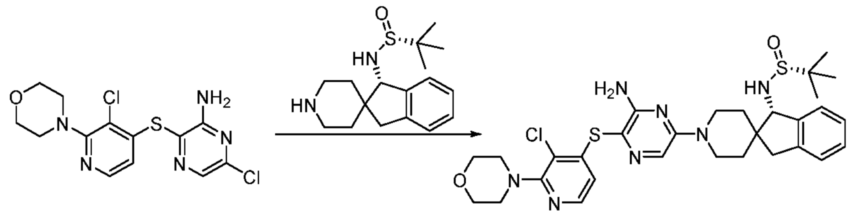

6-Chloro-3-((3-chloro-2-morpholinopyridin-4-yl)thio)pyrazin-2-amine (60 mg, 0.22 mmol), (R)-N-((S)-1,3-dihydrospiro[indene-2,4'-piperidin]-1-yl)-2-methylpropane-2-sulfinamide (76 mg, 0.25 mmol) and potassium carbonate (231 mg, 1.67 mmol) were dissolved in DMF (5 mL), and the reaction solution was stirred under a nitrogen atmosphere at 100°C under microwave for 2 hours. Water was added, and the reaction solution was extract with ethyl acetate. The organic phase was dried over anhydrous sodium sulfate, and concentrated to dryness to obtain an oil, which was used directly in the next step.

MS m/z (ESI): 628.2[M+H]$^+$.

Step 5: Preparation of (S)-1'-(6-amino-5-((3-chloro-2-morpholinopyridin-4-yl)thio)pyrazin-2-yl)-1,3-dihydrospiro[indene-2,4'-piperidin]-1-amine

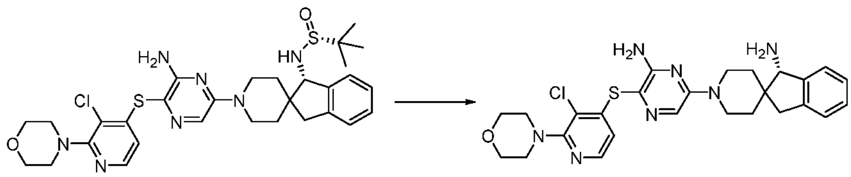

(R)-N-((S)-1'-(6-amino-5-((3-chloro-2-morpholinopyridin-4-yl)thio)pyrazin-2-yl)-1,3-dihydrospiro[indene-2,4'-piperidin]-1-yl)-2-methylpropane-2-sulfinamide (obtained in the previous step) was dissolved in methanol (5 mL). 4M HCl/1,4-dioxane (5 mL) was added, and the reaction solution was stirred at room temperature for 1 hour. The reaction solution was concentrated to dryness, and the resulting residue was purified by column chromatography to obtain the target product (S)-1'-(6-amino-5-((3-chloro-2-morpholinopyridin-4-yl)thio)pyrazin-2-yl)-1,3-dihydrospiro[indene-2,4'-piperidin]-1-amine (6 mg, yield of two steps: 5%).

MS m/z (ESI): 524.1[M+H]$^+$.

**Example 12**

Preparation of (S)-1'-(6-amino-5-((3-chloro-2-(3,3-difluoroazetidin-1-yl)pyridin-4-yl)thio)pyrazin-2-yl)-1,3-dihydrospiro[indene-2,4'-piperidin]-1-amine

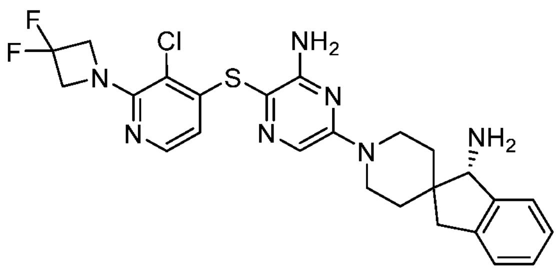

The compound of Example 12 was prepared by referring to the experimental scheme of Example 11.

MS m/z (ESI): 530.1 [M+H]$^+$, 532.1[M+2+H]$^+$.

**Example 13**

Preparation of (S)- 1' -(6-amino-5-((3-chloro-2-(pyrrolidin-1 -yl)pyridin-4-yl)thio)pyrazin-2-yl)-1,3-dihydrospiro[indene-2,4'-piperidin]-1-amine

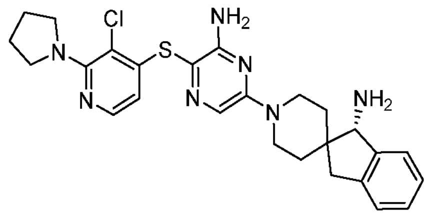

The compound of Example 13 was prepared by referring to the experimental scheme of Example 11.
MS m/z (ESI): 508.1 $[M+H]^+$, 510.1$[M+2+H]^+$.

**Example 14**

Preparation of (S)-1'-(6-amino-5-((2-(azetidin-1-yl)-3-chloropyridin-4-yl)thio)pyrazin-2-yl)-4,6-dihydrospiro[cyclopenta[d]oxazole-5,4'-piperidin]-6-amine

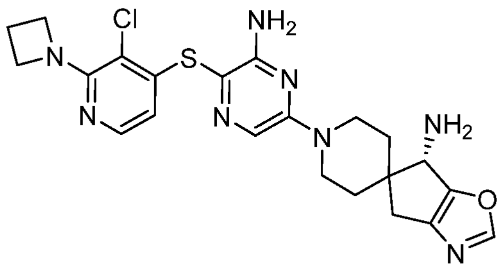

The compound of Example 14 was prepared by referring to the experimental scheme of Example 11.
MS m/z (ESI): 508.1 $[M+H]^+$, 510.1$[M+2+H]^+$.

**Example 15**

Preparation of (S)-1'-(6-amino-5-((2-(azetidin-1-yl)-3-chloropyridin-4-yl)thio)pyrazin-2-yl)-1-methyl-4,6-dihydro-1H-spiro[cyclopenta[d]imidazole-5,4'-piperidin] -6-amine

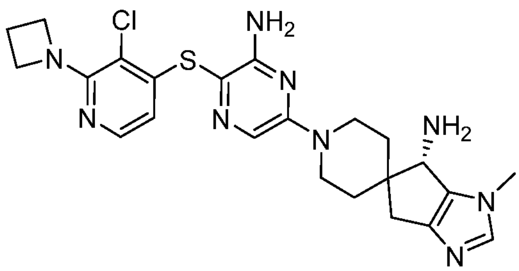

The compound of Example 15 was prepared by referring to the experimental scheme of Example 11.
MS m/z (ESI): 498.1 $[M+H]^+$, 500.1$[M+2+H]^+$.

**Example 16**

Preparation of (S)-1'-(6-amino-5-((2-(azetidin-1-yl)-3-chloropyridin-4-yl)thio)pyrazin-2-yl)-2-methyl-2,6-dihydro-4H-spiro[cyclopenta[c]pyrazole-5,4'-piperidin] -4-amine

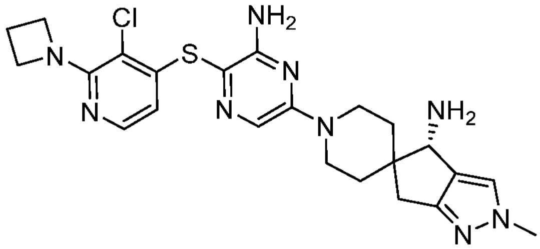

The compound of Example 16 was prepared by referring to the experimental scheme of Example 11.
MS m/z (ESI): 498.1 $[M+H]^+$, 500.1$[M+2+H]^+$.

**Example 17**

Preparation of (S)-1'-(6-amino-5-((3-chloro-2-morpholinopyridin-4-yl)thio)-3-methylpyrazin-2-yl)-1,3-dihydrospiro[indene-2,4'-piperidin]-1-amine

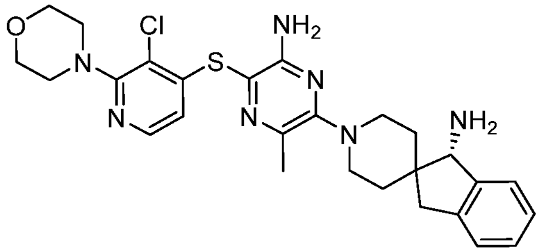

The compound of Example 17 was prepared by referring to the experimental scheme of Example 6.
MS m/z (ESI): 538.1 $[M+H]^+$, 540.1$[M+2+H]^+$.

**Example 18**

Preparation of (S)-1'-(6-amino-5-((3-chloro-2-(pyrrolidin-1-yl)pyridin-4-yl)thio)-3-methylpyrazin-2-yl)-1,3-dihydrospiro[indene-2,4'-piperidin]-1-amine

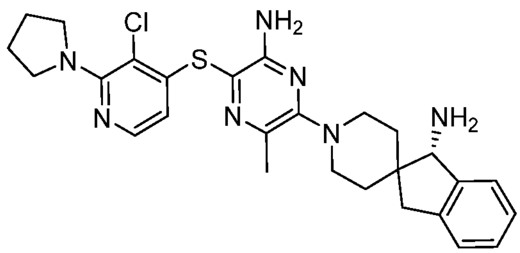

The compound of Example 18 was prepared by referring to the experimental scheme of Example 6.
MS m/z (ESI): 522.1 $[M+H]^+$, 524.1$[M+2+H]^+$.

**Example 19**

Preparation of (S)-1'-(6-amino-5-((3-chloro-2-(pyrrolidin-1-yl)pyridin-4-yl)thio)-3-methylpyrazin-2-yl)-4,6-dihydrospiro[cyclopenta[d]thiazole-5,4'-piperidin]-6-amine

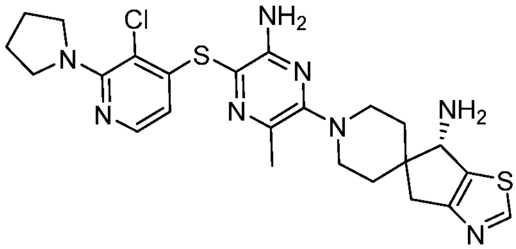

The compound of Example 19 was prepared by referring to the experimental scheme of Example 6.
MS m/z (ESI): 529.1 $[M+H]^+$, 531.1$[M+2+H]^+$.

**Example 20**

Preparation of (S)-1'-(6-amino-5-((3-chloro-2-(pyrrolidin-1-yl)pyridin-4-yl)thio)-3-methylpyrazin-2-yl)-4,6-dihydrospiro[cyclopenta[d]oxazole-5,4'-piperidin]-6-amine

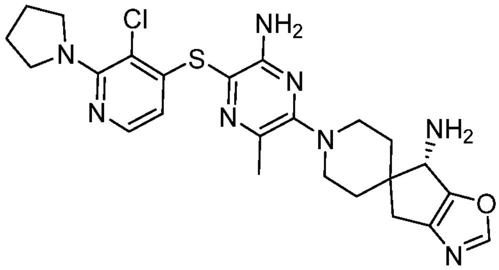

The compound of Example 20 was prepared by referring to the experimental scheme of Example 6.
MS m/z (ESI): 513.1 $[M+H]^+$, 515.1$[M+2+H]^+$.

**Example 21**

Preparation of (S)-1'-(6-amino-5-((3-chloro-2-(pyrrolidin-1-yl)pyridin-4-yl)thio)-3-methylpyrazin-2-yl)-1-methyl-4,6-dihydro-1H-spiro [cyclopenta[d]imidazole-5,4'-piperidin] -6-amine

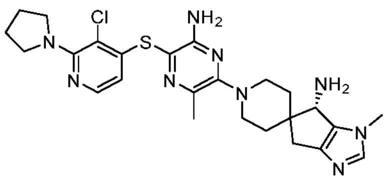

The compound of Example 21 was prepared by referring to the experimental scheme of Example 6.
MS m/z (ESI): 526.1 $[M+H]^+$, 528.1$[M+2+H]^+$.

**Example 22**

Preparation of (S)-1'-(6-amino-5-((3-chloro-2-(pyrrolidin-1-yl)pyridin-4-yl)thio)-3-methylpyrazin-2-yl)-2-methyl-2,6-dihydro-4H-spiro[cyclopenta[c]pyrazole-5,4'-piperidin] -4-amine

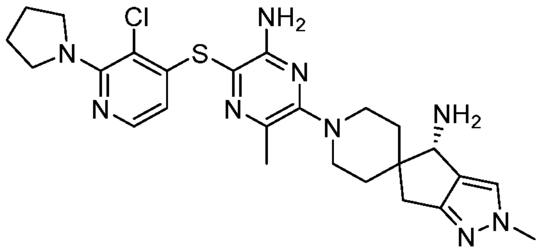

The compound of Example 22 was prepared by referring to the experimental scheme of Example 6.
MS m/z (ESI): 526.1 $[M+H]^+$, 528.1$[M+2+H]^+$.

**Example 23**

Preparation of (S)-1'-(6-amino-5-((3-chloro-2-morpholinopyridin-4-yl)thio)-3-methylpyrazin-2-yl)-4,6-dihydrospiro[cyclopenta[d]oxazole-5,4'-piperidin]-6-amine

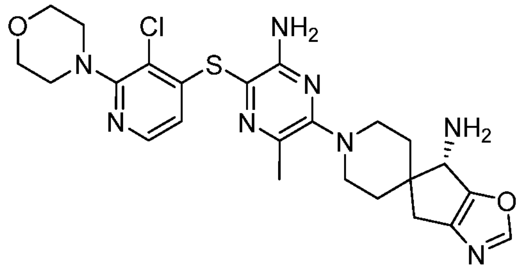

The compound of Example 23 was prepared by referring to the experimental scheme of Example 6.
MS m/z (ESI): 529.1 $[M+H]^+$, 531.1$[M+2+H]^+$.

**Example 24**

Preparation of (S)-1'-(6-amino-5-((3-chloro-2-morpholinopyridin-4-yl)thio)-3-methylpyrazin-2-yl)-2-methyl-2,6-dihydro-4H-spiro[cyclopenta[c]pyrazole-5,4'-piperidin] -4-amine

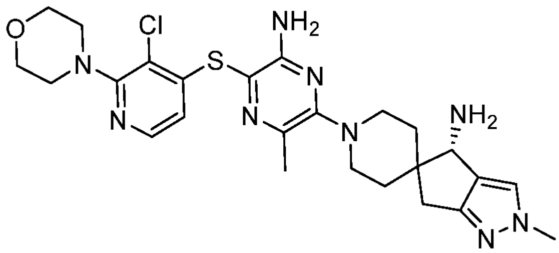

The compound of Example 24 was prepared by referring to the experimental scheme of Example 6.
MS m/z (ESI): 542.1 $[M+H]^+$, 544.1$[M+2+H]^+$.

**Example 25**

Preparation of (S)-1'-(6-amino-5-((3-chloro-2-cyclopropylpyridin-4-yl)thio)-3-methylpyrazin-2-yl)-4,6-dihydrospiro[cyclopenta[d]oxazole-5,4'-piperidin]-6-amine

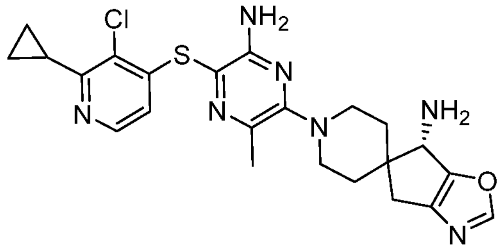

The compound of Example 25 was prepared by referring to the experimental scheme of Example 6.
MS m/z (ESI): 484.1 $[M+H]^+$, 486.1$[M+2+H]^+$.

**Example 26**

Preparation of (S)-1'-(6-amino-5-((3-chloro-2-cyclopropylpyridin-4-yl)thio)pyrazin-2-yl)-4,6-dihydrospiro[cyclopenta[d]oxazole-5,4'-piperidin]-6-amine

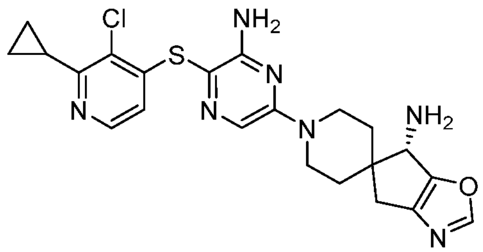

The compound of Example 26 was prepared by referring to the experimental scheme of Example 1.
MS m/z (ESI): 470.1 $[M+H]^+$, 472.1$[M+2+H]^+$.

**Example 27**

Preparation of (S)-1'-(6-amino-5-((2-cyclopropylpyridin-4-yl)thio)pyrazin-2-yl)-4,6-dihydrospiro-[cyclopenta[d]oxazole-5,4'-piperidin]-6-amine

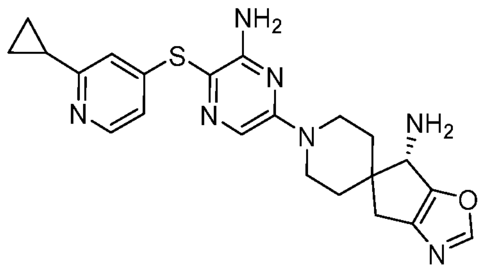

The compound of Example 27 was prepared by referring to the experimental scheme of Example 1.
MS m/z (ESI): 436.1 $[M+H]^+$.

**Example 28**

Preparation of (S)-1'-(6-amino-5-((2-cyclopropyl-3-methylpyridin-4-yl)thio)-pyrazin-2-yl)-4,6-dihydrospiro[cyclopenta[d]oxazole-5,4'-piperidin]-6-amine

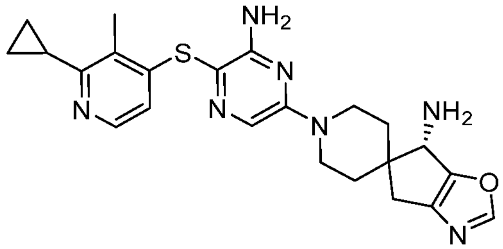

The compound of Example 28 was prepared by referring to the experimental scheme of Example 1.

MS m/z (ESI): 450.1 $[M+H]^+$.

**Example 29**

Preparation of (S)-1'-(6-amino-5-((2-cyclopropyl-3-fluoropyridin-4-yl)thio)-pyrazin-2-yl)-4,6-dihydrospiro[cyclopenta[d]oxazole-5,4'-piperidin]-6-amine

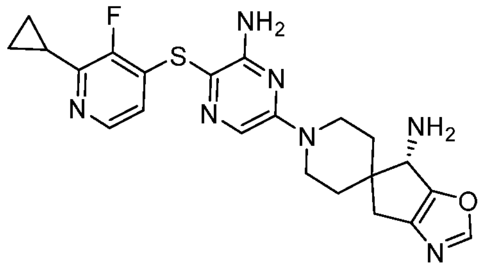

The compound of Example 29 was prepared by referring to the experimental scheme of Example 1.

MS m/z (ESI): 454.1 $[M+H]^+$.

**Example 30**

Preparation of (S)-1-(4-((3-amino-5-(1-amino-1,3-dihydrospiro[indene-2,4'-piperidin]-1'-yl)-pyrazin-2-yl)thio)-3-chloropyridin-2-yl)azetidin-2-one

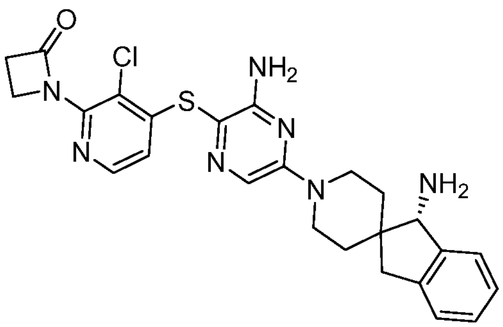

The compound of Example 30 was prepared by referring to the experimental scheme of Example 1.

MS m/z (ESI): 508.1 $[M+H]^+$, 510.1$[M+2+H]^+$.

**Example 31**

Preparation of (S)-1-(4-((3-amino-5-(1-amino-1,3-dihydrospiro[indene-2,4'-piperidin]-1'-yl)pyrazin-2-yl)thio)-3-chloropyridin-2-yl)pyrrolidin-2-one

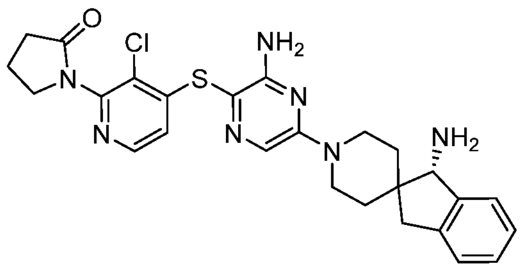

The compound of Example 31 was prepared by referring to the experimental scheme of Example 1.

MS m/z (ESI): 522.1 $[M+H]^+$, 524.1$[M+2+H]^+$.

**Example 32**

Preparation of (S)-1-(3-(1-amino-1,3-dihydrospiro[indene-2,4'-piperidin]-1'-yl)-6-((2-cyclopropyl-pyridin-4-yl)thio)-5-methylpyrazin-2-yl)cyclopropan-1-ol

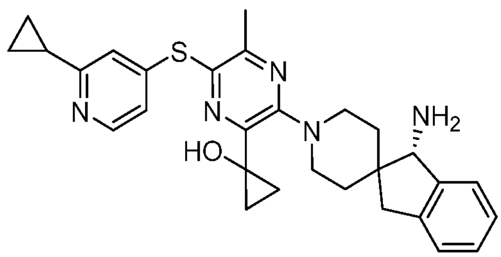

Step 1: Preparation of (S)-1-(3-(1-amino-1,3-dihydrospiro[indene-2,4'-piperidin]-1'-yl)-6-((2-cyclopropylpyridin-4-yl)thio)-5-methylpyrazin-2-yl)cyclopropan-1-ol

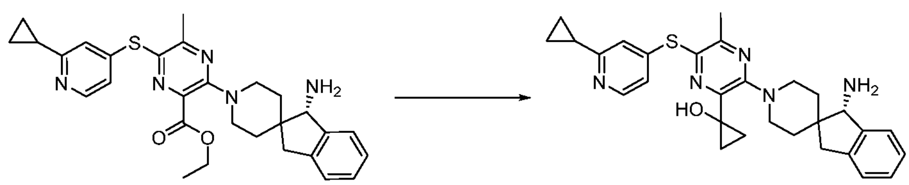

Ethylmagnesium bromide (0.1 mL, 0.30 mmol, 3M) and tetraisopropyl titanate (43 mg, 0.15 mmol) were added dropwise to a solution of ethyl (S)-3-(1-amino-1,3-dihydrospiro[indene-2,4'-piperidin]-1'-yl)-6-(2-cyclopropylpyridin-4-yl)-5-methylpyrazine-2-carboxylate (50 mg, 0.10 mmol) in THF (10 mL) at 0° C. The reaction solution was warmed up to room temperature and stirred for 5 hours. Saturated $NH_4Cl$ aqueous solution was added to quench the reaction, and the reaction solution was extracted with dichloromethane (3*20 mL). The organic phase was dried over anhydrous sodium sulfate, concentrated and purified by preparative HPLC to obtain (S)-1-(3-(1-amino-1,3-dihydrospiro[indene-2,4'-piperidin]-1'-yl)-6-((2-cyclopropyl-pyridin-4-yl)-5-methylpyrazin-2-yl)cyclopropan-1-ol (5 mg, yield: 10%) as a white solid.

MS m/z (ESI): 500.1 $[M+H]^+$.

**Example 33**

Preparation of

(S)-1-(3-(1-amino-1,3-dihydrospiro[indene-2,4'-piperidin]-1'-yl)-6-((3-chloro-2-cyclopropylpyridin-4-yl)thio)-5-methylpyrazin-2-yl)cyclopropan-1-ol

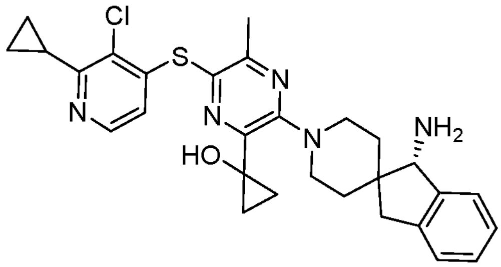

The compound of Example 33 was prepared by referring to the experimental scheme of Example 32.

MS m/z (ESI): 534.1 $[M+H]^+$, 536.1$[M+2+H]^+$.

**Example 34**

Preparation of (S)-1-(3-(1-amino-1,3-dihydrospiro[indene-2,4'-piperidin]-1'-yl)-6-((2-amino-3-chloropyridin-4-yl)thio)-5-methylpyrazin-2-yl)cyclopropan-1-ol

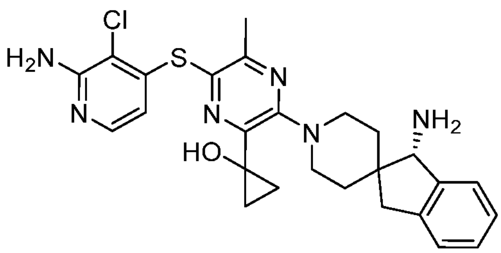

The compound of Example 34 was prepared by referring to the experimental scheme of Example 32.

MS m/z (ESI): 509.1 $[M+H]^+$, 511.1$[M+2+H]^+$.

**Example 35**

Preparation of (S)-8-(6-amino-5-((2-amino-3-chloropyridin-4-yl)thio)pyrazin-2-yl)-2-cyclopropyl-8-azaspiro[4.5]dec-2-en-1-amine

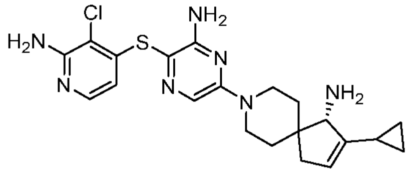

Step 1: Preparation of *tert*-butyl 4-allyl-4-formylpiperidine-1-carboxylate

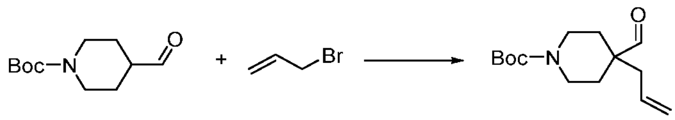

Lithium *tert*-butoxide (13.5 g, 16.9 mmol) was added to a solution of *tert*-butyl 4-formylpiperidine-1-carboxylate (30 g, 14.1 mmol) in N,N-dimethylformamide (300 mL) at 0°C, and the solution was stirred for 30 minutes after completion of the addition. Allyl bromide (19 g, 16.2 mmol) was added, and the reaction solution was stirred at 0°C for 2 hours after completion of the addition. The reaction solution was poured into aqueous ammonium chloride solution (1 L), and extracted with ethyl acetate (1 L×2). The ethyl acetate layer was washed with saturated sodium chloride aqueous solution (500 mL), dried over anhydrous sodium sulfate, and purified by column chromatography [eluent: petroleum ether ~ ethyl acetate/petroleum ether from 0% to 2%] to obtain the product *tert*-butyl 4-allyl-4-formylpiperidine-1-carboxylate (15 g, yield: 42%) as a colorless oil.

Step 2: Preparation of *tert*-butyl 4-allyl-4-(1-hydroxyallyl)piperidine-1-carboxylate

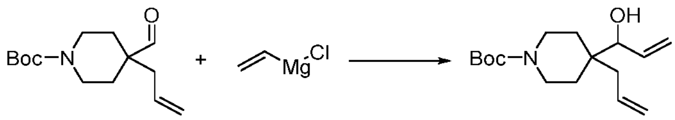

Vinyl magnesium chloride (65 mL, 123.5 mmol) was added dropwise to a solution of *tert*-butyl 4-allyl-4-formylpiperidine-1-carboxylate (25 g, 99 mmol) in tetrahydrofuran (300 mL) at -78°C. After completion of the addition, the reaction solution was slowly warmed up to room temperature and stirred for 30 minutes. The reaction solution was poured into aqueous ammonium chloride solution (1 L), and extracted with ethyl acetate (1 L×2).The ethyl acetate layer was washed with saturated sodium chloride aqueous solution (500 mL), dried over anhydrous sodium sulfate, and purified by column chromatography [eluent: petroleum ether ~ ethyl acetate/petroleum ether from 0% to 5%] to obtain the product *tert*-butyl 4-allyl-4-(1-hydroxyallyl)piperidine-1-carboxylate (25 g, yield: 90%) as a colorless oil.

Step 3: Preparation of *tert*-butyl 4-acryloyl-4-allylpiperidine-1-carboxylate

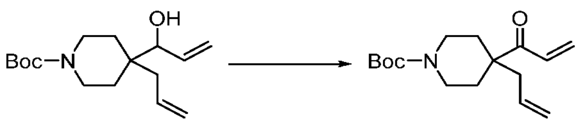

Dess-Martin reagent (41.5 g, 98 mmol) was added to a solution of tert-butyl 4-allyl-4-(1-hydroxyallyl)piperidine-1-carboxylate (25 g, 89 mmol) in dichloromethane (400 mL) at 0°C. After completion of the addition, the reaction solution was stirred at 40°C for 1 hour. The reaction solution was slowly poured into sodium bicarbonate/sodium sulfite aqueous solution (1/1, 1 L), and extracted with dichloromethane (1 L×2). The dichloromethane layer was washed with saturated sodium chloride aqueous solution (500 mL), dried over anhydrous sodium sulfate, and concentrated to dryness by rotary evaporation. *N*-heptane (200 mL) was added, and the solution was stirred for 5 minutes. The solution was filtered to remove insoluble substance, and the filtrate was concentrated to obtain the product *tert*-butyl 4-acryloyl-4-allylpiperidine-1-carboxylate (24.8 g, yield: 100%) as a colorless oil, which was rapidly used in the next step.

Step 4: Preparation of *tert*-butyl 1-oxo-8-azaspiro[4.5]dec-2-ene-8-carboxylate

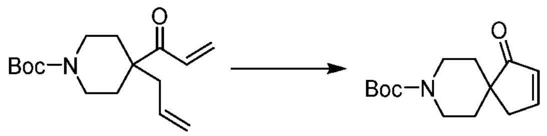

*Tert*-butyl 4-acryloyl-4-allylpiperidine-1-carboxylate (24.8 g, 89 mmol) and dichloro(2-isopropoxyphenylmethylene)(tricyclohexylphosphine)ruthenium(II) (1.2 g, 1.4 mmol) were stirred in toluene (700 mL) at 90°C for 2 hours. The reaction solution was cooled, concentrated and purified by column chromatography [eluent: petroleum ether ~ ethyl acetate/petroleum ether from 0% to 20%] to obtain the product *tert*-butyl 1-oxo-8-azaspiro[4.5]dec-2-ene-8-carboxylate (13 g, yield: 58%) as a reddish black solid.

MS m/z (ESI): 252.1 $[M+H]^+$.

Step 5: Preparation of *tert*-butyl 2-bromo-1-oxo-8-azaspiro[4.5]dec-2-ene-8-carboxylate

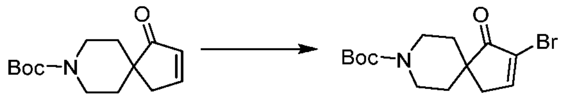

Pyridine N-oxide (11 g, 116 mmol) was added to a solution of *tert*-butyl 1-oxo-8-azaspiro[4.5]dec-2-ene-8-carboxylate (13 g, 51.8 mmol) in acetonitrile (300 mL), followed by the addition of N-bromosuccinimide (28 g, 157 mmol). After completion of the addition, the reaction solution was stirred at 80°C for 18 hours. The reaction solution was cooled, concentrated and purified by column chromatography [eluent: petroleum ether ~ ethyl acetate/petroleum ether from 0% to 20%] to obtain the product *tert*-butyl 2-bromo-1-oxo-8-azaspiro[4.5]dec-2-ene-8-carboxylate (4.2 g, yield: 25%) as a yellow solid.

MS m/z (ESI): 330.1 $[M+H]^+$, 332.1 $[M+2+H]^+$.

Step 6: Preparation of *tert-butyl* 2-cyclopropyl-1-oxo-8-azaspiro[4.5]dec-2-ene-8-carboxylate

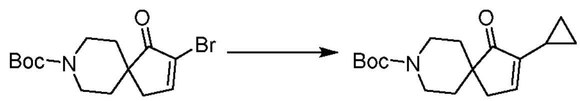

*Tert*-butyl 2-bromo-1-oxo-8-azaspiro[4.5]dec-2-ene-8-carboxylate (4.2 g, 12.8 mmol), potassium cyclopropyl trifluoroborate (3.79 g, 25.6 mmol), cesium carbonate (12.5 g, 38.4 mmol) and [1,1'-bis(diphenylphosphino)ferrocene]dichloropalladium dichloromethane complex (731 mg, 0.9 mmol) were stirred in dioxane (100 mL) and water (10 mL) at 100°C for 18 hours. Water (150 mL) was added to quench the reaction, and the reaction solution was extracted with ethyl acetate (150 mL×2). The ethyl acetate layer was washed with saturated sodium chloride aqueous solution (50 mL), dried over anhydrous sodium sulfate, and purified by column chromatography [eluent: petroleum ether ~ ethyl acetate/petroleum ether from 0% to 12%] to obtain the product *tert*-butyl 2-cyclopropyl-1-oxo-8-azaspiro[4.5]dec-2-ene-8-carboxylate (1 g, yield: 27%) as a yellow oil.

MS m/z (ESI): 292.2 $[M+H]^+$

Step 7: Preparation of *tert*-butyl (R,Z)-1-((*tert*-butylsulfinyl<sulfenyl>)imino)-2-cyclopropyl-8-azaspiro[4.5]dec-2-ene-8-carboxylate

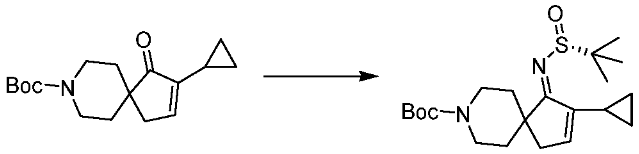

*Tert*-butyl 2-cyclopropyl-1-oxo-8-azaspiro[4.5]dec-2-ene-8-carboxylate (1 g, 3.4 mmol) and (R)-(+)-*tert*-butylsulfinamide (2 g, 17.2 mmol) were stirred in tetraethyl titanate (30 mL) at 100°C for 18 hours. The reaction solution was cooled, and then ethyl acetate (100 mL) was added. The reaction solution was poured into water (150 mL) to precipitate a large amount of white solid. The reaction solution was filtered through diatomaceous earth, and the filtrate was separated into two phases. The organic phase was concentrated to dryness by rotary evaporation, and the resulting residue was purified by column chromatography [eluent: petroleum ether ~ ethyl acetate/petroleum ether from 0% to 50%] obtain the product *tert-butyl* (R,Z)-1-((*tert*-butylsulfinyl<sulfenyl>)imino)-2-cyclopropyl-8-azaspiro[4.5]dec-2-ene-8-carboxylate (190 mg, yield: 14%) as a yellow oil.

MS m/z (ESI): 395.2 $[M+H]^+$.

Step 8: Preparation of *tert*-butyl (S)-1-(((R)-*tert*-butylsulfinyl<sulfenyl>)amino)-2-cyclopropyl-8-azaspiro[4.5]dec-2-ene-8-carboxylate

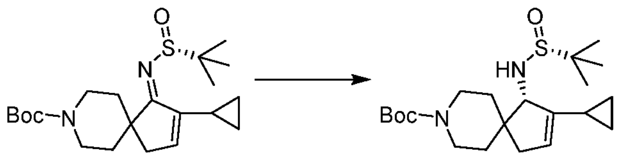

Diisobutyl aluminum hydride (0.9 mL, 1 mmol) was added dropwise to a solution of *tert*-butyl (R,Z)-1-((*tert*-butylsulfinyl<sulfenyl>)imino)-2-cyclopropyl-8-azaspiro[4.5]dec-2-ene-8-carboxylate (210 mg, 0.53 mmol) in tetrahydrofuran (10 mL) at -78°C. After completion of the addition, the reaction solution was stirred for 15 minutes. Sodium sulfate decahydrate was added at -78°C to quench the reaction, and the reaction solution was stirred for 10 minutes. The reaction solution was filtered, and the filtrate was concentrated and purified by column chromatography [eluent: petroleum ether ~ ethyl acetate/petroleum ether from 0% to 5%] to obtain the product *tert*-butyl (S)-1-(((R)-*tert*-butylsulfinyl<sulfenyl>)amino)-2-cyclopropyl-8-azaspiro[4.5]dec-2-ene-8-carboxylate (105 mg, yield: 50%) as a colorless oil.

MS m/z (ESI): 397.2 $[M+H]^+$.

Step 9: Preparation of (S)-2-cyclopropyl-8-azaspiro[4.5]dec-2-en-1-amine

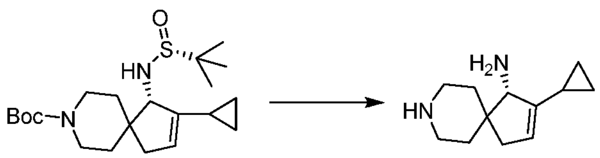

Hydrochloric acid in dioxane (15 mL, 60 mmol) was added to a solution of *tert*-butyl (S)-1-(((R)-*tert*-butylsulfinyl<sulfenyl>)amino)-2-cyclopropyl-8-azaspiro[4.5]dec-2-ene-8-carboxylate (105 mg, 0.27 mmol) in methanol (3 mL). After completion of the addition, the reaction solution was stirred at room temperature for 1 hour. The reaction solution was concentrated to dryness by rotary evaporation to obtain the product (S)-2-cyclopropyl-8-azaspiro[4.5]dec-2-en-1-amine(105 mg, yield: 100%) as a white solid.

MS m/z (ESI): 193.2 $[M+H]^+$.

Step 10: Preparation of (S)-8-(6-amino-5-((2-amino-3-chloropyridin-4-yl)thio)-pyrazin-2-yl)-2-cyclopropyl-8-azaspiro[4.5]dec-2-en-1-amine

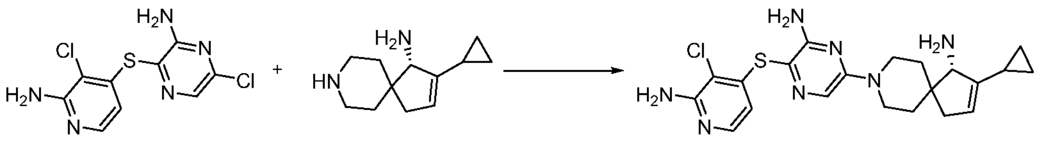

3-((2-Amino-3-chloropyridin-4-yl)thio)-6-chloropyrazin-2-amine (76 mg, 0.26 mmol), (S)-2-cyclopropyl-8-azaspiro[4.5]dec-2-en-1-amine (80 mg, 0.2 mmol) and potassium carbonate (167 mg, 1.2 mmol) were stirred in N,N-dimethylformamide (8 mL) at 100°C for 7 hours. Water (60 mL) was added, and the reaction solution was extracted with ethyl acetate (50 mL×2). The ethyl acetate layer was washed with saturated sodium chloride aqueous solution (20 mL), dried over anhydrous sodium sulfate, and purified by thin layer chromatography [eluent: dichloromethane/methanol=7/1] to obtain the product (S)-8-(6-amino-5-((2-amino-3-chloropyridin-4-yl)thio)pyrazin-2-yl)-2-cyclopropyl-8-azaspiro[4.5]dec-2-en-1-amine (37 mg, yield: 41%) as a yellow oil.

$^{1}$H NMR (400 MHz, MeOD) δ 7.67 - 7.51 (m, 2H), 5.92 (d, *J*= 8 Hz, 1H), 5.27 (s, 1H), 4.26 - 4.04 (m, 2H), 3.33 (s, 1H), 3.27 - 3.08 (m, 2H), 2.44 - 2.18 (m, 2H), 1.81 - 1.69 (m, 1H), 1.68 - 1.47 (m, 2H), 1.42 - 1.24 (m, 2H), 0.81 - 0.60 (m, 2H), 0.57 - 0.28 (m, 2H).

MS m/z (ESI): 444.1 [M+H]$^+$, 446.1 [M+2+H]$^+$.

**Example 36**

Preparation of (S)-8-(5-((2-amino-3-chloropyridin-4-yl)thio)pyrazin-2-yl)-2-cyclopropyl-8-azaspiro[4.5]dec-2-en-1-amine

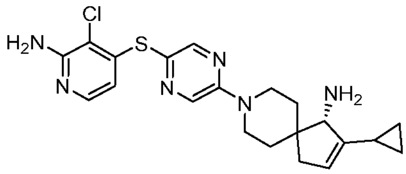

Step 1: Preparation of 3-chloro-4-((5-chloropyrazin-2-yl)thio)pyridin-2-amine

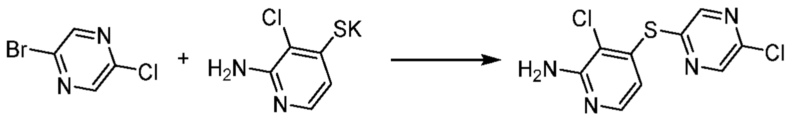

Potassium 2-amino-3-chloropyridine-4-thiolate (1 g, 2.64 mmol), 2-bromo-5-chloropyrazine (597 mg, 3.11 mmol), tris(dibenzylideneacetone)dipalladium (284 mg, 0.311 mmol), chloro[(4,5-bis(diphenylphosphino)-9,9-dimethylxanthene)-2-(2'-amino-1,1'-biphenyl)]palladium(II) (360 mg, 0.622 mmol) and N,N-diisopropylethylamine (1.2 g, 9.33 mmol) were stirred in N,N-dimethylformamide (10 mL) at 90°C under microwave for 1 hour. The reaction solution was concentrated to dryness by rotary evaporation, and the resulting residue was purified by column chromatography [eluent: petroleum ether ~ ethyl acetate/petroleum ether from 0% to 30%] to obtain the product 3-chloro-4-((5-chloropyrazin-2-yl)thio)pyridin-2-amine (250 mg, yield: 35%) as a white solid.

MS m/z (ESI): 273.0 [M+H]$^+$, 275.0 [M+2+H]$^+$.

Step 2: Preparation of (S)-8-(5-((2-amino-3-chloropyridin-4-yl)thio)pyrazin-2-yl)-2-cyclopropyl-8-azaspiro[4.5]dec-2-en-1-amine

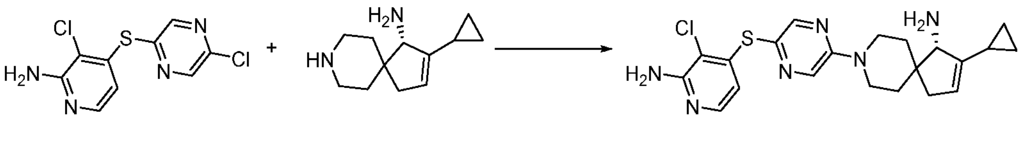

3-Chloro-4-((5-chloropyrazin-2-yl)thio)pyridin-2-amine (100 mg, 0.368 mmol), (S)-2-cyclopropyl-8-azaspiro[4.5]dec-2-en-1-amine hydrochloride (105 mg, 0.265 mmol) and potassium carbonate (254 mg, 1.84 mmol) were stirred in N,N-dimethylformamide (8 mL) at 100°C for 6 hours. Water (40 mL) was added, and the reaction solution was extracted with ethyl acetate (40 mL*2). The organic phase was concentrated, and purified by thin layer chromatography (dichloromethane/methanol=10/1) to obtain the product (S)-8-(5-((2-amino-3-chloropyridin-4-yl)thio)pyrazin-2-yl)-2-cyclopropyl-8-azaspiro[4.5]dec-2-en-1-amine (40 mg, yield: 35%) as a yellow solid.

$^1$H NMR (400 MHz, MeOD) δ 8.29 (d, *J* = 16 Hz, 2H), 7.60 (d, *J* = 4 Hz, 1H), 5.93 (d, *J* = 4 Hz, 1H), 5.31 (s, 1H), 4.34 - 4.14 (m, 2H), 3.43 - 3.35 (m, 2H), 3.28 - 3.21 (m, 1H), 2.45 - 2.21 (m, 2H), 1.86 - 1.49 (m, 3H), 1.44 - 1.22 (m, 2H), 0.82 - 0.61 (m, 2H), 0.58 - 0.30 (m, 2H).

MS m/z (ESI): 429.1 $[M+H]^+$, 431.1 $[M+2+H]^+$.

**Example 37**

Preparation of (S)-8-(6-amino-5-((2-amino-3-chloropyridin-4-yl)thio)-3-methylpyrazin-2-yl)-2-methyl-8-azaspiro[4.5]dec-2-en-1-amine

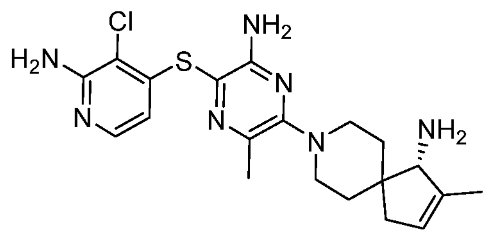

The compound of Example 37 was prepared by referring to the experimental scheme of Example 6.

MS m/z (ESI): 432.1 $[M+H]^+$, 434.1$[M+2+H]^+$.

**Example 38**

Preparation of (S)-8-(6-amino-5-((2-amino-3-chloropyridin-4-yl)thio)-3-methylpyrazin-2-yl)-2-ethyl-8-azaspiro[4.5]dec-2-en-1-amine

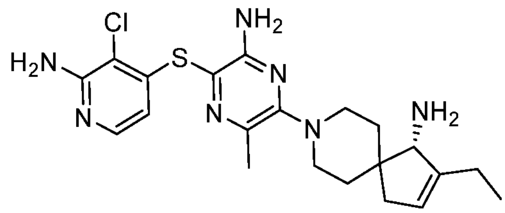

The compound of Example 38 was prepared by referring to the experimental scheme of Example 6.

MS m/z (ESI): 446.1 $[M+H]^+$, 448.1$[M+2+H]^+$.

**Example 39**

Preparation of (S)-8-(6-amino-5-((2-amino-3-chloropyridin-4-yl)thio)-3-methylpyrazin-2-yl)-2-isopropyl-8-azaspiro[4.5]dec-2-en-1-amine

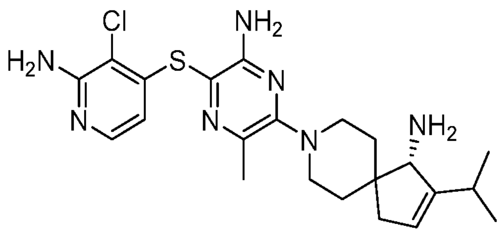

The compound of Example 39 was prepared by referring to the experimental scheme of Example 6.
MS m/z (ESI): 460.1 $[M+H]^+$, 462.1$[M+2+H]^+$.

**Example 40**

Preparation of (S)-8-(6-amino-5-((2-amino-3-chloropyridin-4-yl)thio)-3-methylpyrazin-2-yl)-2-cyclopropyl-8-azaspiro[4.5]dec-2-en-1-amine

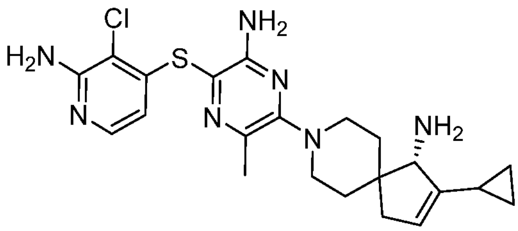

The compound of Example 40 was prepared by referring to the experimental scheme of Example 6.
MS m/z (ESI): 458.1 $[M+H]^+$, 460.1$[M+2+H]^+$.

**Example 41**

Preparation of (S)-1-(6-((2-amino-3-chloropyridin-4-yl)thio)-3-(6-amino-4,6-dihydrospiro[cyclopenta-[d]oxazole-5,4'-piperidin]-1'-yl)-5-methylpyrazin-2-yl)cyclopropan-1-ol

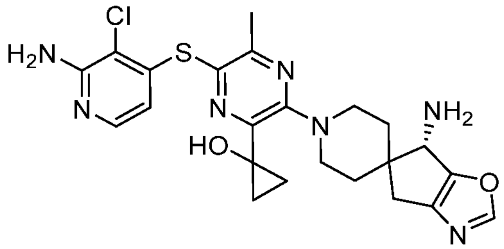

The compound of Example 41 was prepared by referring to the experimental scheme of Example 32.
MS m/z (ESI): 500.1 $[M+H]^+$, 502.1$[M+2+H]^+$.

**Example 42**

Preparation of (R)-1-(6-((2-amino-3-chloropyridin-4-yl)thio)-3-(1-amino-8-azaspiro[4.5]dec-2-en-8-yl)-5-methylpyrazin-2-yl)cyclopropan-1-ol

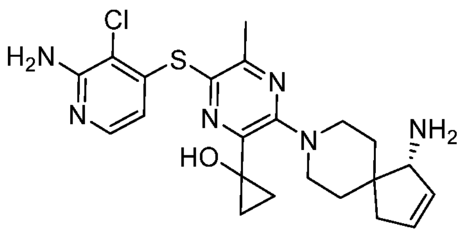

The compound of Example 42 was prepared by referring to the experimental scheme of Example 32.
MS m/z (ESI): 459.1 $[M+H]^+$, 461.1$[M+2+H]^+$.

**Example 43**

Preparation of (S)-1-(3-(1-amino-2-methyl-8-azaspiro[4.5]dec-2-en-8-yl)-6-((2-amino-3-chloropyridin-4-yl)thio)-5-methylpyrazin-2-yl)cyclopropan-1-ol

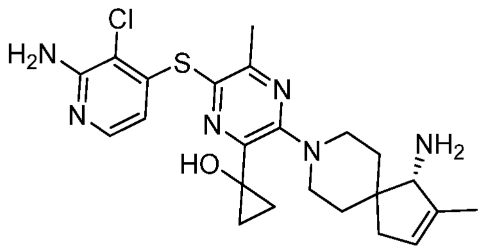

The compound of Example 43 was prepared by referring to the experimental scheme of Example 32.
MS m/z (ESI): 473.1 $[M+H]^+$, 475.1$[M+2+H]^+$.

**Example 44**

Preparation of (S)-1-(3-(1-amino-2-cyclopropyl-8-azaspiro[4.5]dec-2-en-8-yl)-6-((2-amino-3-chloropyridin-4-yl)thio)-5-methylpyrazin-2-yl)cyclopropan-1-ol

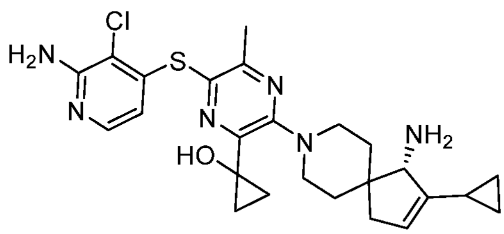

The compound of Example 44 was prepared by referring to the experimental scheme of

Example 32.

MS m/z (ESI): 499.1 $[M+H]^+$, 501.1$[M+2+H]^+$.

**Example 45**

Preparation of (S)-(3-(1-amino-1,3-dihydrospiro[indene-2,4'-piperidin]-1'-yl)-6-((2-amino-3-chloropyridin-4-yl)thio)-5-methylpyrazin-2-yl)methanol

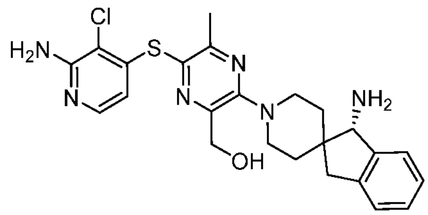

Step 1: Preparation of 2-ethylhexyl 3-((2-amino-3-chloropyridin-4-yl)thio)propanoate

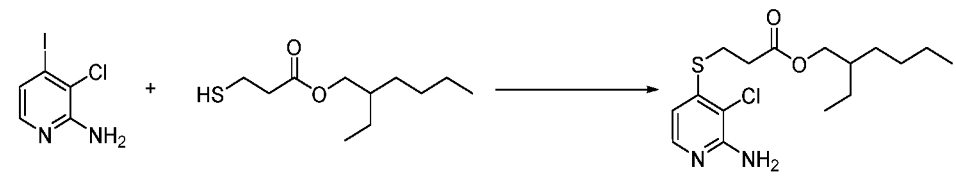

3-Chloro-4-iodopyridin-2-amine (3 g, 11.8 mmol), 2-ethylhexyl 3-mercaptopropanoate (2.9 g, 14.2 mmol), palladium acetate (132 mg, 0.59 mmol), 4,5-bis(diphenylphosphino)-9,9-dimethylxanthene (683 mg, 1.18 mmol) and N,N-diisopropylethylamine (561 mg, 4.3 mmol) were stirred in dioxane (80 mL) at 100°C for 18 hours. The reaction solution was filtered. The filtrate was concentrated, and purified by column chromatography [eluent: petroleum ether ~ petroleum ether/ethyl acetate (60:40)] to obtain 2-ethylhexyl 3-((2-amino-3-chloropyridin-4-yl)thio)propanoate (3.8 g, yield: 94%) as a light yellow solid.

MS m/z (ESI): 345.1 $[M+H]^+$, 347.0 $[M+2+H]^+$.

Step 2: Preparation of potassium 2-amino-3-chloropyridine-4-thiolate

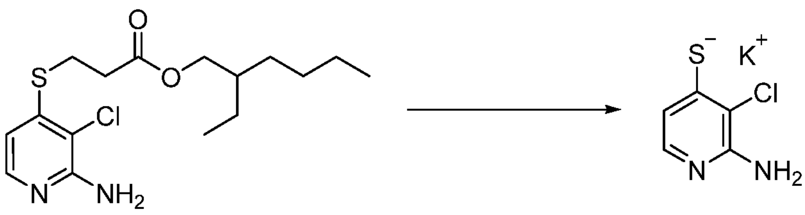

Potassium tert-butoxide (174 mg, 1.53 mmol) was added to a solution of 2-ethylhexyl 3-((2-amino-3-chloropyridin-4-yl)thio)propanoate (500 mg, 1.5 mmol) in ethanol (30 mL). After completion of the addition, the reaction solution was stirred at 40°C for 2 hours. The reaction solution was concentrated to obtain the crude product potassium 2-amino-3-chloropyridine-4-thiolate (500 mg) as a yellow solid, which was used directly in the next step.

MS m/z (ESI): 161.0 $[M+H]^+$, 163.0 $[M+2+H]^+$.

Step 3: Preparation of ethyl 6-bromo-3-((S)-1-(((R)-*tert*-butylsulfinyl<sulfenyl>)amino)-1,3-dihydrospiro-[indene-2,4'-piperidin]-1'-yl)-5-methylpyrazine-2-carboxylate

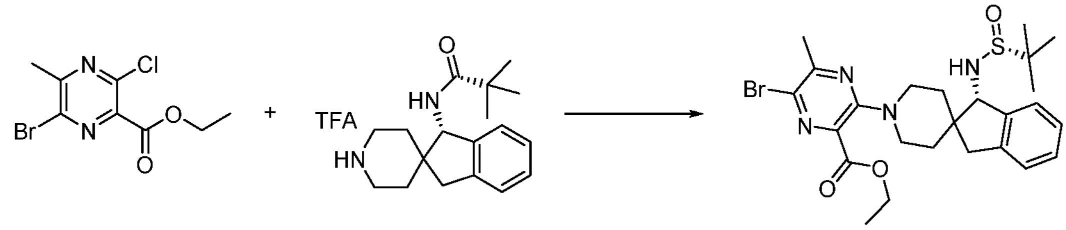

Ethyl 6-bromo-3-chloro-5-methylpyrazine-2-carboxylate (840 mg, 3 mmol), (S)-N-(1,3-dihydrospiro[indene-2,4'-piperidin]-1-yl)pivalamide trifluoroacetate (1.5 g, 3.6 mmol) and potassium carbonate (1.7 g, 12 mmol) were stirred in acetonitrile (40 mL) at 55°C for 18 hours. The reaction solution was filtered. The filtrate was concentrated, and purified by column chromatography [eluent: petroleum ether ~ petroleum ether/ethyl acetate (60:40)] to obtain ethyl 6-bromo-3-((S)-1-(((R)-*tert*-butylsulfinyl<sulfenyl>)amino)-1,3-dihydrospiro[indene-2,4'-piperidin]-1'-yl)-5-methylpyrazine-2-carboxylate (1.5 g, yield: 91%) as a yellow solid.

MS m/z (ESI): 549.2 $[M+H]^+$, 551.2 $[M+2+H]^+$.

Step 4: Preparation of ethyl 6-((2-amino-3-chloropyridin-4-yl)thio)-3-((S)-1-(((R)-*tert*-butylsulfinyl<sulfenyl>)amino)-1,3-dihydrospiro[indene-2,4'-piperidin]-1'-yl)-5-methylpyrazine-2-carboxylate

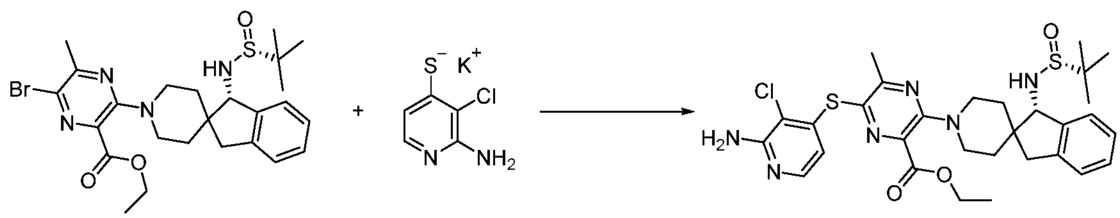

Ethyl 6-bromo-3-((S)-1-(((R)-*tert*-butylsulfinyl<sulfenyl>)amino)-1,3-dihydrospiro-[indene-2,4'-piperidin]-1'-yl)-5-methylpyrazine-2-carboxylate (798 mg, 1.45 mmol), potassium 2-amino-3-chloropyridine-4-thiolate (500 mg, 1.45 mmol, crude), tris(dibenzylideneacetone)dipalladium (66 mg, 0.073 mmol), 4,5-bis(diphenylphosphino)-9,9-dimethylxanthene (84 mg, 0.145 mmol) and N,N-diisopropylethylamine (561 mg, 4.35 mmol) were stirred in N,N-dimethylformamide (15 mL) at 100°C under microwave for 1.5 hours. The reaction solution was filtered. The filtrate was concentrated, and purified by column chromatography [eluent: dichloromethane ~ dichloromethane/methanol (98.5:1.5)] to obtain ethyl 6-((2-amino-3-chloropyridin-4-yl)thio)-3-((S)-1-(((R)-*tert*-butylsulfinyl<sulfenyl>)amino)-1,3-dihydrospiro[indene-2,4'-piperidin]-1'-yl)-5-methylpyrazine-2-carboxylate (500 mg, yield: 55%) as a yellow solid.

MS m/z (ESI): 629.1 $[M+H]^+$, 631.1 $[M+2+H]^+$.

Step 5: Preparation of (R)-N-((S)-1'-(5-((2-amino-3-chloropyridin-4-yl)thio)-3-(hydroxymethyl)-6-methylpyrazin-2-yl)-1,3-dihydrospiro[indene-2,4'-piperidin]-1-yl)-2-methylpropane-2-sulfinamide

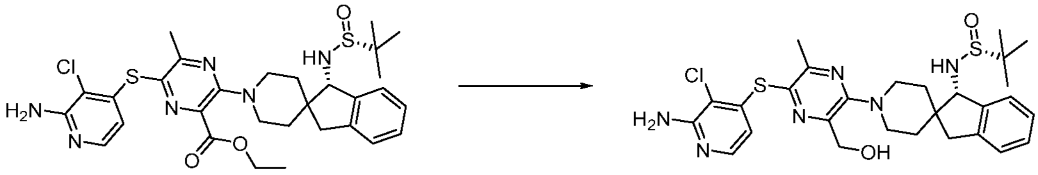

Lithium aluminum hydride (0.7 mL, 2.5 M) was added dropwise to a solution of ethyl 6-((2-amino-3-chloropyridin-4-yl)thio)-3-((S)-1-(((R)-*tert*-butylsulfinyl<sulfenyl>)-amino)-1,3-dihydrospiro[indene-2,4'-piperidin]-1'-yl)-5-methylpyrazine-2-carboxylate (500 mg, 0.795 mmol) in tetrahydrofuran (30 mL) at 0°C. The reaction solution was stirred at 0°C for 30 minutes. Sodium sulfate decahydrate was added to quench the reaction, and the reaction solution was stirred for 10 minutes. The reaction solution was filtered through diatomaceous earth, and the filter cake was washed with tetrahydrofuran (50 mL) twice. The filtrate was concentrated, and purified by column chromatography [eluent: dichloromethane

~ dichloromethane/methanol (95:5)] to obtain (R)-N-((S)-1'-(5-((2-amino-3-chloropyridin-4-yl)thio)-3-(hydroxymethyl)-6-methyl-pyrazin-2-yl)-1,3-dihydrospiro[indene-2,4'-piperidin]-1-yl)-2-methylpropane-2-sulfinamide (220 mg, yield: 47%) as a yellow solid.

MS m/z (ESI): 587.1 $[M+H]^+$, 589.1 $[M+2+H]^+$.

Step 6: Preparation of (S)-(3-(1-amino-1,3-dihydrospiro[indene-2,4'-piperidin]-1'-yl)-6-((2-amino-3-chloropyridin-4-yl)thio)-5-methylpyrazin-2-yl)methanol

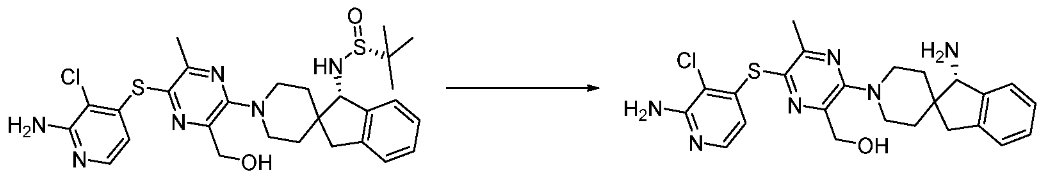

Hydrochloric acid in dioxane (20 mL, 4.0 M) was added to a solution of (R)-N-((S)-1'-(5-((2-amino-3-chloropyridin-4-yl)thio)-3-(hydroxymethyl)-6-methyl-pyrazin-2-yl)-1,3-dihydrospiro[indene-2,4'-piperidin]-1-yl)-2-methylpropane-2-sulfinamide (220 mg, 0.375 mmol) in methanol (3 mL), and the reaction solution was stirred at room temperature for 30 minutes. The reaction solution was concentrated, and dissolved in methanol (10 mL). The reaction solution was adjusted to alkaline with ammonia, and concentrated to dryness by rotary evaporation. The resulting residue was purified by column chromatography [eluent: dichloromethane-dichloromethane/methanol (1% NH3-H2O) (95:5)] to obtain (S)-(3-(1-amino-1,3-dihydrospiro[indene-2,4'-piperidin]-1'-yl)-6-((2-amino-3-chloro-pyridin-4-yl)thio)-5-methylpyrazin-2-yl)methanol (115 mg, yield: 63%) as a white solid.

$^1H$ NMR (400 MHz, MeOD) $\delta$ 7.59 (d, $J$ = 4 Hz, 1H), 7.41 - 7.34 (m, 1H), 7.27 - 7.16 (m, 3H), 5.89 (d, $J$= 4 Hz, 1H), 4.64 (s, 2H), 4.00 (s, 1H), 3.88 (d, $J$= 12 Hz, 2H), 3.28 - 3.20 (m, 2H), 3.14 (d, $J$ = 16 Hz, 1H), 2.82 (d, $J$ = 16 Hz, 1H), 2.48 (s, 3H), 2.01 - 1.85 (m, 2H), 1.67 - 1.58 (m, 1H), 1.52 - 1.43 (m, 1H).

MS m/z (ESI): 483.2 $[M+H]^+$, 485.2 $[M+2+H]^+$.

**Example 46**

Preparation of (S)-(6-((2-amino-3-chloropyridin-4-yl)thio)-3-(6-amino-4,6-dihydro-spiro[cyclopenta[d]thiazole-5,4'-piperidin]-1'-yl)-5-methylpyrazin-2-yl)methanol

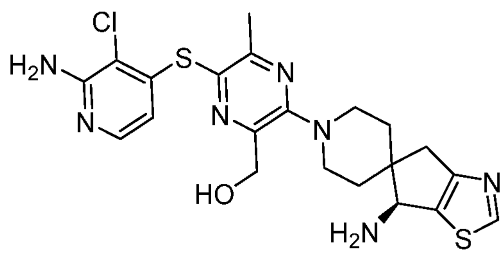

Step 1: Preparation of ethyl 6-bromo-3-((S)-6-(((R)-*tert*-butylsulfinyl<sulfenyl>)amino)-4,6-dihydrospiro-[cyclopenta[d]thiazole-5,4'-piperidin]-1'-yl)-5-methylpyrazine-2-carboxylate

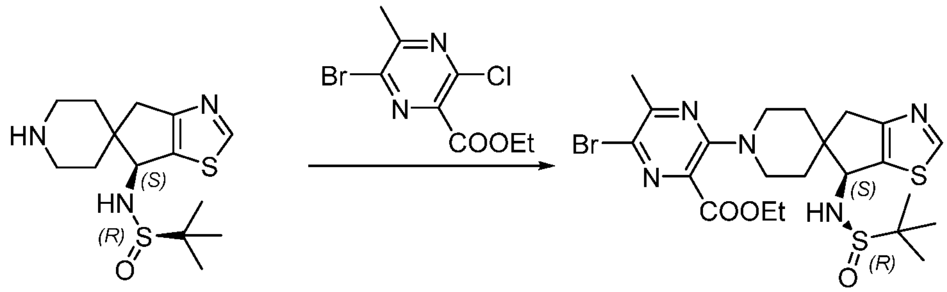

(R)-N-((S)-4,6-Dihydrospiro[cyclopenta[d]thiazole-5,4'-piperidin]-6-yl)-2-methylpropane-2-sulfinamide (470 mg, 1.5 mmol, 1.0 eq), ethyl 6-bromo-3-chloro-5-methylpyrazine-2-carboxylate (398 mg, 1.43 mmol, 0.95 eq) and potassium carbonate (1.04 g, 7.5 mmol, 5.0 eq) were dissolved in DMF (4.0 mL). The reaction solution was heated to 60°C under a nitrogen atmosphere and stirred for 3 hours. After completion of the reaction, the reaction solution was concentrated to remove DMF. The resulting crude product was purified by column chromatography (100% EtOAc in Petro ether) to obtain the product ethyl 6-bromo-3-((S)-6-(((R)-*tert*-butylsulfinyl<sulfenyl>)amino)-4,6-dihydrospiro-[cyclopenta[d]thiazole-5,4'-piperidin]-1'-yl)-5-methylpyrazine-2-carboxylate (600 mg, yield: 72%) as a yellow solid.

MS m/z (ESI): 556.1$[M+H]^+$, 558.1$[M+H+2]^+$.

Step 2: Preparation of ethyl 6-((2-amino-3-chloropyridin-4-yl)thio)-3-((S)-6-(((R)-*tert*-butylsulfinyl<sulfenyl>)-amino)-4,6-dihydrospiro[cyclopenta[d]thiazole-5,4'-piperidin]-1'-yl)-5-methylpyrazine-2-carboxylate

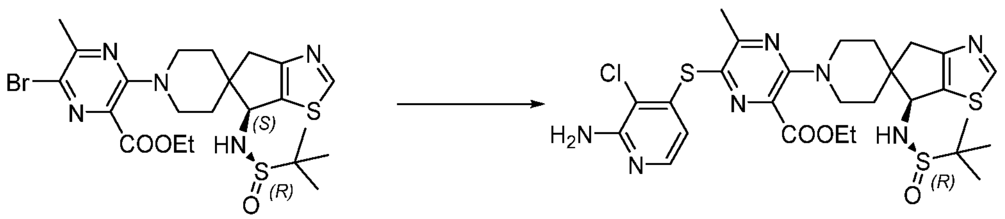

In a microwave reaction tube, ethyl 6-bromo-3-((S)-6-(((R)-*tert*-butylsulfinyl<sulfenyl>)amino)-4,6-dihydrospiro-[cyclopenta[d]thiazole-5,4'-piperidin]-1'-yl)-5-methylpyrazine-2-carboxylate (600 mg, 1.08 mmol, 1.0 eq) and potassium 2-amino-3-chloropyridine-4-thiolate (536 mg, 2.70 mmol, 2.5 eq) were dissolved in 10 mL of DMF, followed by the addition of tris(dibenzylideneacetone)dipalladium (50 mg, 0.054 mmol, 0.05 eq), Xantphos (62 mg, 0.11 mmol, 0.1 eq) and DIPEA (419 mg, 3.24 mmol, 3.0 eq). The reaction solution was bubbled with nitrogen for 3 minutes, heated under microwave to 105°C and reacted for 75 minutes. The reaction solution was cooled to room temperature, diluted with 20 mL of ethyl acetate, and filtered through diatomaceous earth. The filtrate was concentrated, and the resulting crude product was purified by column chromatography (10% MeOH in $CH_2Cl_2$) to obtain the product ethyl 6-((2-amino-3-chloropyridin-4-yl)thio)-3-((S)-6-(((R)-*tert*-butylsulfinyl<sulfenyl>)amino)-4,6-dihydrospiro[cyclopenta[d]-thiazole-5,4'-piperidin]-1'-yl)-5-methylpyrazine-2-carboxylate (360 mg, yield: 52%) as a yellow solid.

MS m/z (ESI): 636.0 $[M+H]^+$, 638.0 $[M+H+2]^+$.

Step 3: Preparation of ethyl 6-((2-amino-3-chloropyridin-4-yl)thio)-3-((S)-6-(((R)-*tert*-butylsulfinyl<sulfenyl>)amino)-4,6-dihydrospiro[cyclopenta[d]thiazole-5,4'-piperidin]-1'-yl)-5-methylpyrazine-2-carboxylate

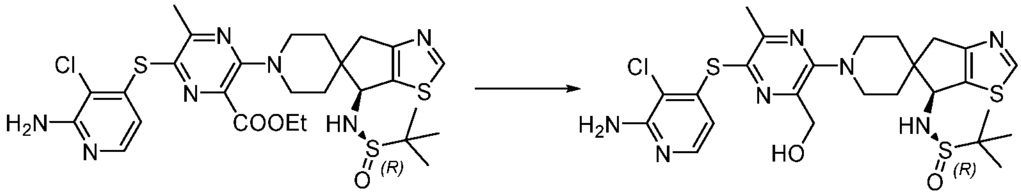

Ethyl 6-((2-amino-3-chloropyridin-4-yl)thio)-3-((S)-6-(((R)-*tert*-butylsulfinyl<sulfenyl>)amino)-4,6-dihydrospiro[cyclopenta[d]thiazole-5,4'-piperidin]-1'-yl)-5-methylpyrazine-2-carboxylate (360 mg, 0.57 mmol, 1.0 eq) was dissolved in 40 mL of anhydrous THF, and the solution was purged with nitrogen. The solution was cooled to 0°C, and then a solution of $LiAlH_4$ in tetrahydrofuran (0.6 mL, 1.14 mmol, 2.0 eq., D=2M) was added dropwise. After completion of the addition, the reaction solution was slowly warmed up to room temperature and stirred for 3 hours. After completion of the reaction, the reaction solution was diluted with ethyl acetate (100 mL), and cooled to 0° C. 2 mL of water was added dropwise, and 4 mL of 15% sodium hydroxide aqueous solution was added. 6 mL of water was added, and the reaction solution was warmed up to room temperature and stirred for 30 minutes. An appropriate amount of anhydrous sodium sulfate was added, and the reaction solution was stirred for 15 minutes. The reaction solution was filtered to remove the solid, and the filtrate was concentrated. The resulting crude product was purified by column chromatography (10% MeOH in $CH_2Cl_2$) to obtain the product ethyl 6-((2-amino-3-chloropyridin-4-yl)thio)-3-((S)-6-(((R)-*tert*-butylsulfinyl<sulfenyl>)-amino)-4,6-dihydrospiro[cyclopenta[d]thiazole-5,4'-piperidin]-1'-yl)-5-methylpyrazine-2-carboxylate (150 mg, yield: 44%) as a yellow solid.

MS m/z (ESI): 594.1$[M+H]^+$, 596.1$[M+H+2]^+$.

Step 4: Preparation of (S)-(6-((2-amino-3-chloropyridin-4-yl)thio)-3-(6-amino-4,6-dihydro spiro [cyclopenta [d] thiazole-5,4' -piperidin] -1' -yl)-5 -methylpyrazin-2-yl)-methanol

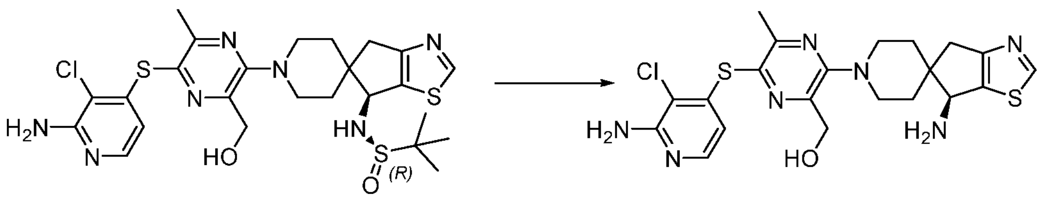

Ethyl 6-((2-amino-3-chloropyridin-4-yl)thio)-3-((S)-6-(((R)-*tert*-butylsulfinyl<sulfenyl>)amino)-4,6-dihydrospiro[cyclopenta[d]thiazole-5,4'-piperidin]-1'-yl)-5-methylpyrazine-2-carboxylate (150 mg, 0.25 mmol, 1.0 eq) was dissolved in MeOH (5 mL). A solution of hydrochloric acid in dioxane (1.0 mL, 4M) was added, and the reaction solution was reacted at room temperature for 1 hour. The reaction solution was concentrated to dryness and dissolved in 5 mL of methanol, and the pH was adjusted to alkaline with 7M $NH_3$ in methanol. The reaction solution was concentrated, and purified by normal-phase column chromatography (10% MeOH in $CH_2Cl_2$) and reversed-phase column chromatography (30% MeCN in $H_2O$ (0.1% $NH_3.H_2O$)) successively to obtain the target compound (S)-(6-((2-amino-3-chloropyridin-4-yl)thio)-3-(6-amino-4,6-dihydrospiro[cyclopenta-[d]thiazole-5,4'-piperidin]-1'-yl)-5-methylpyrazin-2-yl)methanol (52 mg, yield: 42%) as a grey solid.

$^1H$ NMR (400 MHz, DMSO) δ 8.93 (s, 1H), 7.62 (s, 1H), 6.31 (s, 1H), 5.72 (s, 1H), 5.41 (s, 1H), 4.45 (s, 2H), 3.98 (s, 1H), 3.84 (s, 2H), 2.76 (dd, *J* = 47.2, 16.9 Hz, 4H), 2.37 (s, 3H), 1.60 (dd, *J* = 165.2, 116.4 Hz, 7H).

MS m/z (ESI): 490.1 $[M+H]^+$, 492.1 $[M+H+2]^+$.

**Example 47**

Preparation of (S)-(6-((2-amino-3-chloropyridin-4-yl)thio)-3-(6-amino-4,6-dihydro-spiro[cyclopenta[d]oxazole-5,4'-piperidin]-1'-yl)-5-methylpyrazin-2-yl)methanol

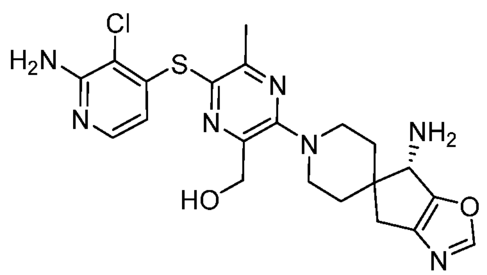

The compound of Example 47 was prepared by referring to the experimental scheme of Example 46.
MS m/z (ESI): 474.1 $[M+H]^+$, 476.1$[M+2+H]^+$.

**Example 48**

Preparation of (R)-8-(6-amino-5-((2-amino-3-chloropyridin-4-yl)thio)pyrazin-2-yl)-2-methylene-8-azaspiro[4.5]decan-1-amine

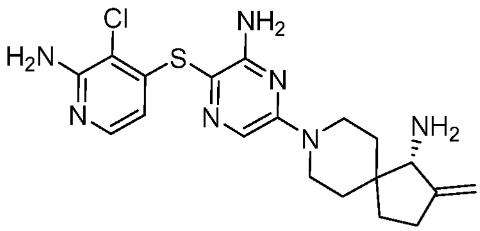

The compound of Example 48 was prepared by referring to the experimental scheme of Example 1.
MS m/z (ESI): 418.1 $[M+H]^+$, 420.1$[M+2+H]^+$.

**Example 49**

Preparation of (S,E)-8-(6-amino-5-((2-amino-3-chloropyridin-4-yl)thio)pyrazin-2-yl)-2-ethylidene-8-azaspiro[4.5]decan-1-amine

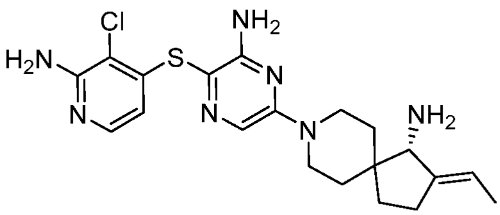

The compound of Example 49 was prepared by referring to the experimental scheme of Example 1.
MS m/z (ESI): 432.1 $[M+H]^+$, 434.1$[M+2+H]^+$.

**Example 50**

Preparation of (S)-8-(6-amino-5-((2-amino-3-chloropyridin-4-yl)thio)pyrazin-2-yl)-2-(propan-2-ylidene)-8-azaspiro[4.5]decan-1-amine

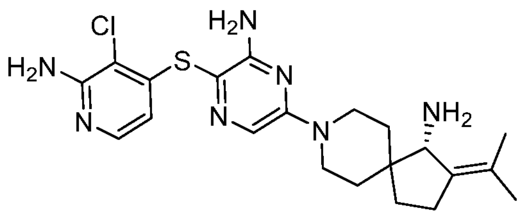

The compound of Example 50 was prepared by referring to the experimental scheme of Example 1.
MS m/z (ESI): 446.1 $[M+H]^+$, 448.1$[M+2+H]^+$.

**Example 51**

Preparation of (S,E)-8-(6-amino-5-((2-amino-3-chloropyridin-4-yl)thio)-3-methyl-pyrazin-2-yl)-2-ethylidene-8-azaspiro[4.5]decan-1-amine

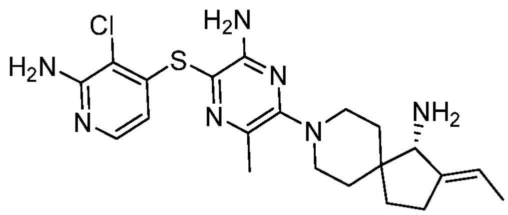

The compound of Example 51 was prepared by referring to the experimental scheme of Example 6.
MS m/z (ESI): 446.1 $[M+H]^+$, 448.1$[M+2+H]^+$.

**Example 52**

Preparation of (R)-8-(6-amino-5-((2-amino-3-chloropyridin-4-yl)thio)-3-methyl-pyrazin-2-yl)-2-methylene-8-azaspiro[4.5]decan-1-amine

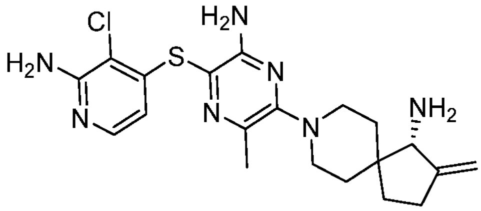

The compound of Example 52 was prepared by referring to the experimental scheme of Example 6.
MS m/z (ESI): 432.1 $[M+H]^+$, 434.1$[M+2+H]^+$.

**Example 53**

Preparation of (S)-1'-(6-amino-5-((2-amino-3-chloropyridin-4-yl)thio)-3-fluoro-pyrazin-2-yl)-1,3-dihydrospiro[indene-2,4'-piperidin]-1-amine

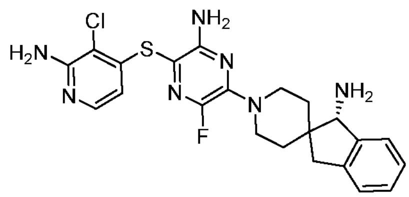

Step 1: Preparation of (R)-N-((S)-1'-(6-amino-5-((2-amino-3-chloropyridin-4-yl)-thio)pyrazin-2-yl)-1,3-dihydrospiro[indene-2,4'-piperidin]-1-yl)-2-methylpropane-2-sulfinamide

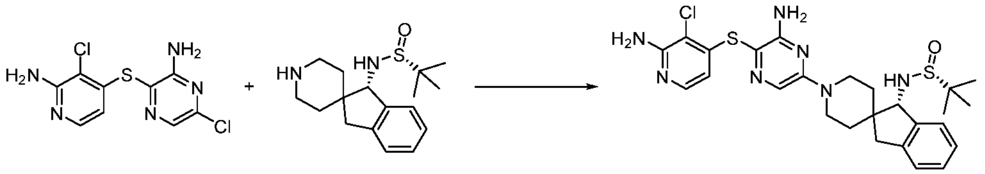

3-((2-Amino-3-chloropyridin-4-yl)thio)-6-chloropyrazin-2-amine (200 mg, 0.694 mmol), (R)-N-((S)-1,3-dihydrospiro[indene-2,4'-piperidin]-1-yl)-2-methylpropane-2-sulfinamide (350 mg, 0.833 mmol, $[\alpha]^{20}{}_D$=1.773) and potassium carbonate (479 mg, 3.47 mmol) were stirred in N,N-dimethylformamide (7 mL) at 100°C for 8 hours. After cooling, 40 mL of water was added, and the reaction solution was extracted with ethyl acetate (40 mL×2). The ethyl acetate layer was washed with saturated sodium chloride aqueous solution (20 mL), dried over anhydrous sodium sulfate, and purified by column chromatography [eluent: dichloromethane ~ dichloromethane/methanol from 0% to 3%] to obtain the product (R)-N-((S)-1'-(6-amino-5-((2-amino-3-chloropyridin-4-yl)thio)pyrazin-2-yl)-1,3-dihydrospiro[indene-2,4'-piperidin]-1-yl)-2-methylpropane-2-sulfinamide (200 mg, yield: 52%) as an orange solid.

MS m/z (ESI): 558.2 $[M+H]^+$.

Step 2: Preparation of (S)-1'-(6-amino-5-((2-amino-3-chloropyridin-4-yl)thio)pyrazin-2-yl)-1,3-dihydrospiro[indene-2,4'-piperidin]-1-amine

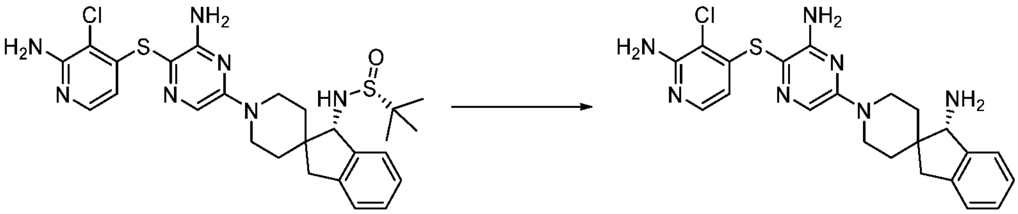

Hydrochloric acid in dioxane (20 mL, 80 mmol) was added to a solution of (R) -N-((S)-1 ' -(6-amino-5-((2-amino-3-chloropyridin-4-yl)thio)pyrazin-2-yl)-1,3-dihydrospiro[indene-2,4'-piperidin]-1-yl)-2-methylpropane-2-sulfinamide (188 mg, 0.337 mmol) in methanol (5 mL), and the reaction solution was stirred at room temperature for 1 hour. The reaction solution was concentrated to dryness, and dissolved in 5 mL of methanol. The pH was adjusted to alkaline with ammonia. The reaction solution was concentrated and purified by column chromatography [eluent: dichloromethane ~ dichloromethane/methanol from 0% to 7.5%] to obtain the product (S)-1'-(6-amino-5-((2-amino-3-chloropyridin-4-yl)thio)pyrazin-2-yl)-1,3-dihydrospiro-[indene-2,4'-piperidin]-1-amine (130 mg, yield: 85%) as a white solid.

MS m/z (ESI): 454.2 $[M+H]^+$.

Step 3: Preparation of (S)-1'-(6-amino-5-((2-amino-3-chloropyridin-4-yl)thio)-3-fluoropyrazin-2-yl)-1,3-dihydrospiro[indene-2,4'-piperidin]-1-amine

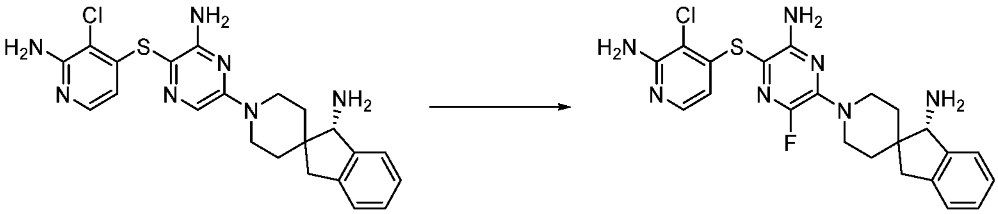

N-Fluorobisbenzenesulfonamide (69 mg, 0.22 mmol) was added to a solution of (S)-1'-(6-amino-5-((2-amino-3-chloropyridin-4-yl)thio)pyrazin-2-yl)-1,3-dihydrospiro-[indene-2,4'-piperidin]-1-amine (100 mg, 0.22 mmol) in acetonitrile (10 mL) at -10°C. After completion of the addition, the reaction solution was slowly warmed up to room temperature and stirred for 7 hours. The reaction solution was concentrated to dryness by rotary evaporation, and the resulting residue was purified by thin layer chromatography (dichloromethane/methanol=10/1) to obtain the product (S)-1'-(6-amino-5-((2-amino-3-chloropyridin-4-yl)thio)-3-fluoropyrazin-2-yl)-1,3-dihydrospiro[indene-2,4'-piperidin]-1-amine (28 mg, yield:

27%) as a khaki solid.

$^1H$ NMR (400 MHz, MeOD) δ 7.62 (d, *J* = 4 Hz, 1H), 7.42 - 7.33 (m, 1H), 7.26 - 7.15 (m, 3H), 6.00 (d, *J* = 4 Hz, 1H), 4.67 - 4.54 (m, 2H), 4.30 (d, J = 16 Hz, 2H), 3.97 (s, 1H), 3.16 (d, *J* = 16 Hz, 1H), 2.82 (d, *J* = 16 Hz, 1H), 1.95 - 1.78 (m, 2H), 1.65 - 1.53 (m, 1H), 1.48 - 1.38 (m, 1H).

MS m/z (ESI): 472.1 $[M+H]^+$, 474.1 $[M+2+H]^+$.

$$[\alpha]^{20}_{D}=-36.893.$$

**Example 54**

(S)-1'-(6-Amino-5-((2-amino-3-chloropyridin-4-yl)thio)-3-fluoropyrazin-2-yl)-5,7-dihydrospiro[cyclopenta[b]pyridine-6,4'-piperidin]-5-amine

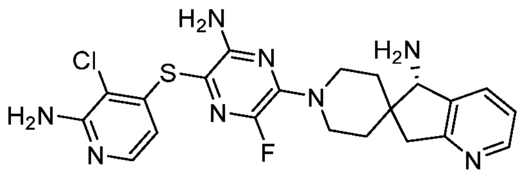

Step 1: Preparation of 3-bromo-2-(chloromethyl)pyridine

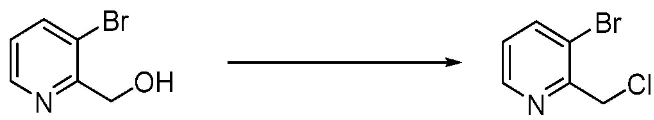

(3-Bromopyridin-2-yl)methanol (7.5 g, 40 mmol) was dissolved in dichloromethane (20 mL) and mixed well. $SOCl_2$(9.5 g, 80 mmol) was added dropwise in an ice water bath, and the reaction solution was stirred for 1 hour. The reaction solution was concentrated under reduced pressure, and the resulting crude product was purified by rapid silica gel column chromatography to obtain the target compound 3-bromo-2-(chloromethyl)pyridine (5 g, yield: 61%) as a colorless liquid.

MS *m/z* (ESI): 205.9 $[M+H]^+$.

Step 2: Preparation of 1-(*tert*-butyl) 4-methyl 4-((3-bromopyridin-2-yl)methyl)piperidine-1,4-dicarboxylate

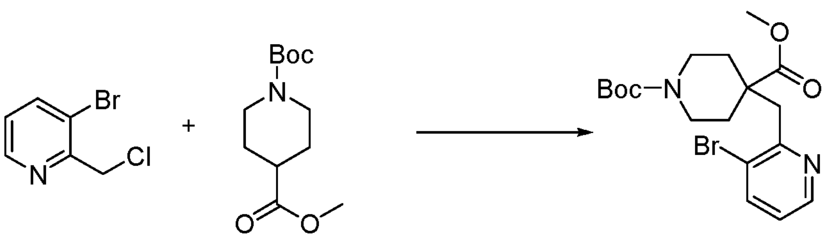

1-(*Tert*-butyl) 4-methyl piperidine-1,4-dicarboxylate (7.7 g, 31.7 mmol) and LDA (18.3 mL, 36.6 mmol) were added to THF (20 mL), and the reaction solution was reacted at -60°C for 1 hour. 3-Bromo-2-(chloromethyl)pyridine (5 g, 24.4 mmol) was added, and the reaction solution was reacted at -60°C for 2 hours. The reaction solution was concentrated under reduced pressure, and the resulting crude product was purified by rapid silica gel column chromatography to obtain the target compound 1-(*tert*-butyl) 4-methyl 4-((3-bromopyridin-2-yl)methyl)piperidine-1,4-dicarboxylate (9 g, yield: 89%) as a light yellow solid.

MS *m/z* (ESI):413.0 [M+H]+.

Step 3: Preparation of *tert-butyl* 5-oxo-5,7-dihydrospiro[cyclopenta[b]pyridine-6,4'-piperidine]-1'-carboxylate

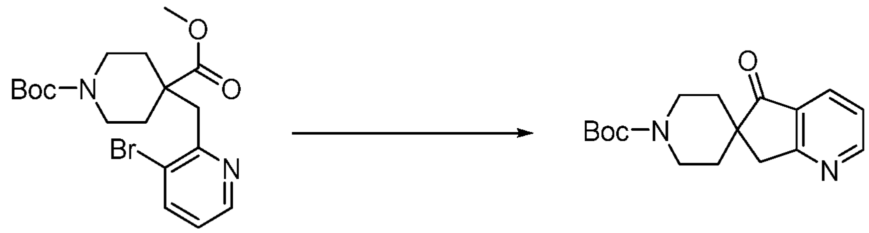

THF (100 mL) was added to 1-(*tert*-butyl) 4-methyl 4-((3-bromopyridin-2-yl)methyl)piperidine-1,4-dicarboxylate (9 g, 21.84 mmol), and the solution was cooled to -78°C. N-butyl lithium solution (1.6 N, 17 mL) was slowly added dropwise, and the reaction solution was reacted at -78°C for 1 hour. Ammonium chloride aqueous solution was added to quench the reaction, and the reaction solution was extracted with ethyl acetate. The organic phase was dried over anhydrous sodium sulfate and filtered, and the filtrate was concentrated under reduced pressure. The resulting crude product was purified by rapid silica gel column chromatography to obtain the target compound *tert*-butyl 5-oxo-5,7-dihydrospiro[cyclopenta[b]-pyridine-6,4'-piperidine]-1'-carboxylate (3 g, yield: 45%) as a light yellow solid.
MS *m/z* (ESI):303.1 [M+H]+.

Step 4: Preparation of *tert*-butyl (R,Z)-5-((*tert*-butylsulfinyl<sulfenyl>)imino)-5,7-dihydrospiro[cyclopenta[b]pyridine-6,4'-piperidine]-1'-carboxylate

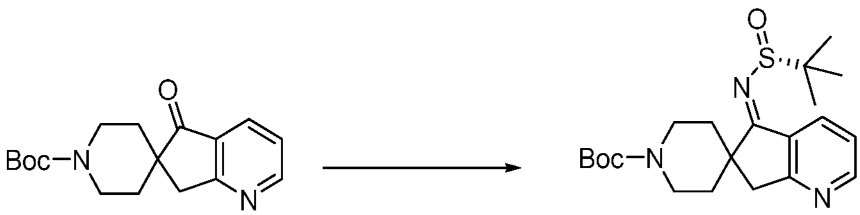

*Tert*-butyl 5-oxo-5,7-dihydrospiro[cyclopenta[b]pyridine-6,4'-piperidine]-1'-carboxylate (3 g, 9.93 mmol) and (R)-2-methylpropane-2-sulfinamide (3.6 g, 29.8 mmol) were added to ethyl titanate (50 mL). The reaction solution was stirred well, and reacted at 110°C for 13 hours. The reaction solution was cooled to room temperature, and water (100 mL) was slowly added dropwise to precipitate a solid. Ethyl acetate (200 mL) was added, and the reaction solution was filtered. The organic phase was concentrated under reduced pressure to obtain the target compound *tert*-butyl (R,Z)-5-((*tert*-butylsulfinyl<sulfenyl>)imino)-5,7-dihydrospiro[cyclopenta[b]pyridine-6,4'-piperidine]-1'-carboxylate (3 g, yield: 75%), which was used directly in the next step.
MS *m/z* (ESI):406.1 [M+H] +.

Step 5: Preparation of *tert*-butyl (S)-5-(((R)-*tert*-butylsulfinyl<sulfenyl>)amino)-5,7-dihydrospiro[cyclopenta[b]pyridine-6,4'-piperidine]-1'-carboxylate

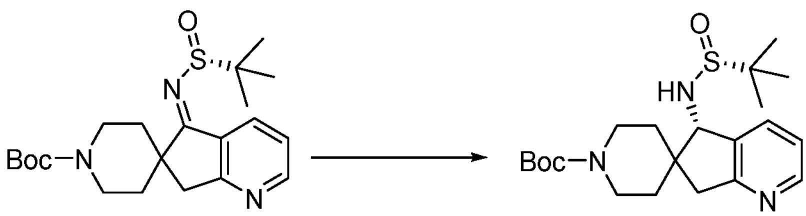

*Tert*-butyl (R,Z)-5-((*tert*-butylsulfinyl<sulfenyl>)imino)-5,7-dihydrospiro[cyclopenta[b]pyridine-6,4'-piperidine]-1'-carboxylate (3 g, 7.41 mmol) was added to THF (40 mL). The solution was cooled to 0°C, and $BH_3$ (7.5 mL, 2N in THF) was slowly added dropwise. After completion of the addition, the reaction solution was kept at 0°C and reacted for 2 hours. Methanol (10 mL) was added dropwise, and the reaction solution was concentrated under reduced pressure. The resulting crude product was purified by rapid silica gel column chromatography to obtain the target compound *tert*-butyl (S)-5-(((R)-*tert*-butylsulfinyl<sulfenyl>)amino)-5,7-dihydrospiro[cyclopenta[b]pyridine-6,4'-piperidine]-1'-carboxylate (2.5 g, yield: 83%) as a light yellow solid.

MS *m/z* (ESI):408.1 $[M+H]^+$.

Step 6: Preparation of (S)-5,7-dihydrospiro[cyclopenta[b]pyridine-6,4'-piperidin]-5-amine

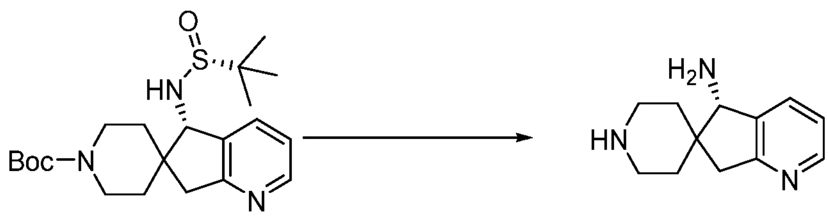

*Tert*-butyl (S)-5-(((R)-*tert*-butylsulfinyl<sulfenyl>)amino)-5,7-dihydrospiro[cyclopenta-[b]pyridine-6,4'-piperidine]-1'-carboxylate (2.5 g, 6.14 mmol) and hydrochloric acid in dioxane (10 mL, 4N) were added to $CH_2Cl_2$ (40 mL). The reaction solution was stirred at room temperature for 3 hours, and then concentrated under reduced pressure to obtain the target compound (S)-5,7-dihydrospiro[cyclopenta[b]pyridine-6,4'-piperidin]-5-amine (1.5 g), which was used directly in the next step.

MS *m/z* (ESI):204.1 $[M+H]^+$.

Step 7: Preparation of (S)-1'-(6-amino-5-((2-amino-3-chloropyridin-4-yl)thio)pyrazin-2-yl)-5,7-dihydrospiro[cyclopenta[b]pyridine-6,4'-piperidin]-5-amine

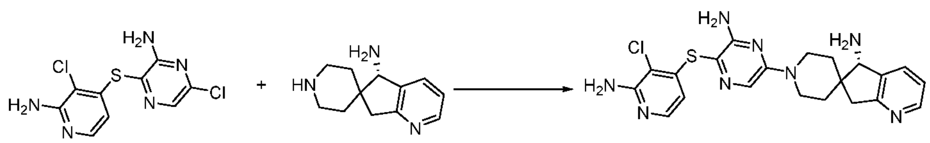

(S)-5,7-Dihydrospiro[cyclopenta[b]pyridine-6,4'-piperidin]-5-amine (1.5 g, 7.39 mmol), 3-((2-amino-3-chloropyridin-4-yl)thio)-6-bromopyrazine-2-amine (3.7 g, 11.09 mmol) and cesium carbonate (7.2 g, 22.17 mmol) were added to DMF (50 mL). The reaction solution was reacted at 90°C for 13 hours. The reaction solution was concentrated under reduced pressure, and the resulting crude product was purified by rapid silica gel column chromatography to obtain the target compound (S)-1'-(6-amino-5-((2-amino-3-chloropyridin-4-yl)thio)pyrazin-2-yl)-5,7-dihydrospiro-[cyclopenta[b]pyridine-6,4'-piperidin]-5-amine (1 g, yield: 29%) as a light yellow solid. MS *m/z* (ESI): 455.1 $[M+H]^+$, 457.1 $[M+H+2]^+$.

Step 8: Preparation of (S)-1'-(6-amino-5-((2-amino-3-chloropyridin-4-yl)thio)-3-fluoro-pyrazin-2-yl)-5,7-dihydrospiro[cyclopenta[b]pyridine-6,4'-piperidin]-5-amine

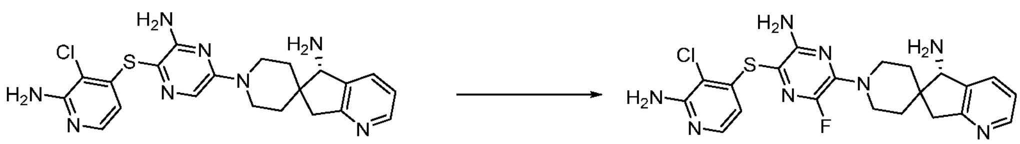

(S)-1'-(6-Amino-5-((2-amino-3-chloropyridin-4-yl)thio)pyrazin-2-yl)-5,7-dihydrospiro-[cyclopenta[b]pyridine-6,4'-piperidin]-5-amine (600 mg, 1.32 mmol) and NFSI (830 mg, 2.64 mmol) were added to THF (10 mL). The reaction solution was reacted at room temperature for 3 hours. The reaction solution was concentrated under reduced pressure, and the resulting crude product was purified by rapid silica gel column chromatography to obtain the target compound (S)-1'-(6-amino-5-((2-amino-3-chloropyridin-4-yl)thio)-3-fluoropyrazin-2-yl)-5,7-dihydrospiro[cyclopenta[b]pyridine-6,4'-piperidin]-5-amine (90 mg, yield: 14%) as a white solid.

MS *m/z* (ESI): 473.0 [M+H] $^+$, 475.1 [M+H+2] $^+$.

$^1$H NMR (400 MHz, MeOH-$d_4$) $\delta$8.33 (d, *J*=4.8 Hz, 1H), 7.82 (d, *J*=7.2 Hz, 1H), 7.62 (d, *J*=5.6 Hz, 1H), 7.28-7.25 (m, 1 H), 6.01 (d, *J*=5.6 Hz, 1H), 4.35-4.31(m,2 H), 4.03 (s, 1 H), 3.36-3.31(m, 2 H),3.25-3.21 (m,1H), 2.92 (d, *J*=16.8 Hz, 1H), 1.98-1.84 (m, 2 H), 1.64-1.60 (m,1 H), 1.42-1.39(m, 1 H).

**Example 55**

Preparation of (S)-1'-(6-amino-5-((2-amino-3-chloropyridin-4-yl)thio)-3-fluoro-pyrazin-2-yl)-4,6-dihydrospiro[cyclopenta[d]thiazole-5,4'-piperidin]-6-amine

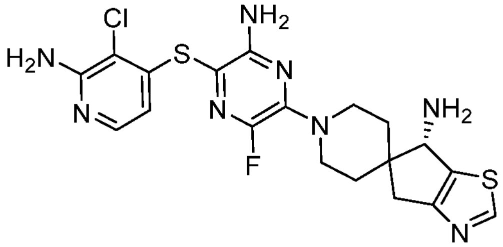

Step 1: Preparation of (R)-N-((S)-1'-(6-amino-5-((3-chloro-2-cyclopropyl-pyridin-4-yl)thio)pyrazin-2-yl)-1,3-dihydrospiro[indene-2,4'-piperidin]-1-yl)-2-methylpropane-2-sulfinamide

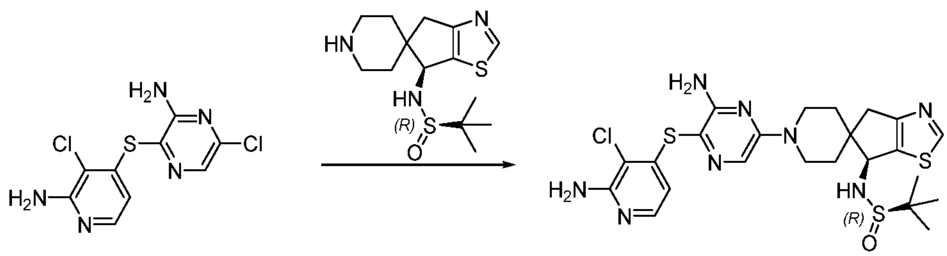

(R)-N-((S)-4,6-Dihydrospiro[cyclopenta[d]thiazole-5,4'-piperidin]-6-yl)-2-methylpropane-2-sulfinamide (260 mg, 0.83 mmol, 1.0 eq), 3-((2-amino-3-chloropyridin-4-yl)thio)-6-chloropyrazin-2-amine (227 mg, 0.79 mmol, 0.95 eq) and potassium carbonate (574 mg, 4.15 mmol, 5.0 eq) were dissolved in DMF (2 mL). The reaction solution was heated to 105°C under a nitrogen atmosphere and stirred for 10 hours. After completion of the reaction, the reaction solution was concentrated to remove DMF. The resulting crude product was purified by column chromatography (8% MeOH in $CH_2Cl_2$) to obtain the product (R)-N-((S)-1'-(6-amino-5-((3-chloro-2-cyclopropylpyridin-4-yl)thio)pyrazin-2-yl)-1,3-dihydrospiro[indene-2,4'-piperidin]-1-yl)-2-methylpropane-2-sulfinamide (340 mg, yield: 76%) as a yellow solid.

MS m/z (ESI): 565.1[M+H]$^+$, 567.1[M+H+2]$^+$.

Step 2: Preparation of (S)-1'-(6-amino-5-((2-amino-3-chloropyridin-4-yl)thio)-pyrazin-2-yl)-4,6-dihydrospiro[cyclopenta[d]thiazole-5,4'-piperidin]-6-amine

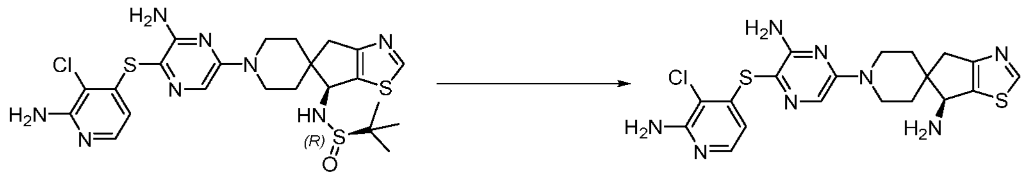

(R)-N-((S)-1'-(6-Amino-5-((3-chloro-2-cyclopropylpyridin-4-yl)thio)pyrazin-2-yl)-1,3-dihydrospiro[indene-2,4'-piperidin]-1-yl)-2-methylpropane-2-sulfinamide (340 mg, 0.60 mmol, 1.0 eq) was dissolved in MeOH (5 mL). A solution of HCl in dioxane (1.0 mL, 4M) was added, and the reaction solution was reacted at room temperature for 1 hour. The reaction solution was concentrated to dryness and dissolved in 5 mL of methanol, and the pH was adjusted to alkaline with 7M $NH_3$ in methanol. The reaction solution was concentrated, and purified by normal-phase column chromatography (10% MeOH in $CH_2Cl_2$) and reversed-phase column chromatography (40% MeCN in $H_2O$ (0.1% $NH_3.H_2O$)) successively to obtain the target compound (S)-1'-(6-amino-5-((2-amino-3-chloropyridin-4-yl)thio)pyrazin-2-yl)-4,6-dihydrospiro-[cyclopenta[d]thiazole-5,4'-piperidin]-6-amine (220 mg, yield: 80%) as a grey solid. MS m/z (ESI): 461.1 $[M+H]^+$, 463.1 $[M+H+2]^+$.

Step 3: Preparation of (S)-1'-(6-amino-5-((2-amino-3-chloropyridin-4-yl)thio)-3-fluoropyrazin-2-yl)-4,6-dihydrospiro[cyclopenta[d]thiazole-5,4'-piperidin]-6-amine

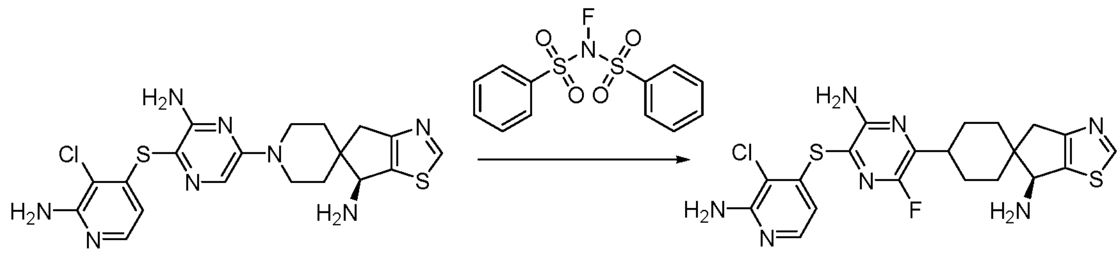

(S)-1'-(6-Amino-5-((2-amino-3-chloropyridin-4-yl)thio)pyrazin-2-yl)-4,6-dihydrospiro-[cyclopenta[d]thiazole-5,4'-piperidin]-6-amine (220 mg, 0.48 mmol, 1.0 eq) was dissolved in anhydrous DMF (2 mL) and anhydrous MeCN (10 mL). The reaction solution was purged with nitrogen three times, and the reagent N-fluoro-N-(benzenesulfonyl)benzenesulfonamide (159 mg, 0.5 mmol, 1.05 eq.) was added in batches under a nitrogen atmosphere. After completion of the addition, the reaction solution was stirred at room temperature for 3 hours. The reaction solution was added dropwise to MeOH (50 mL) under stirring, and 1 mL of water was added. The reaction solution was concentrated to obtain a crude product, which was purified by preparative HPLC to obtain the product (S)-1'-(6-amino-5-((2-amino-3-chloropyridin-4-yl)thio)-3-fluoropyrazin-2-yl)-4,6-dihydrospiro[cyclopenta[d]thiazole-5,4'-piperidin]-6-amine (40 mg, yield: 17%) as a grey solid.

$^1H$ NMR (400 MHz, DMSO) $\delta$ 8.96 (s, 1H), 7.67 (d, $J$= 5.3 Hz, 1H), 6.28 (s, 2H), 6.17 (s, 2H), 5.86 (d, $J$ = 5.4 Hz, 1H), 4.10 (s, 2H), 3.98 (s, 1H), 2.81 (dd, $J$ = 52.7, 15.2 Hz, 4H), 1.88 (s, 2H), 1.75 - 1.52 (m, 4H).

$^{19}F$ NMR (376 MHz, DMSO) $\delta$ -88.17 (s).

MS m/z (ESI): 479.0 $[M+H]^+$, 481.0 $[M+H+2]^+$.

**Example 56**

Preparation of (S)-8-(6-amino-5-((2-amino-3-chloropyridin-4-yl)thio)-3-fluoro-pyrazin-2-yl)-2-cyclopropyl-8-azaspiro[4.5]dec-2-en-1-amine

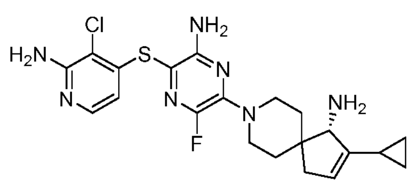

Step 1: Preparation of (S)-8-(6-amino-5-((2-amino-3-chloropyridin-4-yl)thio)-3-fluoropyrazin-2-yl)-2-cyclopropyl-8-azaspiro [4.5] dec-2-en-1-amine

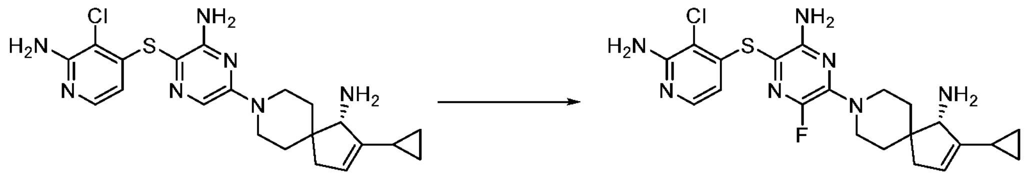

N-fluorobisbenzenesulfonamide (40 mg, 0.128 mmol) was added to a solution of (S)-8-(6-amino-5-((2-amino-3-chloropyridin-4-yl)thio)pyrazin-2-yl)-2-cyclopropyl-8-az aspiro[4.5]dec-2-en-1-amine (35 mg, 0.085 mmol) in tetrahydrofuran (18 mL) at 25°C. After completion of the addition, the reaction solution was stirred for 1 hour. TLC showed that the reaction was not complete, additional N-fluorobisbenzenesulfonamide (27 mg, 0.085 mmol) was added, and the reaction solution was stirred for another 1 hour. TLC showed that the reaction was not complete, additional N-fluorobisbenzenesulfonamide (27 mg, 0.085 mmol) was added, and the reaction solution was stirred for another 1 hour. TLC showed the presence of impurities. The reaction solution was quenched with hydrochloric acid (40 mL, 0.5 N), and washed with ethyl acetate (20 mL*2). The aqueous phase was adjusted to the pH about 8 with sodium carbonate, and extracted with ethyl acetate (30 mL*2). The organic phase was concentrated to dryness by rotary evaporation, and the resulting residue was purified by preparative chromatography (dichloromethane/methanol=10/1) to obtain (S)-8-(6-amino-5-((2-amino-3-chloropyridin-4-yl)thio)-3-fluoropyrazin-2-yl)-2-cyclopropyl-8-azaspiro[4.5]dec-2-en-1-amine (28 mg, yield: 27%) as a light yellow solid.

$^1H$ NMR (400 MHz, MeOD) δ 7.61 (d, *J* = 8 Hz, 1H), 5.99 (d, *J* = 8 Hz, 1H), 5.36 (s, 1H), 4.31 - 4.13 (m, 2H), 3.45 (s, 1H), 3.30 - 3.15 (m, 2H), 2.43 - 2.24 (m, 2H), 1.89 - 1.48 (m, 5H), 0.84 - 0.65 (m, 2H), 0.57 - 0.36 (m, 2H).

MS m/z (ESI): 462.1 $[M+H]^+$, 474.1 $[M+2+H]^+$.

**Example 57**

Preparation of (S)-8-(6-amino-5-((2-amino-3-chloropyridin-4-yl)thio)-3-fluoropyrazin-2-yl)-2-methyl-8-azaspiro[4.5]dec-2-en-1-amine

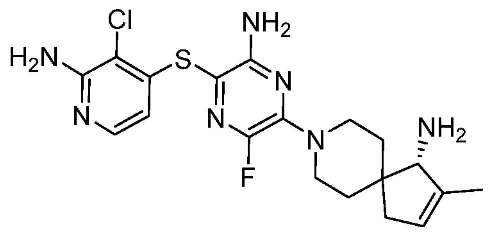

The compound of Example 57 was prepared by referring to the experimental scheme of Example 53.

MS m/z (ESI): 436.1 $[M+H]^+$, 438.1$[M+2+H]^+$.

**Example 58**

Preparation of (S)-(3-(1-amino-2-cyclopropyl-8-azaspiro[4.5]dec-2-en-8-yl)-6-((2-amino-3-chloropyridin-4-yl)thio)-5-methylpyrazin-2-yl)methanol

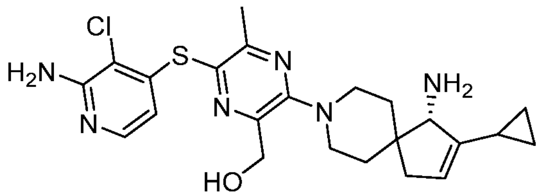

The compound of Example 58 was prepared by referring to the experimental scheme of Example 45.
MS m/z (ESI): 473.1 $[M+H]^+$, 475.1$[M+2+H]^+$.

**Example 59**

Preparation of (S)-1'-(6-amino-5-((2-amino-3-chloropyridin-4-yl)thio)pyrazin-2-yl)-1,3-dihydrospiro[indene-2,4'-piperidin]-1-amine

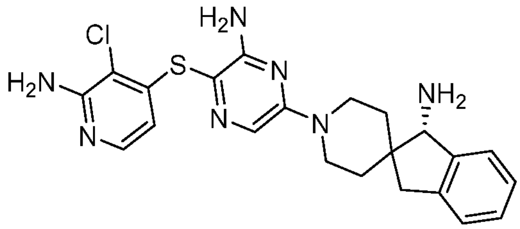

The compound of Example 59 was prepared by referring to the experimental scheme of Example 53.
$^1$H NMR (400 MHz, MeOD) δ 7.66 - 7.54 (m, 2H), 7.42 - 7.33 (m, 1H), 7.29 - 7.16 (m, 3H), 5.93 (d, *J* = 5.5 Hz, 1H), 4.37 - 4.19 (m, 2H), 3.98 (s, 1H), 3.29 - 3.20 (m, 2H), 3.15 (d, *J* = 16 Hz, 1H), 2.83 (d, *J* = 16 Hz, 1H), 1.91 - 1.68 (m, 2H), 1.65 - 1.53 (m, 1H), 1.50 - 1.39 (m, 1H).
MS m/z (ESI): 454.1 $[M+H]^+$, 456.1$[M+2+H]^+$.

**II. Biological Assay and Evaluation of the Compounds**

The present invention is further described below in combination with the following test examples, which are not intended to limit the scope of the present invention.

**Test Example 1. Determination of the Inhibitory Effect of the Compounds of the Present Invention on SHP-2 Kinase Activity**

Experimental Objective:

The objective of this test is to determine the inhibitory effect of the compounds on SHP-2 full-length protein allosteric activity.
Experimental instruments: Centrifuge (5810R, purchased from Eppendorf), pipette (purchased from Eppendorf or Rainin), and microplate reader (Model: SynergyH1 full-function microplate reader, purchased from BioTek, USA).
Experimental method: Homogeneous Full Length SHP-2 Assay Kit (BPS Bioscience, #79330) was used to determine the *in vitro* SHP-2 activity. First, 18 μL of Master Mix was added to a 96-well low adsorption microplate (NUNC, #267342), *i.e.,* the reaction buffer with a final concentration of 1× comprised 0.5 μM SHP-2 activating Peptide and 5 mM DTT. After centrifugation, 5 μL of test compound/DMSO was added to each well (the final DMSO content was 1%, V/V, the test compound was dissolved in DMSO to 1 mM, a three-fold serial dilution was carried out to obtain ten concentrations, the final concentration of the reaction system ranged from 1 μM to 0.05 nM). SHP-2 was diluted in the 1× reaction buffer to a final concentration of 0.06 nM, and the resulting solution was added to the reaction microplate (2 μL per well). Full activity control (compound only with DMSO) and full inhibition control (no SHP-2) were set up on the reaction plate. After centrifugation, the reaction mixture was incubated at room temperature for 60 minutes.
After completion of the incubation, 25 μL of Substrate solution, which comprised Substrate with a final concentration of 10 μM and 5 mM DTT, was added to each well. After centrifugation, the reaction mixture was incubated at

room temperature for 30 minutes. After completion of the reaction, readings were measured on the Synergy H1 full-function microplate reader (BioTek) (excitation wavelength: 340 nm, emission wavelength: 455 nM, gain value: 75).

Experimental Data Processing Method:

Based on the full activity control and full inhibition control used as the Max and Min values, the percentage inhibition rate data of the wells treated by the compounds was calculated through the positive control well (DMSO control well) and negative control well (no kinase) on the plate {% inhibition rate=100-[(test compound value-Min average)/(Max average-Min average)]×100}. The $IC_{50}$ value of the test compounds was calculated by fitting the percentage inhibition rate and data of ten concentrations to a four-parameter nonlinear logic formula with GraphPad prism.

Experimental Conclusion:

According to the above scheme, the compounds of the examples of the present invention showed the following biological activities in Table 2.1 in the SHP-2 kinase activity test.

Table 2.1: Relative $IC_{50}$ value of the compounds on inhibiting SHP-2 kinase activity

| Example No. | SHP-2 $IC_{50}$ (nM) |
|---|---|
| Example 1 | 17.8 |
| Example 2 | 4.17 |
| Example 3 | 4.33 |
| Example 4 | 11.7 |
| Example 5 | 6.27 |
| Example 6 | 9.28 |
| Example 7 | 13.2 |
| Example 8 | 3.93 |
| Example 9 | 29.6 |
| Example 10 | 0.98 |
| Example 11 | 5.22 |
| Example 12 | 3.75 |
| Example 13 | 0.66 |
| Example 35 | 3.80 |
| Example 36 | 1.48 |
| Example 45 | 6.81 |
| Example 46 | 1.73 |
| Example 53 | 1.75 |
| Example 54 | 9.29 |
| Example 55 | 3.07 |
| Example 56 | 3.78 |
| Example 57 | 4.25 |

The above data show that the compounds of the examples of the present invention have a good activity in inhibiting SHP-2 kinase activity.

**Test Example 2. Determination of the Proliferation Inhibitory Activity of the Compounds of the Present Invention on KYSE520 Cells**

The objective of this test example is to determine the proliferation inhibitory effect of the compounds of the present invention on tumor cells. The proliferation inhibitory activity of the compounds on tumor cells was determined by the CellTiter-Glo method, and the half inhibitory concentration $IC_{50}$ of the compounds on inhibiting cell proliferation was obtained. 2000 cells (90 μL of cell suspension) were inoculated to each well of a 96-well cell culture plate (Corning, #3610). The culture plate was incubated in an incubator overnight (37°C, 5% $CO_2$). On the next day, 10 μL of gradient dilutions of the test compound solution (the final DMSO content was 0.1%, V/V, the final concentration of the reaction system ranged from 100 μM to 45 nM) was added to each well of the culture plate. At the same time, full activity control (compound only with DMSO) and full inhibition control (no cells) were set up on the reaction plate. The culture plate was incubated in the incubator for 3 days (37°C, 5% $CO_2$).

After completion of the incubation, 50 μL of CellTiter-Glo reagent was added to each well of the cell culture plate, which was left to stand at room temperature for 10 minutes. The chemiluminescence signal value was measured on the Synergy H1 full-function microplate reader (BioTek). Based on the full activity control and full inhibition control used as the Max and Min values, the percentage inhibition rate data of the wells treated by the compounds was calculated {% inhibition rate=100-[(test compound value-Min average)/(Max average-Min average)]×100}. The $IC_{50}$ value of the test compounds was calculated by fitting the percentage inhibition rate and data of eight concentrations to a four-parameter nonlinear logic formula with GraphPad prism.

According to the above scheme, the compounds of the present invention showed a biological activity of about 0.1 nM to 200 nM ($IC_{50}$) in the proliferation inhibitory activity test on KYSE520 cells.

In some embodiments, the $IC_{50}$ of the compounds of the present invention on inhibiting the proliferation of KYSE520 cells was less than about 200 nM, preferably less than about 100 nM, further preferably less than about 10 nM, more preferably less than about 1 nM, and most preferably less than 1 nM.

**Test Example 3. Determination of the Proliferation Inhibitory Activity of the Compounds of the Present Invention on NCI-H358 Cells**

Experimental objective: The objective of this test example is to determine the proliferation inhibitory effect of the compounds of the present invention on tumor cells. Experimental instruments: Centrifuge (5810R, purchased from Eppendorf), carbon dioxide incubator (purchased from Thermo), biological safety cabinet (purchased from Shanghai Boxun Company), pipette (purchased from Eppendorf or Rainin), and microplate reader (Model: SynergyH1 full-function microplate reader, purchased from BioTek, USA).

Experimental method: 3000 cells (90 μL of cell suspension) were inoculated to each well of a 96-well cell culture plate (Corning, #3610). The culture plate was incubated in an incubator overnight (37°C, 5% $CO_2$). On the next day, 10 μL of gradient dilutions of the test compound solution (the final DMSO content was 0.2%, V/V, the final concentration of the reaction system ranged from 10 μM to 4.5 nM) was added to each well of the culture plate. At the same time, full activity control (compound only with DMSO) and full inhibition control (no cells) were set up on the reaction plate. The culture plate was incubated in the incubator for 3 days (37°C, 5% $CO_2$).

After completion of the incubation, 50 μL of CellTiter-Glo reagent was added to each well of the cell culture plate, which was left to stand at room temperature for 10 minutes. The chemiluminescence signal value was measured in the Synergy H1 full-function microplate reader (BioTek). Based on the full activity control and full inhibition control used as the Max and Min values, the percentage inhibition rate data of the wells treated by the compounds was calculated {% inhibition rate=100-[(test compound value-Min average)/(Max average-Min average)]×100}. The $IC_{50}$ value of the test compounds was calculated by fitting the percentage inhibition rate and data of eight concentrations to a four-parameter nonlinear logic formula with GraphPad prism.

Experimental Data Processing Method:

The percentage inhibition data of the wells treated by the compounds of the examples was calculated through the vehicle control well on the plate {% inhibition rate=100-(test compound value/vehicle control value)×100]. The $IC_{50}$ value was calculated by fitting the data of different concentrations and corresponding percentage inhibition rates to a four-parameter nonlinear logic formula with GraphPad prism.

Experimental Conclusion:

According to the above scheme, the compounds of the examples of the present invention showed the following biological activities in Table 2.2 in the NCI-H358 cell proliferation inhibition test.

Table 2.2: $IC_{50}$ value of the compounds on inhibiting the proliferation of NCI-H358 cells

| Example No. | NCI-H358 $IC_{50}$ (nM) |
|---|---|
| Example 1 | 37.0 |
| Example 2 | 10.5 |
| Example 3 | 31.1 |
| Example 4 | 184 |
| Example 5 | 6.39 |
| Example 6 | 13.4 |
| Example 7 | 41.87 |
| Example 8 | 18.5 |
| Example 9 | 32.8 |
| Example 10 | 24.5 |
| Example 11 | 37.7 |
| Example 12 | 42.6 |
| Example 13 | 47.0 |
| Example 35 | 23.3 |
| Example 36 | 9.19 |
| Example 45 | 25.9 |
| Example 46 | 11.9 |
| Example 53 | 20.9 |
| Example 54 | 79.9 |
| Example 55 | 46.5 |
| Example 56 | 78.5 |
| Example 57 | 56.5 |

The above data show that the compounds of the examples of the present invention have a good activity in inhibiting the proliferation of NCI-H358 cells.

**Test Example 4. Pharmacokinetics Assay in Mice**

4.1. Study Objective:

Balb/c mice were used as test animals. The pharmacokinetic behavior of the compounds of Examples 36, 46, 53, 54 and 55 after oral administration in mouse body (plasma) was studied.

4.2. Test Protocol

4.2.1 Test Compounds:

Compounds of Examples 36, 46, 53, 54 and 55, self-prepared;

4.2.2 Test Animals:

Male Balb/c mice were purchased from Shanghai Jiesijie Laboratory Animal Co., LTD, with Certificate No.: SCXK (Shanghai) 2013-0006 N0.311620400001794.

4.2.3 Preparation of the Test Compounds:

5 g of hydroxyethyl cellulose (HEC, CMC-Na, viscosity: 800-1200 Cps) was dissolved in 1000 mL of purified water, and 10 g of Tween 80 was added. The solution was mixed well to form a clear solution.

1.2 mg of the compounds of Examples 36, 46, 53, 54 and 55 were respectively added to a 4-mL glass bottle, followed by the addition of 2.4 mL of the above solution. The solution was subjected to ultrasound for 10 minutes to obtain a colorless and clear solution with a concentration of 0.5 mg/mL.

4.2.4 Administration:

After an overnight fast, male Balb/c mice were administrated orally with the test compounds at an administration dose of 5 mg/kg and an administration volume of 10 mL/kg.

4.2.5 Sample Collection:

Blood was taken before administration and at 0.25 hour, 0.5 hour, 1 hour, 2 hours, 4 hours, 6 hours, 8 hours and 24 hours after administration. The blood samples were stored in EDTA-2K tubes, and centrifuged for 6 minutes at 6000 rpm, at 4°C to separate the blood plasma. The plasma samples were stored at -80°C. The mice were fed 4 hours after administration.

4.2.6 Test Results:

The test results were determined by LCMS/MS method as shown in Table 2.3.

Table 2.3: Pharmacokinetic parameters of the compounds in mice

| Example No. | Tmax (hr) | Cmax (ng/mL) | $AUC_{0-\infty}$ (ng/mL*hr) | $T_{1/2}$ (hr) | MRT (hr) |
|---|---|---|---|---|---|
| Example 36 | 1.0 | 436.0 | 5390.0 | NA | 9.08 |
| Example 46 | 0.5 | 474.7 | 2214.7 | 3.07 | 4.34 |
| Example 53 | 0.5 | 179.0 | 2441.8 | 5.23 | 7.92 |
| Example 54 | 0.5 | 996.3 | 5976.9 | 3.92 | 5.15 |
| Example 55 | 0.5 | 1383.3 | 8156.7 | 4.63 | 5.07 |

The above data show that the compounds of the examples of the present invention have a good metabolic activity at a dose of 5 mg/kg.

**Test Example 5. Pharmacokinetics Assay in Rats**

5.1. Study Objective:

SD rats were used as test animals. The pharmacokinetic behavior of the compounds of Examples 36, 46, 53, 54, 55 and 59 after oral administration in rat body (plasma) was studied.

5.2. Test Protocol

5.2.1 Test Compounds:

Compounds of Examples 36, 46, 53, 54, 55 and 59, self-prepared;

5.2.2 Test Animals:

Male SD rats (3 rats per group) were purchased from Shanghai Jiesijie Laboratory Animal Co., LTD, with Certificate No.: SCXK (Shanghai) 2013-0006 N0.311620400001794.

5.2.3 Preparation of the Test Compounds:

5 g of hydroxyethyl cellulose (HEC, CMC-Na, viscosity: 800-1200 Cps) was dissolved in 1000 mL of purified water, and 10 g of Tween 80 was added. The solution was mixed well to form a clear solution.

3.9 mg of the compounds of Examples 36, 46, 53, 54, 55 and 59 were respectively dissolved in the above solution. The solution was shaked well and subjected to ultrasound for 15 minutes to obtain a colorless and clear solution with a concentration of 0.5 mg/mL.

5.2.4 Administration:

After an overnight fast, male SD rats (3 rats per group) were administrated orally with the test compounds at an administration dose of 5 mg/kg and an administration volume of 10 mL/kg.

5.2.5 Sample Collection:

0.2 mL of blood was taken from jugular vein before administration and at 0.5 hour, 1.0 hour, 2.0 hours, 4.0 hours, 6.0 hours, 8.0 hours and 24.0 hours after administration. The blood samples were stored in EDTA-2K tubes, and centrifuged for 6 minutes at 6000 rpm, at 4°C to separate the blood plasma. The plasma samples were stored at -80°C. The rats were fed 4 hours after administration.

5.3 Test results: The test results were determined by LCMS/MS method as shown in Table 2.4.

Table 2.4: Pharmacokinetic parameters of the compounds of the present invention in rats

| Example No. | Tmax (hr) | Cmax (ng/mL) | $AUC_{0-\infty}$ (ng/mL*hr) | $T_{1/2}$ (hr) | MRT (hr) |
|---|---|---|---|---|---|
| Example 36 | 1.0 | 247.3 | 3305.3 | 6.49 | 9.24 |
| Example 46 | 1.0 | 189.0 | 1783.0 | 3.69 | 5.78 |
| Example 53 | 2.0 | 201.0 | 3842.0 | 10.51 | 15.15 |
| Example 54 | 1.0 | 666.3 | 6252.6 | 4.12 | 5.67 |
| Example 55 | 2.0 | 614.3 | 5891.6 | 3.55 | 5.84 |
| Example 59 | 2.0 | 113.8 | 1537.4 | 5.11 | 7.52 |

Experimental Conclusion: The data in the table show that the compounds of the examples of the present invention reached a relatively high exposure in rat plasma after oral administration at a dose of 5 mg/kg. In addition, perianal pollution was observed in rats administered with the compound of Example 59, indicating that the compound of Example 59 has a gastrointestinal toxicity.

**Test Example 6. Tumor Inhibition Test on MiaPaca 2 Xenograft Model**

6.1 Experimental Objective:

Balb/c nude mice were used as test animals. *In vivo* pharmacodynamic test was carried out on human pancreatic cancer cell MiaPaca 2 xenograft (CDX) model to evaluate the anti-tumor effect of the test compounds.

6.2 Experimental Instruments and Reagents:

6.2.1 Instruments:

Ultra-clean workbench (BSC-1300II A2, Shanghai Boxun Medical Biological Instrument Corp.)
$CO_2$ incubator (Thermo-311, Thermo)
Centrifuge (Centrifuge 5720R, Eppendorf)
Automatic cell counter (Countess II, Life Technologies)
Pipette (10-20 μL, Eppendorf)
Microscope (Ts 2, Nikon)

Vernier caliper (CD-6" AX, Mitutoyo, Japan)
Cell culture flask (T25/T75/T225, Corning)
Constant temperature water tank (HWS12, Shanghai YIHENG Technical Co., Ltd.)

6.2.2 Reagents:

DMEM (11995-065, Gibco)
Fetal Bovine Serum (FBS) (10091-148, Gibco)
0.25% Trypsin (25200-056, Gibco)
Penicillin-streptomycin double antibiotics (P/S) (SV30010, GE)
Phosphate buffered saline (PBS) (10010-023, Gibco)
Matrigel (356234, Corning)
Gln (25030-081, Gibco)

6.3 Experimental Process:

MiaPaca 2 cells were taken from the cell bank and thawed. The cells were added to the DMEM medium (containing 10% FBS, 1% Glu and 1% P/S), and incubated in the $CO_2$ incubator (the temperature of the incubator was 37°C, and the $CO_2$ concentration was 5%). After the cells covered 80-90% of the bottom of the culture flask, the cells were passaged. After passage, the cells continued to be incubated in the $CO_2$ incubator. The process was repeated until the number of cells met the inoculation requirement of the *in vivo* pharmacodynamic test. The cells in logarithmic growth phase were collected, and counted by the automatic cell counter. According to the counting results, the cells were resuspended with PBS and Matrigel (volume ratio: 1:1) to obtain a cell suspension (density: $8\times10^7$/ml), which was placed in an ice box for later use.

Female Balb/c nude mice (6-8 weeks old, body weight: about 18-22 grams) were used as test animals. The mice were maintained in an environment free of special pathogens and in a single ventilated cage (five mice per cage). All cages, bedding and water were disinfected before use. All animals had free access to standard certified commercial laboratory animal diets. The nude mice were marked with a disposable universal ear tag for rats and mice before the test. The skin of the inoculation site was disinfected with 75% medical alcohol before the inoculation. Each mouse was inoculated with 0.1 ml of MiaPaca 2 tumor cells (containing $8*10^6$ cells) subcutaneously on the right back. When the average tumor volume reached 100 to 200 $mm^3$, administration was carried out. The test compounds were administered intragastrically daily. The dose, frequency of administration and the efficacy of each group at the end of the test are shown in Table 5. The tumor volume ($mm^3$) was measured with the vernier caliper twice a week. The tumor volume calculation formula: V=0.5*D*d*d, wherein D and d represent the long diameter and short diameter of the tumor, respectively. The anti-tumor efficacy was determined by dividing the average tumor increase volume of the compound-treated animals by the average tumor increase volume of the untreated animals. Tumor growth inhibition rate calculation formula: TGI (%)=1-[(Vt-V0) administration group/(Vt-V0) vehicle control group] * 100%. All animals were euthanized after the experiment.

6.4 Experimental Results:

Table 2.5: Pharmacodynamic parameters of the compounds in mice with xenograft

| Group | Tumor volume ($mm^3$, Mean ± SD) | | ΔT/ΔC (%) | TGI (%) |
|---|---|---|---|---|
| | Day 0 | Day 21 | Day 21 | Day 21 |
| Example 6<br>6 mg/kg QD × 3w | 170±28(171±25) | 183±70(780±248) | 2.24 | 97.76 |
| Example 35<br>6 mg/kg QD x 3w | 166±27(168±32) | 196±62(847±287) | 4.36 | 95.64 |
| Example 36<br>3 mg/kg QD x 3w | 172±26(173±28) | 216±66(1064±236) | 4.93 | 95.07 |

(continued)

| Group | Tumor volume ($mm^3$, Mean ± SD) | | ΔT/ΔC (%) | TGI (%) |
|---|---|---|---|---|
| | Day 0 | Day 21 | Day 21 | Day 21 |
| Example 46<br>6 mg/kg QD x 3w | 167±26(168±32) | 198±70(847±287) | 4.57 | 95.43 |
| Example 53<br>6 mg/kg QD × 3w | 166±28(168±32) | 227±37(847±287) | 9.00 | 91.00 |
| Example 55<br>10 mg/kg QD × 3w | 176±36(176±39) | 240+86(1078+395) | 7.06 | 92.94 |
| Note: The data in parentheses refers to the tumor volume of the Vehicle QD × 3w group (*i.e.* the control group) corresponding to this Example at the corresponding time (*i.e.* Day 0 and Day 21, respectively). | | | | |

Experimental Conclusion: The above data show that after 21 days of continuous oral administration, the compounds of the examples of the present invention can significantly inhibit the growth of MiaPaca 2 xenograft in nude mice under the administration condition of 3 to 10 mg/kg QD.

**Test Example 7. Pharmacokinetics Assay in Tumor-Bearing Mice**

7.1. Study Objective:

MiaPaca 2 tumor-bearing mice were used as test animals. The pharmacokinetic behavior of the compound of Example 53 after oral administration at a dose of 6 mg/kg in mouse body (plasma, tumor tissue and intestine) was studied.

7.2. Test Protocol

7.2.1 Test Compound:

Compound of Example 53, self-prepared.

7.2.2 Test Animals:

Twenty-four female MiaPaca 2 tumor-bearing mice were used. Three mice were used for each time point (0 hour, 1 hour, 2 hours, 4 hours, 6 hours, 8 hours, 16 hours and 24 hours). The mice were purchased from Shanghai Sippr-BK laboratory animal Co. Ltd., with Certificate No.: SCXK (Shanghai) 2018-0006.

7.2.3 Preparation of the Test Compound:

5 g of hydroxymethyl cellulose was dissolved in 1000 mL of purified water, and 10 g of Tween 80 was added. The solution was mixed well to form a clear solution. 4.57 mg of the compound of Example 53, 22.6 mg was dissolved in the above solution. The solution was shaked well, and subjected to ultrasound for 15 minutes to obtain a uniform suspension with a concentration of 0.6 mg/mL.

7.2.4 Administration:

After fast, MiaPaca 2 tumor-bearing mice were administered orally (p.o.) with the test compound according to body weight at an administration dose of 6 mg/kg and an administration volume of 10 mL/kg (the animals were not administered with the test compound on 0 hour).

7.2.5 Sample Collection:

The mice were euthanized by $CO_2$ before administration and after administration, and 0.5 mL of blood was taken from heart. The blood samples were stored in EDTA-2K tubes, and centrifuged for 6 minutes at 6000 rpm, at 4°C to separate the blood plasma.

The plasma samples were stored at -80°C. The tumor tissue was weighed, placed in a 2 mL centrifuge tube,

and stored at 80°C. An appropriate length of duodenum, ileum and colon tissues were taken and cut open with scissors, and the contents were removed.

The above tissues were washed with PBS twice, and the moisture was absorbed with absorbent paper. The tissues were weighed, placed in a 2 mL centrifuge tube, and stored at -80°C.

7.3 Test results: The test results were determined by LCMS/MS method as shown in Table 2.6.

Table 2.6: Pharmacokinetic parameters of the compound of the present invention in mice

| Compound No. | | Tmax (hr) | Cmax (ng/mL) | $AUC_{0-\infty}$ (ng/mL*hr) | $T_{1/2}$ (hr) | MRT (hr) |
|---|---|---|---|---|---|---|
| Example 53 | Plasma | 1.0 | 385.7 | 2857.0 | 4.8 | 7.5 |
| | Tumor | 8.0 | 2388.3 | 51774.2 | 11.5 | 19.1 |
| | Duodenum | 1.0 | 5416.7 | 16252.0 | 4.8 | 4.8 |
| | Ileum | 2.0 | 1928.3 | 10008.3 | 5.3 | 6.1 |
| | Colon | 4.0 | 1310.0 | 9182.5 | 2.8 | 5.8 |

Experimental Conclusion: The data in the table show that at a dose of 6 mg/kg, the exposure of the compound of the example of the present invention in mice tumor reached a very high level, and the exposure in tumor was significantly higher than that in blood. It can be seen from Tmax and MRT that the concentration of the compound in tumor gradually increased and the $T_{1/2}$ was longer, indicating that the compound will gradually accumulate in tumor and always maintain a higher concentration in tumor, thereby ensuring a better tumor inhibition effect.

**Test Example 8. Test of Inhibitory Activity on hERG Potassium Channel**

8.1 Cell Preparation

8.1.1 CHO-hERG cells were cultured in a 175 $cm^2$ culture flask. When the cells were grown to a density of 60 to 80%, the culture solution was removed, and the cells were washed once with 7 mL of PBS, and then 3 mL of Detachin was added for digestion of the cells.

8.1.2 After the digestion was complete, the cells were neutralized by adding 7 mL of the culture solution and then centrifuged. The supernatant was removed, and then 5 mL of the culture solution was added for resuspension to ensure a cell density of 2 to $5\times10^6$ cells/mL.

8.2 Solution Preparation

Table 2.7: Components of intracellular fluid and extracellular fluid

| Reagent | Extracellular fluid (mM) | Intracellular fluid (mM) |
|---|---|---|
| $CaCl_2$ | 2 | 5.374 |
| $MgCl_2$ | 1 | 1.75 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity~305 mOsm | 7.25 (adjusted with KOH), Osmolarity~290 mOsm |

8.3 Electrophysiological Recording Process

The single-cell high-impedance sealing and whole-cell pattern formation processes were both completed automatically by the Qpatch instrument. After the whole-cell recording pattern was obtained, the cells were clamped at -80 millivolts. A pre-voltage of -50 millivolts was applied for 50 milliseconds prior to the application of a depolarizing stimulus of +40 millivolts for 5 seconds, and then the cells were repolarized to -50 millivolts and maintained for 5 seconds, and then returned back to -80 millivolts. This voltage stimulation was applied every 15 seconds, and data were recorded for 2 minutes, and thereafter the extracellular fluid was administered and data were recorded for 5 minutes, and then the administration process was started. The compound was administered from the lowest test concentration, and 2.5 minutes were given for each test concentration. After all the concentrations of the compound were administered, 3 M Cisapride as a positive control compound was administered. At least three cells (n≥3) were tested for each concentration.

8.4 Compound Preparation

8.4.1 20 mM compound mother solution was diluted with the extracellular fluid. 5 μL of the 20 mM compound mother solution was diluted 500 times to 40 μM by adding 2495 μL of the extracellular fluid, and then 3-fold serial dilutions were performed sequentially in the extracellular fluid containing 0.2% DMSO to obtain a final concentration to be tested.

8.4.2 The highest test concentration was 40 μM, and there were six concentrations in total, *i.e.,* 40, 13.33, 4.44, 1.48, 0.49, and 0.16 μM, respectively.

8.4.3 The DMSO content in the final test concentration was not more than 0.2%. This concentration of DMSO had no effect on the hERG potassium channel.

8.5 Data Analysis

The experimental data were analyzed by XLFit software.

8.6 Quality Control

Environment: Humidity 20 to 50% and temperature 22 to 25°C

Reagents: The experimental reagents used were purchased from Sigma, with a purity >98%.

The experimental data in the report must meet the following criteria:

Whole cell sealing impedance > 100 MΩ

Tail current amplitude > 400 pA

Pharmacological parameters:

The inhibitory effect of multiple concentrations of Cisapride on hERG channel was set as a positive control.

8.7 Experimental Results

The inhibitory effect of the compounds of the examples at multiple concentrations on hERG current is as follows:

Table 2.8: Inhibitory effect of the compounds of the examples at multiple concentrations on hERG current

| Example No. | hERG (uM) |
|---|---|
| Example 6 | 5.24 |
| Example 35 | 6.17 |
| Example 36 | 2.34 |
| Example 46 | 18.78 |
| Example 53 | 4.51 |
| Example 55 | 21.91 |

Experimental Conclusion: The inhibition of a drug on the cardiac hERG potassium channel is the main reason that the drug causes QT prolongation syndrome. It can be seen from the experimental results that the compounds of the examples of the present invention have no obvious inhibitory effect on cardiac hERG potassium channel, thereby avoiding cardiotoxic side effects at high doses.

**Test Example 9. Pharmacokinetics Assay in Mice**

9.1. Study Objective:

Balb/c mice were used as test animals. The pharmacokinetic behavior of the compounds of Examples 53 and 59 after single intravenous injection in mouse body (plasma) was studied.

9.2. Test Protocol

9.2.1 Test Compounds:

Compounds of Examples 53 and 59, self-prepared;

9.2.2 Test Animals:

Male Balb/c mice were purchased from Shanghai Jiesijie Laboratory Animal Co., LTD, with Certificate No.: SCXK (Shanghai) 2013-0006 N0.311620400001794.

9.2.3 Preparation of the Test Compounds:

Aqueous solution containing 10% Solutol HS15: 10 g of Solutol HS15 was added to purified water for injection, and dissolved in it under stirring. The solution was diluted to 100 mL with purified water for injection and shaked well to obtain the desired solution.

Aqueous solution containing 20% HP-β-CD: 20 g of HP-β-CD was added to purified water for injection, and dissolved in it under stirring. The solution was diluted to 100 mL with purified water for injection and shaked well to obtain the desired solution.

0.5 ml of DMSO, 8.5 ml of 20% HP-β-CD aqueous solution and 1 ml of 10% Solutol HS15 aqueous solution were mixed and shaked well to obtain a clear solution.

0.234 mg of the compounds of Examples 53 and 59 were weighed and added to 1.17 mL of the above solution. The solution was subjected to ultrasound to obtain a clear solution. The solution was filtered with a 0.22 μm water filter, and the filtrate was the formulation for intravenous administration (i.v.). The concentration was 0.2 mg/mL.

9.2.4 Administration:

After an overnight fast, male Balb/c mice were administered intravenously with the test compounds at an administration dose of 1 mg/kg and an administration volume of 5 mL/kg.

9.2.5 Sample Collection:

Blood was taken before administration and at 0 minute, 5 minutes, 15 minutes, 0.5 hour, 1.0 hour, 2.0 hours, 4.0 hours, 8.0 hours and 24.0 hours after administration. The blood samples were stored in EDTA-2K tubes, and centrifuged for 6 minutes at 6000 rpm, at 4°C to separate the blood plasma. The plasma samples were stored at -80°C. The mice were fed 4 hours after administration.

9.3 Test results: The test results were determined by LCMS/MS method as shown in Table 2.9.

Table 2.9: Pharmacokinetic parameters of the compounds in mice

| Example No. | $C_0$ (ng/mL) | $AUC_{0-\infty}$ (ng/mL*hr) | $T_{1/2}$ (hr) | MRT (hr) | CL (mL/min/kg) | Vss (L/kg) | F (%) |
|---|---|---|---|---|---|---|---|
| Example 53 | 264.5 | 736.0 | 7.74 | 9.04 | 22.6 | 12.3 | 71 |
| Example 59 | 257.4 | 404.0 | 5.81 | 5.96 | 41.3 | 14.7 | 150 |

Experimental conclusion: The compounds of the examples of the present invention have good metabolic characteristics in mouse body at a dose of 1 mg/kg.

**Test Example 10. Pharmacokinetics Assay in Rats**

10.1. Study Objective:

SD rats were used as test animals. The pharmacokinetic behavior of the compounds of Examples 53 and 59 after single intravenous injection in rat body (plasma) was studied. 10.2. Test Protocol

10.2.1 Test Compounds:

Compounds of Examples 53 and 59, self-prepared.

10.2.2 Test Animals:

Male SD rats (3 rats per group) were purchased from Shanghai Jiesijie Laboratory Animal Co., LTD, with Certificate No.: SCXK (Shanghai) 2013-0006 N0.311620400001794.

10.2.3 Preparation of the Test Compounds:

Aqueous solution containing 10% Solutol HS15: 10 g of Solutol HS15 was added to purified water for injection, and dissolved in it under stirring. The solution was diluted to 100 mL with purified water for injection and shaked well to obtain the desired solution.

Aqueous solution containing 20% HP-β-CD: 20 g of HP-β-CD was added to purified water for injection, and dissolved in it under stirring. The solution was diluted to 100 mL with purified water for injection and shaked well to obtain the desired solution.

0.5 ml of DMSO, 8.5 ml of 20% HP-β-CD aqueous solution and 1 ml of 10% Solutol HS15 aqueous solution were mixed and shaked well to obtain a clear solution.

0.78 mg of the compounds of Examples 53 and 59 were weighed and added to 3.9 mL of the above solution. The solution was subjected to ultrasound to obtain a clear solution. The solution was filtered with a 0.22 μm water filter, and the filtrate was the formulation for intravenous administration (i.v.). The concentration was 0.2 mg/mL.

10.2.4 Administration:

After an overnight fast, male SD rats (3 rats per group) were administered intravenously with the test compounds at an administration dose of 1 mg/kg and an administration volume of 5 mL/kg.

10.2.5 Sample Collection:

0.2 mL of blood was taken from jugular vein before administration and at 0 minute, 5 minutes, 15 minutes, 0.5 hour, 1.0 hour, 2.0 hours, 4.0 hours, 8.0 hours and 24.0 hours after administration. The blood samples were stored in EDTA-2K tubes, and centrifuged for 6 minutes at 6000 rpm, at 4°C to separate the blood plasma. The plasma samples were stored at -80°C. The rats were fed 4 hours after administration.

10.3 Test results: The test results were determined by LCMS/MS method as shown in Table 2.10.

Table 2.10: Pharmacokinetic parameters of the compounds of the present invention in rats

| Example No. | $C_0$ (ng/mL) | $AUC_{0-\infty}$ (ng/mL*hr) | $T_{1/2}$ (hr) | MRT (hr) | CL (mL/min/kg) | Vss (L/kg) | F (%) |
|---|---|---|---|---|---|---|---|
| Example 53 | 311.1 | 612.6 | 3.35 | 4.26 | 27.2 | 7.0 | 118 |
| Example 59 | 516.5 | 427.6 | 1.83 | 1.79 | 39.0 | 4.2 | 72 |

Experimental conclusion: The compounds of the examples of the present invention have good metabolic characteristics in rat body at a dose of 1 mg/kg.

**Test Example 11. Tolerance Assay in Mice**

11.1. Study Objective:

Balb/c mice were used as test animals. The tolerance of the compounds of Examples 53 and 59 after oral administration in female and male mice was studied.

11.2. Test Protocol

11.2.1 Test Compounds:

Compounds of Examples 53 and 59, self-prepared;

11.2.2 Test Animals:

Balb/c mice (9 male and 9 female) were purchased from Shanghai Jiesijie Laboratory Animal Co., LTD, with Certificate No.: SCXK (Shanghai) 2013-0006 N0.311620400001794.

11.2.3 Preparation of the Test Compounds:

5 g of hydroxyethyl cellulose (HEC, CMC-Na, viscosity: 800-1200 Cps) was dissolved in 1000 mL of purified water, and 10 g of Tween 80 was added. The solution was mixed well to form a clear solution.

41.0 mg of the compound of Example 53 and 25.9 mg of the compound of Example 59 were respectively added to a 100-mL glass bottle, followed by the addition of 41.0 mL and 25.9 mL of the above solution. The solution was subjected to ultrasound for 10 minutes to obtain a colorless and clear solution with a concentration of 1 mg/mL.

11.2.4 Administration:

After an overnight fast, Balb/c mice (3 male and 3 female) were administrated orally with the test compound at an administration dose of 10 mg/kg and an administration volume of 10 mL/kg.

11.2.5 Data Collection:

The mice were weighed daily before and after administration, and abnormal responses were observed. The mice were fed 4 hours after administration.

11.2.6 Test Results:

The body weight was weighed with a balance, and the test results were shown in Table 2.11.

Table 2.11: Comparison of mouse body weight changes

| Group | Body weight (g, Mean) | | Body weight changes △W/W1 (%, Mean) |
|---|---|---|---|
| | Day 1 | Day 16 | Day 16 |
| Control group QD male | 22.99 | 27.34 | 18.98 |
| Example 53 10 mg/kg QD male | 23.01 | 28.30 | 23.25 |
| Example 59 10 mg/kg QD male | 23.03 | 21.57 | -8.02 |
| Control group QD female | 22.00 | 23.77 | 7.99 |
| Example 53 10 mg/kg QD female | 22.07 | 25.09 | 13.80 |
| Example 59 10 mg/kg QD female | 22.10 | 18.74 | -15.32 |

Experimental Conclusion: The above data show that the compounds of the examples of the present invention have a good tolerance in mice at a dose of 10 mg/kg. However, the mice administered with the compound of Example 59 showed loose stools, blood in the stool, significant weight loss and the like, indicating that this compound has a poor tolerance.

In summary, the present invention provides a series of highly active and selective SHP2 kinase inhibitors with novel structures. These inhibitors show good pharmacokinetic characteristics in both rats and mice, and also show good efficacy on Miapaca2 tumor-bearing mouse model. These inhibitors have great potential to be developed into single drugs or combination drugs for treating a tumor disease.

**III. Study on the Crystal Forms of (S)-8-(6-Amino-5-((2-amino-3-chloropyridin-4-yl)thio)pyrazin-2-yl)-2-cyclopropyl-8-azaspiro[4.5]dec-2-en-1-amine**

1.1 Experimental Instruments

1.1.1 Some Parameters of Physical and Chemical Testing Instruments

| | | |
|---|---|---|
| XRPD | Instrument model | BRUKER D8 ADVANCE |
| | Diffraction ray | CuK (40 kV, 25 mA) |
| | Scan rate | 0.02°/S (2θ value) |
| | Scan range | 4° to 40° (2θ value) |
| DSC | Instrument model | NETZSCH DSC 214 polyma |
| | Purge gas | Nitrogen |
| | Purge rate | 40 mL/min |
| | Heating rate | 1 0 °C /min |
| | Temperature range | 25 to 350 °C |
| | Plate type | Aluminum plate |
| TGA | Instrument model | NETZSCH TG 209 Tarsus |
| | Purge gas | Nitrogen |
| | Purge rate | 40 mL/min |
| | Heating rate | 1 0 °C /min |
| | Temperature range | Room temperature ~ 400 °C |
| | Plate type | $Al_2O_3$ |

1.2 Instruments and Liquid Chromatography Conditions

1.2.1 Instruments and Devices

| Instrument name | Model |
|---|---|
| Analytical Balances | Sartorius BSA224S-CW |
| Water purifier | Milli-Q Plus, Millipore |
| High performance liquid chromatography | Agilent1260 |
| Pump | Agilent G1311B |
| Injector | G1329B |
| Column oven | G1316A |
| Detector | G1315D |

1.2.2 Chromatographic Conditions

Column: Waters Xbridge C18 (4.6mm*150mm, 3.5 μm)
Flow rate: 1 mL/min
Column temperature: 40°C
Detection wavelength: 275 nm
Injection volume: 5.0 μL
Running time: 30 min
Diluent: DMSO
Mobile phase: A: water (0.05% trifluoroacetic acid); B: acetonitrile (0.05% trifluoroacetic acid)

| T (min) | A (%) | B (%) |
|---|---|---|
| 0.00 | 90 | 10 |
| 15.00 | 70 | 30 |
| 25.00 | 30 | 70 |
| 25.01 | 90 | 10 |
| 30.00 | 90 | 10 |

**2. Preparation of Various Crystal Forms of (S)-8-(6-Amino-5-((2-amino-3-chloropyridin-4-yl)thio)pyrazin-2-yl)-2-cyclopropyl-8-azaspiro[4.5]dec-2-en-1-amine**

**2.1 Preparation of Crystal Form A of (S)-8-(6-Amino-5-((2-amino-3-chloropyridin-4-yl)thio)pyrazin-2-yl)-2-cyclopropyl-8-azaspiro[4.5]dec-2-en-1-amine**

5 g of (S)-2-cyclopropyl-8-azaspiro[4.5]dec-2-en-1-amine hydrochloride was added to 75 mL of NMP under a nitrogen atmosphere, and the temperature was adjusted to 20 to 30°C. 3.17g of $NaHCO_3$ was added, and the resulting solution was stirred for 10 minutes. 4.35 g of 3-((2-amino-3-chloropyridin-4-yl)thio)-6-chloropyrazin-2-amine and 5.2 g of $K_2CO_3$ were added, and the reaction solution was bubbled with nitrogen for 30 minutes. The reaction solution was warmed up to 85°C, and stirred for 8 hours. The reaction solution was cooled to room temperature, and 120 mL of DCM and 150 mL of pure water were added. The solution was stirred and separated into two phases, and the aqueous phase was extracted with 120 mL of DCM once. The organic phases were combined, and washed with pure water (180 mL) five times to remove NMP and 15% aqueous sodium chloride solution (180 mL) once. 0.75 g of activated carbon was added to the DCM phase, which was then stirred for 1 hour and filtered, and the filter cake was rinsed with 30 mL of THF. 1 g of mercapto silica gel was added to the filtrate, which was then stirred for 3 hours and filtered, and the filter cake was rinsed with 30 ml of THF. The filtrate was concentrated to dryness to obtain 9 g of yellow oily solid. 30 mL of EA/MTBE (1/1) was added, and the solution was heated to 50°C and pulped for 0.5 hour. The solution was naturally cooled to 20°C, stirred for 4 hours, and filtered. The filter cake was rinsed with MTBE, and dried under vacuum to obtain 3.6 g of light yellow solid. According to detection and analysis, it had the XRPD data shown in Table 6, the XRPD pattern shown in Figure 12, the DSC spectrum shown in Figure 13, and the TGA spectrum shown in Figure 14.

**2.2 Preparation of Crystal Form B of (S)-8-(6-Amino-5-((2-amino-3-chloropyridin-4-yl)thio)pyrazin-2-yl)-2-cyclopropyl-8-azaspiro[4.5]dec-2-en-1-amine**

About 20 mg of crystal form A of (S)-8-(6-amino-5-((2-amino-3-chloropyridin-4-yl)thio)pyrazin-2-yl)-2-cyclopropyl-8-azaspiro[4.5]dec-2-en-1-amine was weighed, and dissolved in 0.6 mL of 2-methyl-tetrahydrofuran at 70°C under heating to be a clear solution. The solution was quickly placed into 2°C condition, and stirred for 12 hours to precipitate a solid. The mixture was centrifuged at high speed, the supernatant was removed, and the solid was vacuum dried at 40°C to obtain crystal form B of (S)-8-(6-amino-5-((2-amino-3-chloropyridin-4-yl)thio)pyrazin-2-yl)-2-cyclopropyl-8-azaspiro[4.5]dec-2-en-1-amine. According to detection and analysis, it had the XRPD data shown in Table 7, the XRPD pattern shown in Figure 15, and the DSC spectrum shown in Figure 16.

**2.3 Preparation of Crystal Form C of (S)-8-(6-Amino-5-((2-amino-3-chloropyridin-4-yl)thio)pyrazin-2-yl)-2-cyclopropyl-8-azaspiro[4.5]dec-2-en-1-amine**

About 20 mg of crystal form A of (S)-8-(6-amino-5-((2-amino-3-chloropyridin-4-yl)-thio)pyrazin-2-yl)-2-cyclopropyl-8-azaspiro[4.5]dec-2-en-1-amine was weighed in a 2 mL glass flask, and dissolved in 200 μL of NMP at room temperature under stirring to be a clear solution. 0.6 mL of isopropyl ether was added, and the mixture was stirred at room temperature for 12 hours to precipitate a solid. The mixture was centrifuged at high speed, the supernatant was removed, and the solid was dried under vacuum at 40°C to obtain crystal form C of (S)-8-(6-amino-5-((2-amino-3-chloropyridin-4-yl)thio)-pyrazin-2-yl)-2-cyclopropyl-8-azaspiro[4.5]dec-2-en-1-amine. According to detection and analysis, it had the XRPD data shown in Table 8, the XRPD pattern shown in Figure 17, and the DSC spectrum shown in Figure 18.

**3. Screening of Various Crystal Forms of (S)-8-(6-Amino-5-((2-amino-3-chloro-pyridin-4-yl)thio)pyrazin-2-yl)-2-cyclopropyl-8-azaspiro[4.5]dec-2-en-1-amine and**

**Determination of Stable Crystal Forms**

3.1 Experimental Objective:

The objective is to find stable crystal forms of the compound (S)-8-(6-amino-5-((2-amino-3-chloropyridin-4-yl)thio)pyrazin-2-yl)-2-cyclopropyl-8-az aspiro[4.5]dec-2-en-1-amine by polymorphic screening.

3.2 Experimental Method and Results

**(1) Transformation Test in Slurry**

About 20 mg of crystal form A of the compound (S)-8-(6-amino-5-((2-amino-3-chloropyridin-4-yl)thio)pyrazin-2-yl)-2-cyclopropyl-8-az aspiro[4.5]dec-2-en-1-amine was weighed in a 2 mL glass flask, followed by the addition ofg 10 to 200 μL of relative organic solvents respectively, and the samples were uniformly shaken at 50°C for 4 days. If the sample was a clear solution, the solution would be placed into room temperature and stirred. The suspension was centrifuged at high speed, the supernatant was removed, and the solid was dried under vacuum at 40°C. The XRPD, DSC and TGA of the resulting solids were determined and compared. Experimental results are shown in Table 3.1.

Table 3.1 Results of transformation test in slurry

| Solvent | Crystal form |
|---|---|
| MeOH | A |
| EtOH | A |
| ACN | A |
| EA | A |
| IPA | A |
| DCM | A |
| $H_2O$ | A |
| Toluene | A |
| IPAC | A |

The above results show that crystal form A of the compound (S)-8-(6-amino-5-((2-amino-3-chloropyridin-4-yl)thio)pyrazin-2-yl)-2-cyclopropyl-8-az aspiro[4.5]dec-2-en-1-amine didn’t undergo crystal form transformation during pulping in the above solvents, crystal form A is thus stable.

**(2) Cooling Method and Volatilization Method Test**

A certain amount of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was weighed in a 2 mL glass flask, and dissolved

in some good solvents under heating respectively to be a clear solution. The clear solution was cooled, or an anti-solvent was added to the clear solution, and stirred to see if there would be solid precipitation. If there was solid, the solution would be centrifuged at high speed, the supernatant would be removed, and the solid would be vacuum dried at 40°C. If there was no solid, the solution would be kept open at room temperature to evaporate the solvent. The XRPD, DSC and TGA of the resulting solids were determined and compared. Experimental results are shown in Table 3.2.

Table 3.2 Cooling method and volatilization method test

| Solvent | Cooling | Anti-solvent | Volatilization results |
|---|---|---|---|
| 2-Me-THF | Crystal form B | - | - |
| MeOH | No solid precipitation | - | Crystal form A |
| DCM | No solid precipitation | - | Crystal form A |
| THF | No solid precipitation | Still no solid precipitation after adding isopropyl ether | Crystal form A |
| 88% Acetone | No solid precipitation | - | Crystal form A |

The above results show that crystal form B of the compound (S)-8-(6-amino-5-((2-amino-3-chloropyridin-4-yl)thio)pyrazin-2-yl)-2-cyclopropyl-8-azaspiro[4.5]dec-2-en-1-amine can be obtained by the rapid cooling method using 2-Me-THF as the solvent. No solid was precipitated by the rapid cooling method using MeOH, DCM, THF or 88% Acetone as the solvent, and crystal form A was still obtained after volatilization.

**(3) Solventing-out Crystallization Method Test**

A certain amount of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was weighed in a 2 mL glass flask, and dissolved in some solvents (as good solvents) respectively to be a clear solution. Some solvents (as anti-solvents) were added dropwise to the solution respectively, which was stirred to see if there would be solid precipitation. If there was solid, the solution would be centrifuged at high speed, the supernatant would be removed, and the solid would be dried under vacuum at 40°C. The XRPD, DSC and TGA of the resulting solids were determined and compared. Experimental results are shown in Table 3.3.

Table 3.3 Solventing-out crystallization method test

| Good solvent | Anti-solvent | Experimental results |
|---|---|---|
| DMF | $H_2O$ | A |
| DMSO | | A |
| THF | n-Heptane | A |
| NMP | Isopropyl ether | C |

The above results show that crystal forms A and C of the compound (S)-8-(6-amino-5-((2-amino-3-chloropyridin-4-yl)thio)pyrazin-2-yl)-2-cyclopropyl-8-azaspiro[4.5]dec-2-en-1-amine were obtained by the solventing-out crystallization screening.

3.3 Experimental Conclusion:

Three crystal forms of (S)-8-(6-amino-5-((2-amino-3-chloropyridin-4-yl)thio-pyrazin-2-yl)-2-cyclopropyl-8-azaspiro[4.5]dec-2-en-1-amine, *i.e.,* crystal forms A, B and C, were obtained by pulping, changing crystallization solvent and crystallization method. By comparing the DSC spectrums of different crystal forms of the compound (S)-8-(6-amino-5-((2-amino-3-chloropyridin-4-yl)thio)pyrazin-2-yl)-2-cyclopropyl-8-azaspiro[4.5]dec-2-en-1-amine, it can be seen that crystal form A has better thermodynamic stability, and is anhydrous.

**4. Solid Stability Experiment**

4.1 Experimental Objective:

The physicochemical stability of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine under the conditions of high temperature (60°C), room temperature/high humidity (92.5% RH) and high temperature/high humidity (50°C and 75% RH) was investigated, so as to provide a basis for the storage of the compound.

4.2 Experimental Scheme:

About 2 mg of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was investigated under the conditions of high temperature (60°C), room temperature/high humidity (92.5% RH) and high temperature/high humidity (50°C and 75% RH) for 5, 10, 20 and 30 days. The content was determined by HPLC using the external standard method. The related substance change was calculated by chromatographic peak area normalization method.
4.3 Experimental Results: The stability results are shown in Table 3.4.

Table 3.4 Solid stability results

| Days / Condition | Stability results of crystal form A (maximum single impurity increase %) | | | |
|---|---|---|---|---|
| | 5 days | 10 days | 20 days | 30 days |
| High temperature (60°C) | <0.05 | <0.05 | <0.10 | <0.10 |
| Room temperature/high humidity (92.5% RH) | <0.05 | <0.05 | <0.14 | <0.12 |
| High temperature/high humidity (50°C and 75% RH) | <0.05 | <0.05 | <0.13 | <0.10 |

4.4 Experimental Conclusion:

The above data show that crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine is stable under the conditions of high temperature (60°C), room temperature/high humidity (92.5% RH) and high temperature/high humidity (50°C and 75% RH), and the impurity increase is less than 0.15%.

**5. Hygroscopicity Experiment**

5.1 Experimental Objective

The hygroscopicity of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine under different relative humidity conditions was investigated, so as to provide a basis for the crystal form screening and storage of the compound.

5.2 Experimental scheme:

Crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was placed in saturated water vapor with different relative humidity to achieve dynamic equilibrium between the compound and water vapor, and the percentage of hygroscopic weight gain of the compound after the equilibrium was calculated.

5.3 Experimental Results:

Crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine had a hygroscopic weight gain of about 0.544% under RH 80% condition, and had

slight hygroscopicity. After one cycle of humidification and dehumidification under 0 to 95% relative humidity condition, the XRPD pattern of crystal form A of the compound (S)-8-(6-amino-5-((2-amino-3-chloropyridin-4-yl)-thio)pyrazin-2-yl)-2-cyclopropyl-8-azaspiro[4.5]dec-2-en-1-amine didn't change, *i.e.,* the crystal form didn't transform.

**6. Solubility Experiment in Different Media**

6.1 Experimental Objective

The solubility of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine in media such as buffers with pH 1 to 8 (hydrochloric acid buffers with pH=1 and 2, phthalic acid buffers with pH=3 and 4, and phosphate buffers with pH=5, 6, 7 and 8), water, Fasted State Simulated Gastric Fluid (FaSGF), Fasted State Simulated Intestinal Fluid (FaSSIF) and Fed State Simulated Intestinal Fluid (FeSSIF) was compared, so as to provide a basis for evaluating the druggability.

6.2 Experimental Scheme:

About 2 mg of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was suspended in different media for 2 hours, respectively. Thermodynamic solubility of the compound at 37°C was determined by HPLC using the external standard method.

6.3 Experimental results are shown in Table 3.5.

Table 3.5 Solubility experiment in different media

| Media | Solubility of crystal form A (mg/mL) |
|---|---|
| pH 1.0 | > 1.648 |
| pH 2.0 | > 1.485 |
| pH 3.0 | 0.700 |
| pH 4.0 | 0.446 |
| pH 5.0 | 0.223 |
| pH 6.0 | 0.401 |
| pH 7.0 | 0.062 |
| pH 8.0 | 0.017 |
| $H_2O$ | 0.005 |
| FaSSGF | > 1.707 |
| FaSSIF | 0.088 |
| FeSSIF | 0.432 |

6.4 Experimental Conclusion:

By comparing the solubility of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine in different media, it can be seen that crystal form A has better solubility under acidic conditions.

**7. Crystal Transformation Investigation Experiment**

7.1 Experimental Objective:

The objective is to investigate whether crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine will undergo crystal transformation by pressure crystal transformation and grinding crystal transformation experiments.

7.2 Experimental Scheme:

A certain amount of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was weighed, and compressed into tablets with a tablet machine. The tablets were mashed to powder and characterized by XRPD.

A certain amount of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was weighed, vigorously ground with a mortar for 15 minutes, and characterized by XRPD.

7.3 Experimental Conclusion:

By comparing the XRPD patterns, it can be seen that crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine didn't undergo crystal transformation after tabletting and grinding, crystal form A is thus stable.

**8. Pharmacokinetics Study in Rats**

8.1 Experimental Objective:

The pharmacokinetic behavior of crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine after single oral administration in rat body (plasma) was studied, and the bioavailability for oral administration was calculated.

8.2 Experimental Instruments and Reagents:

8.2.1 Instruments:

| Instrument | Manufacturer | Model |
|---|---|---|
| Analytical Balances | Sartorius Scientific Instruments (Beijing) Co., Ltd. | SQP |
| Centrifuge | EPPENDORF | Centrifuge 5415R |
| Ultrasonic cleaner | Kun Shan Ultrasonic Instruments Co., Ltd. | KQ-100B |
| Pipette | Eppendorf Lab Technologies (Shanghai) Co., Ltd. | YE5K655563(1000uL) |
| | | YE5A592012(200uL) |
| | | YE4A298094(100uL) |
| | | YE4A405553(10uL) |
| Triple quadrupole mass spectrometry | Thermo Fisher Scientific Inc. | Thermo TSQ Ultra |
| High performance liquid chromatography | Agilent Technologies, Inc. | Agilent 1100 |
| Ultrasonic cell disruptor | Nanjing Shunma Instrument Equipment Co., Ltd. | SM-650D |
| 4°C refrigerator | Hefei Meiling Co.,Ltd. | SC-316 |
| -20°C refrigerator | Zhongke Meiling Cryogenics Co.,Ltd. | DW-YL450 |

8.2.2 Reagents:

| Reagent | Supplier | Batch number | Note |
|---|---|---|---|
| DMSO | Vetech | WXBD0293V | |
| Acetonitrile | Sigma-Aldrich | WXBD0232V | |

(continued)

| Reagent | Supplier | Batch number | Note |
|---|---|---|---|
| Methyl Alcohol | Sigma-Aldrich | WXBC6573V | |
| Formic Acid | Fisher Scientific | 193497 | |
| Dexamethasone | Solarbio | 822A0523 | Internal Standard |
| HP-β-CD | Shandong Binzhou Zhiyuan Biotechnology Co., Ltd. | 20190517 | |
| HPMC K4M | Damas-beta | P1575251 | |

8.3 Test Animals:

| Animal species | Supplier | Certificate No. |
|---|---|---|
| SD rats (7 weeks old, body weight 180 to 200 g) | JOINN (Suzhou) Laboratories Co., Ltd. | 202009863 |

8.4 Test Compound:

Crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine, self-prepared.

8.5 Experimental Scheme:

Crystal form A of the compound (S)-8-(6-amino-5-((2-amino-3-chloropyridin-4-yl)thio)pyrazin-2-yl)-2-cyclopropyl-8-azaspiro[4.5]dec-2-en-1-amine was suspended evenly in an aqueous solution containing 0.5% HPMC K4M, and then intragastrically administered to rats in triplicate at a dose of 10 mg/kg.

8.6 Experimental Results:

Table 3.6 Results of the pharmacokinetics experiment in rats

| Test compound | Pharmacokinetics experiment (10 mg/kg) | | | | | |
|---|---|---|---|---|---|---|
| | Peak time | Area under curve | plasma concentration | Half life | Average residence time | Bioavailability |
| | $t_{max}$ (h) | $AUC_{0-t}$ (ng/mL*h) | $C_{max}$(ng/mL) | $t_{1/2}$(h) | $MRT_{0-\infty}$(h) | Bio avialibility (%) |
| Crystal form A | 1.00 | 1436.77 | 359.90 | 6.99 | 5.17 | 76.20 |

8.7 Experimental Conclusion:

It can be seen from the results of the pharmacokinetics experiment in rats shown in the table that crystal form A of the compound (S)-8-(6-amino-5-((2-amino-3-chloro-pyridin-4-yl)thio)pyrazin-2-yl)-2-cyclopropyl-8-azaspiro[4.5]dec-2-en-1-amine of the present invention showed good metabolic property at the dose of 10 mg/kg.

**IV. Study on the Crystal Forms of (S)-1'-(6-Amino-5-((2-amino-3-chloro-pyridin-4-yl)thio)-3-fluoropyrazin-2-yl)-1,3-dihydrospiro[indene-2,4'-piperidin1-1-amine**

Scale-up production of the amorphous form of (S)-1'-(6-amino-5-((2-amino-3-chloropyridin-4-yl)thio)-3-fluoropyrazin-2-yl)-1,3-dihydrospiro[indene-2,4'-piperidin]-1-amine (Example 53)

(S)-1'-(6-Amino-5-((2-amino-3-chloropyridin-4-yl)thio)pyrazin-2-yl)-1,3-dihydrospiro[indene-2,4'-piperidin]-1-amine and THF were added to a reactor, then N-fluorobisbenzenesulfonamide (NFSI) was added, and the reaction was

carried out at room temperature for 2 hours. 2 L of 0.5 mol/L hydrochloric acid solution was added, and the reaction solution was extracted with 2 L of EA under stirring and separated into two phases. 3 L of 0.25 mol/L hydrochloric acid solution was added to the organic phase, which was then extracted with 11 L of EA under stirring and separated into two phases. The aqueous phases were combined, and the organic phase was discarded. The aqueous phase was extracted with 1.5 L*2 of EA to remove the impurities, followed by adding 2.65 L of THF and stirring. Solid sodium carbonate was added to adjust pH=9 to 10, and the solution was separated into two phases. The aqueous phase was extracted with 1.5 L of 2-methyltetrahydrofuran. The organic phases were combined, dried, filtered and concentrated to obtain 73.4 g of a tan solid. The solid was dissolved in tetrahydrofuran to be a clear solution, and the resulting product was washed with EA:PE=20%, 60%, EA containing 0.005% triethylamine, and methanol (containing 5% triethylamine):DCM=5% successively. The buffer was concentrated under reduced pressure to obtain 41.1 g of a yellow solid for later use.

1.1 Experimental Instruments

1.1.1 Some Parameters of Physical and Chemical Testing Instruments

| | | |
|---|---|---|
| XRPD | Instrument model | BRUKER D8 ADVANCE |
| | Diffraction ray | CuK (40 kV, 25 mA) |
| | Scan rate | 0.02°/S (2θ value) |
| | Scan range | 4° to 40° (2θ value) |
| DSC | Instrument model | NETZSCH DSC 214 polyma |
| | Purge gas | Nitrogen |
| | Purge rate | 40 mL/min |
| | Heating rate | 1 0 °C /min |
| | Temperature range | 25 to 350 °C |
| | Plate type | Aluminum plate |
| TGA | Instrument model | NETZSCH TG 209 Tarsus |
| | Purge gas | Nitrogen |
| | Purge rate | 40 mL/min |
| | Heating rate | 1 0 °C /min |
| | Temperature range | Room temperature ~ 400 °C |
| | Plate type | $Al_2O_3$ |

1.2 Instruments and Liquid Chromatography Conditions

1.2.1 Instruments and Devices

| Instrument name | Model |
|---|---|
| Analytical Balances | Sartorius BSA224S-CW |
| Water purifier | Milli-Q Plus, Millipore |
| High performance liquid chromatography | Agilent1260 |
| Pump | Agilent G1311B |
| Injector | G1329B |

(continued)

| Instrument name | Model |
|---|---|
| Column oven | G1316A |
| Detector | G1315D |

1.2.2 Chromatographic Conditions

Column: ZORBAX (SB-C8, 3.5 μm, 4.6*75 mm)
Flow rate: 1 mL/min
Column temperature: 40°C
Detection wavelength: 275 nm
Injection volume: 5.0 μL
Running time: 13 min
Diluent: ACN-water (v/v, 1:1)
Mobile phase: A: water (0.05% trifluoroacetic acid); B: acetonitrile (0.05% trifluoroacetic acid)

| T(min) | A (%) | B (%) |
|---|---|---|
| 0.00 | 90 | 10 |
| 3.00 | 70 | 30 |
| 10.00 | 40 | 60 |
| 10.10 | 90 | 10 |
| 13.00 | 90 | 10 |

**1. Preparation of Various Crystal Forms of (S)-1'-(6-Amino-5-((2-amino-3-chloropyridin-4-yl)thio)-3-fluoropyrazin-2-yl)-1,3-dihydrospiro[indene-2,4'-piperidin]-1-amine**

1.1 Preparation of Crystal Form A of (S)-1'-(6-Amino-5-((2-amino-3-chloropyridin-4-yl)thio)-3-fluoropyrazin-2-yl)-1,3-dihydrospiro[indene-2,4'-piperidin]-1-amine 10 mg of the amorphous form of (S)-1'-(6-amino-5-((2-amino-3-chloropyridin-4-yl)thio)-3-fluoropyrazin-2-yl)-1,3-dihydrospiro[indene-2,4'-piperidin]-1-amine was weighed in a 2 mL glass flask, followed by the addition of 100 μL of 3-pentanone, and the mixture was stirred at room temperature for 24 hours. The system was clear, and then a solid was precipitated during stirring. The mixture was centrifuged to remove the supernatant, and the remaining solid was dried in a vacuum drying oven at 40°C to constant weight to obtain crystal form A of (S)-1'-(6-amino-5-((2-amino-3-chloropyridin-4-yl)thio)-3-fluoropyrazin-2-yl)-1,3-dihydrospiro[indene-2,4'-piperidin]-1-amine. According to detection and analysis, it had the XRPD data shown in Table 1, the DSC spectrum shown in Figure 1, and the TGA spectrum shown in Figure 2.

1.2 Preparation of Crystal Form B of (S)-1'-(6-Amino-5-((2-amino-3-chloropyridin-4-yl)thio)-3-fluoropyrazin-2-yl)-1,3-dihydrospiro[indene-2,4'-piperidin]-1-amine 20 mg of the amorphous form of (S)-1'-(6-amino-5-((2-amino-3-chloropyridin-4-yl)-thio)-3-fluoropyrazin-2-yl)-1,3-dihydrospiro[indene-2,4'-piperidin]-1-amine was weighed in a 2 mL glass flask, and dissolved in 200 μL of acetonitrile at room temperature to be a clear solution. 400 μL of $H_2O$ was slowly added to the system at room temperature. $H_2O$ was added under stirring, and a solid was precipitated soon. The mixture was stirred at room temperature for 2 hours, centrifuged to remove the supernatant, and the remaining solid was dried in a vacuum drying oven at 40°C to constant weight to obtain crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine. According to detection and analysis, it had the XRPD data shown in Table 2, the DSC spectrum shown in Figure 4, and the TGA spectrum shown in Figure 5.

1.3 Preparation of Crystal Form C of (S)-1'-(6-Amino-5-((2-amino-3-chloropyridin-4-yl)thio)-3-fluoropyrazin-2-yl)-1,3-dihydrospiro[indene-2,4'-piperidin]-1-amine 20 mg of the amorphous form of (S)-1'-(6-amino-5-((2-amino-3-chloropyridin-4-yl)thio)-3-fluoropyrazin-2-yl)-1,3-dihydrospiro[indene-2,4'-piperidin]-1-amine was weighed in a 2 mL glass flask, and dissolved in 50 μL of dichloromethane at room temperature to be a clear solution. The solution was stirred at room temperature for 24 hours, and a solid was precipitated. The mixture was rapidly centrifuged to remove the supernatant, and the remaining solid was dried in a vacuum drying oven at 40°C to constant weight to obtain crystal form C of (S)-1'-(6-amino-5-((2-amino-3-chloropyridin-4-yl)thio)-3-fluoropyrazin-2-yl)-1,3-dihydrospiro[indene-2,4'-piperidin]-1-amine. According to detection and analysis, it had the XRPD data shown in Table 3, the DSC spectrum shown in Figure 6, and the TGA spectrum shown in Figure 7.

1.4 Preparation of Crystal Form D of (S)-1'-(6-Amino-5-((2-amino-3-chloropyridin-4-yl)thio)-3-fluoropyrazin-2-yl)-1,3-dihydrospiro[indene-2,4'-piperidin]-1-amine 20 mg of the amorphous form of (S)-1'-(6-amino-5-((2-amino-3-chloropyridin-4-yl)thio)-3-fluoropyrazin-2-yl)-1,3-dihydrospiro[indene-2,4'-piperidin]-1-amine was weighed in a 2 mL glass flask, and dissolved in 50 μL of 1,4-dioxane at room temperature to be a clear solution. The solution was stirred at room temperature for 24 hours, and a solid was precipitated. The mixture was rapidly centrifuged to remove the supernatant, and the remaining solid was dried in a vacuum drying oven at 40°C to constant weight to obtain crystal form D of (S)-1'-(6-amino-5-((2-amino-3-chloropyridin-4-yl)thio)-3-fluoropyrazin-2-yl)-1,3-dihydrospiro[indene-2,4'-piperidin]-1-amine. According to detection and analysis, it had the XRPD data shown in Table 4, the DSC spectrum shown in Figure 8, and the TGA spectrum shown in Figure 9.

1.5 Preparation of Crystal Form E of (S)-1'-(6-Amino-5-((2-amino-3-chloropyridin-4-yl)thio)-3-fluoropyrazin-2-yl)-1,3-dihydrospiro[indene-2,4'-piperidin]-1-amine 20 mg of the amorphous form of (S)-1'-(6-amino-5-((2-amino-3-chloropyridin-4-yl)thio)-3-fluoropyrazin-2-yl)-1,3-dihydrospiro[indene-2,4'-piperidin]-1-amine was weighed in a 2 mL glass flask, and dissolved in 100 μL of DMF at room temperature to be a clear solution. 400 μL of $H_2O$ was slowly added to the system at room temperature. $H_2O$ was added under stirring, and a solid was precipitated soon. The mixture was stirred at room temperature for 2 hours, centrifuged to remove the supernatant, and the remaining solid was dried in a vacuum drying oven at 40°C to constant weight to obtain crystal form E of (S)-1'-(6-amino-5-((2-amino-3-chloropyridin-4-yl)thio)-3-fluoropyrazin-2-yl)-1,3-dihydrospiro[indene-2,4'-piperidin]-1-amine. According to detection and analysis, it had the XRPD data shown in Table 5, the DSC spectrum shown in Figure 10, and the TGA spectrum shown in Figure 11.

**2. Solid Stability Experiment**

2.1 Experimental Objective:

The physicochemical stability of the amorphous form, crystal form A and crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine under light condition was investigated, so as to provide a basis for the crystal form screening and storage of the compound.

2.2 Experimental Scheme:

About 2 mg of the amorphous form, crystal form A and crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine were placed in a light box (5000 1× ±500 lx) respectively to investigate for 5 and 10 days. The content was determined by HPLC using the external standard method. The related substance change was calculated by chromatographic peak area normalization method.

2.3 Experimental Results:

The physicochemical stability results of different crystal forms of (S)-1'-(6-amino-5-((2-amino-3-chloropyridin-4-yl)thio)-3-fluoropyrazin-2-yl)-1,3-dihydrospiro[indene-2,4'-piperidin]-1-amine are shown in Table 4.1.

Table 4.1 Physicochemical stability results of different crystal forms

| Condition | | | Light 5000±500 lux (sealed) | | | | | |
|---|---|---|---|---|---|---|---|---|
| Time point | Day 0 | Total impurities | Day 5 | Total impurities | Total impurities gain | Day 10 | Total impurities | Total impurities gain |
| Amorphous form | 98.09 % | 1.91% | - | - | - | 78.75 % | 21.25% | 19.34% |
| Crystal form A | 99.14 % | 0.86% | 98.23 % | 1.77% | 0.91% | 97.29 % | 2.71% | 1.85% |
| Crystal form B | 98.34 % | 1.66% | 97.28 % | 2.72% | 1.06% | 96.46 % | 3.54% | 1.88% |

The above data show that the 10-day total impurities gain of the amorphous form of (S)-1'-(6-amino-5-((2-amino-3-chloropyridin-4-yl)thio)-3-fluoropyrazin-2-yl)-1,3-dihydrospiro[indene-2,4'-piperidin]-1-amine under light condition is 19.34%, whereas the 10-day total impurities gain of crystal form A and crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine under light condition is less than 2%.

2.4 Experimental Conclusion:

The stability under light condition can be improved by preparing (S)-1'-(6-amino-5-((2-amino-3-chloropyridin-4-yl)thio)-3-fluoropyrazin-2-yl)-1,3-dihydrospiro[indene-2,4'-piperidin]-1-amine into a crystal form.

**3. Hygroscopicity Experiment**

3.1 Experimental Objective

The hygroscopicity of crystal form B of the compound (S)-1'-(6-amino-5-((2-amino-3-chloropyridin-4-yl)thio)-3-fluoropyrazin-2-yl)-1,3-dihydrospiro[indene-2,4'-piperidin]-1-amine under different relative humidity conditions was investigated, so as to provide a basis for the crystal form screening and storage of the compound.

3.2 Experimental Scheme:

Crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine was placed in saturated water vapor with different relative humidity to achieve dynamic equilibrium between the compound and water vapor, and the percentage of hygroscopic weight gain of the compound after the equilibrium was calculated.

3.3 Experimental Results:

Crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine had a hygroscopic weight gain of about 1.639% under RH 80% condition, and had slight hygroscopicity. After one cycle of humidification and dehumidification under 0 to 95% relative humidity condition, the XRPD pattern of crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine didn't change, *i.e.,* the crystal form didn't transform.

**4. Solubility Experiment in Different Media**

4.1 Experimental Objective

The solubility of crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine in media such as water, Simulated Gastric Fluid (SGF), Fasted State Simulated Intestinal Fluid (FaSSIF) and Fed State Simulated Intestinal Fluid (FeSSIF) was compared, so as to provide a basis for evaluating the druggability.

4.2 Experimental scheme:

About 2 mg of crystal form B of (S)-1'-(6-amino-5-((2-amino-3-chloropyridin-4-yl)thio)-3-fluoropyrazin-2-yl)-1,3-dihydrospiro[indene-2,4'-piperidin]-1-amine was suspended in different media for 24 hours, respectively. Thermodynamic solubility of the compound at 37°C was determined by HPLC using the external standard method.
4.3 Experimental results are shown in Table 4.2.

Table 4.2 Solubility experiment in different media

| Sample name | Solubility of crystal form B (mg/mL) |
|---|---|
| Fa | 0.170 |
| Fe | 0.064 |
| SGF | 2.050 |

(continued)

| Sample name | Solubility of crystal form B (mg/mL) |
|---|---|
| water | 0.001 |

**5. Polymorphic Screening Experiment and Deteremination of Stable Crystal Form**

5.1 Experimental Objective:

The objective is to find stable crystal forms of (S)-1'-(6-amino-5-((2-amino-3-chloropyridin-4-yl)thio)-3-fluoropyrazin-2-yl)-1,3-dihydrospiro[indene-2,4'-piperidin]-1-amine by polymorphic screening.

5.2 Experimental scheme:

Organic solvents and water with a certain solubility was chosen to suspend (S)-1'-(6-amino-5-((2-amino-3-chloropyridin-4-yl)thio)-3-fluoropyrazin-2-yl)-1,3-dihydrospiro[indene-2,4'-piperidin]-1-amine in the solvent system, and the mixture was stirred and slurried at room temperature for one week, and then centrifuged. The supernatant was discarded, and the solid was dried under vacuum (-0.1 Mpa) at 40°C overnight. The XRPD, DSC and TGA of the solid was determined and compared.

5.3 Experimental Results:

Five crystal forms of (S)-1'-(6-amino-5-((2-amino-3-chloropyridin-4-yl)thio)-3-fluoropyrazin-2-yl)-1,3-dihydrospiro[indene-2,4'-piperidin]-1-amine, *i.e.,* crystal forms A, B, C, D and E, were obtained by pulping, changing crystallization solvent and crystallization method. By comparing the DSC spectrums of different crystal forms of (S)-1'-(6-amino-5-((2-amino-3-chloropyridin-4-yl)thio)-3-fluoropyrazin-2-yl)-1,3-dihydrospiro[indene-2,4'-piperidin]-1-amine, it can be seen that crystal form B has the best thermodynamic stability among the five crystal forms.

## Claims

**1.** A crystal form of a compound of formula (I):

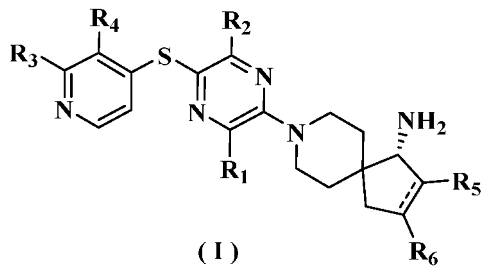

( I )

wherein:

$R_1$ is selected from the group consisting of hydrogen, halogen, $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-12}$ aryl and 5 to 12 membered heteroaryl, wherein the $C_{1-6}$ alkyl, $C_{1-6}$ alkoxy, $C_{1-6}$ hydroxyalkyl, $C_{3-8}$ cycloalkyl, 3 to 12 membered heterocyclyl, $C_{6-12}$ aryl and 5 to 12 membered heteroaryl are each optionally further substituted by one or more substituent(s) selected from the group consisting of deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl;

$R_2$ is selected from the group consisting of hydrogen, amino and $C_{1-6}$ alkyl;

$R_3$ is selected from the group consisting of hydrogen, halogen, amino, cyano, $C_{1-6}$ alkyl, $C_{1-6}$ alkoxy, $C_{1-6}$ hydroxyalkyl, $C_{3-8}$ cycloalkyl and 3 to 12 membered heterocyclyl, wherein the $C_{1-6}$ alkyl, $C_{1-6}$ alkoxy, $C_{1-6}$ hydroxyalkyl, $C_{3-8}$ cycloalkyl and 3 to 12 membered heterocyclyl are each optionally further substituted by one or more substituent(s) selected from the group consisting of deuterium, halogen, amino, nitro, hydroxy, cyano,

oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl;

$R_4$ is selected from the group consisting of hydrogen, deuterium, halogen, amino, nitro, hydroxy, cyano, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{3-8}$ cycloalkyl, 3 to 12 membered heterocyclyl, $C_{6-12}$ aryl and 5 to 12 membered heteroaryl;

$R_5$ and $R_6$ are each independently selected from the group consisting of hydrogen, deuterium, halogen, amino, nitro, hydroxy, cyano, $C_{1-6}$ alkyl, $C_{1-6}$ deuterated alkyl, $C_{1-6}$ haloalkyl, $C_{1-6}$ alkoxy, $C_{1-6}$ haloalkoxy, $C_{1-6}$ hydroxyalkyl, $C_{2-6}$ alkenyl, $C_{3-8}$ cycloalkyl, 3 to 12 membered heterocyclyl, $C_{6-12}$ aryl and 5 to 12 membered heteroaryl, wherein the $C_{1-6}$ alkyl, $C_{1-6}$ deuterated alkyl, $C_{1-6}$ haloalkyl, $C_{1-6}$ alkoxy, $C_{1-6}$ haloalkoxy, $C_{1-6}$ hydroxyalkyl, $C_{2-6}$ alkenyl, $C_{3-8}$ cycloalkyl, 3 to 12 membered heterocyclyl, $C_{6-12}$ aryl and 5 to 12 membered heteroaryl are each optionally further substituted by one or more substituent(s) selected from the group consisting of deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl;

or, $R_5$ and $R_6$ are bonded to form a $C_{3-8}$ cycloalkyl, 3 to 12 membered heterocyclyl, $C_{6-12}$ aryl or 5 to 12 membered heteroaryl, wherein the $C_{3-8}$ cycloalkyl, 3 to 12 membered heterocyclyl, $C_{6-12}$ aryl and 5 to 12 membered heteroaryl are each optionally further substituted by one or more substituent(s) selected from the group consisting of deuterium, halogen, amino, nitro, hydroxy, cyano, oxo, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl.

**2.** The crystal form according to claim 1, wherein $R_1$ is selected from the group consisting of hydrogen, halogen, $C_{1-6}$ alkyl, $C_{1-6}$ hydroxyalkyl, $C_{3-8}$ cycloalkyl and 3 to 12 membered heterocyclyl, wherein the $C_{1-6}$ alkyl, $C_{1-6}$ hydroxyalkyl, $C_{3-8}$ cycloalkyl and 3 to 12 membered heterocyclyl are each optionally further substituted by one or more substituent(s) selected from the group consisting of deuterium, halogen, hydroxy, oxo, $C_{1-6}$ alkyl and $C_{3-8}$ cycloalkyl; $R_1$ is more preferably selected from the group consisting of hydrogen, halogen, $C_{1-3}$ alkyl, $C_{1-3}$ hydroxyalkyl, $C_{3-6}$ cycloalkyl and 3 to 6 membered heterocyclyl, wherein the $C_{1-3}$ alkyl, $C_{1-3}$ hydroxyalkyl, $C_{3-6}$ cycloalkyl and 3 to 6 membered heterocyclyl are each optionally further substituted by one or more substituent(s) selected from the group consisting of deuterium, halogen, hydroxy, oxo, $C_{1-3}$ alkyl and $C_{3-6}$ cycloalkyl; and $R_1$ is further preferably selected from the group consisting of hydrogen, fluorine, bromine, methyl, hydroxyethyl,

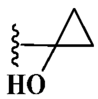 , 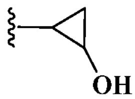 ,

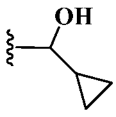 , 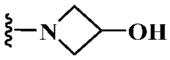 , 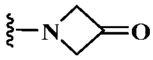 and 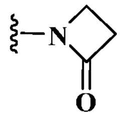 .

**3.** The crystal form according to claim 1, wherein $R_2$ is selected from the group consisting of hydrogen, amino and $C_{1-6}$ alkyl; preferably selected from the group consisting of hydrogen, amino and $C_{1-3}$ alkyl; and more preferably selected from the group consisting of hydrogen, amino, methyl, ethyl and propyl.

**4.** The crystal form according to claim 1, wherein $R_3$ is selected from the group consisting of hydrogen, halogen, amino, $C_{1-6}$ alkyl, $C_{3-8}$ cycloalkyl and 3 to 12 membered heterocyclyl, wherein the $C_{1-6}$ alkyl, $C_{3-8}$ cycloalkyl and 3 to 12 membered heterocyclyl are each optionally further substituted by one or more substituent(s) selected from the group consisting of deuterium, halogen and oxo; $R_3$ is preferably selected from the group consisting of hydrogen, halogen, amino, $C_{1-3}$ alkyl, $C_{3-6}$ cycloalkyl and 3 to 6 membered heterocyclyl, wherein the $C_{1-3}$ alkyl, $C_{3-6}$ cycloalkyl and 3 to 6 membered heterocyclyl are each optionally further substituted by one or more substituent(s) selected from the group consisting of deuterium, halogen and oxo; and $R_3$ is further preferably selected from the group consisting of amino, chlorine, cyclopropyl, azacyclobutyl, tetrahydropyrrolyl, morpholinyl,

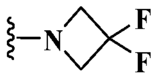, 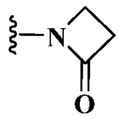

and

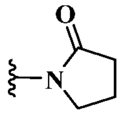.

5. The crystal form according to claim 1, wherein $R_4$ is selected from the group consisting of hydrogen, halogen, $C_{1-6}$ alkyl and $C_{3-8}$ cycloalkyl; preferably selected from the group consisting of hydrogen, halogen, $C_{1-3}$ alkyl and $C_{3-6}$ cycloalkyl; and further preferably selected from the group consisting of hydrogen, fluorine, chlorine, methyl and cyclopropyl.

6. The crystal form according to claim 1, wherein $R_5$ and $R_6$ are each independently selected from the group consisting of hydrogen, $C_{1-6}$ alkyl, $C_{1-6}$ haloalkyl, $C_{2-6}$ alkenyl and $C_{3-8}$ cycloalkyl, wherein the $C_{1-6}$ alkyl, $C_{1-6}$ haloalkyl, $C_{2-6}$ alkenyl and $C_{3-8}$ cycloalkyl are each optionally further substituted by one or more substituent(s) selected from the group consisting of deuterium, halogen and $C_{1-6}$ alkyl; preferably, $R_5$ and $R_6$ are each independently selected from the group consisting of hydrogen, $C_{1-3}$ alkyl, $C_{2-4}$ alkenyl and $C_{3-6}$ cycloalkyl, wherein the $C_{1-3}$ alkyl, $C_{2-4}$ alkenyl and $C_{3-6}$ cycloalkyl are each optionally further substituted by one or more substituent(s) selected from the group consisting of hydrogen, deuterium, halogen and $C_{1-3}$ alkyl; and further preferably, $R_5$ and $R_6$ are each independently selected from the group consisting of hydrogen, methyl, ethyl, isopropyl, vinyl, cyclopropyl,

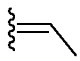 and 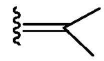;

or, $R_5$ and $R_6$ are bonded to form a $C_{3-8}$ cycloalkyl, $C_{6-12}$ aryl or 5 to 12 membered heteroaryl, wherein the $C_{3-8}$ cycloalkyl, $C_{6-12}$ aryl and 5 to 12 membered heteroaryl are each optionally further substituted by one or more substituent(s) selected from the group consisting of hydrogen, deuterium, halogen and $C_{1-6}$ alkyl; preferably, $R_5$ and $R_6$ are bonded to form a $C_{3-6}$ cycloalkyl, phenyl or 5 to 6 membered heteroaryl, wherein the $C_{3-6}$ cycloalkyl, phenyl and 5 to 6 membered heteroaryl are each optionally further substituted by one or more substituent(s) selected from the group consisting of deuterium, halogen and $C_{1-3}$ alkyl; and further preferably, $R_5$ and $R_6$ are bonded to form

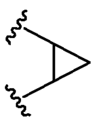, 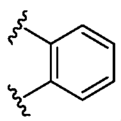, 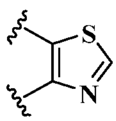, 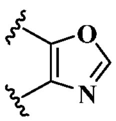, 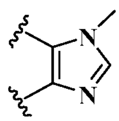, 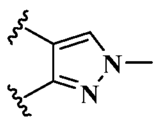 or 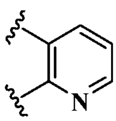.

7. The crystal form according to claim 1, wherein the structure of the compound is as shown in formula (II):

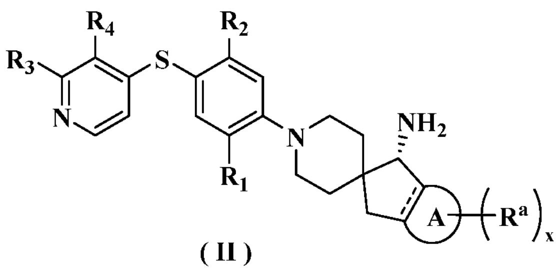

( II )

wherein:

ring A is selected from the group consisting of $C_{3-8}$ cycloalkyl, 3 to 12 membered heterocyclyl, $C_{6-10}$ aryl and 5 to 12 membered heteroaryl;
$R^a$ is selected from the group consisting of hydrogen, deuterium, halogen, amino, nitro, hydroxy, cyano, $C_{1-6}$ alkyl, $C_{1-6}$ deuterated alkyl, $C_{1-6}$ haloalkyl, $C_{1-6}$ alkoxy, $C_{1-6}$ hydroxyalkyl, $C_{1-6}$ haloalkoxy, $C_{2-6}$ alkenyl, $C_{2-6}$ alkynyl, $C_{3-8}$ cycloalkyl, 3 to 12 membered heterocyclyl, $C_{6-12}$ aryl and 5 to 12 membered heteroaryl; and
x is 0, 1, 2 or 3.

**8.** The crystal form according to claim 1, wherein ring A is phenyl.

**9.** The crystal form according to claim 1, wherein the structure of the compound is as follows:

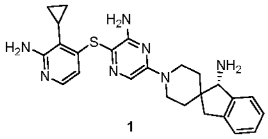
1
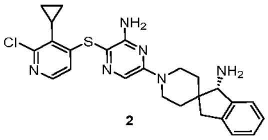
2
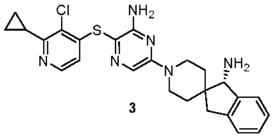
3
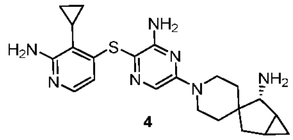
4
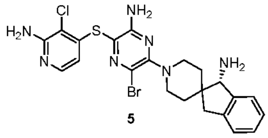
5
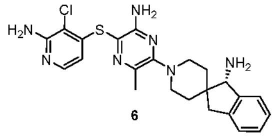
6
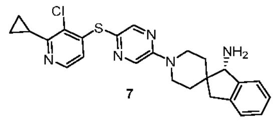
7
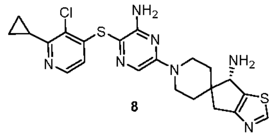
8
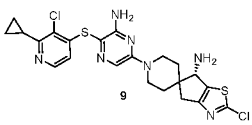
9
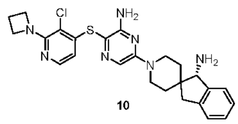
10
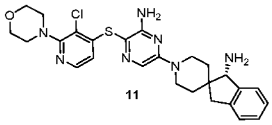
11
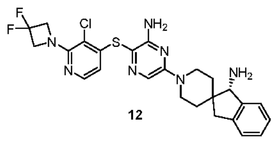
12
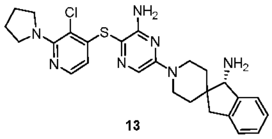
13
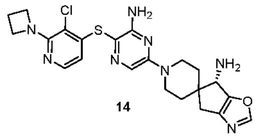
14
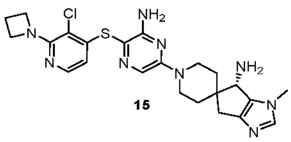
15
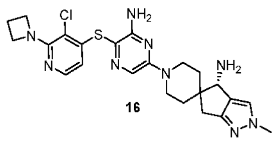
16
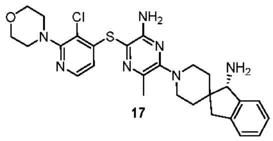
17
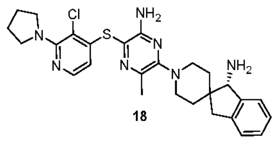
18
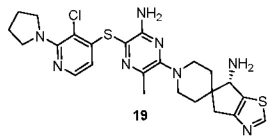
19
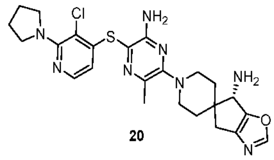
20
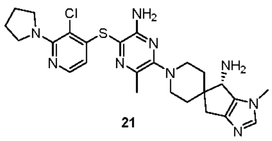
21
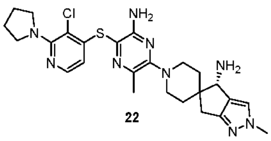
22
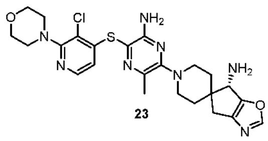
23
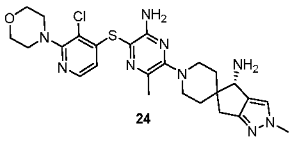
24
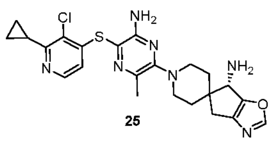
25
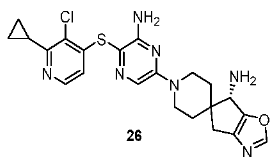
26
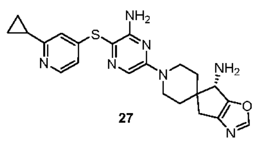
27
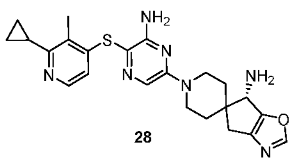
28

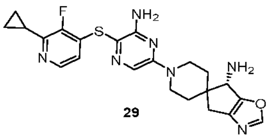
29
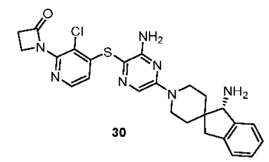
30
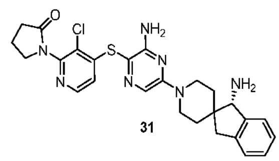
31
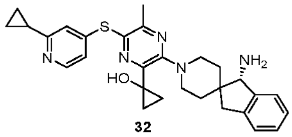
32

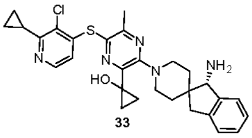
33
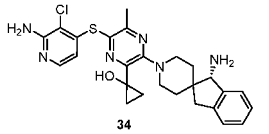
34
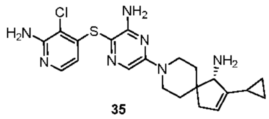
35
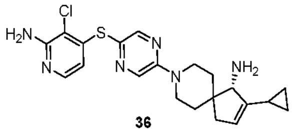
36

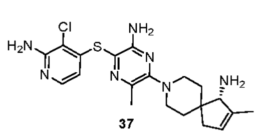
37
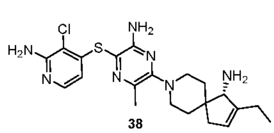
38
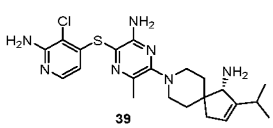
39
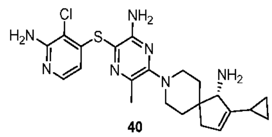
40

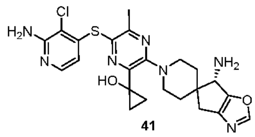
41
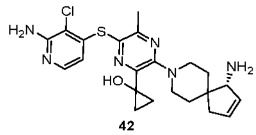
42
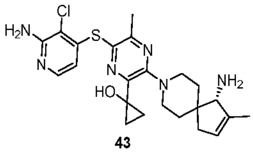
43
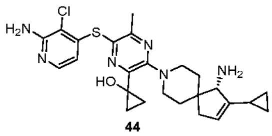
44

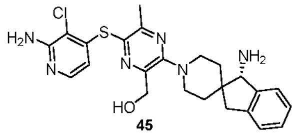
45
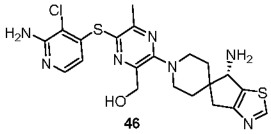
46
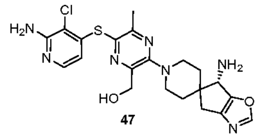
47
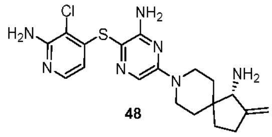
48

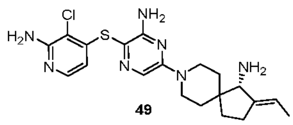
49
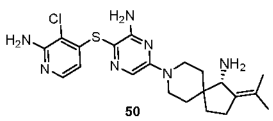
50
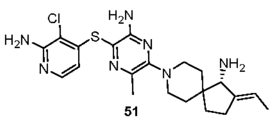
51
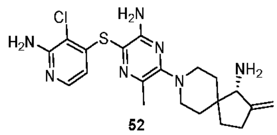
52

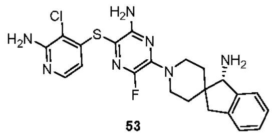
53
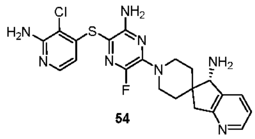
54
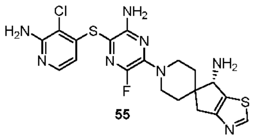
55
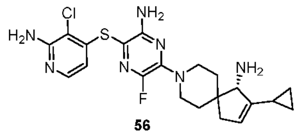
56

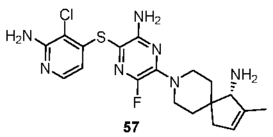
57
or
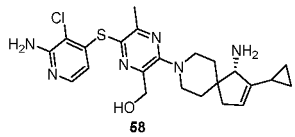
58
.

**10.** The crystal form according to claim 1, wherein the compound is selected from the group consisting of (S)-1'-(6-amino-5-((2-amino-3-chloropyridin-4-yl)thio)-3-fluoropyrazin-2-yl)-1,3-dihydrospiro[indene-2,4'-piperidin]-1-amine and (S)-8-(6-amino-5-((2-amino-3-chloropyridin-4-yl)thio)pyrazin-2-yl)-2-cyclopropyl-8-azaspiro[4.5]dec-2-en-1-amine.

**11.** The crystal form according to claim 10, wherein the compound (S)-1'-(6-amino-5-((2-amino-3-chloropyridin-4-yl)thio)-3-fluoropyrazin-2-yl)-1,3-dihydrospiro[indene-2,4'-piperidin]-1-amine comprises crystal forms A, B, C, D and E, wherein,

being crystal form A, the X-ray powder diffraction pattern thereof has diffraction peaks at 2θ of 9.0°, 15.2°, 20.2° and 23.0°, preferably further has diffraction peaks at 2θ of 9.7°, 12.3°, 15.4°, 19.8°, 23.5° and 27.1°, and more preferably further has diffraction peaks at 2θ of 4.5°, 13.7°, 14.6°, 16.8°, 18.1°, 21.5°, 27.7° and 28.2°,
more preferably, the DSC spectrum thereof is substantially as shown in Figure 1, or the TGA spectrum thereof is substantially as shown in Figure 2;
being crystal form B, the X-ray powder diffraction pattern thereof has diffraction peaks at 2θ of 7.9°, 14.6° and 16.2°, preferably also has diffraction peaks at 2θ of 18.1°, 19.2°, 20.2°, 24.7° and 26.8°, more preferably further has diffraction peaks at 2θ of 14.9°, 27.2°, 28.1° and 30.5°, further preferably further has diffraction peaks at 2θ of 13.9°, 15.6°, 16.9° and 19.8°, and most preferably, the XRPD pattern thereof is substantially as shown in Figure 3,
preferably, the DSC spectrum thereof is substantially as shown in Figure 4, or the TGA spectrum thereof is substantially as shown in Figure 5;
being crystal form C, the X-ray powder diffraction pattern thereof has diffraction peaks at 2θ of 9.4°, 14.5° and 20.3°, preferably also has diffraction peaks at 2θ of 22.2°, 22.7°, 22.9° and 26.2°, more preferably further has diffraction peaks at 2θ of 8.2°, 14.9°, 23.8°, 27.5°, 28.1°, 29.3°, 30.2° and 31.7°, and further preferably further has diffraction peaks at 2θ of 12.0°, 16.4°, 25.5°, 28.6°, 34.8° and 35.4°,
more preferably, the DSC spectrum thereof is substantially as shown in Figure 6, or the TGA spectrum thereof is substantially as shown in Figure 7;
being crystal form D, the X-ray powder diffraction pattern thereof has diffraction peaks at 2θ of 5.1°, 13.4° and 18.3°, preferably also has diffraction peaks at 2θ of 18.0°, 19.9°, 20.7° and 22.6°, more preferably further has diffraction peaks at 2θ of 13.7°, 16.8°, 17.7°, 19.0°, 22.3° and 27.5°, and further preferably further has diffraction peaks at 2θ of 16.1°, 22.9°, 24.9°, 25.5° and 28.9°,
more preferably, the DSC spectrum thereof is substantially as shown in Figure 8, or the TGA spectrum thereof is substantially as shown in Figure 9;
being crystal form E, the X-ray powder diffraction pattern thereof has diffraction peaks at 2θ of 8.4°, 15.3° and 18.3°, preferably also has diffraction peaks at 2θ of 20.7°, 22.5°, 26.7° and 27.6°, more preferably further has diffraction peaks at 2θ of 13.5°, 14.6°, 15.8°, 16.0°, 16.9°, 19.2°, 20.4°, 24.0°, 24.4°, 25.5°, 28.2°, 28.9°, 31.8° and 32.2°, and further preferably further has diffraction peaks at 2θ of 5.2°, 9.5°, 10.4°, 17.2°, 17.7°, 19.5°, 20.0°, 26.3°, 28.7°, 30.5°, 31.0° and 34.1°,
more preferably, the DSC spectrum thereof is substantially as shown in Figure 10, or the TGA spectrum thereof is substantially as shown in Figure 11.

**12.** The crystal form according to claim 10, wherein the crystal form of (S)-8-(6-amino-5-((2-amino-3-chloropyridin-4-yl)thio)pyrazin-2-yl)-2-cyclopropyl-8-azaspiro[4.5]dec-2-en-1-amine comprises crystal form A, crystal form B and crystal form C, wherein:

the X-ray powder diffraction pattern of crystal form A has a diffraction peak at 2θ of 20.0±0.2°; or has a diffraction peak at 19.4±0.2°; or has a diffraction peak at 17.1±0.2°; or has a diffraction peak at 22.6±0.2°; or has a diffraction peak at 10.7±0.2°; or has a diffraction peak at 25.6±0.2°; or has a diffraction peak at 12.7±0.2°; or has a diffraction peak at 24.5±0.2°; or has a diffraction peak at 19.1±0.2°; or has a diffraction peak at 18.4±0.2°; preferably comprises any 2 to 5, or 3 to 5, or 3 to 6, or 3 to 8, or 5 to 8, or 6 to 8 of the above diffraction peaks; and more preferably comprises any 6, 7, or 8 of the above diffraction peaks;
the X-ray powder diffraction pattern of crystal form B has a diffraction peak at 2θ of 20.2±0.2°; or has a diffraction peak at 22.2±0.2°; or has a diffraction peak at 12.2±0.2°; or has a diffraction peak at 25.1±0.2°; or has a diffraction peak at 21.5±0.2°; or has a diffraction peak at 18.5±0.2°; or has a diffraction peak at 17.8±0.2°; or has a diffraction peak at 20.0±0.2°; or has a diffraction peak at 28.6±0.2°; or has a diffraction peak at 5.1±0.2°; preferably comprises any 2 to 5, or 3 to 5, or 3 to 6, or 3 to 8, or 5 to 8, or 6 to 8 of the above diffraction peaks; and more preferably comprises any 6, 7, or 8 of the above diffraction peaks;
the X-ray powder diffraction pattern of crystal form C has a diffraction peak at 2θ of 17.8±0.2°; or has a diffraction peak at 19.8±0.2°; or has a diffraction peak at 26.1±0.2°; or has a diffraction peak at 18.2±0.2°; or has a diffraction peak at 24.0±0.2°; or has a diffraction peak at 22.8±0.2°; or has a diffraction peak at 10.5±0.2°; or has a diffraction peak at 21.5±0.2°; or has a diffraction peak at 17.5±0.2°; or has a diffraction peak at 21.8±0.2°; preferably comprises any 2 to 5, or 3 to 5, or 3 to 6, or 3 to 8, or 5 to 8, or 6 to 8 of the above diffraction peaks; and more preferably comprises any 6, 7, or 8 of the above diffraction peaks.

**13.** The crystal form according to claim 12, wherein:

the X-ray powder diffraction pattern of crystal form A at least comprises one or more diffraction peaks at 2θ of

20.0±0.2°, 19.4±0.2° and 17.1±0.2°, preferably comprises 2 of the above diffraction peaks, and more preferably comprises 3 of the above diffraction peaks; optionally, can further comprises at least one diffraction peak at 2θ of 22.6±0.2°, 10.7±0.2°, 25.6±0.2°, 12.7±0.2°, 24.5±0.2, and preferably comprises 2, 3, 4, or 5 of the above diffraction peaks;
for example, the X-ray powder diffraction pattern of crystal form A has diffraction peaks at 2θ of the following positions:

20.0±0.2°, 19.4±0.2°, 17.1±0.2°, 22.6±0.2°, 10.7±0.2° and 25.6±0.2°;
or, 20.0±0.2°, 19.4±0.2°, 22.6±0.2°, 10.7±0.2°, 25.6±0.2° and 12.7±0.2°;
or, 20.0±0.2°, 17.1±0.2°, 22.6±0.2°, 10.7±0.2°, 25.6±0.2° and 12.7±0.2°;

the X-ray powder diffraction pattern of crystal form B at least comprises one or more diffraction peaks at 2θ of 20.2±0.2°, 22.2±0.2° and 12.2±0.2°, preferably comprises 2 of the above diffraction peaks, and more preferably comprises 3 of the above diffraction peaks; optionally, can further comprises at least one diffraction peak at 2θ of 25.1±0.2°, 21.5±0.2°, 18.5±0.2°, 17.8±0.2°, 20.0±0.2, and preferably comprises 2, 3, 4, or 5 of the above diffraction peaks;
for example, the X-ray powder diffraction pattern of crystal form B has diffraction peaks at 2θ of the following positions:

20.2±0.2°, 22.2±0.2°, 12.2±0.2°, 25.1±0.2°, 21.5±0.2° and 18.5±0.2°;
or, 20.2±0.2°, 22.2±0.2°, 25.1±0.2°, 21.5±0.2°, 18.5±0.2° and 17.8±0.2°;
or, 20.2±0.2°, 12.2±0.2°, 25.1±0.2°, 21.5±0.2°, 18.5±0.2° and 17.8±0.2°;

the X-ray powder diffraction pattern of crystal form C at least comprises one or more diffraction peaks at 2θ of 17.8±0.2°, 19.8±0.2° and 26.1±0.2°, preferably comprises 2 of the above diffraction peaks, and more preferably comprises 3 of the above diffraction peaks; optionally, can further comprises at least one diffraction peak at 2θ of 18.2±0.2°, 24.0±0.2°, 22.8±0.2°, 10.5±0.2°, 21.5±0.2, and preferably comprises 2, 3, 4, or 5 of the above diffraction peaks;
for example, the X-ray powder diffraction pattern of crystal form C has diffraction peaks at 2θ of the following positions:

17.8±0.2°, 19.8±0.2°, 26.1±0.2°, 18.2±0.2°, 24.0±0.2° and 22.8±0.2°;
or, 17.8±0.2°, 19.8±0.2°, 18.2±0.2°, 24.0±0.2°, 22.8±0.2° and 10.5±0.2°;
or, 17.8±0.2°, 26.1±0.2°, 18.2±0.2°, 24.0±0.2°, 22.8±0.2° and 10.5±0.2°.

**14.** The crystal form according to claim 12, wherein:

the X-ray powder diffraction pattern of crystal form A optionally also comprises one or more diffraction peaks at 2θ of 19.1±0.2°, 18.4±0.2°, 6.2±0.2°, 31.5±0.2°, 29.2±0.2°, 20.6±0.2° and 21.4±0.2°; preferably at least comprises any 2 to 3, or 4 to 5, or 6 to 7 of the above diffraction peaks; and further preferably comprises any 2, 3, 4, 5, 6, 7 of the above diffraction peaks;
for example, the X-ray powder diffraction pattern of crystal form A has diffraction peaks at 2θ of the following positions:

20.0±0.2°, 19.4±0.2°, 17.1±0.2°, 22.6±0.2°, 10.7±0.2°, 25.6±0.2°, 19.1±0.2° and 18.4±0.2°;
or, 20.0±0.2°, 19.4±0.2°, 22.6±0.2°, 10.7±0.2°, 25.6±0.2°, 12.7±0.2°, 19.1±0.2°, 18.4±0.2° and 6.2±0.2°;
or, 20.0±0.2°, 17.1±0.2°, 22.6±0.2°, 10.7±0.2°, 25.6±0.2°, 12.7±0.2°, 19.1±0.2°, 18.4±0.2°, 6.2±0.2° and 31.5±0.2°;

the X-ray powder diffraction pattern of crystal form B optionally also comprises one or more diffraction peaks at 2θ of 28.6±0.2°, 5.1±0.2°, 14.3±0.2°, 24.5±0.2°, 13.2±0.2°, 12.4±0.2° and 29.2±0.2°; preferably at least comprises any 2 to 3, or 4 to 5, or 6 to 7 of the above diffraction peaks; and further preferably comprises any 2, 3, 4, 5, 6, 7 of the above diffraction peaks;
for example, the X-ray powder diffraction pattern of crystal form B has diffraction peaks at 2θ of the following positions:

20.2±0.2°, 22.2±0.2°, 12.2±0.2°, 25.1±0.2°, 21.5±0.2°, 18.5±0.2°, 28.6±0.2° and 5.1±0.2°;

or, 20.2±0.2°, 22.2±0.2°, 25.1±0.2°, 21.5±0.2°, 18.5±0.2°, 17.8±0.2°, 28.6±0.2°, 5.1±0.2° and 14.3±0.2°;
or, 20.2±0.2°, 12.2±0.2°, 25.1±0.2°, 21.5±0.2°, 18.5±0.2°, 17.8±0.2°, 28.6±0.2°, 5.1±0.2°, 14.3±0.2° and 24.5±0.2°;

the X-ray powder diffraction pattern of crystal form C optionally also comprises one or more diffraction peaks at 2θ of 17.5±0.2°, 21.8±0.2°, 19.5±0.2°, 22.3±0.2°, 24.3±0.2°, 14.5±0.2° and 30.1±0.2°; preferably at least comprises any 2 to 3, or 4 to 5, or 6 to 7 of the above diffraction peaks; and further preferably comprises any 2, 3, 4, 5, 6, 7 of the above diffraction peaks;
for example, the X-ray powder diffraction pattern of crystal form C has diffraction peaks at 2θ of the following positions:

17.8±0.2°, 19.8±0.2°, 26.1±0.2°, 18.2±0.2°, 24.0±0.2°, 22.8±0.2°, 17.5±0.2° and 21.8±0.2°;
or, 17.8±0.2°, 19.8±0.2°, 18.2±0.2°, 24.0±0.2°, 22.8±0.2°, 10.5±0.2°, 17.5±0.2°, 21.8±0.2° and 19.5±0.2°;
or, 17.8±0.2°, 26.1±0.2°, 18.2±0.2°, 24.0±0.2°, 22.8±0.2°, 10.5±0.2°, 17.5±0.2°, 21.8±0.2°, 19.5±0.2° and 22.3±0.2°.

**15.** The crystal form according to claim 12, wherein:

the X-ray powder diffraction pattern of crystal form A comprises one or more diffraction peaks at 2θ of 20.0±0.2°, 19.4±0.2°, 17.1±0.2°, 22.6±0.2°, 10.7±0.2°, 25.6±0.2°, 12.7±0.2°, 24.5±0.2, 19.1±0.2°, 18.4±0.2°, 6.2±0.2°, 31.5±0.2°, 29.2±0.2°, 20.6±0.2° and 21.4±0.2°; and preferably comprises any 4, 5, 6, 8, or 10 of the above diffraction peaks;
for example, the X-ray powder diffraction pattern of crystal form A has diffraction peaks at 2θ of the following positions:

20.0±0.2°, 19.4±0.2°, 17.1±0.2° and 22.6±0.2°;
or, 20.0±0.2°, 19.4±0.2°, 17.1±0.2° and 10.7±0.2°;
or, 20.0±0.2°, 19.4±0.2°, 17.1±0.2°, 22.6±0.2°, 10.7±0.2° and 19.1±0.2°;
or, 20.0±0.2°, 19.4±0.2°, 17.1±0.2°, 10.7±0.2°, 25.6±0.2° and 19.1±0.2°;
or, 20.0±0.2°, 19.4±0.2°, 17.1±0.2°, 22.6±0.2°, 10.7±0.2°, 25.6±0.2°, 19.1±0.2° and 6.2±0.2°;
or, 20.0±0.2°, 19.4±0.2°, 17.1±0.2°, 22.6±0.2°, 10.7±0.2°, 25.6±0.2°, 18.4±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 24.5±0.2, 19.1±0.2° and 6.2±0.2°;
or, 20.0±0.2°, 19.4±0.2°, 17.1±0.2°, 22.6±0.2°, 10.7±0.2°, 25.6±0.2°, 12.7±0.2°, 24.5±0.2, 18.4±0.2° and 6.2±0.2°;

the X-ray powder diffraction pattern of crystal form B comprises one or more diffraction peaks at 2θ of 20.2±0.2°, 22.2±0.2°, 12.2±0.2°, 25.1±0.2°, 21.5±0.2°, 18.5±0.2°, 17.8±0.2°, 20.0±0.2, 28.6±0.2°, 5.1±0.2°, 14.3±0.2°, 24.5±0.2°, 13.2±0.2°, 12.4±0.2° and 29.2±0.2°; and preferably comprises any 4, 5, 6, 8, or 10 of the above diffraction peaks;
for example, the X-ray powder diffraction pattern of crystal form B has diffraction peaks at 2θ of the following positions:

20.2±0.2°, 22.2±0.2°, 12.2±0.2° and 25.1±0.2°;
or, 20.2±0.2°, 22.2±0.2°, 12.2±0.2° and 21.5±0.2°;
or, 20.2±0.2°, 22.2±0.2°, 12.2±0.2°, 25.1±0.2°, 21.5±0.2° and 28.6±0.2°;
or, 20.2±0.2°, 22.2±0.2°, 12.2±0.2°, 21.5±0.2°, 18.5±0.2° and 28.6±0.2°;
or, 20.2±0.2°, 22.2±0.2°, 12.2±0.2°, 25.1±0.2°, 21.5±0.2°, 18.5±0.2°, 28.6±0.2° and 14.3±0.2°;
or, 20.2±0.2°, 22.2±0.2°, 12.2±0.2°, 25.1±0.2°, 21.5±0.2°, 18.5±0.2°, 5.1±0.2° and 14.3±0.2°;
or, 20.2±0.2°, 22.2±0.2°, 12.2±0.2°, 25.1±0.2°, 21.5±0.2°, 18.5±0.2°, 17.8±0.2°, 20.0±0.2, 28.6±0.2° and 14.3±0.2°;
or, 20.2±0.2°, 22.2±0.2°, 12.2±0.2°, 25.1±0.2°, 21.5±0.2°, 18.5±0.2°, 17.8±0.2°, 20.0±0.2, 5.1±0.2° and 14.3±0.2°;

the X-ray powder diffraction pattern of crystal form C comprises one or more diffraction peaks at 2θ of 17.8±0.2°, 19.8±0.2°, 26.1±0.2°, 18.2±0.2°, 24.0±0.2°, 22.8±0.2°, 10.5±0.2°, 21.5±0.2, 17.5±0.2°, 21.8±0.2°,

19.5±0.2°, 22.3±0.2°, 24.3±0.2°, 14.5±0.2° and 30.1±0.2°; and preferably comprises any 4, 5, 6, 8, or 10 of the above diffraction peaks;
for example, the X-ray powder diffraction pattern of crystal form C has diffraction peaks at 2θ of the following positions:

17.8±0.2°, 19.8±0.2°, 26.1±0.2° and 18.2±0.2°;
or, 17.8±0.2°, 19.8±0.2°, 26.1±0.2° and 24.0±0.2°;
or, 17.8±0.2°, 19.8±0.2°, 26.1±0.2°, 18.2±0.2°, 24.0±0.2° and 17.5±0.2°;
or, 17.8±0.2°, 19.8±0.2°, 26.1±0.2°, 24.0±0.2°, 22.8±0.2° and 17.5±0.2°;
or, 17.8±0.2°, 19.8±0.2°, 26.1±0.2°, 18.2±0.2°, 24.0±0.2°, 22.8±0.2°, 17.5±0.2° and 19.5±0.2°;
or, 17.8±0.2°, 19.8±0.2°, 26.1±0.2°, 18.2±0.2°, 24.0±0.2°, 22.8±0.2°, 21.8±0.2° and 19.5±0.2°;
or, 17.8±0.2°, 19.8±0.2°, 26.1±0.2°, 18.2±0.2°, 24.0±0.2°, 22.8±0.2°, 10.5±0.2°, 21.5±0.2, 17.5±0.2° and 19.5±0.2°;
or, 17.8±0.2°, 19.8±0.2°, 26.1±0.2°, 18.2±0.2°, 24.0±0.2°, 22.8±0.2°, 10.5±0.2°, 21.5±0.2, 21.8±0.2° and 19.5±0.2°.

**16.** The crystal form according to claim 12, wherein:

the X-ray powder diffraction pattern of crystal form A is as shown in Figure 12, or crystal form A has the DSC spectrum as shown in Figure 13, or has the TGA spectrum as shown in Figure 14;
the X-ray powder diffraction pattern of crystal form B is as shown in Figure 15, or crystal form B has the DSC spectrum as shown in Figure 16;
the X-ray powder diffraction pattern of crystal form C is as shown in Figure 17, or crystal form C has the DSC spectrum as shown in Figure 18.

**17.** The crystal form according to any one of claims 1 to 16, wherein the X-ray powder diffraction pattern of the crystal form, the 2θ error between the diffraction peak position having top-ten relative peak intensity and the reference diffraction peak position is ±0.2° to ±0.5°, preferably ±0.2° to ±0.3°, and most preferably ±0.2°.

**18.** A method for preparing the crystal form according to any one of claims 1 to 17, comprising the steps of:

1) weighing an appropriate amount of free base, and suspending it in a poor solvent to obtain a suspension; the suspension density is preferably 50 to 200 mg/mL;
2) shaking the suspension obtained in step 1) at 0 to 40°C for 1 to 10 days;
3) rapidly centrifuging the suspension obtained in step 2), removing the supernatant, and drying the residue to obtain the target product;

wherein:
the poor solvent in step 1) is selected from the group consisting of acetone, ethyl acetate, acetonitrile, ethanol, 88% acetone, tetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone and 3-pentanone; and preferably selected from the group consisting of 3-pentanone, acetonitrile, dichloromethane and 1,4-dioxane.

**19.** A method for preparing the crystal form according to any one of claims 1 to 17, comprising the steps of:

1) weighing an appropriate amount of free base, and dissolving it in a good solvent;
2) adding an anti-solvent to the solution obtained in step 1), and stirring the solution until a solid is precipitated;
3) rapidly centrifuging the suspension obtained in step 2), removing the supernatant, and drying the residue to obtain the target product;

wherein:

the good solvent in step 1) is selected from the group consisting of methanol, acetone, ethyl acetate, acetonitrile, ethanol, 88% acetone, tetrahydrofuran, dichloromethane, 1,4-dioxane, benzene, toluene, isopropanol, n-butanol, isobutanol, N,N-dimethylformamide, N,N-dimethylacetamide, n-propanol, tert-butanol, 2-butanone and 3-pentanone; and preferably selected from the group consisting of N,N-dimethylformamide and acetonitrile;
the anti-solvent in step 2) is selected from the group consisting of heptane, water, methyl tert-butyl ether, toluene and isopropyl ether; and preferably water.

**20.** A method for preparing the crystal form according to any one of claims 1 to 17, comprising the steps of:

1) weighing an appropriate amount of free base, and suspending it in a poor solvent;
2) shaking the suspension obtained in step 1) at a certain temperature for a certain period of time;
3) rapidly centrifuging the suspension obtained in step 2), removing the supernatant, and drying the remaining solid to constant weight to obtain the target product;

wherein:

the poor solvent is one or more selected from the group consisting of acetone, ethyl acetate, isopropyl acetate, acetonitrile, ethanol, 88% acetone, tetrahydrofuran, 2-methyltetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone, 3-pentanone, methyl *tert*-butyl ether and water, and preferably one or more selected from the group consisting of acetone, ethyl acetate, isopropyl acetate, acetonitrile, ethanol, 88% acetone, tetrahydrofuran, 2-methyltetrahydrofuran, dichloromethane, 1,4-dioxane, toluene, isopropanol, 2-butanone, 3-pentanone, methyl *tert*-butyl ether and water;
or, comprising the steps of:

1) weighing an appropriate amount of free base, and dissolving it in a good solvent;
2) adding an anti-solvent to the solution obtained in step 1) at a certain temperature, and stirring the solution until a solid is precipitated;
3) rapidly centrifuging the suspension obtained in step 2), removing the supernatant, and drying the remaining solid to constant weight to obtain the target product;

wherein:

the good solvent is one or more selected from the group consisting of methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone, 3-pentanone and N-methylpyrrolidone, and preferably N-methylpyrrolidone;
the anti-solvent is selected from the group consisting of heptane, water, methyl *tert*-butyl ether, toluene and isopropyl ether;
or, comprising the steps of:

1) weighing an appropriate amount of free base, and dissolving it in a good solvent under heating;
2) rapidly cooling the solution obtained in step 1), and stirring it until a solid is precipitated;
3) rapidly centrifuging the suspension obtained in step 2), removing the supernatant, and drying the remaining solid to constant weight to obtain the target product;

wherein:
the good solvent is one or more selected from the group consisting of methanol, acetone, ethyl acetate, acetonitrile, ethanol, 88% acetone, tetrahydrofuran, 2-methyltetrahydrofuran, dichloromethane, 1,4-dioxane, benzene, toluene, isopropanol, *n*-butanol, isobutanol, N,N-dimethylformamide, N,N-dimethylacetamide, *n*-propanol, *tert*-butanol, 2-butanone, 3-pentanone and N-methylpyrrolidone, and preferably 2-methyltetrahydrofuran.

**21.** A pharmaceutical composition, comprising a therapeutically effective amount of the crystal form according to any one of claims 1 to 17, and one or more pharmaceutically acceptable carrier(s), diluent(s) or excipient(s).

**22.** Use of the crystal form according to any one of claims 1 to 17 or the pharmaceutical composition according to claim 21 in the preparation of a SHP-2 inhibitor medicament.

**23.** The use according to claim 22, wherein the use is a use in the preparation of a medicament for treating a disease or condition of Noonan syndrome, leopard skin syndrome, leukemia, neuroblastoma, melanoma, esophageal cancer, head and neck tumor, breast cancer, lung cancer and colon cancer; and preferably non-small cell lung cancer, esophageal cancer or head and neck tumor.

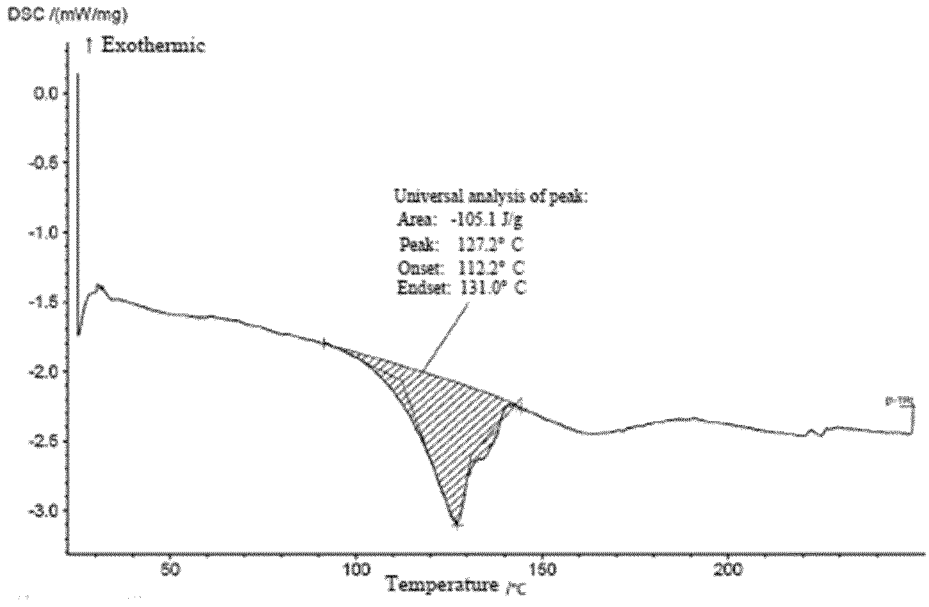


Figure 1

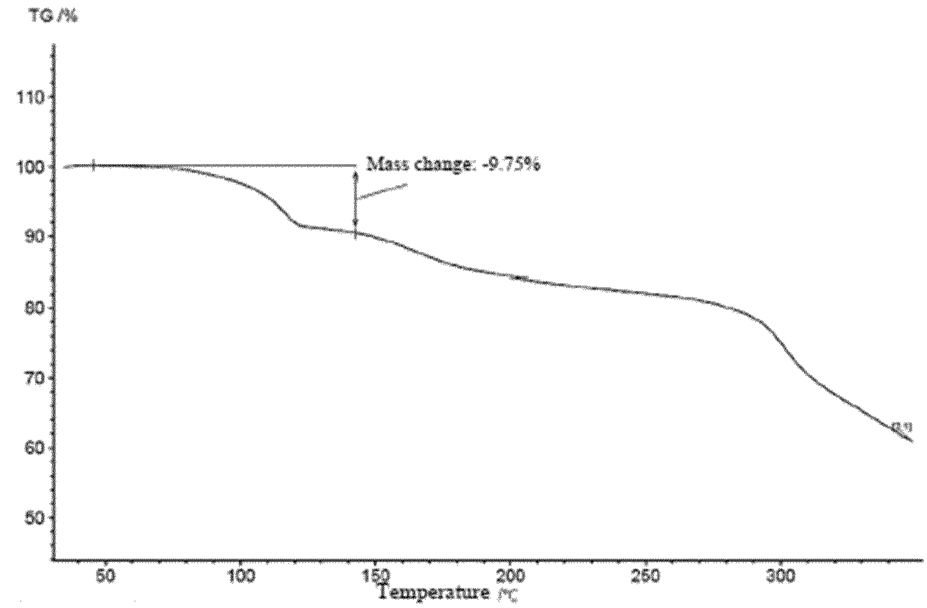


Figure 2

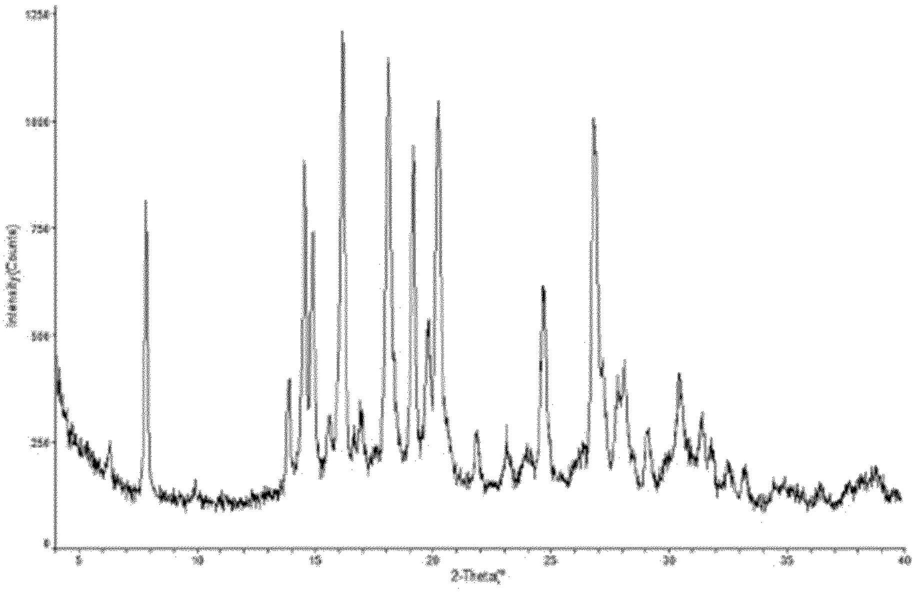

Figure 3

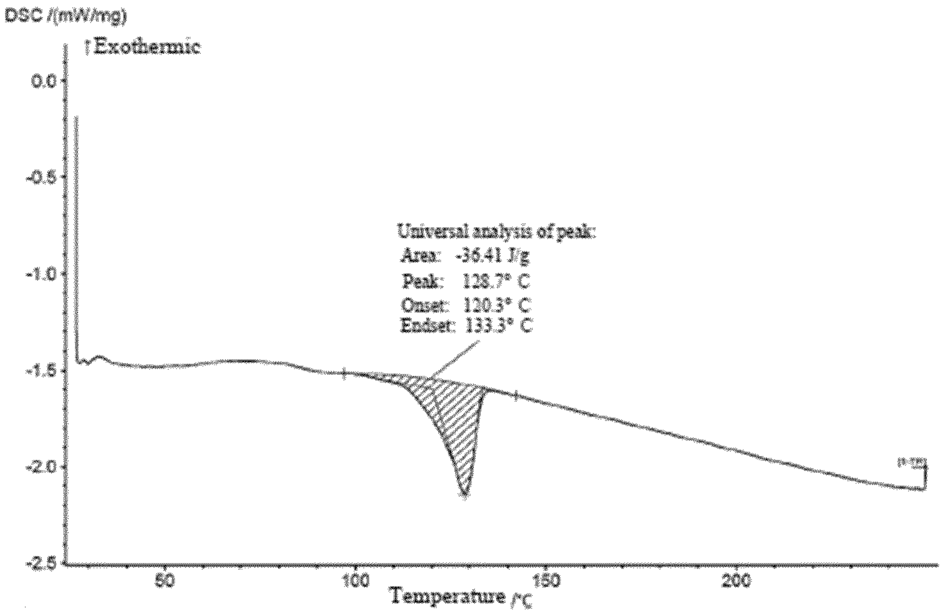

Figure 4

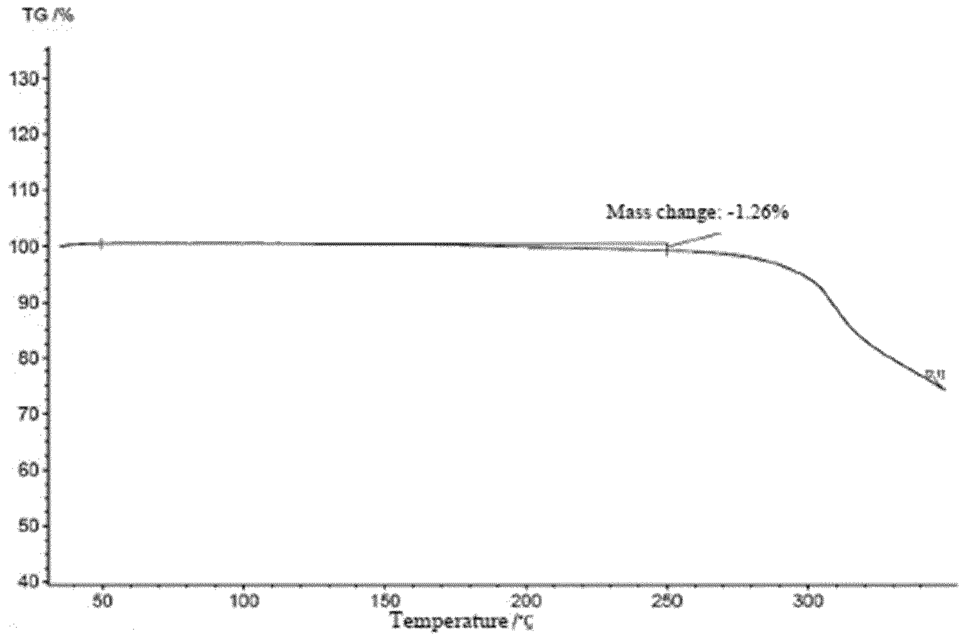


Figure 5

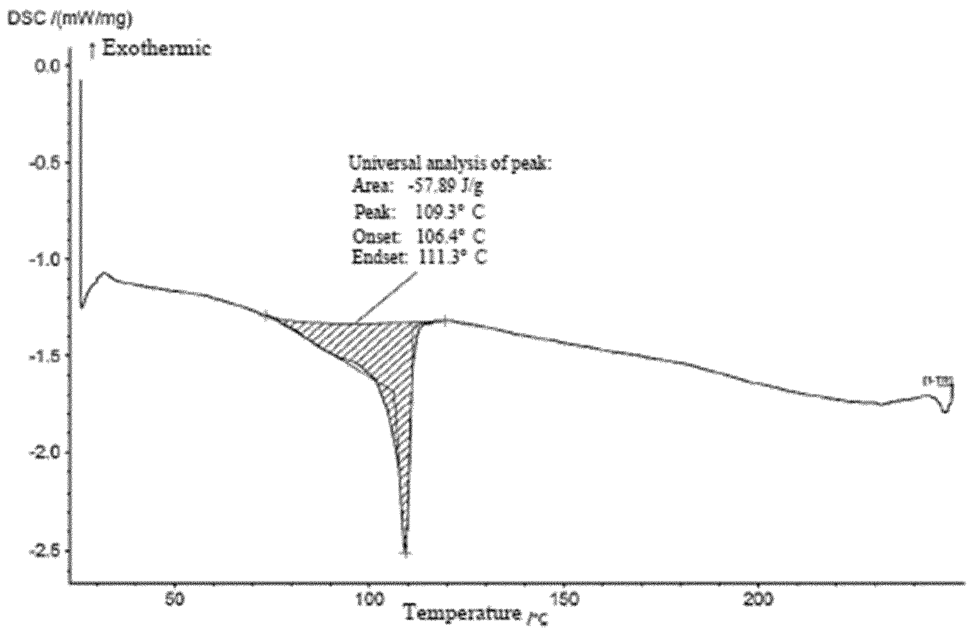


Figure 6

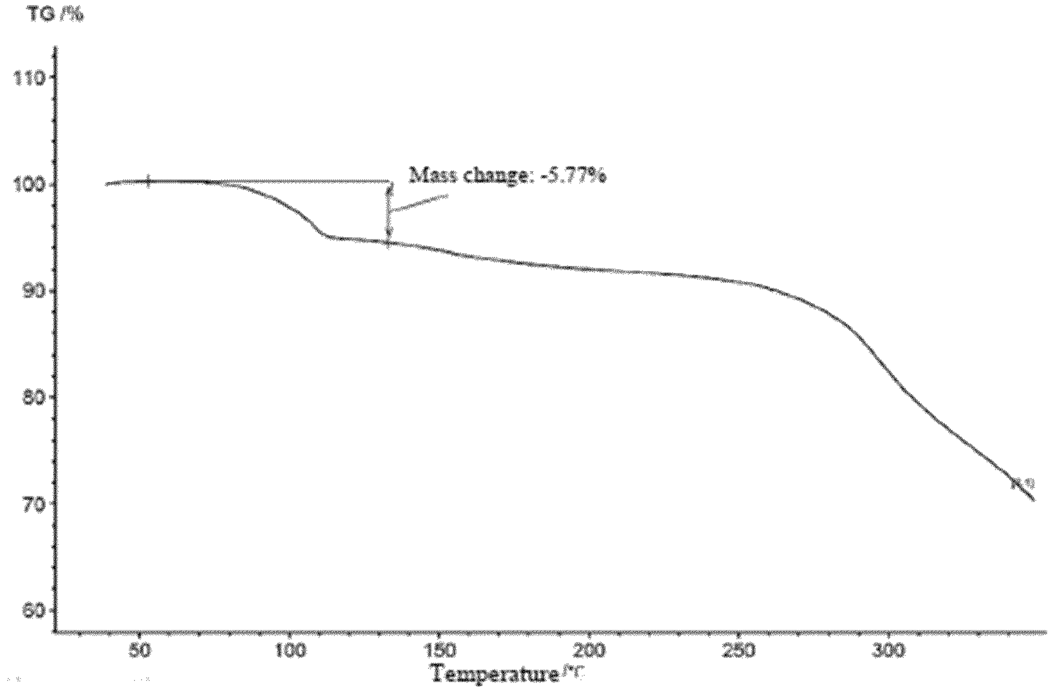


Figure 7

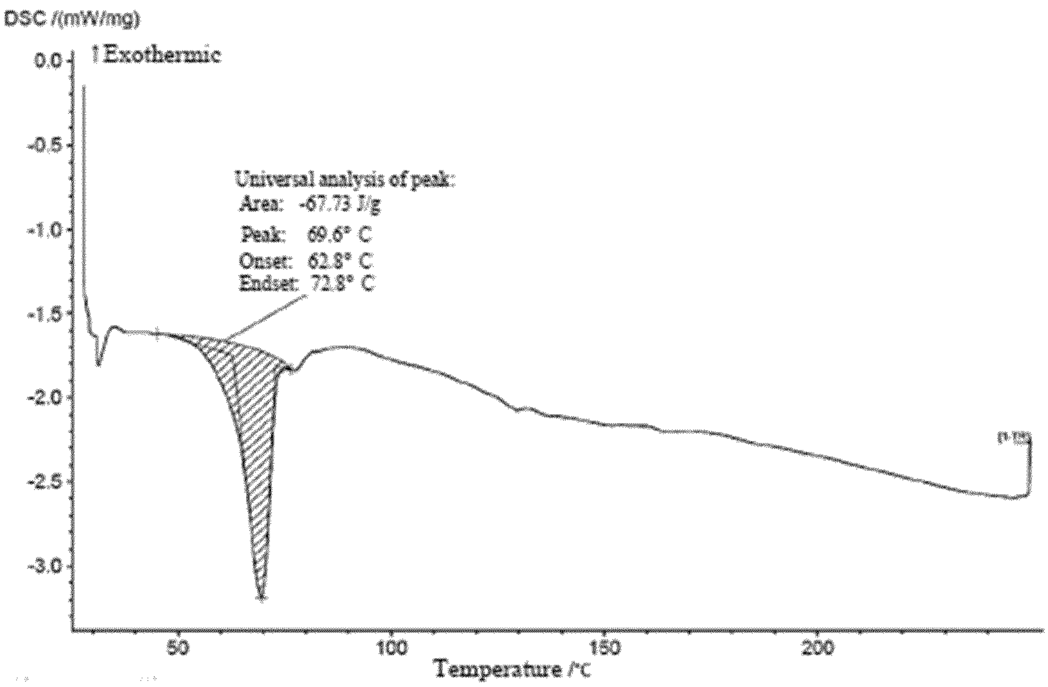


Figure 8

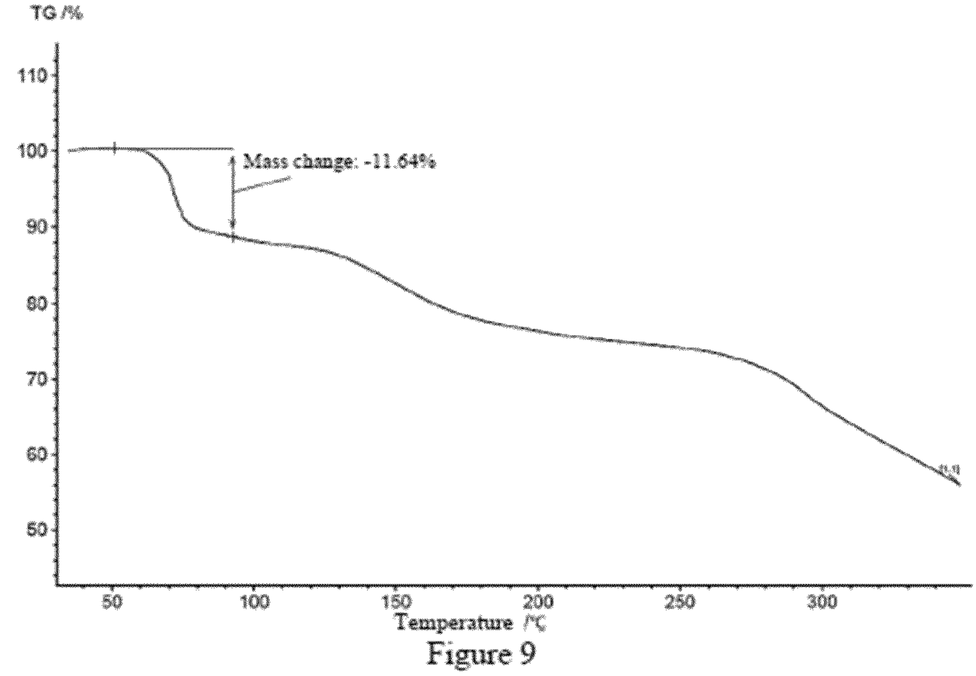

Figure 9

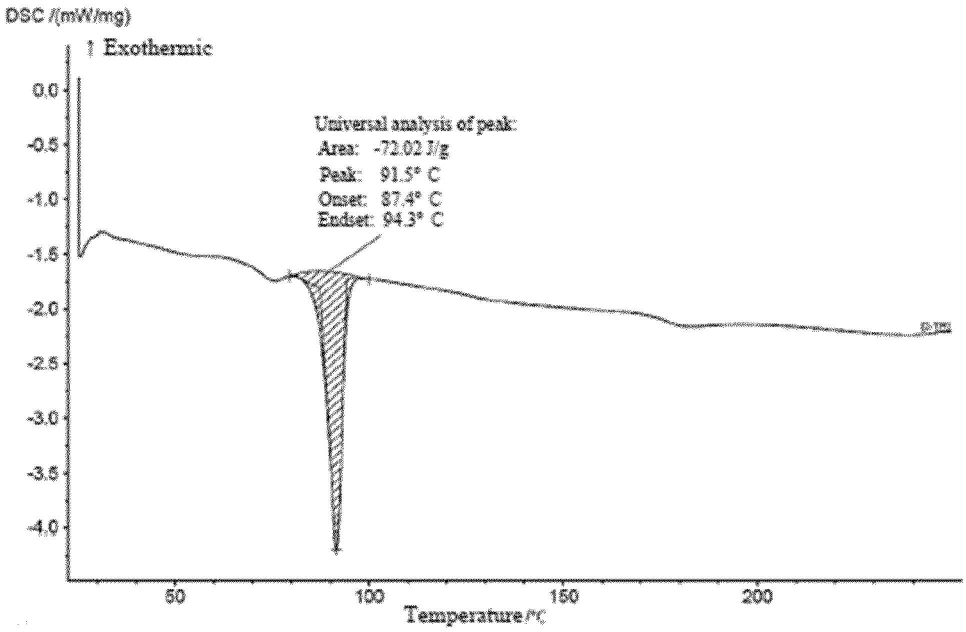

Figure 10

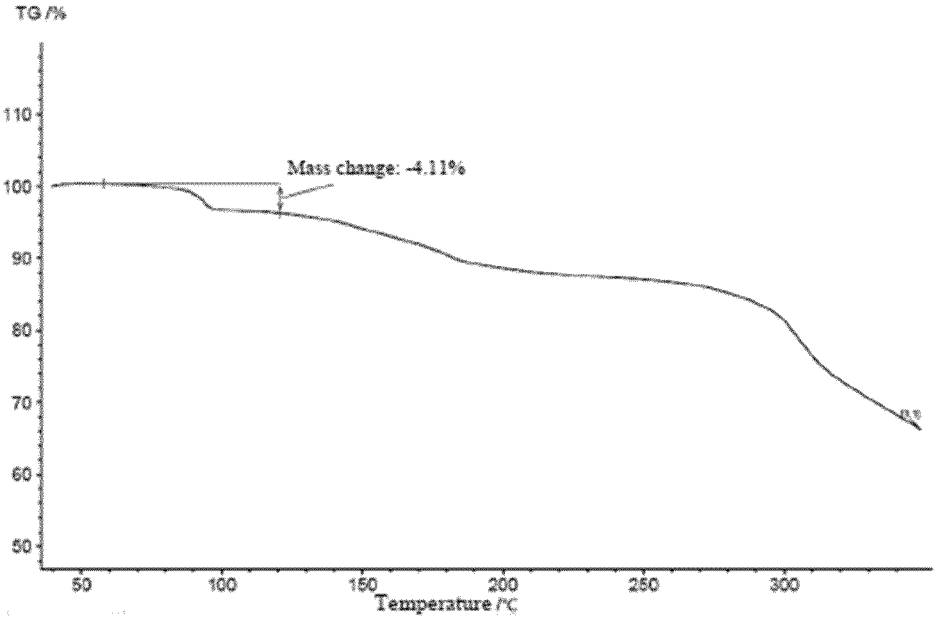


Figure 11

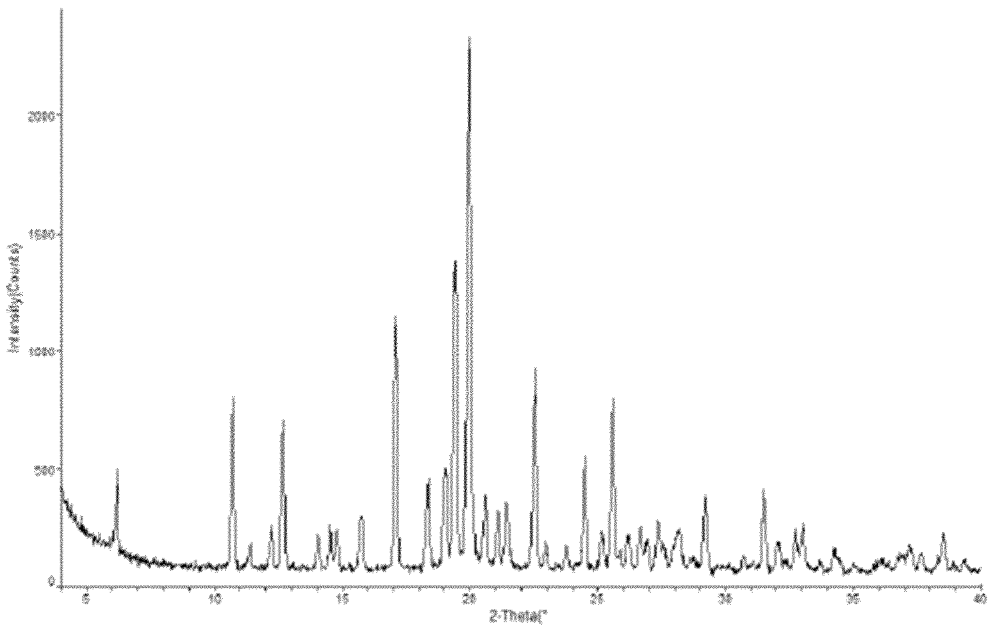


Figure 12

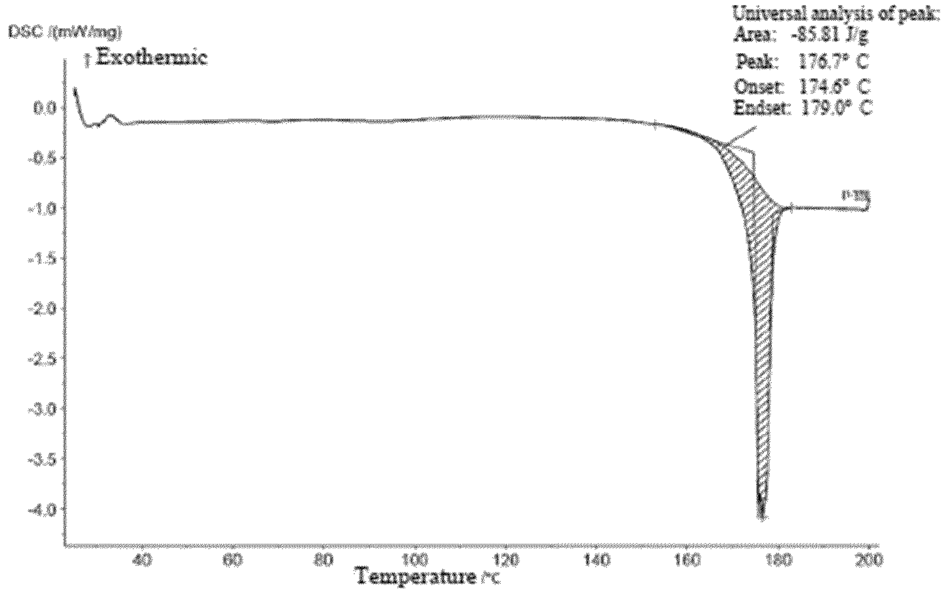


Figure 13

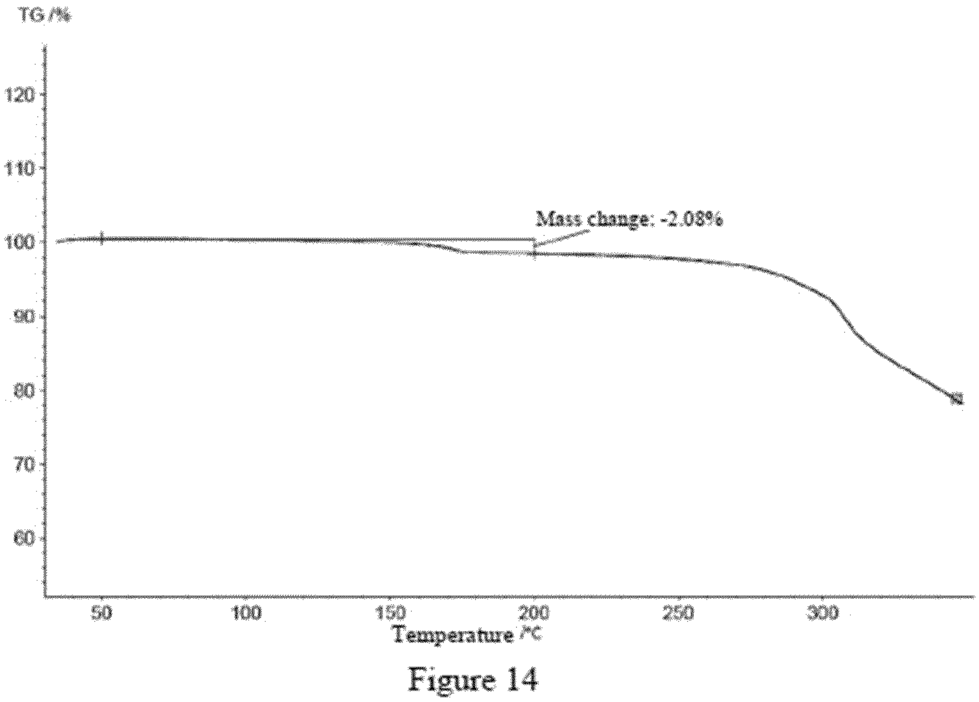


Figure 14

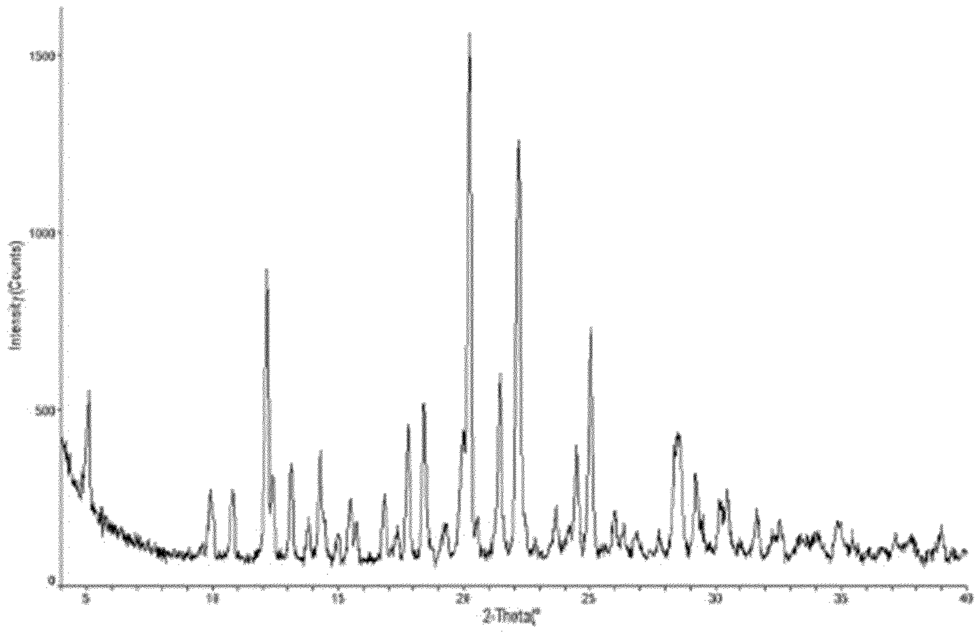


Figure 15

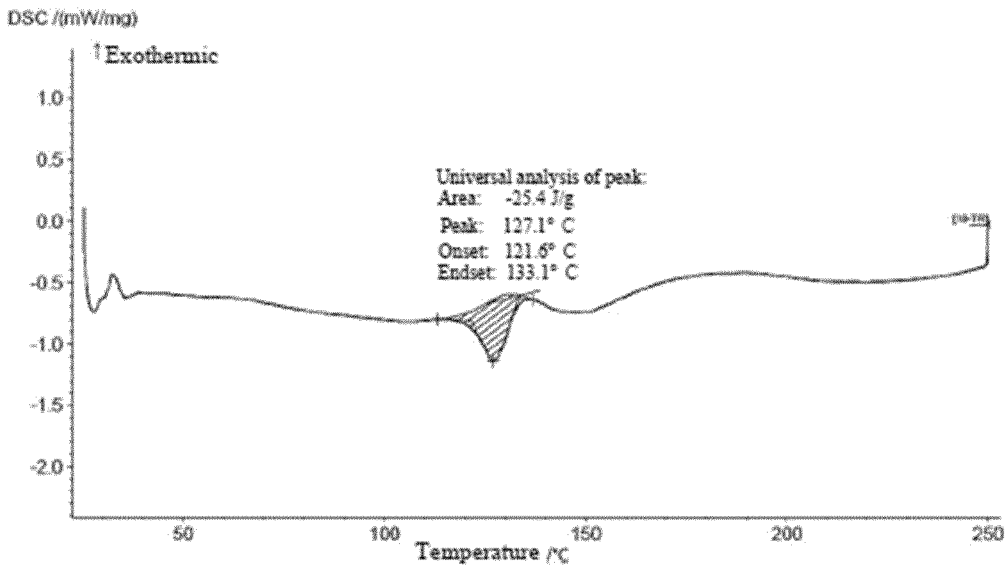


Figure 16

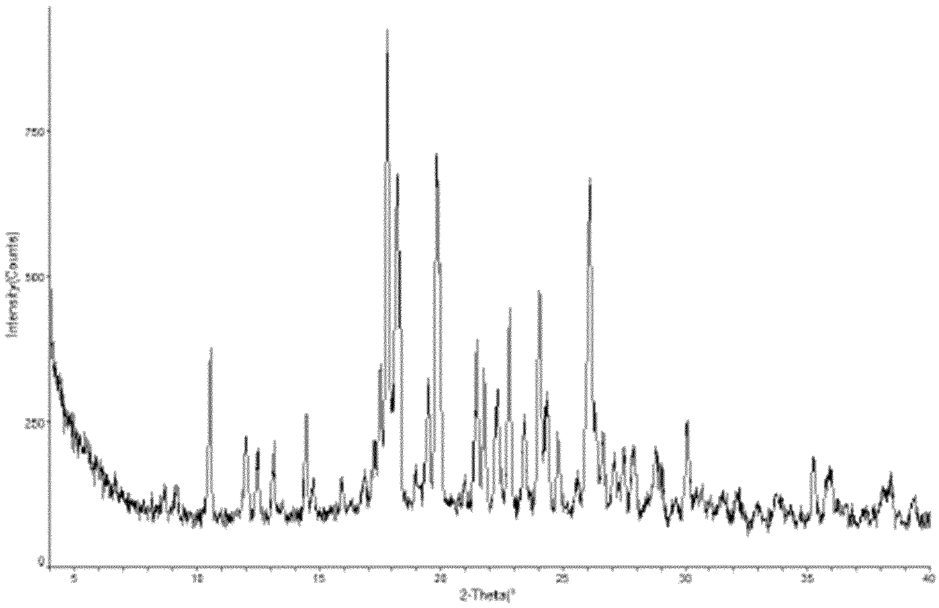


Figure 17

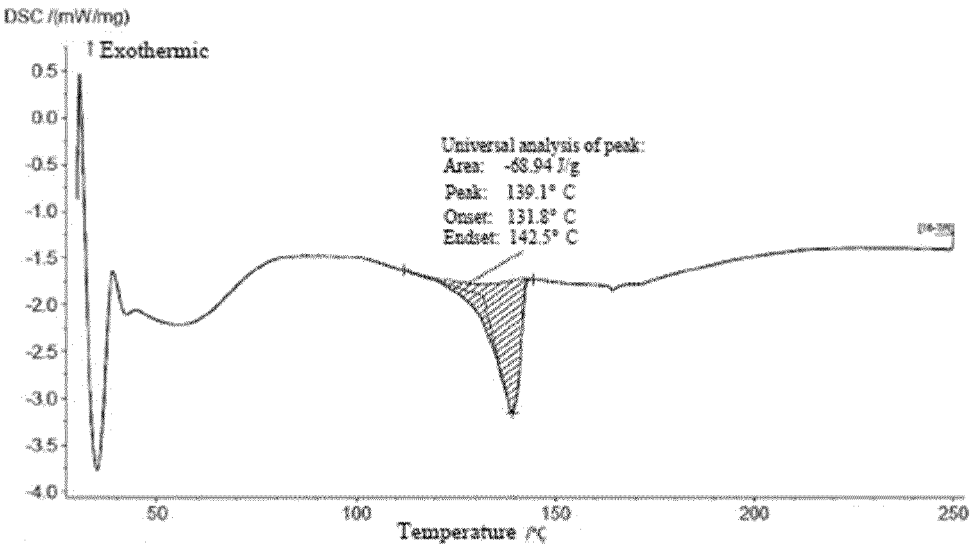


Figure 18

**INTERNATIONAL SEARCH REPORT**

International application No.
**PCT/CN2021/085155**

**A. CLASSIFICATION OF SUBJECT MATTER**

C07D 241/18(2006.01)i; C07D 498/10(2006.01)i; C07D 471/10(2006.01)i; A61K 31/495(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS; DWPI; SIPOABS; CJFD; CNKI; CNTXT; USTXT; WOTXT; EPTXT; STN-REGISTRY; STN-CAPLUS; PATENTICS; 超星读秀; ISI-Web of Science: 上海翰森生物医药科技有限公司, 江苏豪森药业集团有限公司, 詹小兰, 呙临松, 吡啶, 吡嗪, SHP-2, PTPN11, 努南氏症候群, 豹皮症候群, 白血病, 癌, 肿瘤, 晶型, 晶体, +pyridin+, +pyrazin+, Noonan, Leopard, Leukemia, cancer, tumour, tumor, +crystal+

**C. DOCUMENTS CONSIDERED TO BE RELEVANT**

| Category* | Citation of document, with indication, where appropriate, of the relevant passages | Relevant to claim No. |
|---|---|---|
| X | CN 105916845 A (NOVARTIS AG) 31 August 2016 (2016-08-31)<br>description embodiments 30, 41, 71, claims 1, 21-23 | 1-10, 12-23 |
| X | CN 109983001 A (REVOLUTION MEDICINES INC.) 05 July 2019 (2019-07-05)<br>description embodiment 6, claims 1, 48-50 | 1-10, 12-23 |
| X | WO 2018172984 A1 (JACOBIO PHARMACEUTICALS CO., LTD. et al.) 27 September 2018 (2018-09-27)<br>description embodiment 137, claims 1, 117-122 | 1-23 |
| X | WO 2019183367 A1 (RELAY THERAPEUTICS INC. et al.) 26 September 2019 (2019-09-26)<br>description embodiments 81, 83, 84, 259, 260, claims 1, 49-65 | 1-11, 17-23 |
| PX | WO 2020073949 A1 (JIANGSU HANSOH PHARMACEUTICAL GROUP CO., LTD. et al.) 16 April 2020 (2020-04-16)<br>description page 25 lines 2-4, embodiment 143, claims 1-31 | 1-23 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 May 2021** | **23 June 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/085155**

**C. DOCUMENTS CONSIDERED TO BE RELEVANT**

| Category* | Citation of document, with indication, where appropriate, of the relevant passages | Relevant to claim No. |
|---|---|---|
| X | CN 110143949 A (BEIJING JACOBIO PHARMACEUTICALS CO., LTD.) 20 August 2019 (2019-08-20) claims 1-51 | 1-23 |
| X | CN 107922388 A (NOVARTIS AG) 17 April 2018 (2018-04-17) claims 1-14 | 1-10, 12-23 |
| X | WO 2019051084 A1 (REVOLUTION MEDICINES, INC.) 14 March 2019 (2019-03-14) description paragraphs [00119]-[00139] | 1-10, 12-23 |

Form PCT/ISA/210 (second sheet) (January 2015)

**INTERNATIONAL SEARCH REPORT**
**Information on patent family members**

International application No.
**PCT/CN2021/085155**

| Patent document cited in search report | Publication date (day/month/year) | Patent family member(s) | Publication date (day/month/year) |
|---|---|---|---|
| CN 105916845 A | 31 August 2016 | EP 3094628 B1 | 08 August 2018 |
| | | PH 12016501336 A1 | 15 August 2016 |
| | | IL 246436 A | 31 October 2019 |
| | | CA 2935695 A1 | 23 July 2015 |
| | | AU 2015207757 A1 | 07 July 2016 |
| | | TW 201612170 A | 01 April 2016 |
| | | JP 6534389 B2 | 26 June 2019 |
| | | US 2020181168 A1 | 11 June 2020 |
| | | AU 2015207757 B8 | 11 May 2017 |
| | | ES 2695242 T3 | 02 January 2019 |
| | | KR 20160103137 A | 31 August 2016 |
| | | JP 2017502993 A | 26 January 2017 |
| | | EP 3094628 A1 | 23 November 2016 |
| | | TW I657083 B | 21 April 2019 |
| | | NZ 721598 A | 31 August 2018 |
| | | PH 12016501336 B1 | 15 August 2016 |
| | | CN 105916845 B | 12 November 2019 |
| | | US 2017015680 A1 | 19 January 2017 |
| | | AR 100033 A1 | 07 September 2016 |
| | | AU 2015207757 A8 | 11 May 2017 |
| | | US 10336774 B2 | 02 July 2019 |
| | | AU 2015207757 B2 | 06 April 2017 |
| | | EA 031573 B1 | 31 January 2019 |
| | | WO 2015107495 A1 | 23 July 2015 |
| | | US 10077276 B2 | 18 September 2018 |
| | | IN 201617027369 A | 31 August 2016 |
| | | VN 10024576 B | 27 July 2020 |
| | | GC 10854 A | 29 February 2020 |
| | | BR 112016016432 A2 | 08 August 2017 |
| | | VN 49285 A | 25 October 2016 |
| | | MX 2016009223 A | 05 October 2016 |
| | | US 2018201623 A1 | 19 July 2018 |
| | | MX 356895 B | 19 June 2018 |
| | | HK 1228375 A1 | 06 June 2019 |
| | | SG 11201605272 B | 31 July 2019 |
| | | HK 1228375 A0 | 03 November 2017 |
| | | ZA 201604258 A | 28 February 2018 |
| | | SG 11201605272 A1 | 30 August 2016 |
| CN 109983001 A | 05 July 2019 | AU 2017296289 A1 | 31 January 2019 |
| | | US 10590090 B2 | 17 March 2020 |
| | | EP 3484856 A1 | 22 May 2019 |
| | | TW 201808931 A | 16 March 2018 |
| | | CA 3030167 A1 | 18 January 2018 |
| | | JP 2019527728 A | 03 October 2019 |
| | | WO 2018013597 A1 | 18 January 2018 |
| | | KR 20190026893 A | 13 March 2019 |
| | | BR 112019000494 A2 | 24 April 2019 |
| | | US 2019210977 A1 | 11 July 2019 |
| | | PH 12019500056 A1 | 14 October 2019 |
| | | WO 2018013597 A4 | 05 April 2018 |

Form PCT/ISA/210 (patent family annex) (January 2015)

**INTERNATIONAL SEARCH REPORT**
**Information on patent family members**

International application No.
**PCT/CN2021/085155**

| Patent document cited in search report | Publication date (day/month/year) | Patent family member(s) | Publication date (day/month/year) |
|---|---|---|---|
| | | SG 11201900157 A1 | 27 February 2019 |
| | | IN 201817049772 A | 18 October 2019 |
| | | AR 109021 A1 | 17 October 2018 |
| | | ID 201906411 A | 30 August 2019 |
| | | HK 40008949 A0 | 19 June 2020 |
| | | VN 63902 A | 25 June 2019 |
| | | HK 40009464 A0 | 26 June 2020 |
| WO 2018172984 A1 | 27 September 2018 | EP 3601239 A4 | 13 May 2020 |
| | | AU 2018239542 A1 | 14 November 2019 |
| | | AU 2020267305 A1 | 10 December 2020 |
| | | AU 2018239542 C1 | 11 February 2021 |
| | | CA 3057582 A1 | 27 September 2018 |
| | | PH 12019502175 A1 | 08 June 2020 |
| | | US 2020392128 A1 | 17 December 2020 |
| | | TW I664175 B | 01 July 2019 |
| | | KR 20190140931 A | 20 December 2019 |
| | | AU 2018239542 B2 | 20 August 2020 |
| | | EP 3601239 A1 | 05 February 2020 |
| | | JP 2020515538 A | 28 May 2020 |
| | | TW 201840553 A | 16 November 2018 |
| | | SG 11201908820 A1 | 30 October 2019 |
| | | IN 201947042842 A | 25 October 2019 |
| | | IN 202148002849 A | 29 January 2021 |
| | | VN 69294 A | 25 March 2020 |
| WO 2019183367 A1 | 26 September 2019 | KR 20210015758 A | 10 February 2021 |
| | | CN 112166110 A | 01 January 2021 |
| | | EP 3768668 A1 | 27 January 2021 |
| | | AU 2019240299 A1 | 15 October 2020 |
| | | AR 114591 A1 | 23 September 2020 |
| | | CA 3094690 A1 | 26 September 2019 |
| | | SG 11202009245 A1 | 29 October 2020 |
| WO 2020073949 A1 | 16 April 2020 | CN 111295374 A | 16 June 2020 |
| CN 110143949 A | 20 August 2019 | HK 40013065 A0 | 07 August 2020 |
| CN 107922388 A | 17 April 2018 | ES 2805232 T3 | 11 February 2021 |
| | | CN 107922388 B | 29 December 2020 |
| | | JP 6718889 B2 | 08 July 2020 |
| | | US 10287266 B2 | 14 May 2019 |
| | | WO 2016203406 A1 | 22 December 2016 |
| | | JP 2018517752 A | 05 July 2018 |
| | | US 2018186770 A1 | 05 July 2018 |
| | | EP 3310774 A1 | 25 April 2018 |
| | | EP 3310774 B1 | 29 April 2020 |
| WO 2019051084 A1 | 14 March 2019 | AU 2018328273 A1 | 12 March 2020 |
| | | CA 3074690 A1 | 14 March 2019 |
| | | KR 20200051684 A | 13 May 2020 |
| | | IN 202037007385 A | 22 May 2020 |
| | | SG 11202001282 A1 | 30 March 2020 |
| | | US 20200368238 A1 | 26 November 2020 |
| | | BR 112020004246 A2 | 01 September 2020 |

Form PCT/ISA/210 (patent family annex) (January 2015)

**INTERNATIONAL SEARCH REPORT**
**Information on patent family members**

International application No.
**PCT/CN2021/085155**

| Patent document cited in search report | Publication date (day/month/year) | Patent family member(s) | | | Publication date (day/month/year) |
|---|---|---|---|---|---|
| | | TW | 201918260 | A | 16 May 2019 |
| | | EP | 3678703 | A1 | 15 July 2020 |
| | | JP | 2020533315 | W | 19 November 2020 |
| | | CN | 111344017 | A | 26 June 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2019110314 W